(19) Europäisches Patentamt
European Patent Office
Office européen des brevets

(11) EP 4 039 257 B1

(12) **EUROPEAN PATENT SPECIFICATION**

(45) Date of publication and mention of the grant of the patent:
**27.05.2026 Bulletin 2026/22**

(21) Application number: **20873212.3**

(22) Date of filing: **02.10.2020**

(51) International Patent Classification (IPC):
***A61K 31/43*** *(2006.01)* ***A61K 31/497*** *(2006.01)*
***A61K 31/407*** *(2006.01)* ***A61K 31/424*** *(2006.01)*
***A61K 31/431*** *(2006.01)* ***A61K 31/433*** *(2006.01)*
***A61K 31/4439*** *(2006.01)* ***A61K 31/454*** *(2006.01)*
***A61K 31/496*** *(2006.01)* ***A61K 31/505*** *(2006.01)*
***A61K 31/546*** *(2006.01)* ***A61K 38/12*** *(2006.01)*
***A61K 38/14*** *(2006.01)* ***A61K 45/06*** *(2006.01)*
***A61P 31/04*** *(2006.01)* ***A61P 43/00*** *(2006.01)*
*A61K 31/7036 (2006.01)* *A61K 31/69 (2006.01)*
*A61K 31/65 (2006.01)* *A61K 31/551 (2006.01)*
*A61K 31/5383 (2006.01)* *A61K 31/55 (2006.01)*

(52) Cooperative Patent Classification (CPC):
(C-Sets available)
**A61K 31/43; A61K 31/407; A61K 31/424; A61K 31/431; A61K 31/433; A61K 31/4439; A61K 31/454; A61K 31/496; A61K 31/505; A61K 31/5383; A61K 31/546; A61K 31/55; A61K 31/551; A61K 31/65; A61K 31/69;** (Cont.)

(86) International application number:
**PCT/JP2020/037563**

(87) International publication number:
**WO 2021/066149 (08.04.2021 Gazette 2021/14)**

(54) **PHARMACEUTICAL COMPOSITION AND KIT CONTAINING A PENAM DERIVATIVE OR SALT THEREOF AND ONE OR MORE COMPOUNDS SELECTED FROM BETA-LACTAMASE INHIBITOR COMPOUND, ANTIBACTERIAL COMPOUND AND SALTS OF THESE**

PHARMAZEUTISCHE ZUSAMMENSETZUNG UND KIT MIT EINEM PENAM-DERIVAT ODER EINEM SALZ DAVON UND EINER ODER MEHREREN VERBINDUNGEN, AUSGEWÄHLT AUS EINER BETA-LACTAMASE-INHIBITORVERBINDUNG, EINER ANTIBAKTERIELLEN VERBINDUNG ODER SALZEN DAVON

COMPOSITION PHARMACEUTIQUE ET KIT CONTENANT UN NOUVEAU DÉRIVÉ PENAM OU UN SEL DE CELUI-CI ET UN OU PLUSIEURS COMPOSÉS CHOISIS PARMI UN COMPOSÉ INHIBITEUR DE BETA-LACTAMASE, UN COMPOSÉ ANTIBACTÉRIEN ET DES SELS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2019 JP 2019184111**

(43) Date of publication of application:
**10.08.2022 Bulletin 2022/32**

(73) Proprietor: **FUJIFILM Corporation**
**Tokyo 106-8620 (JP)**

(72) Inventors:
- **NAKAGAWA Satoshi**
  **Toyama-shi, Toyama 930-8508 (JP)**
- **SHOJI Muneo**
  **Toyama-shi, Toyama 930-8508 (JP)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Hoffmann Eitle**
**Patent- und Rechtsanwälte PartmbB**
**Arabellastraße 30**
**81925 München (DE)**

(56) References cited:
**WO-A1-2019/070052** **WO-A1-2019/194306**
**JP-A- 2018 104 463** **JP-A- H 072 870**
**JP-A- H0 474 182** **JP-A- H10 130 272**
**JP-A- S63 183 588**

(52) Cooperative Patent Classification (CPC): (Cont.)
**A61K 31/7036; A61K 38/12; A61K 38/14;**
**A61K 45/06; A61P 31/04; A61P 43/00;** Y02P 20/55

C-Sets
**A61K 31/407, A61K 2300/00;**
**A61K 31/424, A61K 2300/00;**
**A61K 31/43, A61K 2300/00;**
**A61K 31/431, A61K 2300/00;**
**A61K 31/433, A61K 2300/00;**
**A61K 31/4439, A61K 2300/00;**
**A61K 31/454, A61K 2300/00;**
**A61K 31/496, A61K 2300/00;**
**A61K 31/505, A61K 2300/00;**
**A61K 31/5383, A61K 2300/00;**
**A61K 31/546, A61K 2300/00;**
**A61K 31/55, A61K 2300/00;**
**A61K 31/551, A61K 2300/00;**
**A61K 31/65, A61K 2300/00;**
**A61K 31/69, A61K 2300/00;**
**A61K 31/7036, A61K 2300/00;**
**A61K 38/12, A61K 2300/00;**
**A61K 38/14, A61K 2300/00**

## Description

### Technical Field

The present invention relates to a pharmaceutical composition which exhibits strong antibacterial activity against Gram-negative bacteria, particularly, Pseudomonas aeruginosa and contains a novel penam derivative or a salt thereof as defined in the claims and one or more compounds selected from a β-lactamase inhibitory compound and an antibacterial compound or salts thereof.

Furthermore, the present invention relates to a kit which exhibits strong antibacterial activity against Gram-negative bacteria, particularly, Pseudomonas aeruginosa and contains a novel penam derivative or a salt thereof as defined in the claims and one or more compounds selected from a β-lactamase inhibitory compound and an antibacterial compound or salts thereof.

### Background Art

β-lactam-based drugs are clinically very important antibacterial agents, and various β-lactam-based drugs have been developed so far. Meanwhile, Gram-negative bacteria highly resistant to many β-lactam-based drugs such as cephalosporin-based drugs and/or carbapenem-based drugs have been isolated. The infections caused by these resistant bacteria have a high fatality rate and tend to result in a long hospital stay for patients, which are major clinical and economical issues (Non-Patent Document 1).

As a resistance mechanism of β-lactam-based drugs, β-lactamase that decomposes β-lactam-based drugs is known. According to the Ambler's molecular classification method, the β-lactamase is roughly classified into Class A (such as TEM, SHV, CTX-M, KPC, and GES-type β-lactamases), Class B (such as IMP, VIM, and NDM-type β-lactamases), Class C (such as AmpC-type β-lactamase), and class D (OXA-type β-lactamase), which decompose β-lactam-based drugs with different substrate specificities (Non-Patent Document 2). Particularly, each type of β-lactamase of class B and KPC, GES, and OXA-type β-lactamases are called carbapenemase, and many of bacteria having these exhibit high resistance to almost all β-lactam-based drugs including carbapenem-based drugs.

Furthermore, in recent years, Pseudomonas aeruginosa strains have been isolated which exhibit high resistance to ceftolozane/tazobactam and ceftazidime/avibactam that are newly developed cephalosporin-based combination medicines (Non-Patent Document 3). Patent Document 1 describes penam derivatives and antibacterial agents comprising the same. Patent Document 2 describes 2-carboxypenam compounds having antibacterial activity against gram-negative bacteria and drug-resistant strains thereof.

### Prior Art Documents

#### Patent Documents

Patent Document 1 : FR 2648818
Patent Document 2 : WO 2019/070052 A1

#### Non-Patent Documents

Non-Patent Document 1: Antimicrobial Agents and Chemotherapy, 2008, No. 52, pp. 813-821
Non-Patent Document 2: Scientific Reports, 2017, No. 24, Article No. 43232
Non-Patent Document 3: International Journal of Antimicrobial Agents, 2014, No. 43, pp. 533-539

### SUMMARY OF THE INVENTION

#### Object to be solved by the Invention

Infections caused by multidrug-resistant Pseudomonas aeruginosa have few effective therapeutic agents and are a major problem worldwide as intractable diseases.

There is a need for the provision of a pharmaceutical composition that exhibits strong antibacterial activity against Gram-negative bacteria and drug-resistant Gram-negative bacteria. Particularly, there is a desperate need for the

provision of a pharmaceutical composition and a kit that exhibit strong antibacterial activity against enterobacteria or Pseudomonas aeruginosa producing carbapenemase.

Means for solving the object

Under such circumstances, the inventors of the present invention conducted intensive studies. As a result, the inventors have found that a pharmaceutical composition and a kit containing a compound represented by General Formula [1] or a salt thereof and one or more compounds selected from a β-lactamase inhibitory compound and an antibacterial compound or salts thereof exhibit strong antibacterial activity against Gram-negative bacteria such as Pseudomonas aeruginosa and/or drug-resistant Gram-negative bacteria such as multidrug-resistant Pseudomonas aeruginosa, for example, enterobacteria and/or Pseudomonas aeruginosa producing carbapenemase and are useful for treating infections caused by these bacteria. Based on what they found, the inventors have accomplished the present invention.
The present invention provides the following.

<1>
A pharmaceutical composition containing a compound represented by General Formula [1] or a salt thereof and one or more compounds selected from a β-lactamase inhibitory compound and an antibacterial compound or salts thereof,

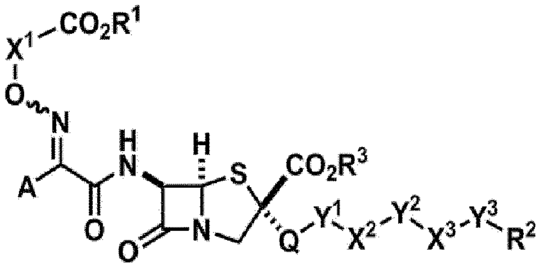
[1]

"in the formula,

$R^1$ represents a hydrogen atom;
$R^2$ represents an aryl group which may be substituted;
$R^3$ represents a hydrogen atom;
$X^1$ represents a $C_{1-6}$ alkylene group which may be substituted, a $C_{2-6}$ alkenylene group which may be substituted, a $C_{2-6}$ alkynylene group which may be substituted, a divalent cyclic hydrocarbon group which may be substituted, or a divalent monocyclic saturated heterocyclic group which may be substituted;
A represents a monocyclic heterocyclic group which may be substituted;
Q represents a divalent cyclic amino group which may be substituted or a divalent heterocyclic group which may be substituted;
$Y^1$ represents a $C_{1-6}$ alkylene group which may be substituted, a $C_{2-6}$ alkenylene group which may be substituted, a $C_{2-6}$ alkynylene group which may be substituted, a group represented by Formula -N=CH-CH=N-, a group represented by Formula -N=CH-CH=N-O-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula -NHC(=O)C(=O)N(OH)-, a group represented by Formula $-NHCH_2C(=O)-$, a group represented by Formula $-NHS(=O)_2NH-C(=O)-$, a group represented by Formula $-NHC(=O)NHS(=O)_2-$, or a bond;
$X^2$ represents a group represented by General Formula $-NR^4-$ (where $R^4$ represents a hydrogen atom, a carbamoyl group, a $C_{1-6}$ alkyl group which may be substituted, or a hydroxyl group which may be protected), a group represented by General Formula $-N^+R^5R^6-$ (where $R^5$ and $R^6$ are the same as or different from each other and each represent a $C_{1-6}$ alkyl group which may be substituted, or in combination represent a $C_{2-6}$ alkylene group which may be substituted or a $C_{2-6}$ alkenylene group which may be substituted), a group represented by General Formula $-NR^7-C(=O)-NR^8-$ (where $R^7$ and $R^8$ are the same as or different from each other and each represent a hydrogen atom, a $C_{1-6}$ alkyl group which may be substituted, or a hydroxyl group which may be protected), a divalent cyclic amino group which may be substituted, a divalent heterocyclic group which may be substituted, or a bond;
$Y^2$ represents a $C_{1-6}$ alkylene group which may be substituted, or a bond;
$X^3$ represents a group represented by General Formula $-NR^9-$ (where $R^9$ represents a hydrogen atom); and

$Y^3$ represents a group represented by Formula -C(=O)-, a group represented by Formula -C(=O)-C(=O)-, or a group represented by General Formula -C(=O)-C(=$NR^{10a}$)-(where $R^{10a}$ represents a hydroxyl group).

<2>
The pharmaceutical composition described in <1>, in which $X^1$ represents a $C_{1-6}$ alkylene group which may be substituted or a divalent cyclic hydrocarbon group which may be substituted.
<3>
The pharmaceutical composition described in <1> or <2>, in which Q represents a divalent heterocyclic group which may be substituted.
<4>
The pharmaceutical composition described in any one of <1> to <3>, in which $Y^1$ represents a $C_{1-6}$ alkylene group which may be substituted, a group represented by Formula -N=CH-CH=N-, a group represented by Formula -N=CH-$CH_2$-, a group represented by Formula -N=CHC(=O)-, a group represented by Formula -NHC(=O)-, a group represented by Formula -NHC(=O)$CH_2$-, a group represented by Formula -NHC(=O)NH-, a group represented by Formula -NHC(=O)NH-O-, a group represented by Formula -NHC(=O)C(=O)NH-, a group represented by Formula -$NHCH_2C$(=O)-, or a bond.
<5>
The pharmaceutical composition described in any one of <1> to <4>, in which $X^2$ represents a group represented by General Formula -$NR^{4a}$- (where $R^{4a}$ represents a hydrogen atom or a carbamoyl group), a group represented by General Formula -$N^+R^{5a}R^{6a}$- (where $R^{5a}$ and $R^{6a}$ in combination represent a $C_{2-6}$ alkylene group which may be substituted), a group represented by General Formula -$NR^{7a}$-C(=O)-$NR^{8a}$- (where $R^{7a}$ and $R^{8a}$ each represent a hydrogen atom), a divalent cyclic amino group which may be substituted, a divalent heterocyclic group which may be substituted, or a bond.
<6>

The pharmaceutical composition described in any one of <1> to <5>, in which $R^2$ represents a phenyl group which may be substituted;
A represents a monocyclic nitrogen and sulfur-containing heterocyclic group which may be substituted;
Q represents a divalent monocyclic heterocyclic group which may be substituted;
$Y^1$ represents a group represented by Formula -NHC(=O)-, a group represented by Formula -NHC(=O)C(=O)NH-, or a bond;
$X^2$ represents a group represented by General Formula -$NR^{4b}$- (where $R^{4b}$ represents a hydrogen atom) or a bond;
$Y^2$ represents a $C_{1-3}$ alkylene group or a bond; and
$Y^3$ represents a group represented by Formula -C(=O)- or a group represented by Formula -C(=O)-C(=O)-.

<7>
The pharmaceutical composition described in <1>, in which the compound represented by General Formula [1] is a compound selected from (3R,5R,6R)-6-((Z)-2-(2-aminothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-a zabicyclo [3.2.0]heptane-3-carboxylic acid, (3R,5R,6R)-6-((Z)-2-(2-aminothiazol-4-yl)-2-(((2-carboxypropan-2-yl)oxy)imino) acetamido)-3-(3-(2-(2-chloro-3,4-dihydroxyphenyl)-2-oxoacetamido)-2-oxoimidazolidin-1-yl)-7-oxo-4-thi a-1-azabicyclo[3.2.0]heptane-3-carboxylic acid, (3R,5R,6R)-6-((Z)-2-(2-amino-5-chlorothiazol-4-yl)-2-(((2-carboxypropan-2-yl)oxy)imino)ac etamido)-3-(3-(2-(2-chloro-3,4-dihydroxyphenyl)-2-oxoacetamido)-2-oxoimidazolidin-1-yl)-7 -oxo-4-thia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-((S)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-((R)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetatamido)-2,3-dioxopiperazin-1-yl)-7-oxo-4-thia-1-azabicyclo[3.2.0]heptane-3-carboxylic acid, and (3R,5R,6R)-6-((Z)-2-(2-amino-5-chlorothiazol-4-yl)-2-(((2-carboxypropan-2-yl)oxy)imino)ac etamido)-3 -(3 -(2-(2-(2-(2-chloro-3,4-dihydroxyphenyl)-2-oxoacetyl)hydradienyl)-2-oxoaceta mido)-2-oxoimidazolidin-1-yl)-7-oxo-4-thia-1-azabicyclo[3.2.0]heptane-3-carboxylic acid.
<8>
The pharmaceutical composition described in any one of <1> to <7>, in which the one or more compounds selected from a β-lactamase inhibitory compound and an antibacterial compound or salts thereof are a β-lactamase inhibitory compound or a salt thereof.

<9>

The pharmaceutical composition described in any one of <1> to <8>, in which the β-lactamase inhibitory compound is one or more compounds selected from a diazabicyclo[3.2.1]octane-type inhibitory compound, a boronic acid-type inhibitory compound, and a clavam-type inhibitory compound.

<10>

The pharmaceutical composition described in any one of <1> to <9>, in which the β-lactamase inhibitory compound is one or more compounds selected from avibactam, nacubactam, relebactam, zidebactam, vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid, sulbactam, tazobactam, clavulanic acid, (1aR,7bS)-5-fluoro-2-hydroxy-1,1a,2,7b-tetrahydrocyclopropan[c][1,2]benzoxaborinine-4-car boxylic acid, and (25,3S,5R)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide.

<11>

The pharmaceutical composition described in any one of <1> to <10> for use in treating infections caused by Gram-negative bacteria.

<12>

An agent for use in treating infections caused by Gram-negative bacteria, including the pharmaceutical composition described in any one of <1> to <10>.

<13>

A kit for use in treating infections, including a compound represented by General Formula [1] or a salt thereof, and one or more compounds selected from a β-lactamase inhibitory compound and an antibacterial compound, or salts thereof,

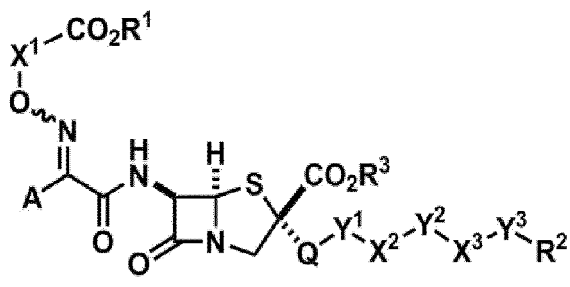

[1]

"in the formula,

$R^1$ represents a hydrogen atom;
$R^2$ represents an aryl group which may be substituted;
$R^3$ represents a hydrogen atom;
$X^1$ represents a $C_{1-6}$ alkylene group which may be substituted, a $C_{2-6}$ alkenylene group which may be substituted, a $C_{2-6}$ alkynylene group which may be substituted, a divalent cyclic hydrocarbon group which may be substituted, or a divalent monocyclic saturated heterocyclic group which may be substituted;
A represents a monocyclic heterocyclic group which may be substituted;
Q represents a divalent cyclic amino group which may be substituted or a divalent heterocyclic group which may be substituted;
$Y^1$ represents a $C_{1-6}$ alkylene group which may be substituted, a $C_{2-6}$ alkenylene group which may be substituted, a $C_{2-6}$ alkynylene group which may be substituted, a group represented by Formula -N=CH-CH=N-, a group represented by Formula -N=CH-CH=N-O-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula -NHC(=O)C(=O)N(OH)-, a group represented by Formula $-NHCH_2C(=O)-$, a group represented by Formula $-NHS(=O)_2NH-C(=O)-$, a group represented by Formula $-NHC(=O)NHS(=O)_2-$, or a bond;
$X^2$ represents a group represented by General Formula $-NR^4-$ (where $R^4$ represents a hydrogen atom, a carbamoyl group, a $C_{1-6}$ alkyl group which may be substituted, or a hydroxyl group which may be protected), a group represented by General Formula $-N^+R^5R^6-$ (where $R^5$ and $R^6$ are the same as or different from each other and each represent a $C_{1-6}$ alkyl group which may be substituted, or in combination represent a $C_{2-6}$ alkylene group which may be substituted or a $C_{2-6}$ alkenylene group which may be substituted), a group represented by General Formula $-NR^7-C(=O)-NR^8-$ (where $R^7$ and $R^8$ are the same as or different from each other and each represent a hydrogen atom, a $C_{1-6}$ alkyl group which may be substituted, or a hydroxyl group which may be protected), a

divalent cyclic amino group which may be substituted, a divalent heterocyclic group which may be substituted, or a bond;

$Y^2$ represents a $C_{1-6}$ alkylene group which may be substituted, or a bond;

$X^3$ represents a group represented by General Formula -$NR^9$- (where $R^9$ represents a hydrogen atom); and

$Y^3$ represents a group represented by Formula -C(=O)-, a group represented by Formula -C(=O)-C(=O)-, or a group represented by General Formula -C(=O)-C(=$NR^{10a}$)-(where $R^{10a}$ represents a hydroxyl group).

<14>

The kit for use described in <13>, in which $R^2$ represents an aryl group which may be substituted, in which A represents a monocyclic heterocyclic group which may be substituted, in which $X^1$ represents a $C_{1-6}$ alkylene group which may be substituted or a divalent cyclic hydrocarbon group which may be substituted, in which Q represents a divalent heterocyclic group which may be substituted, in which $Y^1$ represents a $C_{1-6}$ alkylene group which may be substituted, a group represented by Formula -N=CH-CH=N-, a group represented by Formula -N=CH-$CH_2$-, a group represented by Formula -N=CHC(=O)-, a group represented by Formula -NHC(=O)-, a group represented by Formula -NHC(=O)$CH_2$-, a group represented by Formula -NHC(=O)NH-, a group represented by Formula -NHC(=O)NH-O-, a group represented by Formula -NHC(=O)C(=O)NH-, a group represented by Formula -$NHCH_2C$(=O)-, or a bond, in which $X^2$ represents a group represented by General Formula -$NR^{4a}$- (where $R^{4a}$ represents a hydrogen atom or a carbamoyl group), a group represented by General Formula -$N^+R^{5a}R^{6a}$- (where $R^{5a}$ and $R^{6a}$ in combination represent a $C_{2-6}$ alkylene group which may be substituted), a group represented by General Formula -$NR^{7a}$-C(=O)-$NR^{8a}$- (where $R^{7a}$ and $R^{8a}$ each represent a hydrogen atom), a divalent cyclic amino group which may be substituted, a divalent heterocyclic group which may be substituted, or a bond, in which $Y^2$ represents a $C_{1-6}$ alkylene group which may be substituted or a bond, in which $X^3$ represents a group represented by General Formula -$NR^{9a}$- (where $R^{9a}$ represents a hydrogen atom), in which $R^3$ represents a hydrogen atom, in which $R^1$ represents a hydrogen atom.

<15>

The kit for use described in any one of <13> or <14>, in which $R^2$ represents a phenyl group which may be substituted;

A represents a monocyclic nitrogen and sulfur-containing heterocyclic group which may be substituted;

Q represents a divalent monocyclic heterocyclic group which may be substituted;

$Y^1$ represents a group represented by Formula -NHC(=O)-, a group represented by Formula -NHC(=O)C(=O)NH-, or a bond;

$X^2$ represents a group represented by General Formula -$NR^{4b}$- (where $R^{4b}$ represents a hydrogen atom) or a bond;

$Y^2$ represents a $C_{1-3}$ alkylene group or a bond; and

$Y^3$ represents a group represented by Formula -C(=O)- or a group represented by Formula -C(=O)-C(=O)-.

<16>

The kit for use described in <13>, in which the compound represented by General Formula [1] is a compound selected from (3R,5R,6R)-6-((Z)-2-(2-aminothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-a zabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thi a-1-azabicyclo[3.2.0]heptane-3-carboxylic acid, (3R,5R,6R)-6-((Z)-2-(2-amino-5-chlorothiazol-4-yl)-2-(((2-carboxypropan-2-yl)oxy)imino)ac etamido)-3-(3-(2-(2-chloro-3,4-dihydroxyphenyl)-2-oxoacetamido)-2-oxoimidazolidin-1-yl)-7 -oxo-4-thia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-((S)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-((R)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetatamido)-2,3-dioxopiperazin-1-yl)-7-oxo-4-thia-1-azabicyclo[3.2.0]heptane-3-carboxylic acid, and (3R,5R,6R)-6-((Z)-2-(2-amino-5-chlorothiazol-4-yl)-2-(((2-carboxypropan-2-yl)oxy)imino)ac etamido)-3 -(3 -(2-(2-(2-(2-chloro-3,4-dihydroxyphenyl)-2-oxoacetyl)hydradienyl)-2-oxoaceta mido)-2-oxoimidazolidin-1-yl)-7-oxo-4-thia-1-azabicyclo[3.2.0]heptane-3-carboxylic acid.

<17>

The kit for use described in any one of <13> to <16>, in which the one or more compounds selected from a β-lactamase inhibitory compound and an antibacterial compound or salts thereof are a β-lactamase inhibitory compound or a salt thereof.

<18>

The kit for use described in any one of <13> to <17>, in which the β-lactamase inhibitory compound is one or more compounds selected from a diazabicyclo[3.2.1]octane-type inhibitory compound, a boronic acid-type inhibitory compound, and a clavam-type inhibitory compound.

<19>

The kit for use described in any one of <13> to <17>, in which the β-lactamase inhibitory compound is one or more compounds selected from avibactam, nacubactam, relebactam, zidebactam, vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid, sulbactam, tazobactam, clavulanic acid, (1aR,7bS)-5-fluoro-2-hydroxy-1,1a,2,7b-tetrahydrocyclopropan[c][1,2]benzoxaborinine-4-car boxylic acid, and (2S,3S,5R)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide.

The pharmaceutical composition and the kit according to an embodiment of the present invention exhibit strong antibacterial activity against Gram-negative bacteria and/or drug-resistant Gram-negative bacteria such as enterobacteria or Pseudomonas aeruginosa producing carbapenemase, and are useful as a medicine.

In addition, the pharmaceutical composition and the kit according to the embodiment of the present invention are useful for treating infections caused by Gram-negative bacteria and/or drug-resistant Gram-negative bacteria.

## DESCRIPTION OF THE PREFERRED EMBODIMENTS

Hereinafter, the present invention will be specifically described.

In the present specification, unless otherwise specified, "%" means "% by mass".

In the present specification, unless otherwise specified, each term has the following meaning.

The halogen atom means a fluorine atom, a chlorine atom, a bromine atom, or an iodine atom.

The $C_{1-6}$ alkyl group means, for example, a linear or branched $C_{1-6}$ alkyl group such as a methyl, ethyl, propyl, isopropyl, butyl, sec-butyl, isobutyl, tert-butyl, pentyl, isopentyl, or hexyl group.

The $C_{1-3}$ alkyl group means a methyl, ethyl, propyl, or isopropyl group.

The $C_{2-6}$ alkenyl group means, for example, a linear or branched $C_{2-6}$ alkenyl group such as a vinyl, allyl, propenyl, isopropenyl, butenyl, isobutenyl, 1,3-butadienyl, pentenyl, or hexenyl group.

The $C_{2-6}$ alkynyl group means a linear or branched $C_{2-6}$ alkynyl group such as an ethynyl, propynyl, butynyl, pentynyl, or hexynyl group.

The $C_{3-8}$ cycloalkyl group means a $C_{3-8}$ cycloalkyl group such as a cyclopropyl, cyclobutyl, cyclopentyl, or cyclohexyl group.

The aryl group means, for example, a $C_{6-18}$ aryl group such as a phenyl or naphthyl group.

The aryl $C_{1-6}$ alkyl group means, for example, an aryl $C_{1-6}$ alkyl group such as a benzyl, diphenylmethyl, trityl, phenethyl, or naphthylmethyl group.

The $C_{1-6}$ alkylene group means a linear or branched $C_{1-6}$ alkylene group such as a methylene, ethylene, propylene, butylene, or hexylene group.

The $C_{1-3}$ alkylene group means a methylene, ethylene, or propylene group.

The $C_{2-6}$ alkylene group means a linear or branched $C_{2-6}$ alkylene group such as an ethylene, propylene, butylene, or hexylene group.

The $C_{2-6}$ alkenylene group means a linear or branched $C_{2-6}$ alkenylene group such as a vinylene, propenylene, butenylene, or pentenylene group.

The $C_{2-6}$ alkynylene group means a linear or branched $C_{2-6}$ alkynylene group such as an ethynylene, propynylene, butynylene, or pentynylene group.

The $C_{1-6}$ alkoxy group means, for example, a linear or branched $C_{1-6}$ alkyloxy group such as a methoxy, ethoxy, propoxy, isopropoxy, butoxy, isobutoxy, sec-butoxy, tert-butoxy, pentyloxy, or hexyloxy group.

The $C_{1-6}$ alkoxy $C_{1-6}$ alkyl group means, for example, a $C_{1-6}$ alkyloxy $C_{1-6}$ alkyl group such as a methoxymethyl or 1-ethoxyethyl group.

The $C_{2-12}$ alkanoyl group means, for example, a linear or branched $C_{2-12}$ alkanoyl group such as an acetyl, propionyl, valeryl, isovaleryl, or pivaloyl group.

The aroyl group means, for example, a benzoyl or naphthoyl group.

The acyl group means, for example, a formyl group, a succinyl group, a glutaryl group, a maleoyl group, a phthaloyl group, a $C_{2-12}$ alkanoyl group, or an aroyl group.

The $C_{1-6}$ alkoxycarbonyl group means, for example, a linear or branched $C_{1-6}$ alkyloxycarbonyl group such as a methoxycarbonyl, ethoxycarbonyl, isopropoxycarbonyl, tert-butoxycarbonyl, or 1,1-dimethylpropoxycarbonyl group.

The aryl $C_{1-6}$ alkoxycarbonyl group means, for example, an ar $C_{1-6}$ alkyloxycarbonyl group such as a benzyloxycarbonyl or phenethyloxycarbonyl group.

The aryloxycarbonyl group means, for example, a phenyloxycarbonyl or naphthyloxycarbonyl group.

The $C_{1-6}$ alkylamino group means a linear or branched $C_{1-6}$ alkylamino group such as a methylamino, ethylamino, propylamino, isopropylamino, butylamino, sec-butylamino, tert-butylamino, pentylamino, or hexylamino group.

The di($C_{1-6}$ alkyl) amino group means a linear or branched di($C_{1-6}$ alkyl) amino group such as a dimethylamino, diethylamino, dipropylamino, diisopropylamino, dibutylamino, di (tert-butyl) amino, dipentylamino, dihexylamino, (ethyl) (methyl) amino, or (methyl)(propyl) amino group.

The $C_{1-6}$ alkylthio group means, for example, a $C_{1-6}$ alkylthio group such as a methylthio, ethylthio, or propylthio group.

The $C_{1-6}$ alkylsulfonyl group means, for example, a $C_{1-6}$ alkylsulfonyl group such as a methylsulfonyl, ethylsulfonyl, or propylsulfonyl group.

The arylsulfonyl group means, for example, a benzenesulfonyl, p-toluenesulfonyl, or naphthalenesulfonyl group.

The $C_{1-6}$ alkylsulfonyloxy group means, for example, a $C_{1-6}$ alkylsulfonyloxy group such as a methylsulfonyloxy an ethylsulfonyloxy group.

The arylsulfonyloxy group means a benzenesulfonyloxy or p-toluenesulfonyloxy group.

The silyl group means, for example, a trimethylsilyl, triethylsilyl, or tributylsilyl group.

The cyclic amino group means a cyclic amino group which contains one or more nitrogen atoms as hetero atoms forming a ring, such as a aziridinyl, azetidinyl, pyrrolyl, dihydropyrrolyl, pyrrolidinyl, tetrahydropyridyl, piperidinyl, homopiperidinyl, pyrazolyl, pyrazolinyl, pyrazolidinyl, imidazolyl, imidazolinyl, imidazolidinyl, thiazolinyl, thiazolidinyl, dihydrothiadiazolyl, piperazinyl, homopiperazinyl, morpholinyl, homomorpholinyl, or thiomorpholinyl group, and may further contain one or more oxygen atoms or sulfur atoms.

The monocyclic nitrogen-containing heterocyclic group means a monocyclic nitrogen-containing heterocyclic group containing only nitrogen atoms as hetero atoms forming a ring. Examples of the monocyclic nitrogen-containing heterocyclic group include an azetidinyl group; a 5-membered nitrogen-containing heterocyclic group such as a pyrrolidinyl, pyrrolinyl, pyrrolyl, imidazolidinyl, imidazolinyl, imidazolyl, pyrazolidinyl, pyrazolinyl, pyrazolyl, triazolyl, or tetrazolyl group; a 6-membered nitrogen-containing heterocyclic group such as a piperidyl, tetrahydropyridyl, pyridyl, piperazinyl, pyrazinyl, pyridazinyl, pyrimidinyl, tetrahydropyrimidyl, or homopiperazinyl group; a 7-membered nitrogen-containing heterocyclic group such as a homopiperidinyl group; and an 8-membered nitrogen-containing heterocyclic group such as an octahydroazocinyl group.

The monocyclic oxygen-containing heterocyclic group means a monocyclic oxygen-containing heterocyclic group containing only oxygen atoms as hetero atoms forming a ring. Examples of the monocyclic oxygen-containing heterocyclic group include a 5-membered oxygen-containing heterocyclic group such as a tetrahydrofuranyl or furanyl group; and a 6-membered oxygen-containing heterocyclic group such as a tetrahydropyranyl or pyranyl group.

The monocyclic sulfur-containing heterocyclic group means a thienyl group or the like.

The monocyclic nitrogen and oxygen-containing heterocyclic group means a monocyclic nitrogen and oxygen-containing heterocyclic group containing only a nitrogen atom and an oxygen atom as hetero atoms forming a ring. Examples of the monocyclic nitrogen and oxygen-containing heterocyclic group include a 5-membered nitrogen and oxygen-containing heterocyclic group such as an oxazolyl, oxazolidinyl, isoxazolyl, or oxadiazolyl group; and a 6-membered nitrogen and oxygen-containing heterocyclic group such as a homomorpholinyl group.

The monocyclic nitrogen and sulfur-containing heterocyclic group means a monocyclic nitrogen and sulfur-containing heterocyclic group containing only a nitrogen atom and a sulfur atom as hetero atoms forming a ring. Examples of the monocyclic nitrogen and sulfur-containing heterocyclic group include a 5-membered nitrogen and sulfur-containing heterocyclic group such as a thiazolyl, isothiazolyl, or thiadiazolyl group; and a 6-membered nitrogen and sulfur-containing heterocyclic group such as a thiomorpholinyl, 1-oxidethiomorpholinyl, or 1,1-dioxidethiomorpholinyl group.

The monocyclic heterocyclic group means a monocyclic nitrogen-containing heterocyclic group, a monocyclic oxygen-containing heterocyclic group, a monocyclic sulfur-containing heterocyclic group, a monocyclic nitrogen and oxygen-containing heterocyclic group, or a monocyclic nitrogen and sulfur-containing heterocyclic group.

The monocyclic saturated heterocyclic group means a monocyclic heterocyclic group not containing a multiple bond. Examples of the monocyclic saturated heterocyclic group include aziridinyl, azetidinyl, pyrrolidinyl, piperidinyl, imidazolidinyl, pyrazolidinyl, piperazinyl, oxazolidinyl, tetrahydropyrimidyl, tetrahydrofuranyl, tetrahydropyranyl, and morpholinyl groups.

The bicyclic nitrogen-containing heterocyclic group means a bicyclic nitrogen-containing heterocyclic group which contains only nitrogen atoms as hetero atoms forming a ring such as an indolinyl, indolyl, isoindolinyl, isoindolyl, benzimidazolyl, indazolyl, benzotriazolyl, quinolyl, tetrahydroquinolinyl, tetrahydroisoquinolinyl, isoquinolinyl, quinolizinyl, cinnolinyl, phthalazinyl, quinazolinyl, dihydroquinoxalinyl, quinoxalinyl, naphthyridinyl, purinyl, pyrrolopyridinyl, dihydrocyclopentapyridinyl, pteridinyl, or quinuclidinyl group.

The bicyclic oxygen-containing heterocyclic group means a bicyclic oxygen-containing heterocyclic group containing only oxygen atoms as hetero atoms forming a ring such as a 2,3-dihydrobenzofuranyl, benzofuranyl, isobenzofuranyl, chromanyl, chromenyl, isochromanyl, 1,3-benzodioxolyl, 1,3-benzodioxanyl, or 1,4-benzodioxanyl

group.

The bicyclic sulfur-containing heterocyclic group means a bicyclic sulfur-containing heterocyclic group containing only sulfur atoms as hetero atoms forming a ring such as a 2,3-dihydrobenzothienyl or benzothienyl group.

The bicyclic nitrogen and oxygen-containing heterocyclic group means a bicyclic nitrogen and oxygen-containing heterocyclic group containing only a nitrogen atom and an oxygen atom as hetero atoms forming a ring such as a benzoxazolyl, benzisoxazolyl, benzoxadiazolyl, benzomorpholinyl, dihydropyranopyridyl, dihydrodioxynopyridyl, or dihydropyridoxazinyl group.

The bicyclic nitrogen and sulfur-containing heterocyclic group means a bicyclic nitrogen and sulfur-containing heterocyclic group containing a nitrogen atom and a sulfur atom as hetero atoms forming a ring such as a benzothiazolyl, benzisothiazolyl, or benzothiadiazolyl group.

The bicyclic heterocyclic group means a bicyclic nitrogen-containing heterocyclic group, a bicyclic oxygen-containing heterocyclic group, a bicyclic sulfur-containing heterocyclic group, a bicyclic nitrogen and oxygen-containing heterocyclic group, or a bicyclic nitrogen and sulfur-containing heterocyclic group.

The heterocyclic group means a monocyclic heterocyclic group or a bicyclic heterocyclic group.

The divalent cyclic hydrocarbon group means a group formed by removing any two hydrogen atoms from cyclic hydrocarbons such as cyclopropane-1,1-diyl, cyclobutane-1,1-diyl, cyclopentane-1,1-diyl, cyclohexane-1,1-diyl, cyclopropane-1,2-diyl, cyclobutane-1,3-diyl, cyclobutene-1,3-diyl, cyclopentane-1,3-diyl, cyclopentene-1,3-diyl, cyclopentadiene-1,3-diyl, cyclohexane-1,3-diyl, cyclohexane-1,4-diyl, cyclohexene-1,3-diyl, cyclohexene-1,4-diyl, cyclohexadiene-1,3-diyl, cyclohexadiene-1,4-diyl, cycloheptane-1,3-diyl, cycloheptene-1,4-diyl, cyclooctane-1,3-diyl, benzene-1,3-diyl, and benzene-1,4-diyl.

The divalent monocyclic saturated heterocyclic group is a divalent group formed by further removing any one hydrogen atom from the aforementioned monocyclic heterocyclic group not containing a multiple bond. For example, the divalent monocyclic saturated heterocyclic group means a group formed by further removing any one hydrogen atom from an aziridinyl, azetidinyl, pyrrolidinyl, piperidinyl, imidazolidinyl, pyrazolidinyl, piperazinyl, tetrahydrofuranyl, tetrahydropyranyl, or morpholinyl group, and the like.

The divalent heterocyclic group is a divalent group formed by further removing any one hydrogen atom from the aforementioned heterocyclic group. The divalent heterocyclic group includes a divalent monocyclic heterocyclic group and a divalent bicyclic heterocyclic group.

The divalent monocyclic heterocyclic group is a divalent group formed by further removing any one hydrogen atom from the aforementioned monocyclic heterocyclic group. The divalent monocyclic heterocyclic group includes a divalent monocyclic nitrogen-containing heterocyclic group, a divalent monocyclic oxygen-containing heterocyclic group, a divalent monocyclic sulfur-containing heterocyclic group, a divalent monocyclic nitrogen and oxygen-containing heterocyclic group, and a divalent monocyclic nitrogen and sulfur-containing heterocyclic group.

The divalent bicyclic heterocyclic group is a divalent group formed by further removing any one hydrogen atom from the aforementioned bicyclic heterocyclic group. The divalent bicyclic heterocyclic group includes a divalent bicyclic nitrogen-containing heterocyclic group, a divalent bicyclic oxygen-containing heterocyclic group, a divalent bicyclic sulfur-containing heterocyclic group, a divalent bicyclic nitrogen and oxygen-containing heterocyclic group, and a divalent bicyclic nitrogen and sulfur-containing heterocyclic group.

The divalent cyclic amino group is a divalent group formed by further removing any one hydrogen atom from the aforementioned cyclic amino group. Examples of the divalent cyclic amino group include a divalent group formed by removing any two hydrogen atoms from aziridine, azetidine, pyrrole, dihydropyrrole, pyrrolidine, tetrahydropyridine, piperidine, homopiperidine, pyrazolyl, pyrazoline, pyrazolidine, imidazole, imidazoline, imidazolidine, thiazoline, thiazolidine, dihydrothiadiazole, piperazine, homopiperazine, morpholine, homomorpholine, and thiomorpholine.

Examples of the leaving group include a halogen atom, a $C_{1-6}$ alkylsulfonyloxy group, an arylsulfonyloxy group, and an imidazole group. The $C_{1-6}$ alkylsulfonyloxy group, the arylsulfonyloxy group, or the imidazole group may have a substituent.

The hydroxyl protecting group includes all groups that can be used as a protecting group of general hydroxyl groups. Examples of the hydroxyl protecting group include the groups described in "Protective Groups in Organic Synthesis, W. Greene et al., 4th Edition, pp. 16-299, 2007, John Wiley & Sons, INC". Specifically, examples thereof include a $C_{1-6}$ alkyl group, a $C_{2-6}$ alkenyl group, an aryl $C_{1-6}$ alkyl group, a $C_{1-6}$ alkoxy $C_{1-6}$ alkyl group, an acyl group, a $C_{1-6}$ alkoxycarbonyl group, an aryl $C_{1-6}$ alkoxycarbonyl group, a $C_{1-6}$ alkylsulfonyl group, an arylsulfonyl group, a silyl group, a tetrahydrofuranyl group, and a tetrahydropyranyl group. These groups may be substituted with one or more groups selected from the substituent group A1.

The amino protecting group includes all groups that can be used as a protecting group of general amino groups. Examples of the amino protecting group include the groups described in "Protective Groups in Organic Synthesis, W. Greene et al., 4th Edition, pp. 696-926, 2007, John Wiley & Sons, INC". Specifically, examples thereof include an aryl $C_{1-6}$ alkyl group, a $C_{1-6}$ alkoxy $C_{1-6}$ alkyl group, an acyl group, a $C_{1-6}$ alkoxycarbonyl group, an aryl $C_{1-6}$ alkoxycarbonyl group, an aryloxycarbonyl group, a $C_{1-6}$ alkylsulfonyl group, an arylsulfonyl group, and a silyl group. These groups may be

substituted with one or more groups selected from the substituent group A1.

The imino protecting group includes all groups that can be used as a protecting group of general imino groups. Examples of the imino protecting group include the groups described in "Protective Groups in Organic Synthesis, W. Greene et al., 4th Edition, pp. 696-868, 2007, John Wiley & Sons, INC". Specifically, examples thereof include an aryl $C_{1-6}$ alkyl group, a $C_{1-6}$ alkoxy $C_{1-6}$ alkyl group, an acyl group, a $C_{1-6}$ alkoxycarbonyl group, an aryl $C_{1-6}$ alkoxycarbonyl group, an aryloxycarbonyl group, a $C_{1-6}$ alkylsulfonyl group, an arylsulfonyl group, and a silyl group. These groups may be substituted with one or more groups selected from the substituent group A1.

The carboxyl protecting group includes all groups that can be used as a protecting group of general carboxyl groups. Examples of the carboxyl protecting group include the groups described in "Protective Groups in Organic Synthesis, W. Greene et al., 4th Edition, pp. 533-643, 2007, John Wiley & Sons, INC". Specifically, examples thereof include a $C_{1-6}$ alkyl group, a $C_{2-6}$ alkenyl group, an aryl $C_{1-6}$ alkyl group, a $C_{1-6}$ alkoxy $C_{1-6}$ alkyl group, and a silyl group. These groups may be substituted with one or more groups selected from the substituent group A1.

Examples of aliphatic hydrocarbons include pentane, hexane, cyclohexane, heptane, and decahydronaphthalene.

Examples of halogenated hydrocarbons include methylene chloride, chloroform, and dichloroethane.

Examples of alcohols include methanol, ethanol, propanol, 2-propanol, butanol, and 2-methyl-2-propanol.

Examples of ethers include diethyl ether, diisopropyl ether, dioxane, tetrahydrofuran, anisole, ethylene glycol dimethyl ether, diethylene glycol dimethyl ether, and diethylene glycol diethyl ether.

Examples of ketones include acetone, 2-butanone, and 4-methyl-2-pentanone.

Examples of esters include methyl acetate, ethyl acetate, propyl acetate, and butyl acetate.

Examples of amides include N,N-dimethylformamide, N,N-dimethylacetamide, and 1-methyl-2-pyrrolidone.

Examples of nitriles include acetonitrile and propionitrile.

Examples of aromatic hydrocarbons include benzene, toluene, and xylene.

In the present specification, the substituent group means the following.

Substituent group A1:

a hydrogen atom,
a halogen atom,
a cyano group,
a nitro group,
an oxo group,
a $C_{1-6}$ alkyl group which may be substituted with one or more groups selected from a substituent group B2,
a $C_{2-6}$ alkenyl group which may be substituted with one or more groups selected from the substituent group B2,
a $C_{2-6}$ alkynyl group which may be substituted with one or more groups selected from the substituent group B2,
a $C_{1-6}$ alkoxy group which may be substituted with one or more groups selected from the substituent group B2,
an aryloxy group which may be substituted with one or more groups selected from a substituent group B1,
an acyl group which may be substituted with one or more groups selected from the substituent group B1,
a $C_{1-6}$ alkylamino group which may be substituted with one or more groups selected from the substituent group B2,
a di($C_{1-6}$ alkyl)amino group which may be substituted with one or more groups selected from the substituent group B2,
an imino group which may be protected or substituted with one or more groups selected from the substituent group B1,
a $C_{1-6}$ alkylthio group which may be substituted with one or more groups selected from the substituent group B2,
an arylthio group which may be substituted with one or more groups selected from the substituent group B1,
a $C_{1-6}$ alkylsulfonyl group which may be substituted with one or more groups selected from the substituent group B2,
an arylsulfonyl group which may be substituted with one or more groups selected from the substituent group B1,
a $C_{3-8}$ cycloalkyl group which may be substituted with one or more groups selected from the substituent group B1,
an aryl group which may be substituted with one or more groups selected from the substituent group B1,
a heterocyclic group which may be substituted with one or more groups selected from the substituent group B1,
a carbamoyl group which may be substituted with one or more groups selected from the substituent group B1,
a sulfamoyl group which may be substituted with one or more groups selected from the substituent group B1,
a hydroxyl group which may be protected,
an amino group which may be protected, and
a carboxyl group which may be protected.

Substituent group A2:

a hydrogen atom,
a halogen atom,
a cyano group,

a nitro group,
an oxo group,
a $C_{1-6}$ alkoxy group which may be substituted with one or more groups selected from the substituent group B2,
an aryloxy group which may be substituted with one or more groups selected from a substituent group B1,
an acyl group which may be substituted with one or more groups selected from the substituent group B1,
a $C_{1-6}$ alkylamino group which may be substituted with one or more groups selected from the substituent group B2,
a di($C_{1-6}$ alkyl)amino group which may be substituted with one or more groups selected from the substituent group B2,
a $C_{1-6}$ alkylthio group which may be substituted with one or more groups selected from the substituent group B2,
an arylthio group which may be substituted with one or more groups selected from the substituent group B1,
a $C_{1-6}$ alkylsulfonyl group which may be substituted with one or more groups selected from the substituent group B2,
an arylsulfonyl group which may be substituted with one or more groups selected from the substituent group B1,
a $C_{3-8}$ cycloalkyl group which may be substituted with one or more groups selected from the substituent group B1,
an aryl group which may be substituted with one or more groups selected from the substituent group B1,
a heterocyclic group which may be substituted with one or more groups selected from the substituent group B1,
a carbamoyl group which may be substituted with one or more groups selected from the substituent group B1,
a sulfamoyl group which may be substituted with one or more groups selected from the substituent group B1,
a hydroxyl group which may be protected,
an amino group which may be protected, and
a carboxyl group which may be protected.

Substituent group B1:

a hydrogen atom,
a halogen atom,
a cyano group,
a nitro group,
an oxo group,
a $C_{1-6}$ alkyl group which may be substituted with one or more groups selected from a substituent group C,
a $C_{2-6}$ alkenyl group which may be substituted with one or more groups selected from the substituent group C,
a $C_{2-6}$ alkynyl group which may be substituted with one or more groups selected from the substituent group C,
a $C_{1-6}$ alkoxy group which may be substituted with one or more groups selected from the substituent group C,
an aryloxy group which may be substituted with one or more groups selected from the substituent group C,
an acyl group which may be substituted with one or more groups selected from the substituent group C,
a $C_{1-6}$ alkylamino group which may be substituted with one or more groups selected from the substituent group C,
a di($C_{1-6}$ alkyl)amino group which may be substituted with one or more groups selected from the substituent group C,
a $C_{1-6}$ alkylthio group which may be substituted with one or more groups selected from the substituent group C,
an arylthio group which may be substituted with one or more groups selected from the substituent group C,
a $C_{1-6}$ alkylsulfonyl group which may be substituted with one or more groups selected from the substituent group C,
an arylsulfonyl group which may be substituted with one or more groups selected from the substituent group C,
a $C_{3-8}$ cycloalkyl group which may be substituted with one or more groups selected from the substituent group C,
an aryl group which may be substituted with one or more groups selected from the substituent group C,
a heterocyclic group which may be substituted with one or more groups selected from the substituent group C,
a carbamoyl group which may be substituted with one or more groups selected from the substituent group C,
a sulfamoyl group which may be substituted with one or more groups selected from the substituent group C,
a hydroxyl group which may be protected,
an amino group which may be protected, and
a carboxyl group which may be protected.

Substituent group B2:

a hydrogen atom,
a halogen atom,
a cyano group,
a nitro group,
an oxo group,
a $C_{1-6}$ alkoxy group which may be substituted with one or more groups selected from the substituent group C,
an aryloxy group which may be substituted with one or more groups selected from the substituent group C,
an acyl group which may be substituted with one or more groups selected from the substituent group C,

a $C_{1-6}$ alkylamino group which may be substituted with one or more groups selected from the substituent group C,
a di($C_{1-6}$ alkyl)amino group which may be substituted with one or more groups selected from the substituent group C,
a $C_{1-6}$ alkylthio group which may be substituted with one or more groups selected from the substituent group C,
an arylthio group which may be substituted with one or more groups selected from the substituent group C,
a $C_{1-6}$ alkylsulfonyl group which may be substituted with one or more groups selected from the substituent group C,
an arylsulfonyl group which may be substituted with one or more groups selected from the substituent group C,
a $C_{3-8}$ cycloalkyl group which may be substituted with one or more groups selected from the substituent group C,
an aryl group which may be substituted with one or more groups selected from the substituent group C,
a heterocyclic group which may be substituted with one or more groups selected from the substituent group C,
a carbamoyl group which may be substituted with one or more groups selected from the substituent group C,
a sulfamoyl group which may be substituted with one or more groups selected from the substituent group C,
a hydroxyl group which may be protected,
an amino group which may be protected, and
a carboxyl group which may be protected.

Substituent group C:

a halogen atom,
a cyano group,
a carbamoyl group,
a $C_{1-6}$ alkyl group,
a $C_{1-6}$ alkoxy group,
an amino group which may be protected, and
an imino group which may be protected,
a hydroxyl group which may be protected,
a carboxyl group which may be protected.

The $C_{1-6}$ alkylene group, the $C_{2-6}$ alkenylene group, and the $C_{2-6}$ alkynylene group represented by $X^1$, $Y^1$, and $Y^2$ may be substituted with one or more groups selected from the substituent group A2.

The $C_{1-6}$ alkyl group represented by $R^4$, $R^5$, $R^6$, $R^7$, and $R^8$ may be substituted with one or more groups selected from the substituent group A2.

The $C_{2-6}$ alkylene and $C_{2-6}$ alkenylene groups that $R^5$ and $R^6$ form in combination may be substituted with one or more groups selected from the substituent group A1.

The $C_{2-6}$ alkylene group that $R^{5a}$ and $R^{6a}$ form in combination may be substituted with one or more groups selected from the substituent group A1.

The aryl group represented by $R^2$ may be substituted with one or more groups selected from the substituent group A1.

The monocyclic heterocyclic group represented by A may be substituted with one or more groups selected from the substituent group A1.

The divalent cyclic hydrocarbon group represented by $X^1$ may be substituted with one or more groups selected from the substituent group A1.

The divalent monocyclic saturated heterocyclic group represented by $X^1$ may be substituted with one or more groups selected from the substituent group A1.

The divalent heterocyclic group and the divalent cyclic amino group represented by $X^2$ and Q may be substituted with one or more groups selected from the substituent group A1.

As the compound represented by General Formula [1] according to the embodiment of the present invention, for example, the following compounds are preferable.

According to the present invention, $R^1$ represents a hydrogen atom and $R^2$ represents an aryl group that may be substituted. A compound in which $R^2$ represents a phenyl group that may be substituted is preferable.

As the substituent of the aryl group represented by $R^2$, one or more groups selected from a halogen atom and a hydroxyl group which may be protected are preferable.

According to the present invention, $R^3$ represents a hydrogen atom.

$X^1$ is preferably a $C_{1-6}$ alkylene group which may be substituted or a divalent cyclic hydrocarbon group which may be substituted, and more preferably a $C_{1-3}$ alkylene group which may be substituted or a divalent cyclic hydrocarbon group which may be substituted.

A is a compound that is a monocyclic heterocyclic group which may be substituted, preferably a monocyclic nitrogen-containing heterocyclic group which may be substituted or a monocyclic nitrogen and sulfur-containing heterocyclic group which may be substituted, more preferably a monocyclic nitrogen and sulfur-containing heterocyclic group,

even more preferably 2-amino-5-chlorothiazol-4-yl, 5-amino-1,2,4-thiadiazole-3-yl, or 2-aminothiazol-4-yl, and particularly preferably 2-aminothiazol-4-yl.

Q is preferably a divalent heterocyclic group which may be substituted, more preferably a divalent monocyclic heterocyclic group which may be substituted, even more preferably a divalent imidazolidine group, a divalent piperazine group, a divalent pyrrolidine group, or a divalent oxazolidine group which may be substituted, and still more preferably a divalent imidazolidine group, a divalent piperazine group, or a divalent pyrrolidine group which may be substituted.

Q is preferably, for example, a 2-oxoimidazolidin-1-yl group, a 2,3-dioxopiperazin-1-yl group, a 2-oxopyrrolidin-1-yl group, or a 2-oxooxazolidin-3-yl group.

$Y^1$ is preferably a $C_{1-6}$ alkylene group which may be substituted, a group represented by Formula -N=CH-CH=N-, a group represented by Formula -N=CH-CH=N-O-, a group represented by $-N=CH-CH_2-$, a group represented by Formula -N=CHC(=O)-, a group represented by Formula -NHC(=O)-, a group represented by Formula $-NHC(=O)CH_2$, a group represented by Formula -NHC(=O)NH-, a group represented by Formula -NHC(=O)NH-O-, a group represented by Formula -NHC(=O)C(=O)NH-, a group represented by Formula $-NHCH_2C(=O)-$, a group represented by Formula $-NHS(=O)_2NHC(=O)-$, a group represented by Formula $-NHC(=O)NHS(=O)_2-$, or a bond, more preferably a $C_{1-6}$ alkylene group which may be substituted, a group represented by Formula -N=CH-CH=N-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula $-NHCH_2C(=O)-$, or a bond, and even more preferably a group represented by formula -NHC(=O)-, a group represented by formula -NHC(=O)C(=O)NH-, or a bond.

$X^2$ is preferably a group represented by General Formula $-NR^{4a}-$ (where $R^{4a}$ represents a hydrogen atom or a carbamoyl group), a group represented by General Formula $-N^+R^{5a}R^{6a}-$ (where $R^{5a}$ and $R^{6a}$ in combination represent a $C_{2-6}$ alkylene group which may be substituted), a group represented by General Formula$-NR^{7a}-C(=O)-NR^{8a}-$ (where $R^{7a}$ and $R^{8a}$ each represent a hydrogen atom), a divalent cyclic amino group which may be substituted, a divalent heterocyclic group which may be substituted, or a bond, and more preferably a group represented by General Formula $-NR^{4b}-$ (where $R^{4b}$ represents a hydrogen atom) or a bond.

$Y^2$ is a $C_{1-6}$ alkylene group which may be substituted or a bond, and preferably a $C_{1-3}$ alkylene group or a bond.

$X^3$ is a group represented by General Formula $-NR9^a-$ (where $R9^a$ represents a hydrogen atom).

$Y^3$ is a group represented by Formula -C(=O)-, a group represented by Formula -C(=O)-C(=O)-, a group represented by General Formula $-C(=O)-C(=NR^{10a})-$ (where $R^{10a}$ represents a hydroxyl group), and preferably a group represented by Formula -C(=O)- or a group represented by Formula -C(=O)-C(=O)-.

More specifically, the following compounds are preferable.

(3R,5R,6R)-6-((Z)-2-(2-aminothiazol-4-yl)-2-(((2-carboxypropan-2-yl)oxy)imino)ace tamido)-3-(3-(2-chloro-3,4-dihydroxybenzamido)-2-oxoimidazolidin-1-yl)-7-oxo-4-thia-1-aza bicyclo[3.2.0]heptane-3-carboxylic acid (compound of Example 2)

(3R,5R,6R)-6-((Z)-2-(2-aminothiazol-4-yl)-2-((1-carboxycyclopropoxy)imino)acetam ido)-3-(3-(2-chloro-3,4-dihydroxybenzamido)-2-oxoimidazolidin-1-yl)-7-oxo-4-thia-1-azabicy clo[3.2.0]heptane-3-carboxylic acid (compound of Example 8)

(3R,5R,6R)-6-((Z)-2-(2-aminothiazol-4-yl)-2-((1-carboxycyclobutoxy)imino)acetami do)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 19)

(3R,5R,6R)-6-((Z)-2-(2-aminothiazol-4-yl)-2-(((2-carboxypropan-2-yl)oxy)imino)ace tamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 20)

(3R,5R,6R)-6-((Z)-2-(2-aminothiazol-4-yl)-2-((1-carboxycyclopropoxy)imino)acetam ido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 21)

(3R,5R,6R)-6-((Z)-2-(2-amino-5-chlorothiazol-4-yl)-2-(((2-carboxypropan-2-yl)oxy)imino)ac etamido)-3-(3-(2-(2-chloro-3,4-dihydroxyphenyl)-2-oxoacetamido)-2-oxoimidazolidin-1-yl)-7 -oxo-4-thia-1-azabicyclo[3.2.0]heptane-3-carboxylic acid (compound of Example 22)

(3R,5R,6R)-6-((Z)-2-(5-amino-1,2,4-thiadiazol-3-yl)-2-(((2-carboxypropan-2-yl)oxy) imino)acetamido)-3-(3-(2-(2-chloro-3,4-dihydroxyphenyl)-2-oxoacetamido)-2-oxoimidazolidi n-1-yl)-7-oxo-4-thia-1-azabicyclo[3.2.0]heptane-3-carboxylic acid (compound of Example 23)

(3R,5R,6R)-6-((Z)-2-(2-aminothiazol-4-yl)-2-(((2-carboxypropan-2-yl)oxy)imino)ace tamido)-3-(4-(2-(2-chloro-3,4-dihydroxyphenyl)-2-oxoacetamido)-5-oxo-4,5-dihydro-1H-1,2, 4-triazol-1-yl)-7-oxo-4-thia-1-azabicyclo[3.2.0]heptane-3-carboxylic acid (compound of Example 26)

(3R,5R,6R)-6-((Z)-2-(2-aminothiazol-4-yl)-2-(((2-carboxypropan-2-yl)oxy)imino)ace tamido)-3-(3-(3-(2-chloro-3,4-dihydroxybenzamido)propanamido)-2-oxoimidazolidin-1-yl)-7-oxo-4-thia-1-azabicyclo[3.2.0]heptane-3-carboxylic acid (compound of Example 28)

(3R,5R,6R)-6-((Z)-2-(2-aminothiazol-4-yl)-2-(((2-carboxypropan-2-yl)oxy)imino)acetamido)-3-(3-2-(2-(2-chloro-3,4-dihydroxyphenyl)-2-oxoacetamido)acetamido)-2-oxoimidazolidin-1-yl)-7-oxo-4-thia-1-azabicyclo[3.2.0]heptane-3-carboxylic acid (compound of Example 29)

(3R,5R,6R)-6-((Z)-2-(2-aminothiazol-4-yl)-2-(((2-carboxypropan-2-yl)oxy)imino)acetamido)-3-(3-(3-(2-(2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid (compound of Example 30)

(3R,5R,6R)-6-((Z)-2-(2-aminothiazol-4-yl)-2-((1-carboxycyclopropoxy)imino)acetamido)-3-(3-(3-(2-(2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid (compound of Example 31)

(3R,5R,6R)-6-((Z)-2-(2-aminothiazol-4-yl)-2-(((2-carboxypropan-2-yl)oxy)imino)acetamido)-3-((S)-(3-(((E)-2-(2-chloro-3,4-dihydroxybenzamido)ethylidene)amino)-5-methyl-2-oxoimidazolidin-1-yl)-7-oxo-4-thia-1-azabicyclo[3.2.0]heptane-3-carboxylic acid (compound of Example 45)

(3R,5R,6R)-6-((Z)-2-(2-aminothiazol-4-yl)-2-(((2-carboxypropan-2-yl)oxy)imino)acetamido)-3-((S)-(3-(((E)-2-(2-(2-chloro-3,4-dihydroxyphenyl)-2-oxoacetamido)ethylidene)amino)-5-methyl-2-oxoimidazolidin-1-yl)-7-oxo-4-thia-1-azabicyclo[3.2.0]heptane-3-carboxylic acid (compound of Example 47)

(3R,5R,6R)-6-((Z)-2-(2-aminothiazol-4-yl)-2-(((2-carboxypropan-2-yl)oxy)imino)acetamido)-3-(3-(((1E,2E)-2-(2-((2-(2-chloro-3,4-dihydroxybenzamido)ethyl)carbamoyl)hydrazono)ethylidene)amino)-2-oxoimidazolidin-1-yl)-7-oxo-4-thia-1-azabicyclo[3.2.0]heptane-3-carboxylic acid (compound of Example 53)

(3R,5R,6R)-6-((Z)-2-(2-aminothiazol-4-yl)-2-(((2-carboxypropan-2-yl)oxy)imino)acetamido)-3-(3-(2-((2-(2-chloro-3,4-dihydroxybenzamido)ethyl)amino)-2-oxoacetamido)-2-oxoimidazolidin-1-yl)-7-oxo-4-thia-1-azabicyclo[3.2.0]heptane-3-carboxylic acid (compound of Example 68)

(3R,5R,6R)-6-((Z)-2-(2-aminothiazol-4-yl)-2-(((2-carboxypropan-2-yl)oxy)imino)acetamido)-3-(5-((2-chloro-3,4-dihydroxybenzamido)methyl)-2-oxooxazolidin-3-yl)-7-oxo-4-thia-1-azabicyclo[3.2.0]heptane-3-carboxylic acid (compound of Example 73)

(3R,5R,6R)-6-((Z)-2-(2-aminothiazol-4-yl)-2-(((2-carboxypropan-2-yl)oxy)imino)acetamido)-3-(4-((2-chloro-3,4-dihydroxybenzamido)methyl)-1H-1,2,3-triazol-1-yl)-7-oxo-4-thia-1-azabicyclo[3.2.0]heptane-3-carboxylic acid (compound of Example 74)

(3R,5R,6R)-6-((Z)-2-(2-amino-5-chlorothiazol-4-yl)-2-((1-carboxycyclopropoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 76)

(3R,5R,6R)-6-((Z)-2-(2-amino-5-chlorothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 78)

(3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetamido)-2,5-dioxopiperazin-1-yl)-7-oxo-4-thia-1-azabicyclo[3.2.0]heptane-3-carboxylic acid (compound of Example 82)

(3R,5R,6R)-6-((Z)-2-(2-aminothiazol-4-yl)-2-(((2-carboxypropan-2-yl)oxy)imino)acetamido)-3-(3-(2,5-dichloro-3,4-dihydroxybenzamido)-2-oxoimidazolidin-1-yl)-7-oxo-4-thia-1-azabicyclo[3.2.0]heptane-3-carboxylic acid (compound of Example 83)

(3R,5R,6R)-6-((Z)-2-(2-aminothiazol-4-yl)-2-(((2-carboxypropan-2-yl)oxy)imino)acetamido)-3-((S)-3-(2-(2-chloro-3,4-dihydroxyphenyl)-2-oxoacetamido)-2-oxopyrrolidin-1-yl)-7-oxo-4-thia-1-azabicyclo[3.2.0]heptane-3-carboxylic acid (compound of Example 84)

(3R,5R,6R)-6-((Z)-2-(2-aminothiazol-4-yl)-2-(((2-carboxypropan-2-yl)oxy)imino)acetamido)-3-((R)-3-(2-(2-chloro-3,4-dihydroxyphenyl)-2-oxoacetamido)-2-oxopyrrolidin-1-yl)-7-oxo-4-thia-1-azabicyclo[3.2.0]heptane-3-carboxylic acid (compound of Example 85)

(3R,5R,6R)-6-((Z)-2-(2-aminothiazol-4-yl)-2-(((2-carboxypropan-2-yl)oxy)imino)acetamido)-3-(3(-2-(2-chloro-3,4-dihydroxyphenyl)-2-oxoacetamido)-2-oxotetrahydropyrimidin-1(2H)-yl)-7-oxo-4-thia-1-azabicyclo[3.2.0]heptane-3-carboxylic acid (compound of Example 86)

(3R,5R,6R)-6-((Z)-2-(2-aminothiazol-4-yl)-2-(((2-carboxypropan-2-yl)oxy)imino)acetamido)-3-(3-(2-(2-(2-(2-chloro-3,4-dihydroxyphenyl)-2-oxoacetyl)hydradienyl)-2-oxoacetamido)-2-oxoimidazolidin-1-yl)-7-oxo-4-thia-1-azabicyclo[3.2.0]heptane-3-carboxylic acid (compound of Example 88)

(3R,5R,6R)-6-((Z)-2-(2-aminothiazol-4-yl)-2-(((2-carboxypropan-2-yl)oxy)imino)acetamido)-3-(3-(2-((2-(2-chloro-3,4-dihydroxybenzamido)ethyl)(hydroxy)amino)-2-oxoacetamido)-2-oxoimidazolidin-1-yl)-7-oxo-4-thia-1-azabicyclo[3.2.0]heptane-3-carboxylic acid (compound of Example 104)

(3R,5R,6R)-6-((Z)-2-(2-aminothiazol-4-yl)-2-(((S)-1-carboxypropoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid

(compound of Example 105)
(3R,5R,6R)-6-((Z)-2-(2-amino-5-chlorothiazol-4-yl)-2-((1-carboxycyclopropoxy)imi no)acetamido)-3-(3-(3-(2-(2-chloro-3,4-dihydroxyphenyl)-2-oxoacetamido)propanamido)-2-o xoimidazolidin-1-yl)-7-oxo-4-thia-1-azabicyclo[3.2.0]heptane-3-carboxylic acid (compound of Example 107)
(3R,5R,6R)-6-((Z)-2-(2-aminothiazol-4-yl)-2-(((S)-1-carboxy-2-hydroxyethoxy)imin o)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 113)
(3R,5R,6R)-6-((Z)-2-(2-aminothiazol-4-yl)-2-(((S)-1-carboxy-2-methylpropoxy)imin o)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (compound of Example 114)
(3R,5R,6R)-6-((Z)-2-(2-aminothiazol-4-yl)-2-(((S)-1-carboxybutoxy)imino)acetamid o)-3-(3-(2-(2-chloro-3,4-dihydroxyphenyl)-2-oxoacetamido)-2-oxoimidazolidin-1-yl)-7-oxo-4 -thia-1-azabicyclo[3.2.0]heptane-3-carboxylic acid (compound of Example 115)
(3R,5R,6R)-6-((Z)-2-(2-aminothiazol-4-yl)-2-(((2-carboxypropan-2-yl)oxy)imino)acetamido)-3-(3-((Z)-2-(2-chloro-3,4-dihydroxyphenyl)-2-(hydroxyimino)acetamido)-2-oxoimidazolidin-1-yl)-7-oxo-4-thia-1-azabicyclo[3.2.0]heptane-3-carboxylic acid (compound of Example 117)
(3R,5R,6R)-6-((Z)-2-(2-aminothiazol-4-yl)-2-(((2-carboxypropan-2-yl)oxy)imino)ace tamido)-3-(3-(2-((2-(2-(2-chloro-3,4-dihydroxyphenyl)-2-oxoacetamido)ethyl)amino)-2-oxoac etamido)-2-oxoimidazolidin-1-yl)-7-oxo-4-thia-1-azabicyclo[3.2.0]heptane-3-carboxylic acid (compound of Example 121)
(3R,5R,6R)-6-((Z)-2-(2-aminothiazol-4-yl)-2-(((2-carboxypropan-2-yl)oxy)imino)ace tamido)-3-(3-(3-((Z)-2-(2-chloro-3,4-dihydroxyphenyl)-2-(hydroxyimino)acetamido)propana mido)-2-oxoimidazolidin-1-yl)-7-oxo-4-thia-1-azabicyclo[3.2.0]heptane-3-carboxylic acid (compound of Example 122)
(3R,5R,6R)-6-((Z)-2-(2-aminothiazol-4-yl)-2-(((2-carboxypropan-2-yl)oxy)imino)ace tamido)-3-(4-(2-(2-chloro-3,4-dihydroxyphenyl)-2-oxoacetamido)-2,3-dioxopiperazin-1-yl)-7-oxo-4-thia-1-azabicyclo[3.2.0]heptane-3-carboxylic acid (compound of Example 126)
(3R,5R,6R)-6-((Z)-2-(2-aminothiazol-4-yl)-2-(((2-carboxypropan-2-yl)oxy)imino)ace tamido)-3-(4-(2-chloro-3,4-dihydroxybenzamido)-2,3-dioxopiperazin-1-yl)-7-oxo-4-thia-1-aza bicyclo[3.2.0]heptane-3-carboxylic acid (compound of Example 132)
(3R,5R,6R)-6-((Z)-2-(2-amino-5-chlorothiazol-4-yl)-2-(((2-carboxypropan-2-yl)oxy)i mino)acetamido)-3-(3-(2-(2-(2-(2-chloro-3,4-dihydroxyphenyl)-2-oxoacetyl)hydradienyl)-2-o xoacetamido)-2-oxoimidazolidin-1-yl)-7-oxo-4-thia-1-azabicyclo[3.2.0]heptane-3-carboxylic acid (compound of Example 136)
(3R,5R,6R)-6-((Z)-2-(2-aminothiazol-4-yl)-2-(((2-carboxypropan-2-yl)oxy)imino)ace tamido)-3-(3-((R)-2-(2-(2-chloro-3,4-dihydroxyphenyl)-2-oxoacetamido)-3-methoxypropanam ido)-2-oxoimidazolidin-1-yl)-7-oxo-4-thia-1-azabicyclo[3.2.0]heptane-3-carboxylic acid (compound of Example 139)
(3R,5R,6R)-6-((Z)-2-(2-aminothiazol-4-yl)-2-(((2-carboxypropan-2-yl)oxy)imino)ace tamido)-3-(5-((2-chloro-3,4-dihydroxybenzamido)methyl)-2H-tetrazol-2-yl)-7-oxo-4-thia-1-az abicyclo[3.2.0]heptane-3-carboxylic acid (compound of Example 141)

Examples of the salt of the compound represented by General Formula [1], the β-lactamase inhibitory compound, and the antibacterial compound include salts in a basic group such as a generally known amino group or in an acidic group such as a hydroxyl or carboxyl group.
Examples of the salts in the basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.
Examples of the salts in the acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine, and the like.
Among the above salts, for example, pharmacologically acceptable salts are preferable.
In a case where there are isomers (such as optical isomers, geometric isomers, and tautomers) of the compound represented by General Formula [1], the β-lactamase inhibitory compound, and the antibacterial compound or salts thereof, the compound represented by General Formula [1], the β-lactamase inhibitory compound, and the antibacterial compound or salts thereof include such isomers, solvates, hydrates, and various types of crystals.
One or two or more pharmaceutically acceptable carriers, excipients, or diluents can be additionally incorporated into the pharmaceutical composition and the kit according to the embodiment of the present invention.

The carriers, excipients, and diluents include, for example, water, lactose, dextrose, fructose, sucrose, sorbitol, mannitol, polyethylene glycol, propylene glycol, starch, gum, gelatin, alginate, calcium silicate, calcium phosphate, cellulose, aqueous syrup, methylcellulose, polyvinylpyrrolidone, alkyl parahydroxybenzosorbate, talc, magnesium stearate, stearic acid, glycerin, various oils such as sesame oil, olive oil, and soybean oil, and the like.

Furthermore, if necessary, by being mixed with the aforementioned carriers, excipients, and diluents as well as additives such as a bulking agent, a binder, a disintegrant, a pH adjuster, and a solubilizing agent that are generally used, the compound or a salt thereof can be made into oral or parenteral medicines such as tablets, pills, capsules, granules, powders, solutions, emulsions, suspensions, ointments, injections, or skin patches through commonly used formulation techniques.

Examples of the infections caused by Gram-negative bacteria include infections caused by Gram-negative bacteria selected from the group consisting of genus Pseudomonas, genus Stenotrophomonas, genus Burkholderia, genus Acinetobacter, genus Alcaligenes, genus Bordetella, genus Brucella, genus Bacteroides, genus Fusobacterium, genus Neisseria, genus Moraxella, genus Campylobacter, genus Helicobacter, genus Vibrio, genus Aeromonas, genus Haemophilus, genus Chryseobacterium, genus Elizabethkingia, genus Flavobacterium, and genus Enterobacteriaceae.

A single preparation of the pharmaceutical composition according to the embodiment of the present invention contains the compound represented by General Formula [1] or a salt thereof and one or more compounds selected from a β-lactamase inhibitory compound and an antibacterial compound or salts thereof.

The route of administration of the pharmaceutical composition is not particularly limited. The pharmaceutical composition can be given by intravenous administration, oral administration, intramuscular administration, subcutaneous administration, inhalation, or spraying or through other routes of administration.

A single package of the kit according to the embodiment of the present invention contains the compound represented by General Formula [1] or a salt thereof and one or more compounds selected from a β-lactamase inhibitory compound and an antibacterial compounds or salts thereof. In addition, the kit may include, for example, instruments for administration, directions, instructions, attached documents and/or product labels.

The kit is particularly useful in a case where the compound represented by General Formula [1] or a salt thereof and one or more compounds selected from a β-lactamase inhibitory compound and an antibacterial compound or salts thereof have different routes of administration or in a case where it is preferable that the dose of each component be set by physicians.

The route of administration of the kit according to the embodiment of the present invention is not particularly limited. The kit can be given by intravenous administration, oral administration, intramuscular administration, subcutaneous administration, inhalation, or spraying or through other routes of administration. Furthermore, the compound represented by General Formula [1] or a salt thereof may be administered simultaneously with one or more compounds selected from a β-lactamase inhibitory compound and an antibacterial compound or salts thereof, administered separately, or administered in a specific order.

The pharmaceutical composition and the kit according to the embodiment of the present invention are useful for treating infections caused by Gram-negative bacteria, particularly, infections caused by drug-resistant Gram-negative bacteria.

The pharmaceutical composition and the kit according to the embodiment of the present invention are also useful for treating infections caused by Escherichia coli, Klebsiella pneumoniae, Pseudomonas aeruginosa, Acinetobacter baumannii and/or Stenotrophomonas maltophilia. Especially, the pharmaceutical composition and the kit according to the embodiment of the present invention are useful for treating infections caused by Escherichia coli, Klebsiella pneumoniae and/or Pseudomonas aeruginosa.

In addition, the pharmaceutical composition and the kit according to the embodiment of the present invention are useful for treating infections caused by drug-resistant bacteria producing Class A β-lactamases (such as TEM, SHV, CTX-M, KPC, and GES-type β-lactamases), Class B β-lactamases (such as IMP, VIM, and NDM-type β-lactamases), Class C β-lactamases (such as AmpC and CMY-type β-lactamases), and class D β-lactamases (OXA-type β-lactamase).

The pharmaceutical composition and the kit according to the embodiment of the present invention make it possible to treat more serious infections caused by Gram-negative bacteria. For example, by administering the pharmaceutical composition and the kit in combination with drugs at a dose that does not cause side effects, it is possible to treat more serious infections caused by Gram-negative bacteria.

Furthermore, even though the dose of each drug used is reduced, the pharmaceutical composition and the kit administered exhibit strong antibacterial activity. Therefore, it is possible to reduce the side effects of each drug.

The treatment with the pharmaceutical composition and the kit according to the embodiment of the present invention includes remedy and prevention, and is preferably remedy.

The administration method, dosage, and number of doses of the pharmaceutical composition and the kit according to the embodiment of the present invention can be appropriately selected according to the age, body weight, and symptom of the patient. Usually, to adults, the pharmaceutical composition and the kit may be given by oral or parenteral administration (for example, injection, instillation, administration to a rectal site, and the like) at 0.01 to 300

mg/kg, which is a dose of the compound represented by General Formula [1] and one or more compounds selected from a β-lactamase inhibitory compound and an antibacterial compound or salts thereof, once a day or in divided portions a day.

The pharmaceutical composition and the kit according to the embodiment the present invention are preferably administered as an injection.

The pharmaceutical composition and the kit according to the embodiment of the present invention are preferably manufactured as a solution, a frozen solution, or a lyophilized preparation, and are more preferably a lyophilized preparation.

Examples of the β-lactamase inhibitory compound used in the present invention include a diazabicyclo[3.2.1]octane-type inhibitory compound, a boronic acid-type inhibitory compound, a clavam-type inhibitory compound, and the like. Examples thereof include avibactam, nacubactam, relebactam, zidebactam, vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid (development code: VNRX-5133), sulbactam, tazobactam, clavulanic acid, (1aR,7bS)-5-fluoro-2-hydroxy-1,1a,2,7b-tetrahydrocyclopropan[c][1,2]benzoxaborinine-4-car boxylic acid (development code: QPX7728), and (2S,3S,5R)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide (development code: AAI-101).

Examples of the diazabicyclo[3.2.1]octane-type inhibitory compound include avibactam, nacubactam, relebactam, zidebactam, and the like. Among these, avibactam, nacubactam, and zidebactam are preferable, and avibactam is more preferable.

Examples of the boronic acid-type inhibitory compound include vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid (development code: VNRX-5133), and the like. Among these, vaborbactam is preferable.

Examples of the clavam-type inhibitory compound include sulbactam, tazobactam, clavulanic acid, (laR,7bS)-5-fluoro-2-hydroxy-1,1a,2,7b-tetrahydrocyclopropan[c][1,2]benzoxaborinine-4-car boxylic acid (development code: QPX7728), (2S,3S,5R)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide (development code: AAI-101), and the like. Among these, sulbactam, tazobactam, and clavulanic acid are preferable, and tazobactam is more preferable.

Examples of the antibacterial compound described in the present invention include a β-lactam-based compound, a macrolide-based compound, a lincomycin-based compound, a streptogramin-based compound, a quinolone-based compound, an aminoglycoside-based compound, a rifamycin-based compound, a tetracycline-based compound, an amphenicol-based compound, a sulfonamide-based compound, a trimethoprim-based compound, an oxazolidinone-based compound, a polymyxin-based compound, a pleuromutilin-based compound, a glycopeptide-based compound, an imidazole-based compound, a nitrofuran-based compound, and the like. Examples thereof include piperacillin, ceftazidime, imipenem, mecillinam, levofloxacin, amikacin, polymyxin B, tetracycline, trimethoprim, and rifampicin.

Examples of the β-lactam-based compound include penicillin-based compounds such as benzylpenicillin, phenoxymethylpenicillin, cloxacillin, oxacillin, nafcillin, methicillin, amoxicillin, ampicillin, ticarcillin, carbenicillin, piperacillin, azlocillin, mezlocillin, and mecillinam; cephalosporin-based compounds such as cefazolin, cefalexin, cefalotin, cefaclor, cefuroxime, cefotiam, loracarbef, cefepime, cefozopran, cefpirome, ceftazidime, ceftaroline, ceftolozane, and ceftobiprole; cephamycin-based compounds such as cefotetan, cefoxitin, and cefmetazole; penem-based compounds such as faropenem and ritipenem; carbapenem-based compounds such as ertapenem, doripenem, imipenem, meropenem, biapenem, and panipenem; monobactam-based compounds such as aztreonam, tigemonam, and carumonam, and the like.

As the penicillin-based compound, ampicillin, piperacillin, and mecillinam are preferable, and piperacillin is more preferable.

As the cephalosporin-based compound, cefotiam, cefmetazole, loracarbef, cefepime, cefozopran, cefpirome, ceftazidime, ceftaroline, ceftolozane, and ceftobiprole are preferable, and ceftazidime is more preferable.

As the cephamycin-based compound, cefmetazole is preferable.

As the penem-based compound, faropenem is preferable.

As the carbapenem-based compound, ertapenem, doripenem, imipenem, meropenem, and biapenem are preferable, and imipenem is more preferable.

As the monobactam-based compound, aztreonam, tigemonam, and carumonam are preferable, and aztreonam is more preferable.

Examples of the macrolide-based compound include erythromycin, spiramycin, josamycin, clarithromycin, azithromycin, rokitamycin, telithromycin, solithromycin, and the like. Among these, erythromycin, clarithromycin, azithromycin, and solithromycin are preferable.

Examples of the lincomycin-based compound include clindamycin, lincosamide, and the like.

Examples of the streptogramin-based compound include quinupristin/dalfopristin and the like.

Examples of quinolone-based compound include ofloxacin, ciprofloxacin, enoxacin, norfloxacin, sparfloxacin, grepafloxacin, levofloxacin, moxifloxacin, gemifloxacin, gatifloxacin, prulifloxacin, pazufloxacin, garenoxacin, sitafloxacin, tosufloxacin, delafloxacin, nalidixic acid, and the like. Among these, ofloxacin, ciprofloxacin, norfloxacin, levofloxacin, moxifloxacin, gatifloxacin, pazufloxacin, garenoxacin, sitafloxacin, and tosufloxacin are preferable, levofloxacin, garenoxacin, tosufloxacin, and pazufloxacin are more preferable, and levofloxacin is even more preferable.

Examples of the aminoglycoside-based compound include streptomycin, tobramycin, gentamicin, kanamycin, neomycin, amikacin, dibekacin, arbekacin, apramycin, and the like. Among these, tobramycin, gentamicin, and amikacin are preferable, and amikacin is more preferable.

Examples of the rifamycin-based compound include rifaximin, rifabutin, rifampicin, and the like. Among these, rifampicin is preferable.

Examples of the tetracycline-based compound include doxycycline, tetracycline, minocycline, tigecycline, eravacycline, and the like. Among these, tetracycline and minocycline are preferable, and tetracycline is more preferable.

Examples of the amphenicol-based compound include chloramphenicol and the like.

Examples of the sulfonamide- and trimethoprim-based compounds include trimethoprim, iclaprim, sulfamethoxazole, and the like. Among these, trimethoprim is preferable.

Examples of the oxazolidinone-based compound include linezolid, tedizolid, and the like.

Examples of the polymyxin-based compound include colistin, polymyxin B, and the like. Among these, polymyxin B is preferable.

Examples of the pleuromutilin-based compound include tiamulin and the like.

Examples of the glycopeptide-based compound include vancomycin, teicoplanin, telavancin, dalbavancin, oritavancin, and the like. Among these, vancomycin is preferable.

Examples of the imidazole-based compound include metronidazole and the like.

Examples of the nitrofuran-based compound include nitrofurantoin.

Next, a manufacturing method of the compound represented by General Formula [1] according to the embodiment of the present invention will be described.

The compound represented by General Formula [1] according to the embodiment of the present invention is manufactured by combining known methods. For example, the compound can be manufactured according to a manufacturing method described below.

[Manufacturing method 1]

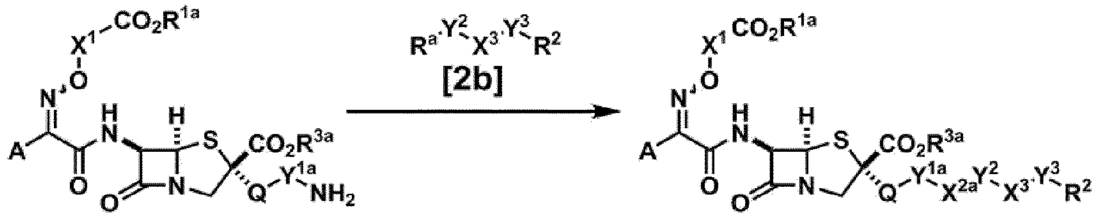

[2a] [2b] [1a]

"In the formula, $R^a$ represents a halogen atom; $Y^{1a}$ represents a bond; $X^{2a}$ is a group represented by Formula -NH-; $R^{1a}$ and $R^{3a}$ each represent a carboxyl protecting group; and $R^2$, Q, $Y^2$, $Y^3$, $X^1$, $X^3$, and A have the same definitions as $R^2$, Q, $Y^2$, $Y^3$, $X^1$, $X^3$, and A described above."

The compound represented by General Formula [1a] can be manufactured by reacting the compound represented by General Formula [2a] with the compound represented by General Formula [2b] in the presence of a base.

Examples of the compound represented by General Formula [2b] include acid halides such as 2-chloro-3,4-bis((4-methoxybenzyl)oxy)benzoyl chloride and 2-(2-chloro-3,4-bis((4-methoxybenzyl)oxy)phenyl)-2-oxoacetyl chloride described in the present specification.

The amount of the compound represented by General Formula [2b] used is not particularly limited, but may be 0.9 to 10 times and preferably 0.9 to 2.0 times the molar amount of the compound represented by General Formula [2a].

The amount of the solvent used is not particularly limited, but may be 1 to 500 times (v/w) the amount of the compound represented by General Formula [2a].

The amount of the base used may be 1 to 50 times and preferably 1 to 10 times the molar amount of the compound represented by General Formula [2a].

This reaction may be carried out at -30°C to 150°C for 30 minutes to 72 hours, and preferably carried out at 0°C to 40°C for 1 to 4 hours.

The solvent used in this reaction is not particularly limited as long as it does not affect the reaction. Examples thereof include halogenated hydrocarbons, ethers, esters, amides, nitriles, sulfoxides, aromatic hydrocarbons, and water.

These solvents may be used by being mixed together. As the solvent, for example, tetrahydrofuran, acetonitrile, and water are preferable. The solvent is more preferably a mixed solvent of tetrahydrofuran and water.

Examples of the base used in this reaction include an inorganic base and an organic base. As the base, for example, an inorganic base is preferable. The base is preferably sodium hydrogen carbonate.

[Manufacturing method 2]

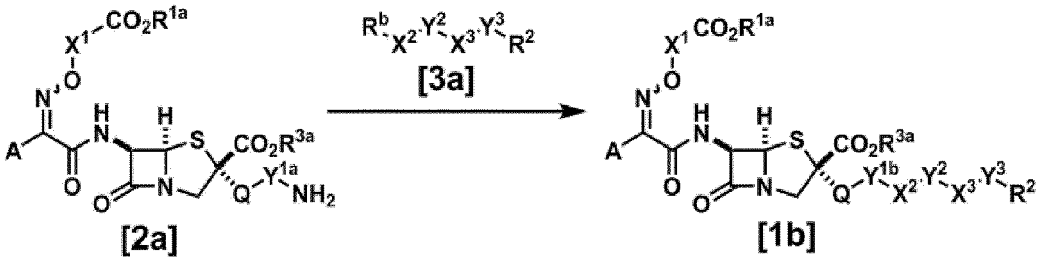


[2a] [1b]

"In the formula, $R^b$ represents a hydroxyl group or a carboxyl group; $Y^{1b}$ is a group represented by -NHC(=O)-; and $R^{1a}$, $R^{3a}$, $R^2$, Q, $Y^{1a}$, $Y^2$, $Y^3$, $X^1$, $X^2$, $X^3$, and A have the same definitions as $R^{1a}$, $R^{3a}$, $R^2$, Q, $Y^{1a}$, $Y^2$, $Y^3$, $X^1$, $X^2$, $X^3$, and A described above."

The compound represented by General Formula [1b] can be manufactured by reacting the compound represented by General Formula [2a] with the compound represented by General Formula [3a] in the presence of a condensing agent or an acid halide or in the presence of a base.

The amount of the compound represented by General Formula [3a] used is not particularly limited, but may be 0.9 to 10 times and preferably 0.9 to 2.0 times the molar amount of the compound represented by General Formula [2a].

The amount of the solvent used is not particularly limited, but may be 1 to 500 times (v/w) the amount of the compound represented by General Formula [2a].

The amount of the base used may be 1 to 50 times and preferably 1 to 10 times the molar amount of the compound represented by General Formula [2a].

This reaction may be carried out at -30°C to 150°C for 30 minutes to 72 hours, and preferably carried out at 0°C to 40°C for 1 to 24 hours.

The solvent used in this reaction is not particularly limited as long as it does not affect the reaction. Examples thereof include halogenated hydrocarbons, ethers, esters, amides, nitriles, sulfoxides, aromatic hydrocarbons, and water. These solvents may be used by being mixed together.

In a case where a condensing agent is used, as the solvent, dimethylacetamide and DMF are preferable.

In a case where an acid halide is used, as the solvent, for example, tetrahydrofuran, acetonitrile, and water are preferable. As the solvent, a mixed solvent of tetrahydrofuran and water is more preferable.

Examples of the base used in this reaction include an inorganic base and an organic base.

In a case where a condensing agent is used, as the base, for example, an organic base is preferable. The condensing agent is more preferably N-methylmorpholine.

In a case where an acid halide is used, as the base, for example, an inorganic base is preferable. As the base, sodium hydrogen carbonate is preferable.

In a case where the condensing agent is used, examples of the condensing agent include carbodiimides such as N,N'-diisopropylcarbodiimide (DIC), N,N'-di-(tert-butyl)carbodiimide, N,N'-dicyclohexylcarbodiimide (DCC), N-(tert-butyl)-N'-ethylcarbodiimide (BEC), N-cyclohexyl-N'-(2-morpholinoethyl)carbodiimide (CMC), and 1-ethyl-3-(3-dimethylaminopropyl)carbodiimide (EDC); imidazoliums such as 1,1'-carbonyldiimidazole (CDI) and 1,1'-carbonyl di(1,2,4-triazole) (CDT); acid azides such as diphenylphosphoryl azide; acid cyanides such as diethylphosphoryl cyanide; 2-ethoxy-1-ethoxycarbonyl-1,2-dihydroquinoline; uroniums such as O-(benzotriazol-1-yl)-N,N,N',N'-tetramethyluronium hexafluorophosphate (HBTU), O-(7-azabenzotriazol-1-yl)-N,N,N',N'-tetramethyluronium hexafluorophosphate (HATU), O-(benzotriazol-1-yl)-N,N,N',N'-bis(tetramethylene)uronium hexafluorophosphate (HBPyU), O-(benzotriazol-1-yl)-N,N,N',N'-bis(pentamethylene)uronium hexafluorophosphate (HBPipU), O-(6-chlorobenzotriazol-1-yl)-N,N,N',N'-tetramethyluronium hexafluorophosphate (HCTU), O-(3,4-dihydro-4-oxo-1,2,3-benzotriazin-3-yl)-N,N,N',N'-tetramethyluronium hexafluorophosphate (HDBTU), O-(2-oxo-1(2H)pyridyl)-N,N,N',N'-tetramethyluronium hexafluorophosphate (TPTU), O-((ethoxycarbonyl)cyanomethyleneamino)-N,N,N',N'-tetramethyluronium hexafluorophosphate (HOTU), O-((ethoxycarbonyl)cyanomethyleneamino)-N,N,N',N'-tetramethyluronium tetrafluoroborate (TOTU), N,N,N',N'-tetramethyl-O-(N-succinimidyl)uronium hexafluorophosphate (HSTU), N,N,N',N'-tetramethyl-O-(N-succinimidyl)uronium tetrafluoroborate

(TSTU), dipyrrolidino(N-succinimidyloxy)carbenium hexafluorophosphate (HSPyU), and S-(1-oxide-2-pyridyl)-N,N,N',N'-tetramethylthiouronium tetrafluoroborate (TOTT); and triazines such as 4-(4,6-dimethoxy-1,3,5-triazin-2-yl)-4-methylmorpholinium chloride (DMT-MM).

As a condensing agent, for example, carbodiimides, uroniums, and triazines are preferable. The condensing agent is more preferably EDC, HATU, or DMT-MM.

In a case where a condensing agent is used, the amount of the condensing agent used may be 1 to 50 times and preferably 1 to 5 times the molar amount of the compound represented by General Formula [2a].

In a case where carbodiimides are used as a condensing agent, it is preferable to further add additives thereto.

Examples of the additives include 1-hydroxybenzotriazole (HOBT), 1-hydroxy-7-azabenzotriazole (HOAT), and ethyl(hydroxyimino)cyanoacetate. Among these, HOBT and ethyl(hydroxyimino)cyanoacetate are preferable.

The amount of the additives used may be 0.01 to 10 times and preferably 0.1 to 1 time the molar amount of the compound represented by General Formula [2a].

In a case where an acid halide is used, examples of the acid halide include oxalyl chloride; carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; halides of sulfites such as thionyl chloride and thionyl bromide; and halides of phosphate such as phosphorus oxychloride, phosphorus oxybromide, phosphorus trichloride, and phosphorus pentachloride. Among these, oxalyl chloride is preferable.

The amount of the acid halide used may be 0.9 to 3 times and preferably 0.9 to 1.5 times the molar amount of the compound represented by General Formula [3a].

[Manufacturing method 3]

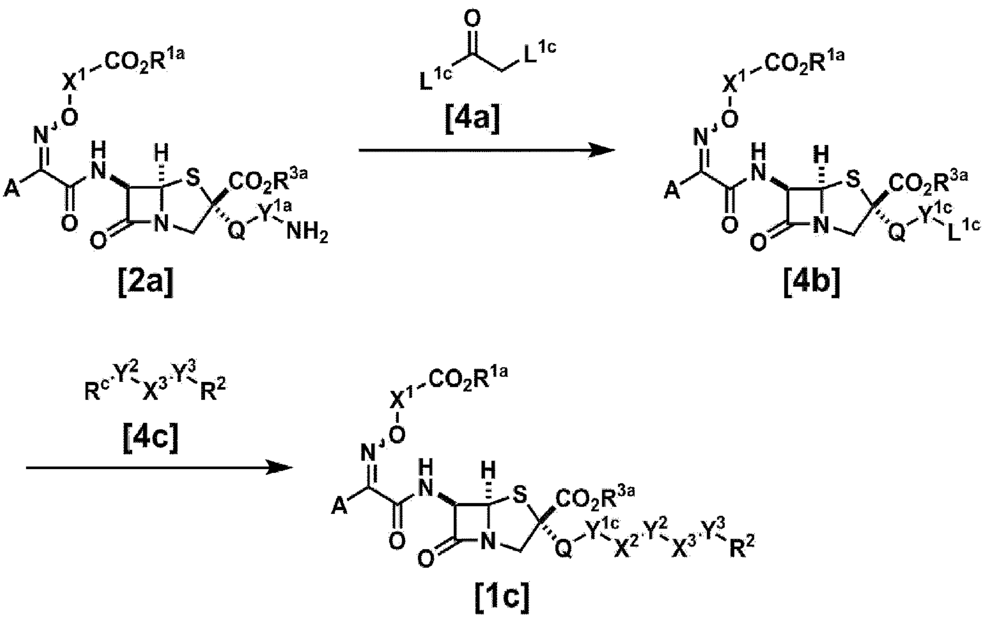

[2a] [4a] [4b] [4c] [1c]

"In the formula, $L^{1c}$ represents a leaving group; $Y^{1c}$ is a group represented by Formula $-NHC(=O)CH_2-$; $R^c$ represents a tertiary amino group or a heterocyclic group; $X^2$ is a group represented by General Formula $-N^+R^5R^6-$ (where $R^5$ and $R^6$ have the same definitions as $R^5$ and $R^6$ described above); and $R^{1a}$, $R^{3a}$, $R^2$, Q, $Y^{1a}$, $Y^2$, $Y^3$, $X^1$, $X^3$, and A have the same definitions as $R^{1a}$, $R^{3a}$, $R^2$, Q, $Y^{1a}$, $Y^2$, $Y^3$, $X^1$, $X^3$, and A described above."

The compound represented by General Formula [1c] can be manufactured by the following method.

(3-1) Condensation

As the compound represented by General Formula [4a], for example, chloroacetyl chloride and the like are known.

The compound represented by General Formula [4b] can be manufactured by reacting the compound represented by General Formula [2a] with the compound represented by General Formula [4a] in the presence of a base.

The amount of the compound represented by General Formula [4a] used is not particularly limited, but may be 0.9

to 20 times and preferably 0.9 to 10 times the molar amount of the compound represented by General Formula [2a].

The amount of the solvent used is not particularly limited, but may be 1 to 500 times (v/w) the amount of the compound represented by General Formula [2a].

The amount of the base used may be 1 to 50 times and preferably 1 to 20 times the molar amount of the compound represented by General Formula [2a].

This reaction may be carried out at -30°C to 150°C for 30 minutes to 48 hours, and preferably carried out at 0°C to 40°C for 1 to 5 hours.

The solvent used in this reaction is not particularly limited as long as it does not affect the reaction. Examples thereof include halogenated hydrocarbons, ethers, esters, amides, nitriles, sulfoxides, aromatic hydrocarbons, and water. These solvents may be used by being mixed together.

Examples of the base used in this reaction include an inorganic base and an organic base. As the base, for example, an organic base is preferable. The base is more preferably pyridine.

(3-2) Alkylation

Examples of the compound represented by General Formula [4c] include 2-chloro-3,4-bis((4-methoxybenzyl)oxy)-N-(2-(pyrrolidin-1-yl)ethyl)benzamide described in the present specification and the like.

The compound represented by General Formula [1c] can be manufactured by reacting the compound represented by General Formula [4b] with the compound represented by General Formula [4c].

The amount of the compound represented by General Formula [4c] used is not particularly limited, but may be 1 to 20 times and preferably 1 to 5 times the molar amount of the compound represented by General Formula [4b].

The amount of the solvent used is not particularly limited, but may be 1 to 500 times (v/w) the amount of the compound represented by General Formula [4b].

This reaction may be carried out at -30°C to 150°C for 30 minutes to 72 hours, and preferably carried out at 0°C to 50°C for 1 to 24 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

[Manufacturing method 4]

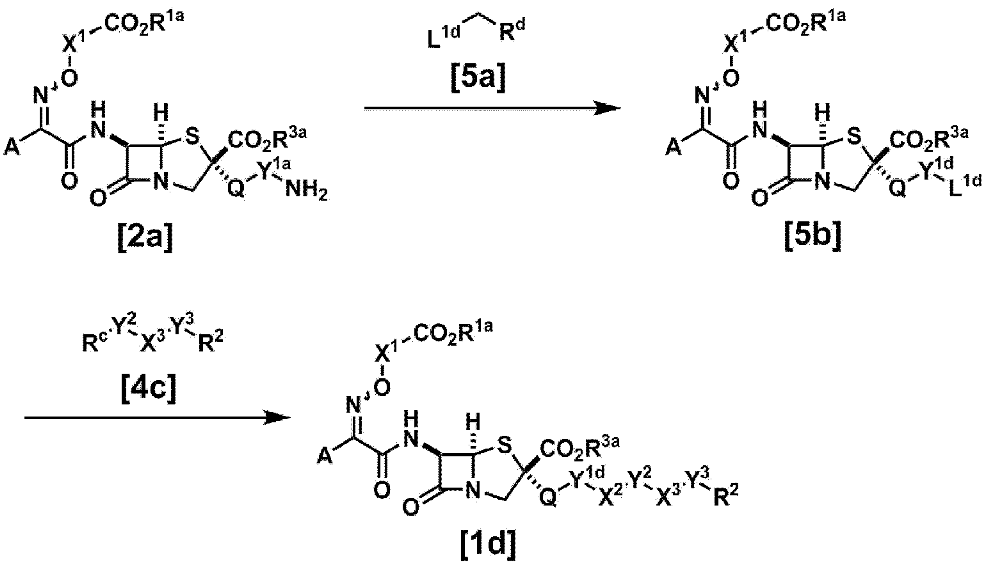

[2a]
[5a]
[5b]
[4c]
[1d]

"In the formula, $L^{1d}$ represents a leaving group; $R^d$ represents an aldehyde group which may be protected; $Y^{1d}$ is a group represented by Formula $-N=CH-CH_2-$; and $R^{1a}$, $R^{3a}$, $R^2$, $R^c$, Q, $Y^{1a}$, $Y^2$, $Y^3$, $X^1$, $X^2$, $X^3$, and A have the same definitions as $R^{1a}$, $R^{3a}$, $R^2$, $R^c$ , Q, $Y^{1a}$, $Y^2$, $Y^3$, $X^1$, $X^2$, $X^3$, and A described above."

The compound represented by General Formula [1d] can be manufactured by the following method.

(4-1) Alkylation

As the compound represented by General Formula [5a], for example, chloroacetaldehyde, acetal-protected chloroacetaldehyde, and the like are known.

The compound represented by General Formula [5b] can be manufactured by reacting the compound represented by General Formula [2a] with the compound represented by General Formula [5a].

The amount of the compound represented by General Formula [5a] used is not particularly limited, but may be 0.9 to 20 times and preferably 0.9 to 10 times the molar amount of the compound represented by General Formula [2a].

The amount of the solvent used is not particularly limited, but may be 1 to 500 times (v/w) the amount of the compound represented by General Formula [2a].

This reaction may be carried out at -30°C to 150°C for 30 minutes to 48 hours, and preferably carried out at 0°C to 50°C for 1 to 4 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

In a case where acetal-protected halogenated acetaldehyde is used as the compound represented by General Formula [5a], it is desirable to further add an acid catalyst thereto. Examples of the acid catalyst include p-toluenesulfonic acid monohydrate, pyridinium p-toluenesulfonate, 10-camphorsulfonic acid, and the like. Among these, p-toluenesulfonic acid monohydrate is preferable.

The amount of the acid catalyst used may be 0.01 to 10 times and preferably 0.1 to 1 time the molar amount of the compound represented by General Formula [5a].

(4-2) Alkylation

Examples of the compound represented by General Formula [4c] include 2-chloro-3,4-bis((4-methoxybenzyl)oxy)-N-(2-(pyrrolidin-1-yl)ethyl)benzamide described in the present specification and the like.

The compound represented by General Formula [1d] can be manufactured by reacting the compound represented by General Formula [5b] with the compound represented by General Formula [4c].

The amount of the compound represented by General Formula [4c] used is not particularly limited, but may be 1 to 20 times and preferably 1 to 5 times the molar amount of the compound represented by General Formula [5b].

The amount of the solvent used is not particularly limited, but may be 1 to 500 times (v/w) the amount of the compound represented by General Formula [5b].

This reaction may be carried out at -30°C to 150°C for 30 minutes to 72 hours, and preferably carried out at 0°C to 50°C for 1 to 4 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

[Manufacturing method 5]

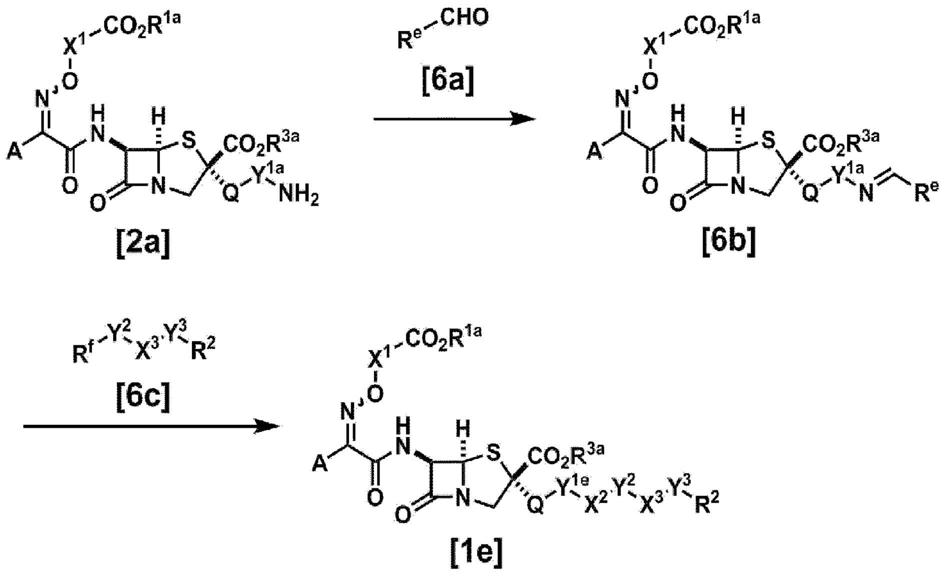


"In the formula, $R^e$ represents an aldehyde group and a carboxyl group; $R^f$ represents a substituted primary amino group; $Y^{1e}$ is a group represented by Formula -N=CH-CH=N-, a group represented by Formula -N=CH-CH=N-O-, or a group represented by Formula -N=CH-C(=O)-, $X^2$ is a group represented by Formula -NH-, a group represented by Formula -NHC(=O)NH-, or a bond; and $R^{1a}$, $R^{3a}$, $R^2$, Q, $Y^{1a}$, $Y^2$, $Y^3$, $X^1$, $X^3$, and A have the same definitions as $R^{1a}$, $R^{3a}$, $R^2$, Q, $Y^{1a}$, $Y^2$, $Y^3$, $X^1$, $X^3$, and A described above."

The compound represented by General Formula [1e] can be manufactured by the following method.

(5-1) Iminoization

As the compound represented by General Formula [6a], for example, glyoxal, glyoxylic acid, hydrates of these, and the like are known.

The compound represented by General Formula [6b] can be manufactured by reacting the compound represented by General Formula [2a] with the compound represented by General Formula [6a].

The amount of the compound represented by General Formula [6a] used is not particularly limited, but may be 1 to 50 times and preferably 1 to 20 times the molar amount of the compound represented by General Formula [2a].

The amount of the solvent used is not particularly limited, but may be 1 to 500 times (v/w) the amount of the compound represented by General Formula [2a].

This reaction may be carried out at -30°C to 150°C for 30 minutes to 48 hours, and preferably carried out at 0°C to 40°C for 1 to 12 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

(5-2) Iminoization

Examples of the compound represented by General Formula [6c] include N-(2-aminoethyl)-2-chloro-3,4-bis((4-methoxybenzyl)oxy)benzamide described in the present specification and the like.

The compound represented by General Formula [1e] can be manufactured by reacting the compound represented by General Formula [6b] with the compound represented by General Formula [6c].

The amount of the compound represented by General Formula [6c] used is not particularly limited, but may be 1 to 20 times and preferably 1 to 10 times the molar amount of the compound represented by General Formula [6b].

The amount of the solvent used is not particularly limited, but may be 1 to 500 times (v/w) the amount of the compound represented by General Formula [6b].

This reaction may be carried out at -30°C to 150°C for 30 minutes to 72 hours, and preferably carried out at 0°C to 40°C for 1 to 12 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

[Manufacturing method 6]

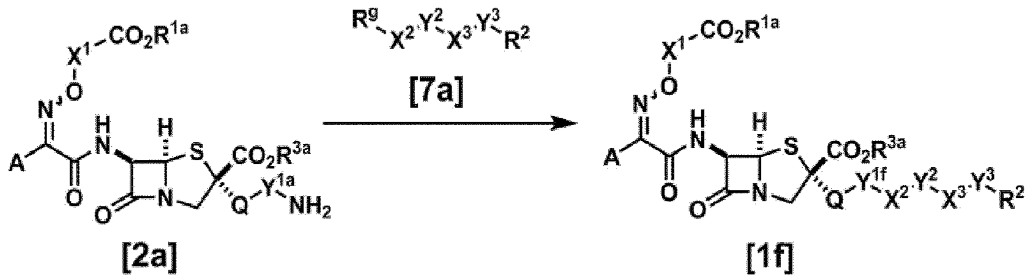

[7a] [2a] [1f]

"In the formula, $R^g$ represents an acetaldehyde group; $Y^{1f}$ is a group represented by Formula $-N=CH-CH_2-$; $X^2$ is a group represented by Formula -NH-; and $R^{1a}$, $R^{3a}$, $R^2$, Q, $Y^{1a}$, $Y^2$, $Y^3$, $X^1$, $X^3$, and A have the same definitions as $R^{1a}$, $R^{3a}$, $R^2$, Q, $Y^{1a}$, $Y^2$, $Y^3$, $X^1$, $X^3$, and A described above."

The compound represented by General Formula [1f] can be manufactured by the following method.

Examples of the compound represented by General Formula [7a] include 2-chloro-3,4-bis((4-methoxybenzyl)oxy)-N-(2-oxoethyl)benzamide described in the present specification and the like.

The compound represented by General Formula [1f] can be manufactured by reacting the compound represented by General Formula [2a] with the compound represented by General Formula [7a].

The amount of the compound represented by General Formula [7a] used is not particularly limited, but may be 1 to 50 times and preferably 1 to 10 times the molar amount of the compound represented by General Formula [2a].

The amount of the solvent used is not particularly limited, but may be 1 to 500 times (v/w) the amount of the compound represented by General Formula [2a].

This reaction may be carried out at -30°C to 150°C for 30 minutes to 72 hours, and preferably carried out at 0°C to 40°C for 1 to 12 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

[Manufacturing method 7]

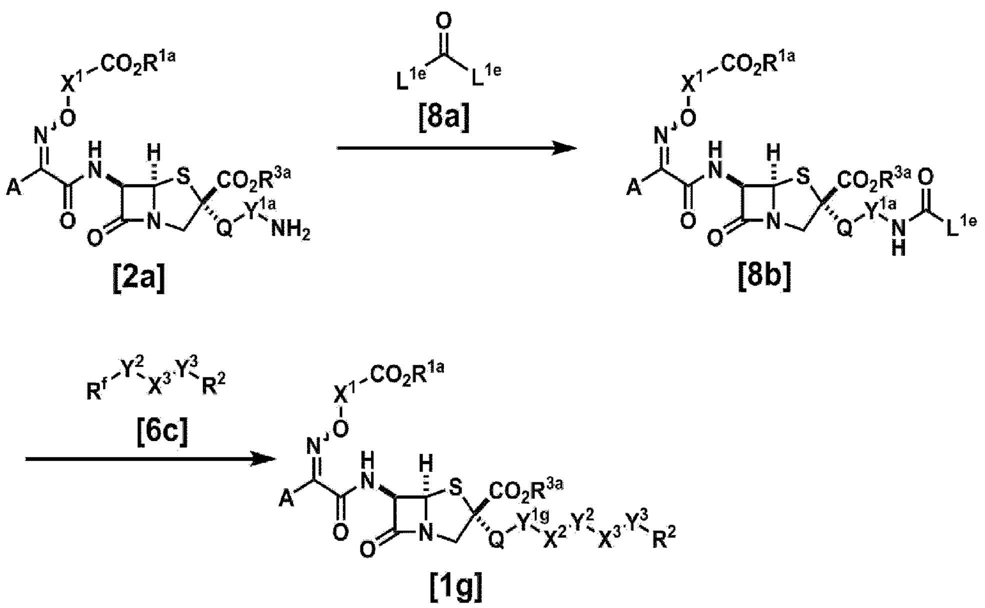

[8a] [2a] [8b] [6c] [1g]

"In the formula, $L^{1e}$ represents a leaving group; $R^f$ represents a primary amino group, a secondary amino group, and a secondary cyclic amino group; $Y^{1g}$ is a group represented by Formula-NHC(=O)-, a group represented by Formula

-NHC(=O)NH-, or a group represented by Formula -NHC(=O)NH-O-, $X^2$ is a group represented by Formula -NH-, a group represented by Formula -NHC(=O)NH-, a heterocyclic group, or a bond; and $R^{1a}$, $R^{3a}$, $R^2$, Q, $Y^{1a}$, $Y^2$, $Y^3$, $X^1$, $X^3$, and A have the same definitions as $R^{1a}$, $R^{3a}$, $R^2$, Q, $Y^{1'a}$, $Y^2$, $Y^3$, $X^1$, $X^3$, and A described above."

The compound represented by General Formula [1g] can be manufactured by the following method.

(7-1) Acyl imidazolation

Examples of the compound represented by General Formula [8a] include phosgene, triphosgene, carbonyldiimidazole, and the like.

The compound represented by General Formula [8b] can be manufactured by reacting the compound represented by General Formula [2a] with the compound represented by General Formula [8a].

The amount of the compound represented by General Formula [8a] used is not particularly limited, but may be 1 to 20 times and preferably 1 to 10 times the molar amount of the compound represented by General Formula [2a].

The amount of the solvent used is not particularly limited, but may be 1 to 500 times (v/w) the amount of the compound represented by General Formula [2a].

This reaction may be carried out at -30°C to 150°C for 30 minutes to 48 hours, and preferably carried out at 0°C to 80°C for 1 to 24 hours.

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

(7-2) Condensation

Examples of the compound represented by General Formula [6c] include N-(2-aminoethyl)-2-chloro-3,4-bis((4-methoxybenzyl)oxy)benzamide described in the present specification and the like.

The compound represented by General Formula [1g] can be manufactured by reacting the compound represented by General Formula [8b] with the compound represented by General Formula [6c] in the presence of a base.

The amount of the compound represented by General Formula [6c] used is not particularly limited, but may be 1 to 20 times and preferably 1 to 5 times the molar amount of the compound represented by General Formula [8b].

Examples of the base used in this reaction include an inorganic base and an organic base.

The amount of the base used may be 1 to 50 times and preferably 1 to 10 times the molar amount of the compound represented by General Formula [2a].

The amount of the solvent used is not particularly limited, but may be 1 to 500 times (v/w) the amount of the compound represented by General Formula [8b].

This reaction may be carried out at -30°C to 150°C for 30 minutes to 48 hours, and preferably carried out at 0°C to 40°C for 1 to 12 hours.

The solvent used in this reaction is not particularly limited as long as it does not affect the reaction. Examples thereof include halogenated hydrocarbons, ethers, esters, amides, nitriles, sulfoxides, aromatic hydrocarbons, and water. These solvents may be used by being mixed together.

[Manufacturing method 8] Deprotection

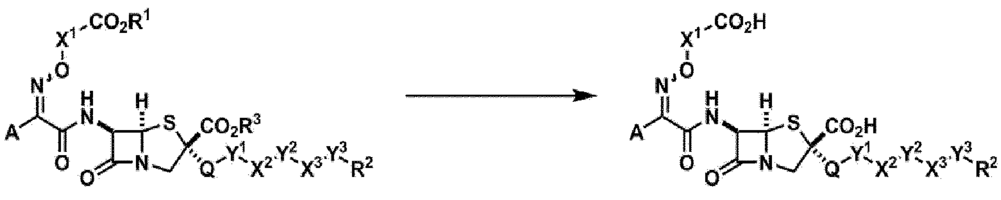

[1h] [1i]

"In the formula, $R^1$, $R^2$, $R^3$, Q, $Y^1$, $Y^2$, $Y^3$, $X^1$, $X^2$, $X^3$, and A have the same definition as $R^1$, $R^2$, $R^3$, Q, $Y^1$, $Y^2$, $Y^3$, $X^1$, $X^2$, $X^3$, and A described above. Here, in General Formula [1h], at least one of $R^1$ or $R^3$ is a protecting group."

The compound represented by General Formula [1i] can be manufactured by performing deprotection by the method described, for example, in "Protective Groups in Organic Synthesis, W. Greene et al., 4th edition, pp. 533-643, 2007, John Wiley & Sons, INC." and the like.

Next, a manufacturing method of raw materials for manufacturing the compound represented by General Formula [1] according to the embodiment of the present invention will be described.

[Manufacturing method A]

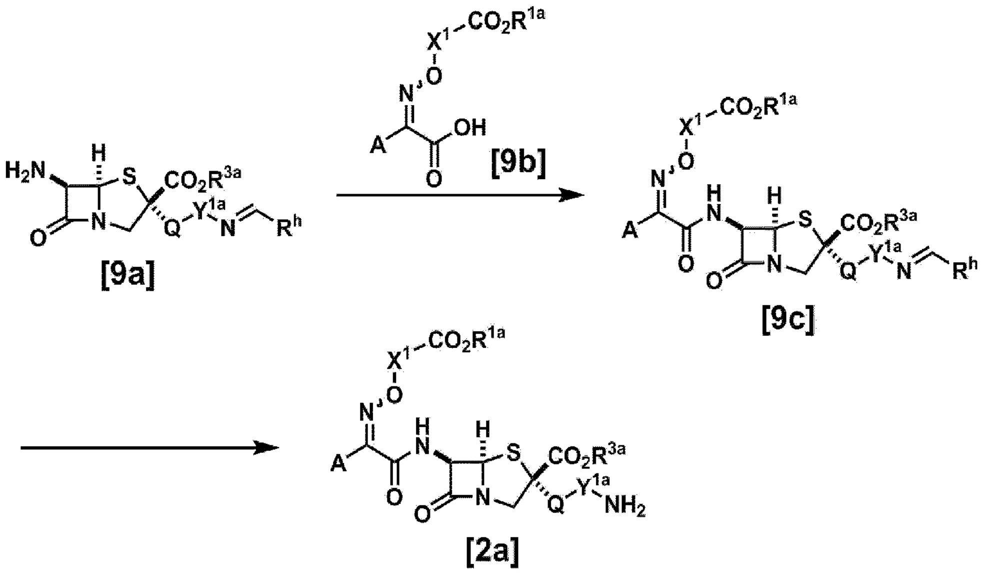

[9a] [9b] [9c] [2a]

"In the formula, $R^h$ represents an aryl group; and $R^{1a}$, $R^{3a}$, Q, $X^1$, $Y^{1a}$, and A have the same definitions as $R^{1a}$, $R^{3a}$, Q, $X^1$, $Y^{1a}$, and A described above."

The compound represented by General Formula [2a] can be manufactured by the following method.

(A-1) Condensation

The compound represented by General Formula [9c] can be manufactured by reacting the compound represented by General Formula [9a] or a hydrochloride thereof with the compound represented by General Formula [9b] in the presence of a condensing agent or an acid halide or in the presence of a base.

Examples of the compound represented by General Formula [9a] include benzhydryl (3R,5R,6R)-6-amino-3-(3-(((E)-benzylidene)amino)-2-oxoimidazolidin-1-yl)-7-oxo-4-thia-1-a zabicyclo[3.2.0]heptane-3-carboxylate hydrochloride described in the present specification.

The compound represented by General Formula [9a] can be manufactured based on the methods described, for example, on pp. 9-14 in JP1992-074182A (JP-H04-074182A), pp. 6-12 in JP1998-182654A (JP-H10-182654A), and pp. 8-20 in US5185330A, in addition to the method described in the present specification.

Examples of the compound represented by General Formula [9b] include (Z)-2-(2-aminothiazol-4-yl)-2-(((1-(tert-butoxy)-2-methyl-1-oxopropan-2-yl)oxy)imino) acetic acid described in the present specification and the like.

Furthermore, the compound represented by General Formula [9c] can also be manufactured by reacting the compound represented by General Formula [9a] with a benzothiazolyl ester as the compound represented by General Formula [9b].

The solvent used in this reaction is not particularly limited as long as it does not affect the reaction. Examples of the solvent include halogenated hydrocarbons, ethers, esters, amides, nitriles, sulfoxides, and aromatic hydrocarbons. These solvents may be used by being mixed together.

As the solvent, for example, halogenated hydrocarbons, ethers, esters, and amides are preferable. Among these, halogenated hydrocarbons and amides are more preferable.

The amount of the solvent used is not particularly limited, but may be 1 to 500 times (v/w) the amount of the compound represented by General Formula [9a].

Examples of the base used in this reaction include an inorganic base and an organic base.

As the base, for example, an organic base is preferable. As the organic base, triethylamine, N,N-diisopropylethylamine, and 4-methylmorpholine are more preferable, and N,N-diisopropylethylamine and 4-methylmorpholine are even more preferable.

The amount of the base used may be 1 to 50 times and preferably 1 to 10 times the molar amount of the compound

represented by General Formula [9a].

Examples of the condensing agent used in this reaction include the condensing agent described in Manufacturing Method 3.

As the condensing agent, for example, carbodiimides are preferable. As the condensing agent, EDC is more preferable.

The amount of the condensing agent used may be 1 to 50 times and preferably 1 to 5 times the molar amount of the compound represented by General Formula [9a].

In a case where carbodiimides are used as the condensing agent, it is preferable to add additives thereto.

Examples of the additives include 1-hydroxybenzotriazole (HOBT), 1-hydroxy-7-azabenzotriazole (HOAT), and ethyl(hydroxyimino)cyanoacetate. Among these, HOBT and ethyl(hydroxyimino)cyanoacetate are preferable.

The amount of the additives used may be 0.01 to 10 times and preferably 0.1 to 1 time the molar amount of the compound represented by General Formula [9a].

Examples of the acid halide used in this reaction include oxalyl chloride; carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; halides of sulfites such as thionyl chloride and thionyl bromide; and halides of phosphate such as phosphorus oxychloride, phosphorus oxybromide, phosphorus trichloride, and phosphorus pentachloride.

The amount of the acid halide used may be 0.9 to 3 times and preferably 0.9 to 1.5 times the molar amount of the compound represented by General Formula [9b].

The amount of the compound represented by General Formula [9b] used is not particularly limited, but may be 1 to 10 times and preferably 1 to 3 times the molar amount of the compound represented by General Formula [9a].

This reaction may be carried out at -30°C to 150°C for 30 minutes to 48 hours, and preferably carried out at 0°C to 50°C for 1 to 12 hours.

(A-2) Deprotection

The compound represented by General Formula [2a] can be manufactured by deprotecting the compound represented by General Formula [9c] by the method described, for example, in "Protective Groups in Organic Synthesis, W. Greene et al., 4th edition, pp. 533-643, 2007, John Wiley & Sons, INC." and the like.

## Examples

Next, the compound represented by General Formula [1] according to the embodiment of the present invention will be described based on examples and reference examples, but the present invention is not limited thereto.

Unless otherwise specified, silica gel column chromatography is flash column chromatography in which B. W. Silica gel, BW-300 manufactured by Fuji Silysia Chemical, Ltd. is used as a carrier.

In the medium-pressure reverse-phase silica gel column chromatography, Isolera SV or Isolera LSV manufactured by Biotage Japan Ltd. was used. Furthermore, as a carrier, SNAP Ultra C18 Cartridge manufactured by Biotage Japan Ltd. was used.

The mixing ratio in the eluent is a volume ratio.

The NMR spectrum was measured using AVANCE III HD400 (Bruker).

The NMR spectrum shows proton NMR, and the internal standard is as follows. The δ value is expressed as ppm.

Deuterated chloroform ($CDCl_3$): tetramethylsilane (0.00 ppm)
Deuterated methanol ($CD_3OD$): methanol ($CH_3OH$) (3.30 ppm)
Deuterated dimethyl sulfoxide ($CD_3SOCD_3$): tetramethylsilane (0.00 ppm)
Heavy water ($D_2O$): water (4.65 ppm)

In the NMR spectrum, for example, the description of [1.45] 1.46 (3H, s) means that the peak derived from each diastereomer in a diastereomer mixture, the peak derived from each isomer in a geometric isomer mixture, or the peak derived from the same protons observed separately in a pH-dependent manner is observed as a singlet at 1.45 and 1.46, and the total number of protons is 3.

Unless otherwise stated, the NMR spectra in reference examples were measured using $CDCl_3$, and the NMR spectra in examples were measured using $D_2O$.

The MS spectrum was measured by an electrospray ionization method (ESI) by using an ACQUITY SQD LC/MS System (Waters Corporation).

The abbreviation in each of the examples and reference examples has the following meaning.
Alloc: allyloxycarbonyl, BH: diphenylmethyl, Boc: tert-butoxycarbonyl, Cbz: benzyloxycarbonyl, DBU: 1,8-diazabicyclo[5.4.0]-7-undecene, DMAC: N,N-dimethylacetamide, DMAP: 4-(dimethylamino)pyridine, DMF: N,N-dimethylformamide, EDC: 1-ethyl-3-(3-dimethylaminopropyl)carbodiimide hydrochloride, ESI: electrospray ionization method, Et: ethyl, HOBt: 1-hydroxybenzotriazole monohydrate, HATU: O-(7-azabenzotriazol-1-yl)-N,N,N',N'-tetramethyluronium hexafluorophosphate, IPE: diisopropyl ether, Me: methyl, Moz: 4-methoxybenzyloxycarbonyl, Ms: methanesulfonyl, MTBE : tert-butyl methyl ether, NMM: N-methylmorpholine, NMP: 1-methyl-2-pyrrolidone, PMB: 4-methoxybenzyl, PNZ: p-nitrobenzyloxycarbonyl, SEM: 2-(trimethylsilyl)ethoxymethyl, TBDPS: tert-butyldiphenylsilyl, TBS: tert-butyldimethylsilyl, t-Bu: tert-butyl, THF: tetrahydrofuran, THP: tetrahydro-2H-pyran-2-yl, Tr: triphenylmethyl, Ts: p-toluenesulfonyl, s: singlet, brs: broad singlet, d: doublet, dd: double doublet, dt: doublet triplet, m: multiplet, t: triplet

Reference Example 1

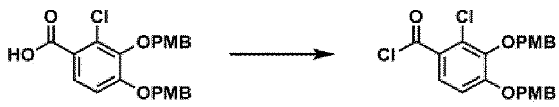

THF (400 mL) was added to 2-chloro-3,4-bis((4-methoxybenzyl)oxy)benzoic acid (40.0 g), and the mixture was stirred under ice cooling. At the same temperature, DMF (361 μL) was added to the reaction mixture, and then oxalyl dichloride (14.2 g) was added dropwise thereto. The reaction mixture was stirred at room temperature for 1 hour, and then oxalyl dichloride (14.2 g) was added dropwise thereto. The reaction mixture was stirred at room temperature overnight, and then IPE (400 mL) was added to the reaction mixture. Solids were collected by filtration and washed with IPE. The solids were dried, thereby obtaining 2-chloro-3,4-bis((4-methoxybenzyl)oxy)benzoyl chloride (30 g) as white solids.

NMR(DMSO-$d_6$): 3.74 (3H, s), 3.78 (3H, s), 4.88 (2H, s), 5.18 (2H, s), 6.82-6.89 (2H, m), 6.95-7.02 (2H, m), 7.23 (1H, d, J = 9.2 Hz), 7.26-7.33 (2H, m), 7.41-7.49 (2H, m), 7.62 (1H, d, J = 8.4 Hz)

Reference example 2

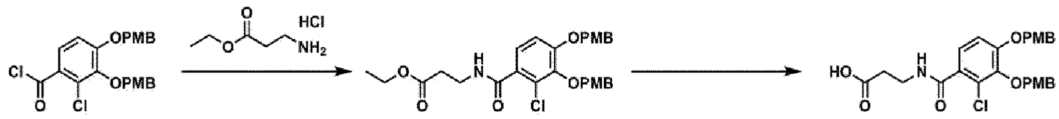

Reference example 2 (1)

THF (20 mL), water (20 mL), sodium hydrogen carbonate (281 mg), and 2-chloro-3,4-bis((4-methoxybenzyl)oxy) benzoyl chloride (500 mg) were sequentially added to ethyl 3-aminopropanoate hydrochloride (257mg). The reaction mixture was stirred at room temperature for 4 hours, ethyl acetate (50 mL) and water (50 mL) were then added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining a target substance (600 mg) as yellow solids.

Reference example 2 (2)

Methanol (5.8 mL), THF (5.8 mL), and a 2 mol/L aqueous sodium hydroxide solution (4.5 mL) were added to the compound (600 mg) obtained in Reference Example 2 (1), and the mixture was stirred at room temperature overnight. Ethyl acetate (20 mL) and water (20 mL) were added to the reaction mixture, 2 mol/L hydrochloric acid was added thereto such that the pH was adjusted to 1.8. The reaction mixture was stirred at room temperature for 30 minutes, and solids were collected by filtration. The solids were dried under reduced pressure, thereby obtaining 3-(2-chloro-3,4-bis((4-methoxybenzyl)oxy)benzamido)propionic acid (488 mg) as white solids.

NMR(DMSO-$d_6$): 2.48 (2H, t, J = 6.8 Hz), 3.39 (2H, q, J = 6.7 Hz), 3.75 (3H, s), 3.77 (3H, s), 4.87 (2H, s), 5.15 (2H, s), 6.87 (2H, dd, J = 6.8, 2.0 Hz), 6.97 (2H, dd, J = 6.6, 1.8 Hz), 7.11 (1H, d, J = 8.8 Hz), 7.18 (1H, d, J = 8.4 Hz), 7.31 (2H, dd, J = 6.8, 2.0 Hz), 7.43 (2H, d, J = 8.8 Hz), 8.33 (1H, t, J = 5.4 Hz), 12.22 (1H, s)

Reference Example 3

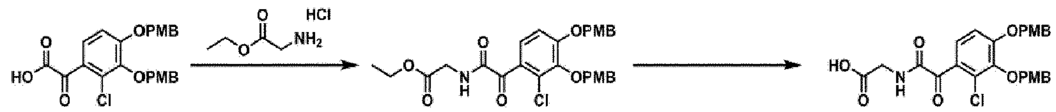


Reference example 3 (1)

2-(2-Chloro-3,4-bis((4-methoxybenzyl)oxy)phenyl)-2-oxoacetic acid (1.64 g), HOBt (532 mg), EDC (755 mg), DMF (10 mL), and NMM (0.47 mL) were sequentially added to ethyl glycinate hydrochloride (500 mg). The reaction mixture was stirred at room temperature overnight. Ethyl acetate (30 mL) and water (30 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 25:75 → 50:50], thereby obtaining a target substance (1.27 g) as light yellow solids.

Reference example 3 (2)

Methanol (13 mL), THF (13 mL), and a 2 mol/L aqueous sodium hydroxide solution (4.7 mL) were added to the compound (1.27 g) obtained in Reference Example 3 (1), and the mixture was stirred at room temperature for 2 hours and 30 minutes. Hydrochloric acid (2 mol/L) was added to the reaction mixture such that the pH was adjusted to 1.9. Ethyl acetate (25 mL) and water (25 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining (2-(2-chloro-3,4-bis((4-methoxybenzyl)oxy)phenyl)-2-oxoacetyl)glycine (1.19 g) as light yellow solids.
MS: 512.10 [M - H]$^-$

The compounds in Table 1 were obtained in the same manner as in Reference Example 3.

[Table 1]

| Ref. Ex. No. | Structural Formula | Name |
|---|---|---|
| 4 | 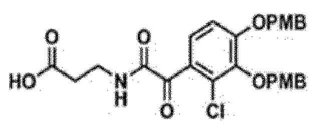 | 3-(2-(2-chloro-3,4-bis((4-methoxybenzyl)oxy)phenyl)-2-oxoacetamido)propionic acid |
| 5 | 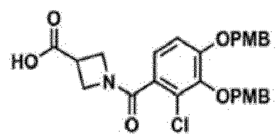 | 1-(2-chloro-3,4-bis((4-methoxybenzyl)oxy)benzoyl)azetidine-3-carboxylic acid |
| 6 | 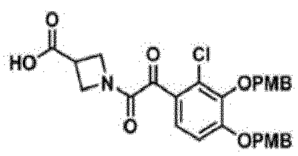 | 1-(2-(2-chloro-3,4-bis((4-methoxybenzyl)oxy)phenyl)-2-oxoacetyl) azetidine-3-carboxylic acid |

The measured values of NMR of the compounds in the table are as follows.

Reference example 4

NMR (DMSO-$d_6$): 2.49-2.53 (2H, m), 3.41 (2H, q, J = 6.5Hz), 3.74 (3H, s), 3.78 (3H, s), 4.90 (2H, s), 5.22 (2H, s), 6.86 (2H, dd, J = 6.8, 2.0 Hz), 6.99 (2H, dd, J = 6.8, 2.0 Hz), 7.29 (2H, d, J = 8.4 Hz), 7.31 (1H, d, J = 8.8 Hz), 7.46 (2H, d, J = 8.8 Hz), 7.52 (1H, d, J = 8.8 Hz), 8.93 (1H, t, J = 5.6 Hz), 12.31 (1H, s)

Reference Example 5

NMR (DMSO-$d_6$): 3.37-3.47 (1H, m), 3.73 (3H, s), 3.78 (3H, s), 3.81-4.06 (3H, m), 4.13-4.23 (1H, m), 4.92 (2H, s), 5.14 (2H, s), 6.80-6.88 (2H, m), 6.95-7.02 (2H, m), 7.11 (1H, d, J = 8.4 Hz), 7.21 (1H, d, J = 8.4 Hz), 7.23-7.30 (2H, m), 7.40-7.48 (2H, m), 12.75 (1H, s)

Reference Example 6

NMR (DMSO-$d_6$): 3.50-3.62 (1H, m), 3.74 (3H, s), 3.78 (3H, s), 4.05-4.13 (1H, m), 4.20-4.29 (2H, m), 4.34 (1H, m), 4.91 (2H, s), 5.23 (2H, s), 6.81-6.89 (2H, m), 6.96-7.03 (2H, m), 7.24-7.38 (3H, m), 7.42-7.50 (2H, m), 7.57 (1H, dd, J = 14.4, 8.8 Hz), 12.85 (1H, s)

Reference Example 7

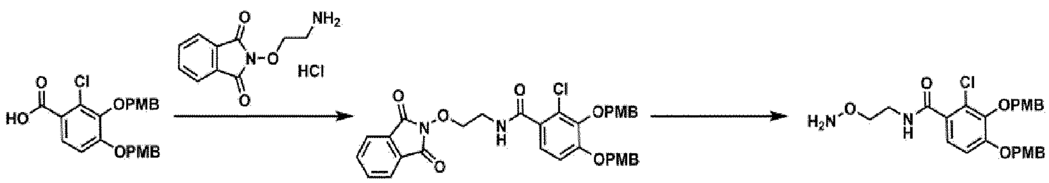

Reference Example 7 (1)

2-Chloro-3,4-bis((4-methoxybenzyl)oxy)benzoic acid (1.6 g), HOBt (555 mg), EDC (858 mg), DMAC (21 mL), and NMM (1.4 mL) were sequentially added to 2-(2-aminoethoxy)isoindoline-1,3-dioxohydrochloride (951 mg). The reaction mixture was stirred at room temperature for 3 hours and 30 minutes. Water (60 mL) was added to the reaction mixture, and solids were collected by filtration. The solids were dried, thereby obtaining a target substance (2.30 g) as light brown solids.

Reference Example 7 (2)

Dichloromethane (20 mL) and methylhydrazine (189 μL) were added to the compound (2.30 g) obtained in Reference Example 7 (1), and the mixture was stirred at room temperature for 2 hours. Then, solids were filtered, and the solvent was distilled off under reduced pressure, thereby obtaining N-(2-(aminooxy)ethyl)-2-chloro-3,4-bis((4-methoxybenzyl)oxy)benzamide (1.97 g) as brown solids.

NMR: 3.63-3.88 (10H, m), 4.94 (2H, s), 5.07 (2H, s), 6.72-6.97 (7H, m), 7.29-7.39 (4H, m), 7.44 (1H, d, J = 8.8 Hz)

Reference Example 8

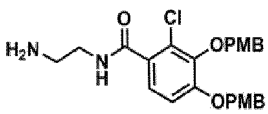

By using 2-(2-aminoethyl)isoindoline-1,3-dione hydrochloride instead of 2-(2-aminoethoxy)isoindoline-1,3-dione hydrochloride in Reference Example 7, N-(2-aminoethyl)-2-chloro-3,4-bis((4-methoxybenzyl)oxy)benzamide was obtained in the same manner as in Reference Example 7.

NMR: 1.40 (2H, s), 2.94 (2H, t, J = 5.8 Hz), 3.46-3.55 (2H, m), 3.80 (3H, s), 3.83 (3H, s), 4.95 (2H, s), 5.08 (2H, s), 6.67 (1H, s), 6.80-6.97 (5H, m), 7.35 (4H, dd, J = 8.6, 3.0 Hz), 7.45 (1H, d, J = 8.8 Hz)

Reference Example 9

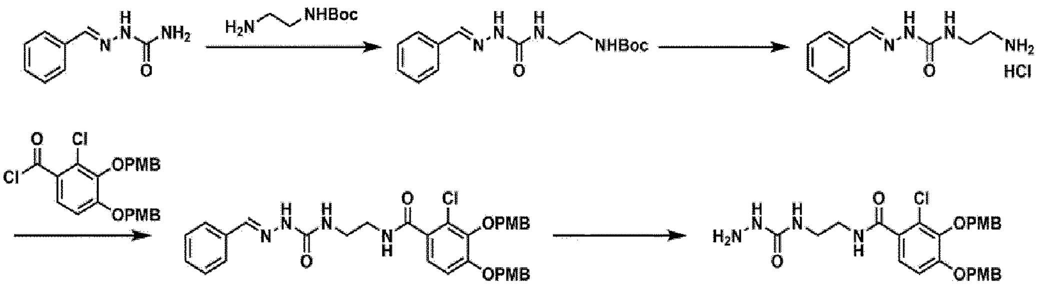

Reference Example 9 (1)

Chlorobenzene (30 mL) and tert-butyl (2-aminoethyl)carbamate (9.82 g) were sequentially added to (E)-2-benzylidenehydrazine-1-carboxamide (10.0 g), and the mixture was stirred. The reaction mixture was heated and stirred under reflux for 3 hours and 15 minutes. The reaction mixture was cooled to room temperature, and chlorobenzene (20 mL) and tert-butyl (2-aminoethyl)carbamate (2.95 g) were added thereto. The reaction mixture was heated and stirred under reflux for 2 hours 40 minutes. The reaction mixture was cooled to room temperature, IPE (200 mL) was added thereto, and the reaction mixture was stirred for 1 hour. Solids were collected by filtration and dried, thereby obtaining a target substance (18.0 g) as white solids.

Reference Example 9 (2)

Ethyl acetate (100 mL) and a 4 mol/L hydrochloric acid in an ethyl acetate solution (16.3 mL) were added to the compound (10.0 g) obtained in Reference Example 9 (1), and the mixture was stirred at room temperature for 1 hour. Methanol (1 mL) was added to the reaction mixture, and the reaction mixture was stirred at room temperature for 1 hour. 1,4-Dioxane (50 mL), 4 mol/L hydrochloric acid in a 1,4-dioxane solution (16.3 mL), and methanol (10 mL) were added to the reaction mixture, and the reaction mixture was stirred at room temperature for 2 hours and 30 minutes. IPE (150 mL) was added to the reaction mixture, and solids were collected by filtration. The solids were dried, thereby obtaining a target substance (5.0 g) as light brown solids.

Reference Example 9 (3)

THF (100 mL), water (39 mL), and sodium hydrogen carbonate (2.2 g) were added to the compound (1.3 g) obtained in Reference Example 9 (2), and the mixture was stirred under ice cooling. At the same temperature, water (39 mL), sodium hydrogen carbonate (2.2 g), and 2-chloro-3,4-bis((4-methoxybenzyl)oxy)benzoyl chloride (7.0 g) were sequentially added to the reaction mixture. At the same temperature, the reaction mixture was stirred for 2 hours and 30 minutes. THF (250 mL) and water (50 mL) were added to the reaction mixture, and the solvent was distilled off under reduced pressure. IPE was added to the residue, and solids were collected by filtration. The solids were dried, thereby obtaining a target substance (7.3 g) as white solids.

Reference Example 9 (4)

Dichloromethane (20 mL) and methanol (10 mL) were added to the compound (1.0 g) obtained in Reference Example 9 (3), and the mixture was stirred under ice cooling. At the same temperature, 2,4-dinitrophenylhydrazine (wetted with 50% water, 1.28 g) and p-toluenesulfonic acid monohydrate (308 mg) were sequentially added to the reaction mixture. The reaction mixture was stirred at room temperature for 2 hours and 30 minutes. Ethyl acetate (220 mL) and water (110 mL) were added to the reaction mixture. A saturated aqueous sodium hydrogen carbonate solution was added to the reaction mixture such that the pH was adjusted to 8.2. The organic layer was separated, washed with a saturated aqueous sodium chloride solution, and then dehydrated and dried over anhydrous sodium sulfate. The solvent was distilled off under reduced pressure, and the residue was purified by silica gel column chromatography [eluent; ethyl acetate:hexane = 90:10 → chloroform: methanol = 90:10 → 80:20], thereby obtaining N-(2-(2-chloro-3,4-bis((4-methoxybenzyl)oxy)benzamido)ethyl)hydrazine carboxamide (250 mg) as light yellow solids.

NMR (DMSO-$d_6$): 3.17-3.27 (4H, m), 3.75 (3H, s), 3.77 (3H, s), 4.08 (2H, s), 4.88 (2H, s), 5.15 (2H, s), 6.57 (1H, s), 6.88 (2H, dd, J = 6.4, 2.0 Hz), 6.98 (2H, dd, J = 6.8, 2.0 Hz), 7.01 (1H, s), 7.17 (2H, dd, J = 10.4, 8.8 Hz), 7.32 (2H, d, J = 8.4 Hz), 7.58 (2H, d, J = 8.4 Hz), 8.32 (1H, t, J = 5.0 Hz)

Reference Example 10

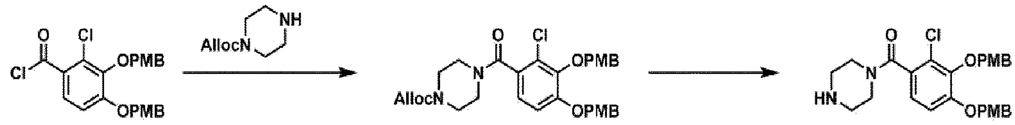


Reference Example 10 (1)

THF (100 mL) and water (75 mL) were added to allylpiperazine-1carboxylate (2.5 g), and the mixture was stirred under ice cooling. Sodium hydrogen carbonate (1.5 g) and 2-chloro-3,4-bis((4-methoxybenzyl)oxy)benzoyl chloride (6.6 g) were sequentially added to the reaction mixture. The reaction mixture was stirred at room temperature overnight, ethyl acetate (100 mL) and water (50 mL) were then added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining a target substance (9.17 g) as light yellow solids.

Reference Example 10 (2)

THF (170 mL), 1,3-dimethylbarbituric acid (2.5 g), and tetrakis(triphenylphosphine)palladium (0) (1.7 g) were sequentially added to the compound (8.5 g) obtained in Reference Example 10 (1), and the mixture was stirred at room temperature for 1 hour. Ethyl acetate (100 mL) and water (100 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [carrier: silica gel NH-DM1020 (Fuji Silysia Chemical, Ltd.), eluent; chloroform], thereby obtaining 2-chloro-3,4-bis((4-methoxybenzyl)oxy)phenyl)(piperazin-1-yl)methanone (4.98g) as a brown oily substance.

NMR: 2.65-2.74 (1H, m), 2.78-2.86 (1H, m), 2.86-2.99 (2H, m), 3.04-3.12 (1H, m), 3.12-3.21 (1H, m), 3.28 (1H, s), 3.66-3.75 (1H, m), 3.75-3.87 (1H, m), 3.79 (3H, s), 3.83 (3H, s), 4.93-5.06 (2H, m), 5.07 (2H, s), 6.78-6.85 (2H, m), 6.90-6.97 (4H, m), 7.29-7.39 (4H, m)

Reference Example 11

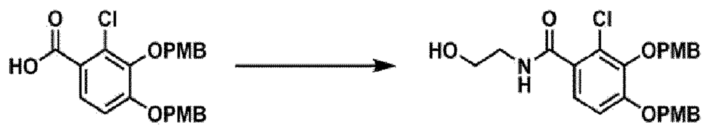

Reference Example 11

DMAC (16mL), 2-chloro-3,4-bis((4-methoxybenzyl)oxy)benzoic acid (10.1 g), HOBt (3.89 g), EDC (6.03 g), and NMM (7.2 mL) were sequentially added to 2-aminoethanol (1.6 g). The reaction mixture was stirred at room temperature for 6 hours. Water (150 mL) and 1 mol/L hydrochloric acid were added to the reaction mixture such that the pH was adjusted to 4.9. The reaction mixture was stirred at room temperature for 1 hour, and solids were collected by filtration. The solids were dried, thereby obtaining 2-chloro-N(2-hydroxyethyl)-3,4-bis((4-methoxybenzyl)oxy)benzamide (11.86 g) as gray solids.

NMR(DMSO-$d_6$): 3.21-3.30 (2H, m), 3.43-3.53 (2H, m), 3.75 (3H, s), 3.77 (3H, s), 4.69 (1H, t, J = 5.6 Hz), 4.88 (2H, s), 5.15 (2H, s), 6.84-6.91 (2H, m), 6.94-7.01 (2H, m), 7.12-7.22 (2H, m), 7.28-7.35 (2H, m), 7.40-7.47 (2H, m), 8.22 (1H, t, J = 5.6 Hz)

The compounds in Table 2 were obtained in the same manner as in Reference Example 11.

[Table 2]

| Ref. Ex. No. | Structural Formula | Name |
|---|---|---|
| 1 2 | 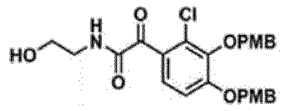 | 2-(2-chloro-3,4-bis((4-methoxybenzyl)oxy)phenyl)-N-(2-hydroxyethyl)-2-oxoacetamide |
| 1 3 | 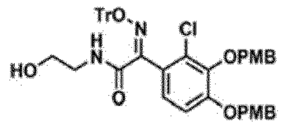 | (Z)-2-(2-chloro-3,4-bis((4-methoxybenzyl)oxy)phenyl)-N-(2-hydroxyethyl)-2-((trityloxy)imino)acetamide |
| 1 4 | 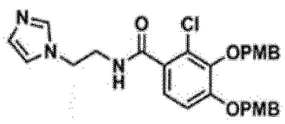 | N-(2-(1H-imidazol-1-yl)ethyl)-2-chloro-3,4-bis((4-methoxybenzyl)oxy) benzamide |
| 1 5 | 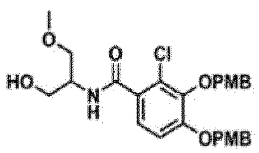 | 2-Chloro-N-(1-hydroxy-3-methoxypropan-2-yl)-3,4-bis((4-methoxybenzyl)oxy)benzamide |

The measured values of NMR of the compounds in the table are as follows.

Reference Example 12

NMR(DMSO-$d_6$): 3.23-3.31 (2H, m), 3.46-3.54 (2H, m), 3.74 (3H, s), 3.78 (3H, s), 4.76 (1H, t, J = 5.6 Hz), 4.90 (2H, s), 5.22 (2H, s), 6.81-6.89 (2H, m), 6.95-7.03 (2H, m), 7.24-7.35 (3H, m), 7.42-7.49 (2H, m), 7.52 (1H, d, J = 8.4 Hz), 8.83 (1H, t, J = 5.6 Hz)

Reference Example 13

NMR(DMSO-$d_6$): 3.10-3.20 (2H, m), 3.31-3.40 (2H, m), 3.66 (3H, s), 3.78 (3H, s), 4.69 (1H, t, J = 5.2 Hz), 4.94 (2H, s), 5.20 (2H, s), 6.75-6.82 (2H, m), 6.96-7.03 (3H, m), 7.13-7.21 (7H, m), 7.25-7.34 (12H, m), 7.44-7.50 (2H, m)

Reference Example 14

NMR: 3.71-3.88 (2H, m), 3.80 (3H, s), 3.83 (3H, s), 4.23 (2H, t, J = 5.8 Hz), 4.93 (2H, s), 5.08 (2H, s), 6.53 (1H, t, J = 5.8 Hz), 6.83 (2H, d, J = 8.4 Hz), 6.88-6.99 (4H, m), 7.07 (1H, s), 7.29-7.42 (5H, m), 7.51 (1H, s)

Reference Example 15

NMR: 3.40 (3H, s), 3.67 (1H, dd, J = 9.6, 4.0 Hz), 3.71 (1H, dd, J = 9.6, 3.6 Hz), 3.76-3.82 (1H, m), 3.81 (3H, s), 3.83 (3H, s), 3.94 (1H, dd, J = 11.2, 4.0 Hz), 4.23-4.32 (1H, m), 4.95 (2H, s), 5.09 (2H, s), 6.84 (2H, dd, J = 6.8, 2.0 Hz), 6.89-7.02 (4H, m), 7.35 (4H, dd, J = 8.8, 1.2 Hz), 7.46 (1H, d, J = 8.8 Hz)

Reference Example 16

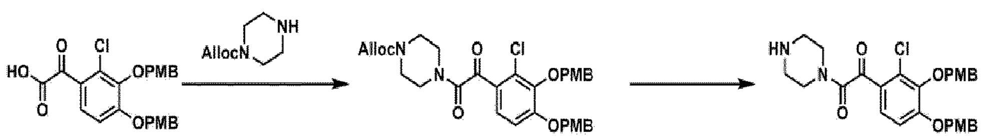

Reference Example 16 (1)

2-(2-Chloro-3,4-bis((4-methoxybenzyl)oxy)phenyl)-2-oxoacetic acid (6.7 g), HOBt (2.2 g), EDC (3.1 g), DMAC (25 mL), and NMM (1.9 mL) were sequentially added to allylpiperazine-1-carboxylate (2.5 g). The reaction mixture was stirred at room temperature overnight. Ethyl acetate (100 mL) and water (100 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 25:75 → 50:50], thereby obtaining a target substance (6.2 g) as light yellow solids.

Reference Example 16 (2)

THF (130 mL), 1,3-dimethylbarbituric acid (1.8 g), and tetrakis(triphenylphosphine)palladium (0) (1.2 g) were sequentially added to the compound (6.3 g) obtained in Reference Example 16 (1), and the mixture was stirred at room temperature for 1 hour. Ethyl acetate (100 mL) and water (100 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [carrier: silica gel NH-DM1020 (Fuji Silysia Chemical, Ltd.), eluent; chloroform], thereby obtaining 1-(2-chloro-3,4-bis((4-methoxybenzyl)oxy)phenyl)-2-(piperazin-1-yl)ethane-1,2-dione (4.6 g) as a brown oily substance.

NMR: 2.90 (2H, t, J = 5.0 Hz), 2.96 (2H, t, J = 5.0 Hz), 3.28 (1H, s), 3.41 (2H, t, J = 5.0 Hz), 3.68 (2H, t, J = 5.0 Hz), 3.80 (3H, s), 3.83 (3H, s), 4.95 (2H, s), 5.13 (2H, s), 6.80-6.85 (2H, m), 6.90-6.95 (2H, m), 6.99 (1H, d, J = 8.8 Hz), 7.30-7.35 (4H, m), 7.67-7.71 (1H, m)

Reference Example 17

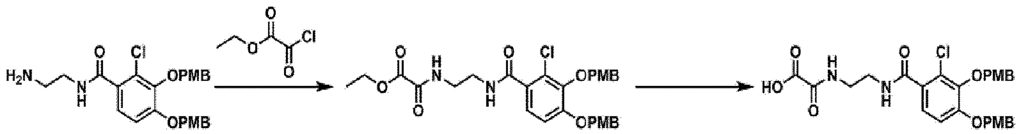

Reference Example 17 (1)

At room temperature, dichloromethane (40 mL), pyridine (310 μL), and ethyl chlorooxoacetate (430 μL) were sequentially added to N-(2-aminoethyl)-2-chloro-3,4-bis((4-methoxybenzyl)oxy)benzamide (1.2 g). The reaction mixture was stirred at a temperature of 40°C to 50°C for 2 hours. At the same temperature, pyridine (100 μL) and ethyl chlorooxoacetate (145 μL) were sequentially added to the reaction mixture. The reaction mixture was stirred at the same temperature for 3 hours. Chloroform and a saturated aqueous ammonium chloride solution were sequentially added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 0:100 → 100:0], thereby obtaining a target substance (0.61 g) as white solids.

Reference Example 17 (2)

THF (20 mL), water (10 mL), and lithium hydroxide (120 mg) were sequentially added to the compound obtained in Reference Example 17 (1), and the mixture was stirred at room temperature for 1 hour. Ethyl acetate and water were sequentially added to the reaction mixture, and the organic layer was separated. The organic layer was washed with a saturated aqueous sodium chloride solution and dehydrated and dried over anhydrous sodium sulfate. The solvent was distilled off under reduced pressure, IPE was added to the residue, and solids were collected by filtration. The solids were dried, thereby obtaining 2-((2-(2-chloro-3,4-bis((4-methoxybenzyl)oxy)benzamido)ethyl)amino)-2-oxoacetic acid (458 mg) as white solids.

NMR: 3.46-3.60 (4H, m), 3.80 (3H, s), 3.84 (3H, s), 4.95 (2H, s), 5.12 (2H, s), 6.83 (2H, d, J = 8.8 Hz), 6.97 (2H, d, J = 8.8 Hz), 7.13 (1H, d, J = 8.4 Hz), 7.22 (1H, d, J = 8.4 Hz), 7.31 (2H, d, J = 8.4 Hz), 7.41 (2H, d, J = 8.4 Hz)

Reference Example 18

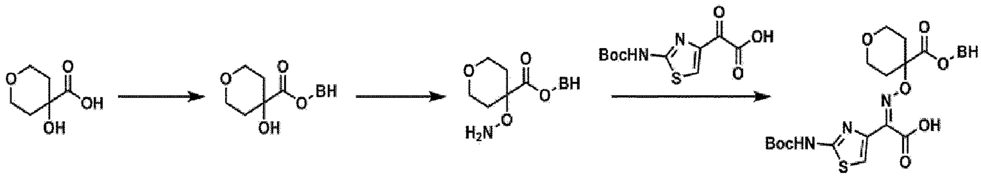

Reference Example 18 (1)

THF (16 mL) was added to 4-hydroxytetrahydro-2H-pyran-4-carboxylic acid (2.0 g), and the mixture was stirred under ice cooling. At the same temperature, 1 mol/L diphenyldiazomethane in a THF solution (16 mL) was added dropwise to the reaction mixture. The reaction mixture was stirred at room temperature overnight. The solvent was distilled off under reduced pressure, and the residue was purified by silica gel column chromatography [eluent; ethyl acetate:hexane = 10:90 → 40:60], thereby obtaining a target substance (4.23 g) as white solids.

Reference Example 18 (2)

THF (40 mL) was added to the compound (4.32 g) obtained in Reference Example 18 (1), and the mixture was stirred under ice cooling. At the same temperature, 60% oily sodium hydride (664 mg) was added to the reaction mixture, and the reaction mixture was stirred for 20 minutes. At the same temperature, O-(mesitylsulfonyl)hydroxylamine (3.87 g) in a THF solution (40 mL) was added dropwise to the reaction mixture. At the same temperature, the reaction mixture was stirred for 3 hours. Ethyl acetate and water were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 10:90 → 80:20], thereby obtaining benzhydryl 4-(aminooxy)tetrahydro-2H-pyran-4-carboxylate (3.85g) as white solids.

NMR: 1.93-2.03 (2H, m), 2.07-2.19 (2H, m), 3.65-3.90 (4H, m), 5.29 (2H, s), 6.95 (1H, s), 7.24-7.42 (10H, m)

Reference Example 18 (3)

Methanol (40 mL) was added to benzhydryl 4-(aminooxy)tetrahydro-2H-pyran-4-carboxylate (2.73 g), and the mixture was stirred under ice cooling. At the same temperature, 2-(2-((tert-butoxycarbonyl)amino)thiazol-4-yl)-2-oxoacetic acid (2.07 g) was added to the reaction mixture, and the reaction mixture was stirred at room temperature for 3 hours. The solvent was distilled off under reduced pressure, ethyl acetate (100 mL), 1 mol/L hydrochloric acid (50 mL), and a saturated aqueous sodium chloride solution were added to the residue, and the organic layer was separated. The aqueous layer was extracted using ethyl acetate, and the organic layers were combined, washed with a saturated aqueous sodium chloride solution, and then dehydrated and dried over anhydrous sodium sulfate. The solvent was distilled off under reduced pressure, IPE (20 mL) and hexane (100 mL) were added to the residue, and solids were collected by filtration. The solids were dried, thereby obtaining (Z)-2-(((4-((benzhydryloxy)carbonyl)tetrahydro-2H-pyran-4-yl)oxy)imino)-2-(2-((tert-butoxy carbonyl)amino)thiazol-4-yl)acetic acid (3.88 g) as white solids.

NMR (DMSO-$d_6$): 1.47 (9H, s), 1.88-2.15 (4H, m), 3.50-3.76 (4H, m), 6.84 (1H, s), 7.19-7.47 (11H, m), 11.81 (1H, s), 14.17-14.22 (1H, brs)

Reference Example 19

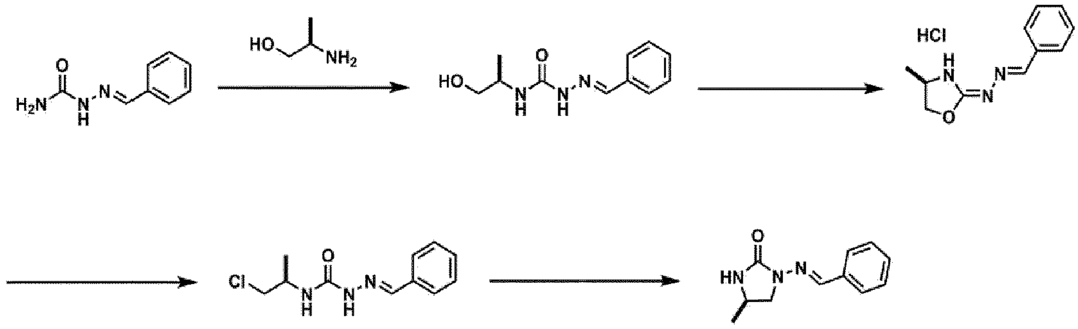

Reference Example 19 (1)

Chlorobenzene (20 mL) and D-alaninol (3.8 mL) were sequentially added to (E)-2-benzylidenehydrazine-1-carboxamide (8.00 g), and the mixture was stirred. The reaction mixture was heated and stirred under reflux for 4 hours and 20 minutes. At the same temperature, the solvent (14 mL) was distilled off. The reaction mixture was cooled to room temperature, thereby obtaining a target substance as a mixture of chlorobenzene.

Reference Example 19 (2)

Benzene (10 mL) was added to the compound obtained in Reference Example 19 (1). Thionyl chloride (5.4 mL) was added to the reaction mixture at room temperature, and the mixture was stirred for 2 hours and 30 minutes. The solvent was distilled off under reduced pressure, diethyl ether was added to the residue, and solids were collected by filtration. The solids were dried, thereby obtaining a target substance (10.93 g) as light yellow solids.

Reference Example 19 (3)

Benzene (120 mL) and toluene (40 mL) were added to the compound (10.93 g) obtained in Reference Example 19 (2), and the mixture was stirred. The reaction mixture was heated and stirred under reflux for 1 hour and 40 minutes. The reaction mixture was cooled to room temperature, and the solvent was distilled off under reduced pressure, thereby obtaining a target substance as light yellow solids.

Reference Example 19 (4)

DMF (80 mL) was added to the compound obtained in Reference Example 19 (3), and the mixture was stirred under ice cooling. At the same temperature, 60% oily sodium hydride (1.76 g) was added to the reaction mixture by being divided into three portions. The reaction mixture was stirred at room temperature for 1 hour. Ethyl acetate, water, and 1 mol/L hydrochloric acid were added to the reaction mixture. Solids were filtered, and the organic layer was separated from the filtrate. The aqueous layer was extracted twice by using ethyl acetate, and the organic layers were combined and washed with a 5% aqueous sodium chloride solution. The organic layer was dehydrated and dried over anhydrous sodium sulfate, and the solvent was distilled off under reduced pressure. IPE was added to the residue, and solids were collected by filtration. The solids were dried, thereby obtaining (R,E)-1-(benzylideneamino)-4-methylimidazolidin-2-one (6.01 g) as light yellow solids.

NMR (DMSO-$d_6$): 1.21 (3H, d, J = 6.0 Hz), 3.21-3.29 (1H, m), 3.78-3.95 (2H, m), 7.29 (1H, s), 7.31-7.45 (3H, m), 7.59 (1H, s), 7.62-7.69 (2H, m)

The compounds in Table 3 were obtained in the same manner as in Reference Example 19.

[Table 3]

| Ref. Ex. No. | Structural Formula | Name |
|---|---|---|
| 2 0 | 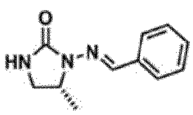 | (R,E)-1(benzylideneamino)-5-methylimidazolidin-2-one |

(continued)

| Ref. Ex. No. | Structural Formula | Name |
|---|---|---|
| 2 1 | 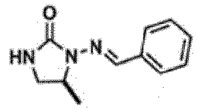 | (S,E)-1(benzylideneamino)-5-methylimidazolidin-2-one |

The measured values of NMR of the compounds in the table are as follows.

Reference Example 20

NMR (DMSO-$d_6$): 1.26 (3H, d, J = 6.0 Hz), 2.95-3.02 (1H, m), 3.51-3.58 (1H, m), 4.23-4.35 (1H, m), 7.07 (1H, s), 7.32-7.45 (3H, m), 7.64-7.70 (2H, m), 8.20 (1H, s)

Reference Example 21

NMR: 1.26 (3H, d, J = 6.0 Hz), 2.94-3.04 (1H, m), 3.55 (1H, t, J = 8.6 Hz), 4.23-4.25 (1H, m), 7.08 (1H, s), 7.32-7.47 (3H, m), 7.67 (2H, d, J = 6.8 Hz), 8.20 (1H, s)

Reference Example 22

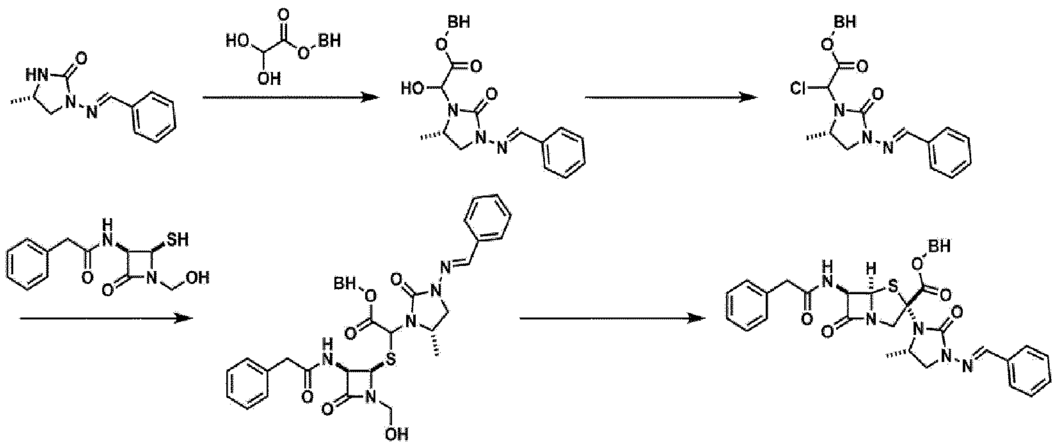

Reference Example 22 (1)

Benzhydryl 2,2-dihydroxyacetate (8.14g) and dichloromethane (61mL) were added to (S,E)-1-(benzylideneamino)-4-methylimidazolidin-2-one (6.10g), and the mixture was stirred under ice cooling. At the same temperature, DBU (226 μL) was added to the reaction mixture, and then the reaction mixture was stirred at room temperature for 5 hours and 30 minutes. At room temperature, benzhydryl 2,2-dihydroxyacetate (1.16 g) was added to the reaction mixture, and the reaction mixture was stirred for 30 minutes. The solvent was distilled off under reduced pressure, thereby obtaining a target substance (13.3 g) as a yellow oily substance.

Reference Example 22 (2)

THF (130 mL) was added to the compound (13.3 g) obtained in Reference Example 22 (1), and the mixture was stirred under ice cooling. At the same temperature, 2,6-lutidine (3.8 mL) and thionyl chloride (2.4 mL) were sequentially added to the reaction mixture. The reaction mixture was stirred at room temperature for 2 hours. The reaction mixture was ice-cooled, 2,6-lutidine (3.1 mL) and thionyl chloride (2.0 mL) were sequentially added thereto, and the reaction mixture was stirred at room temperature for 30 minutes. Insoluble matters were filtered, thereby obtaining a mixture containing a target substance.

Reference Example 22 (3)

DMF (130 mL) was added to the mixture obtained in Reference Example 22 (2), and the mixture was stirred under ice cooling. At the same temperature, N-((2R,3R)-1-(hydroxymethyl)-2-mercapto-4-oxoazetidin-3-yl)-2-phenylacetamide (8.8g) and triethylamine (4.6mL) were sequentially added to the reaction mixture. The reaction mixture was stirred at room temperature for 1 hour. Ethyl acetate (300 mL), water (300 mL), and 1 mol/L hydrochloric acid (20 mL) were added to the reaction mixture. The organic layer was separated and sequentially washed with water and a saturated aqueous sodium chloride solution. After the organic layer was dehydrated and dried over anhydrous sodium sulfate, the solvent was distilled off under reduced pressure. IPE was added to the residue, and solids were collected by filtration. The solids were dried, thereby obtaining a target substance (20.1 g) as light yellow solids.

Reference Example 22 (4)

THF (15 mL) was added to the compound (1.5 g) obtained in Reference Example 22 (3), and the mixture was stirred under ice cooling. At the same temperature, 2,6-lutidine (280 μL) and thionyl chloride (170 μL) were sequentially added to the reaction mixture. The reaction mixture was stirred at room temperature for 1 hour. The reaction mixture was ice-cooled, 2,6-lutidine (76 μL) and thionyl chloride (47 μL) were sequentially added thereto, and the reaction mixture was stirred at room temperature for 50 minutes. Insoluble matters were filtered, and the solvent was distilled off under reduced pressure. DMF (15 mL) was added to the residue, and the mixture was stirred under ice cooling. At the same temperature, DBU (326 μL) was added to the reaction mixture, and the reaction mixture was stirred for 1 hour. Ethyl acetate (50 mL), water (50 mL), and 1 mol/L hydrochloric acid (4 mL) were added to the reaction mixture, and the organic layer was separated. The organic layer was sequentially washed with water and a 5% aqueous sodium chloride solution. After the organic layer was dehydrated and dried over anhydrous sodium sulfate, the solvent was distilled off under reduced pressure. The residue was purified by silica gel column chromatography [eluent; ethyl acetate:hexane = 30:70 → 75:25], thereby obtaining benzhydryl (3R,5R,6R)-3-((S)-3-(((E))-benzylidene)amino)-5-methyl-2-oxoimidazolidin-1-yl)-7-oxo-6-(2 -phenylacetamido)-4-thia-1-azabicyclo[3.2.0]heptane-3-carboxylate (660 mg) as yellow solids.

NMR: 1.27 (3H, d, J = 7.2 Hz), 3.28-3.41 (3H, m), 3.44-3.52 (1H, m), 3.76-3.85 (1H, m), 4.17-4.28 (1H, m), 5.17 (1H, d, J = 13.6 Hz), 5.48-5.56 (2H, m), 6.31 (1H, d, J = 8.4 Hz), 6.89 (1H, s), 7.13-7.19 (2H, m), 7.20-7.45 (16H, m), 7.67 (1H, s), 7.70-7.77 (2H, m)

The compounds in Table 4 were obtained in the same manner as in Reference Example 22.

[Table 4]

| Ref. Ex. No. | Structural Formula | Name |
|---|---|---|
| 2 3 | 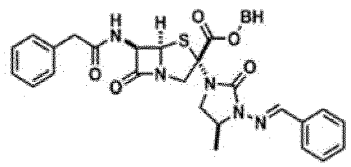 | Benzhydryl (3R,5R,6R)-3-((S)-3-(((E)-benzylidene)amino)-4-methyl-2-oxoimidazolidin-1-yl)-7-oxo-6-(2-phenylacetamido)-4-thia-1-azabicyclo[3.2.0]heptane-3-carboxylate |
| 2 4 | 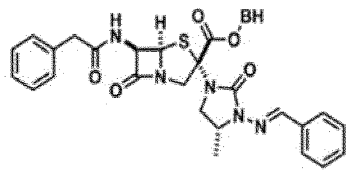 | Benzhydryl (3R,5R,6R)-3-((R)-3-(((E)-benzylidene)amino)-4-methyl-2-oxoimidazolidin-1-yl)-7-oxo-6-(2-phenylacetamido)-4-thia-1-azabicyclo[3.2.0]heptane-3-carboxylate |
| 2 5 | 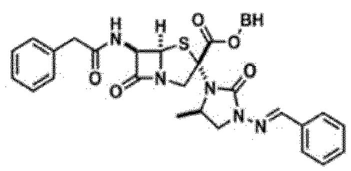 | Benzhydryl (3R,5R,6R)-3-((R)-3-(((E)-benzylidene)amino)-5-methyl-2-oxoimidazolidin-1-yl)-7-oxo-6-(2-phenylacetamido)-4-thia-1-azabicyclo[3.2.0]heptane-3-carboxylate |

The measured values of NMR of the compounds in the table are as follows.

Reference Example 23

NMR: 1.37 (3H, d, J = 6.0 Hz), 3.26 (1H, dd, J = 7.8, 5.4 Hz), 3.36 (1H, dd, J = 13.2, 0.8 Hz), 3.39-3.41 (2H, m), 3.73 (1H, t, J = 8.0 Hz), 4.10-4.22 (1H, m), 4.85 (1H, d, J = 13.2 Hz), 5.50 (1H, d, J = 4.0 Hz), 5.61 (1H, ddd, J = 8.9, 3.9, 0.7 Hz), 6.56 (1H, d, J = 9.2 Hz), 6.88 (1H, s), 7.15-7.43 (17H, m), 7.63-7.72 (3H, m), 8.61 (1H, s)

Reference Example 24

NMR: 1.35 (3H, d, J = 6.0 Hz), 3.19 (1H, t, J = 8.0 Hz), 3.35 (1H, d, J = 13.2 Hz), 3.40 (2H, s), 3.80 (1H, t, J = 8.0 Hz), 4.12-4.18 (1H, m), 4.93 (1H, d, J = 13.2 Hz), 5.50 (1H, d, J = 4.0 Hz), 5.57 (1H, dd, J = 8.8, 3.6 Hz), 6.48 (1H, d, J = 8.4 Hz), 6.90 (1H, s), 7.18 (2H, d, J = 8.0 Hz), 7.21-7.43 (16H, m), 7.65-7.70 (2H, m), 8.73 (1H, s)

Reference Example 25

NMR: 1.45 (3H, d, J = 6.4 Hz), 3.27 (2H, s), 3.34 (1H, dd, J = 13.4, 1.0 Hz), 3.79 (1H, t, J = 8.8 Hz), 4.03-4.11 (2H, m), 5.23 (1H, d, J = 13.2 Hz), 5.45-5.53 (2H, m), 6.05 (1H, d, J = 3.6 Hz), 6.88 (1H, s), 7.15-7.43 (17H, m), 7.54 (1H, s), 7.68-7.77 (2H, m)

Reference Example 26

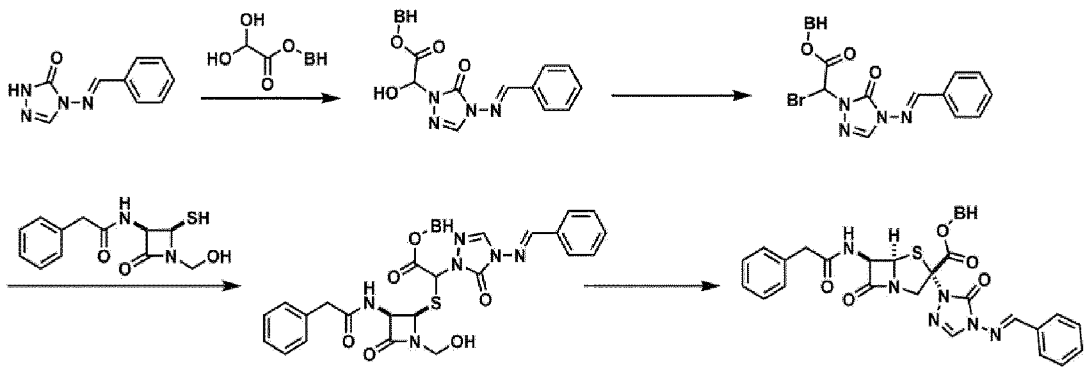

Reference Example 26 (1)

Benzhydryl 2,2-dihydroxyacetate (2.88 g) and dichloromethane (30 mL) were added to (E)-4-(benzylideneamino)-2,4-dihydro-3H-1,2,4-triazol-3-one (2.00 g), and the mixture was stirred under ice cooling. At the same temperature, DBU (79 μL) was added to the reaction mixture, and then the reaction mixture was stirred at room temperature for 1 hour and 15 minutes. Ethyl acetate (30 mL) was added to the reaction mixture, and solids were collected by filtration. The solids were washed with ethyl acetate. The solids were dried under reduced pressure, thereby obtaining a target substance (3.87 g) as white solids.

Reference Example 26 (2)

DMF (19 mL) was added to the compound (1.89 g) obtained in Reference Example 26 (1), and the mixture was stirred under ice cooling. At the same temperature, triphenylphosphine (1.5 g) and bromine (272 μL) were sequentially added to the reaction mixture. The reaction mixture was stirred at room temperature for 1 hour and 30 minutes. The reaction mixture was added to a mixture of ethyl acetate (50 mL) and water (50 mL). The organic layer was separated and sequentially washed with water and a saturated aqueous sodium chloride solution. The organic layer was dehydrated and dried over anhydrous sodium sulfate, and then the solvent was distilled off under reduced pressure. The residue was purified by silica gel column chromatography [eluent; ethyl acetate:hexane = 15:85 → 20:80], thereby obtaining a target substance (2.02 g) as yellow solids.

Reference Example 26 (3)

DMF (40 mL) was added to the compound (2.02 g) obtained in Reference Example 26 (2), and the mixture was

stirred under ice cooling. At the same temperature, N-((2R,3R)-1-(hydroxymethyl)-2-mercapto-4-oxoazetidin-3-yl)-2-phenylacetamide (1.2 g) and triethylamine (630 μL) were sequentially added to the reaction mixture. At the same temperature, the reaction mixture was stirred for 35 minutes. Ethyl acetate (78 mL) and water (62 mL) were added to the reaction mixture. The organic layer was separated and sequentially washed with water and a saturated aqueous sodium chloride solution. The organic layer was dehydrated and dried over anhydrous sodium sulfate, and the solvent was distilled off under reduced pressure. The residue was purified by silica gel column chromatography [eluent; ethyl acetate:hexane = 75:25 → 80:20], thereby obtaining a target substance (2.81 g) as white solids.

Reference Example 26 (4)

THF (56 mL) was added to the compound (2.81 g) obtained in Reference Example 26 (3), and the mixture was stirred under ice cooling. At the same temperature, 2,6-lutidine (773 μL) and thionyl chloride (484 μL) were sequentially added to the reaction mixture. The reaction mixture was stirred at room temperature for 1 hour. Insoluble matters were filtered, and the solvent was distilled off under reduced pressure. DMF (28 mL) was added to the residue, and the mixture was stirred under ice cooling. DBU (749 μL) was added to the reaction mixture, and the reaction mixture was stirred at the same temperature for 1 hour and 30 minutes. Then, at the same temperature, DBU (62 μL) was added to the reaction mixture, and the reaction mixture was stirred for 20 minutes. The reaction mixture was added to a mixture of ethyl acetate (82 mL), water (82 mL), and 1 mol/L hydrochloric acid (5 mL), and the organic layer was separated. The aqueous layer was extracted three times by using ethyl acetate (100 mL), and the organic layers were combined and sequentially washed with water and a saturated aqueous sodium chloride solution. After the organic layer was dehydrated and dried over anhydrous sodium sulfate, the solvent was distilled off under reduced pressure. Ethyl acetate (15 mL) was added to the residue, and solids were collected by filtration. The solids were dried, thereby obtaining benzhydryl (3R,5R,6R)-3-(4-(((E)-benzylidene)amino)-5-oxo-4,5-dihydro-1H-1,2,4-triazol-1-yl)-7-oxo-6-(2-phenylacetamido)-4-thia-1-azabicyclo[3.2.0]heptane-3-carboxylate (1.97 g) as white solids.

NMR: 3.47 (2H, s), 3.93 (1H, dd, J = 13.4, 0.6 Hz), 4.75 (1H, d, J = 13.6 Hz), 5.58 (1H, d, J = 4.0 Hz), 5.73 (1H, dd, J = 9.2,3.6 Hz), 6.75 (1H, d, J = 9.6 Hz), 6.78 (1H, s), 7.16-7.35 (14H, m), 7.44-7.56 (4H, m), 7.75 (2H, dd, J = 8.2, 1.4 Hz), 7.80 (1H, s), 9.54 (1H, s)

Reference Example 27

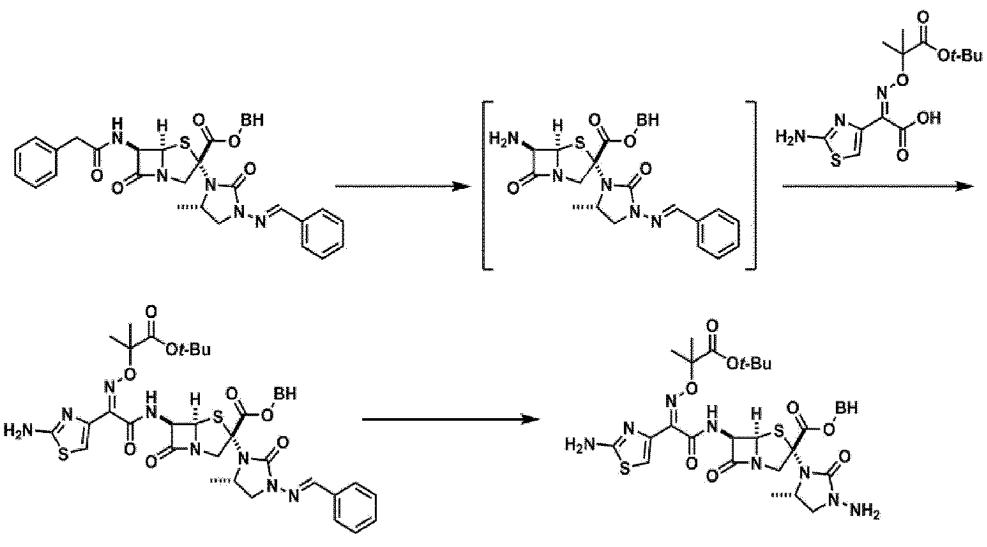

Reference Example 27 (1)

Dichloromethane (6.6 mL) was added to benzhydryl (3R,5R,6R)-3-((S)-3-(((E)-benzylidene)amino)-5-methyl-2-oxoimidazolidin-1-yl)-7-oxo-6-(2-phenylacetamido)-4-thia-1-azabicyclo[3.2.0]heptane-3-carboxylate (660 mg), and the reaction mixture was cooled to a temperature equal to or lower than -30°C. At the same temperature, N,N-dimethylaniline (435 μL) and phosphorus pentachloride (306 mg) were sequentially added to the reaction mixture, and the reaction mixture was stirred at -30°C for 1 hour. Then, at the same temperature, methanol (600 μL) was added to the reaction mixture, and the reaction mixture was stirred for 30 minutes under ice cooling. Dichloromethane (20 mL) and an aqueous

sodium hydrogen carbonate solution (1.07 g of sodium hydrogen carbonate/20 mL of water) were added to the reaction mixture, and the organic layer was separated. The organic layer was dehydrated and dried over anhydrous sodium sulfate, and solids were filtered.

(Z)-2-(2-aminothiazol-4-yl)-2-(((1-(tert-butoxy)-2-methyl-1-oxopropan-2-yl)oxy)imino)acetic acid (355 mg), HATU (410 mg), 2,6-lutidine (251 μL), and DMF (6.6 mL) were added to the filtrate. The reaction mixture was stirred at room temperature under reduced pressure until the reaction mixture became a solution. Water (20 mL) and ethyl acetate (20 mL) were added to the reaction mixture, and the organic layer was separated. The organic layer was washed three times with a 5% aqueous sodium chloride solution. The organic layer was dehydrated and dried over anhydrous sodium sulfate, and then the solvent was distilled off under reduced pressure. The residue was purified by silica gel column chromatography [eluent; ethyl acetate:hexane = 60:40 → 100:0], thereby obtaining a target substance (180 mg) as a yellow oily substance.

Reference Example 27 (2)

Dichloromethane (3.6 mL) and methanol (1.8 mL) were added to the compound (180 mg) obtained in Reference Example 27 (1), and the mixture was stirred under ice cooling. At the same temperature, 2,4-dinitrophenylhydrazine (wetted with 50% water, 165 mg) and p-toluenesulfonic acid monohydrate (40 mg) were sequentially added to the reaction mixture. The reaction mixture was stirred at room temperature for 5 hours. Ethyl acetate (20 mL) and water (10 mL) were added to the reaction mixture. A saturated aqueous sodium hydrogen carbonate solution was added to the reaction mixture such that the pH was adjusted to 7.7. The organic layer was separated, washed with a 5% aqueous sodium chloride solution, and then dehydrated and dried over anhydrous sodium sulfate. The solvent was distilled off under reduced pressure, and the residue was purified by silica gel column chromatography [eluent; ethyl acetate:hexane = 70:30 → 100:0 → chloroform:2-propanol = 100:0 → 80:20], thereby obtaining benzhydryl (3R,5R,6R)-3-((S)-3-amino-5-methyl-2-oxoimidazolidinon-1-yl)-6-((Z)-2-(2-aminothiazol-4-y 1)-2-(((1-(tert-butoxy)-2-methyl-1-oxopropan-2-yl)oxy) imino)acetamido)-7-oxo-4-thia-1-azabi cyclo[3.2.0]heptane-3-carboxylate (90 mg) as a yellow oily substance.

NMR: 1.16-1.23 (3H, m), 1.41 (9H, s), 1.49 (3H, s), 1.51 (3H, s), 3.17 (1H, dd, J = 8.4,2.0Hz), 3.36 (1H, d, J = 13.2 Hz), 3.45-3.54 (1H, m), 3.81 (2H, s), 3.92-4.03 (1H, m), 5.07 (1H, d, J = 13.2 Hz), 5.59 (1H, d, J = 4.0 Hz), 5.80 (1H, dd, J = 8.8,4.0Hz), 6.48 (2H, s), 6.79 (1H, s), 6.88 (1H, s), 6.92-7.01 (1H, m), 7.21-7.42 (10H, m)

The compounds in Table 5 were obtained in the same manner as in Reference Example 27.

[Table 5]

| Ref.Ex. No. | Structural Formula | Name |
|---|---|---|
| 2 8 | 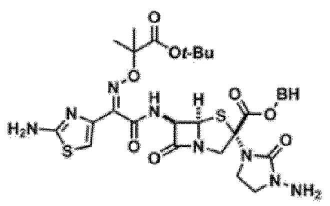 | Benzhydryl (3R.5R,6R)-3-(3-amino-2-oxoimidazolidin-1-yl)-6-((Z)-2-(2-aminothiazol-4-yl)-2-(((1-(tert-butoxy)-2-methyl-1-oxopropan-2-yl)oxy)imino)acetamido)-7-oxo-4-thia-1-azabicyclo [3.2.0]heptane-3-carboxylate |
| 2 9 | 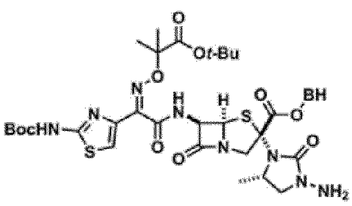 | Benzhydryl (3R,5R,6R)-3-((S)-3-amino-5-methyl-2-oxoimidazolidin-1-yl)-6-((Z)-2-(((1-(tert-butoxy)-2-methyl-1-oxopropan-2-yl) oxy)imino)-2-(2-((tert-butoxycarbonyl)amino)thiazol-4-yl)acetamido)-7-oxo-4-thia-1-azabicyclo[3.2.0]heptane-3-carboxylate |
| 3 0 | 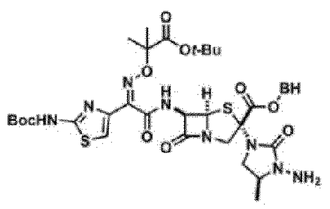 | Benzhydryl (3R,5R,6R)-3-((S)-3-amino-4-methyl-2-oxoimidazolidin-1-yl)-6-((Z)-2-(((1-(tert-butoxy)-2-methyl-1-oxopropan-2-yl) oxy)imino)-2-(2-((tert-butoxycarbonyl)amino)thiazol-4-yl)acetamido)-7-oxo-4-thia-1-azabicyclo[3.2.0]heptane-3-carboxylate |

(continued)

| Ref.Ex. No. | Structural Formula | Name |
|---|---|---|
| 3 1 | 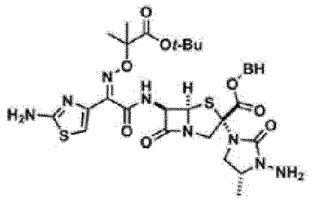 | Benzhydryl (3R,5R,6R)-3-((R)-3-amino-4-methyl-2-oxoimidazolidin-1-yl)-6-((Z)-2-(2-aminothiazol-4-yl)-2-(((1-(tert-butoxy)-2-methyl-1-oxopropan-2-yl)oxy)imino)acetamido)-7-oxo-4-thia-1-azabicyclo[3.2.0]heptane-3-carboxylate |
| 3 2 | 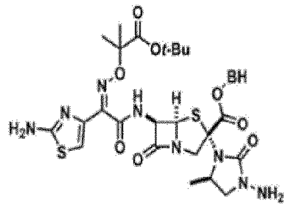 | Benzhydryl (3R,5R,6R)-3-((R)-3-amino-5-methyl-2-oxoimidazolidin-1-yl)-6-((Z)-2-(2-aminothiazol-4-yl)-2-(((1-(tert-butoxy)-2-methyl-1-oxopropan-2-yl)oxy)imino)acetamido)-7-oxo-4-thia-1-azabicyclo[3.2.0]heptane-3-carboxylate |
| 3 3 | 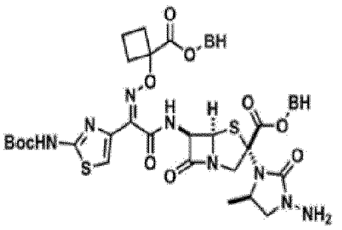 | Benzhydryl (3R,5R,6R)-3-((R)-3-amino-5-methyl-2-oxoimidazolidin-1-yl)-6-((Z)-2-((1-((benzhydryloxy)carbonyl)cyclobutoxy)imino)-2-(2-((tert-butoxy)amino)thiazol-4-yl)acetamido)-7-oxo-4-thia-1-azabicyclo[3.2.0]heptane-3-carboxylate |
| 3 4 | 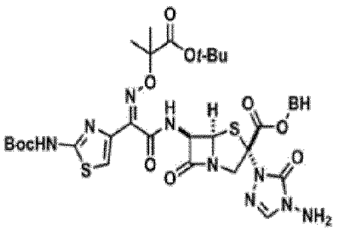 | Benzhydryl (3R,5R,6R)-3-((4-amino-5-oxo-4,5-dihydro-1H-1,2,4-triazol-1-yl))-6-((Z)-2-(((1-(tert-butoxy)-2-methyl-1-oxopropan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |

The measured values of NMR of the compounds in the table are as follows.

Reference Example 28

NMR: 1.39 (9H, s), 1.48 (3H, s), 1.50 (3H, s), 3.40-3.55 (5H, m), 3.83 (2H, s), 4.95 (1H, d, J = 13.2 Hz), 5.57 (1H, d, J = 4.0 Hz), 5.79 (1H, dd, J = 8.4, 3.6 Hz), 6.40 (2H, s), 6.79 (1H, s), 6.87 (1H, s), 7.06 (1H, d, J = 8.8 Hz), 7.16-7.40 (10H, m)

Reference Example 29

NMR: 1.19 (3H, d, J = 6.4 Hz), 1.41 (9H, s), 1.49-1.57 (15H, m), 3.17 (1H, dd, J = 8.0,1.6 Hz), 3.35 (1H, dd, J = 13.2, 0.8 Hz), 3.45-3.54 (1H, m), 3.81 (2H, s), 3.93-4.04 (1H, m), 5.09 (1H, d, J = 13.2 Hz), 5.60 (1H, d, J = 4.0 Hz), 5.78 (1H, dd, J = 8.0,4.0Hz), 6.88 (1H, s), 7.09-7.17 (1H, m), 7.19-7.43 (11H, m), 8.15 (1H, s)

Reference Example 30

NMR: 1.23 (3H, d, J = 6.0 Hz), 1.39 (9H, s), 1.50 (3H, s), 1.52 (3H, s), 1.54 (9H, s), 3.01 (1H, t, J = 6.8 Hz), 3.43-3.57 (3H, m), 3.67 (2H, s), 4.83 (1H, d, J = 12.8 Hz), 5.58 (1H, d, J = 4.0 Hz), 5.82 (1H, dd, J = 8.4,3.6 Hz), 6.86 (1H, s), 7.14-7.44 (11H, m), 7.54 (1H, d, J = 8.8 Hz), 8.19 (1H, s)

Reference Example 31

NMR: 1.22 (3H, d, J = 6.0 Hz), 1.39 (9H, s), 1.50 (3H, s), 1.52 (3H, s), 3.01 (1H, t, J = 8.4 Hz), 3.46-3.58 (2H, m),

3.60-3.73 (3H, m), 4.99 (1H, d, J = 13.2 Hz), 5.57 (1H, d, J = 4.0 Hz), 5.75-5.81 (1H, m), 6.84 (1H, s), 6.85 (1H, s), 7.15-7.38 (13H, m)

Reference Example 32

NMR: 1.35 (3H, d, J = 6.4 Hz), 1.39 (9H, s), 1.48 (3H, s), 1.49 (3H, s), 3.14 (1H, dd, J = 8.2, 3.0 Hz), 3.36 (1H, dd, J = 13.2, 0.8 Hz), 3.54 (1H, t, J = 8.6 Hz), 3.69 (2H, s), 3.83-3.94 (1H, m), 5.15 (1H, d, J = 13.2 Hz), 5.58 (1H, d, J = 4.0 Hz), 5.74 (1H, dd, J = 7.8,3.4 Hz), 6.50 (2H, s), 6.77 (1H, s), 6.88 (1H, s), 6.95 (1H, d, J = 8.4 Hz), 7.14-7.42 (10H, m)

Reference Example 33

NMR: 1.32 (3H, d, J = 6.4 Hz), 1.55 (9H, s), 1.92-2.03 (2H, m), 2.38-2.51 (2H, m), 2.55-2.69 (2H, m), 3.12 (1H, dd, J = 8.0, 2.8 Hz), 3.35 (1H, dd, J = 13.0, 1.0 Hz), 3.49 (1H, t, J = 8.4 Hz), 3.66 (2H, s), 3.77-3.87 (1H, m), 5.17 (1H, d, J = 13.6 Hz), 5.55 (1H, d, J = 3.6 Hz), 5.75 (1H, dd, J = 8.0, 3.6 Hz), 6.86 (1H, s), 6.91 (1H, s), 7.03 (1H, s), 7.11-7.42 (21H, m), 8.16 (1H, s)

Reference Example 34

NMR: 1.38 (9H, s), 1.51 (3H, s), 1.53 (9H, s), 1.54 (3H, s), 4.00 (1H, d, J = 13.6 Hz), 4.24 (2H, s), 4.70 (1H, d, J = 13.6 Hz), 5.64 (1H, d, J = 4.0 Hz), 5.93 (1H, dd, J = 9.2, 4.0 Hz), 6.81 (1H, s), 7.17-7.40 (11H, m), 7.56 (1H, s), 7.63 (1H, d, J = 9.2 Hz), 8.20 (1H, s)

Reference Example 35

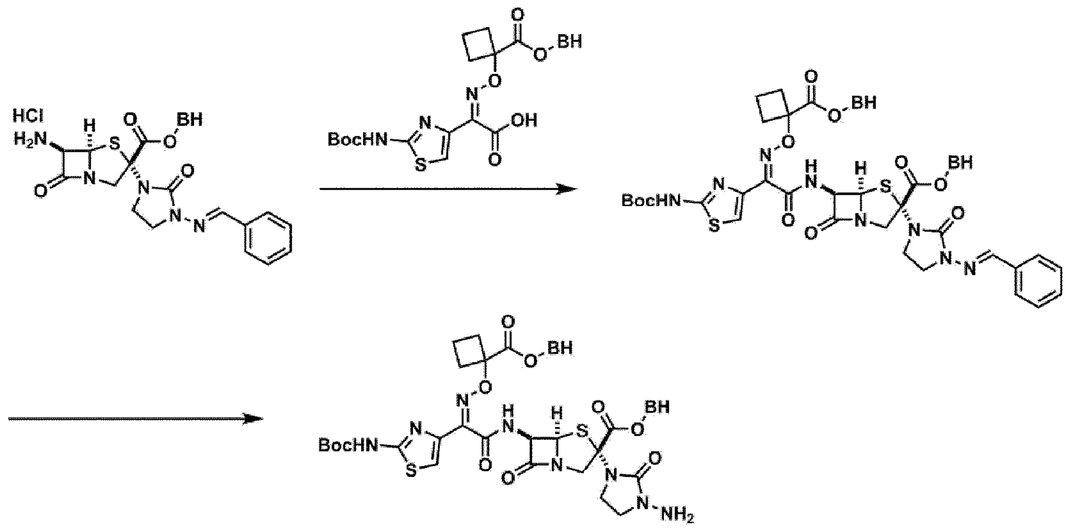

Reference Example 35 (1)

(Z)-2-((1-((benzhydryloxy)carbonyl)cyclobutoxy)imino)-2-(2-((tert-butoxycarbonyl)amino)thiazol-4-yl)acetic acid (191 mg), HOBt (51 mg), EDC (73 mg), NMM (84 μL), and DMF (2 mL) were sequentially added to benzhydryl (3R,5R,6R)-6-amino-3-(3-(((E)-benzylidene)amino)-2-oxoimidazolidin-1-yl)-7-oxo-4-thia-1-azabicyclo[3.2.0]heptane-3-carboxylate hydrochloride (200 mg). The reaction mixture was stirred at room temperature overnight. Ethyl acetate (10 mL) and water (10 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 40:60 → 80:20], thereby obtaining a target substance (159 mg) as light yellow solids.

Reference Example 35 (2)

Dichloromethane (3.2 mL) and methanol (1.6 mL) were added to the compound (159 mg) obtained in Reference Example 35 (1), and the mixture was stirred under ice cooling. At the same temperature, 2,4-dinitrophenylhydrazine (wetted with 50% water, 59 mg) and p-toluenesulfonic acid monohydrate (28 mg) were sequentially added to the reaction mixture. The reaction mixture was stirred at room temperature for 3 hours. Ethyl acetate (20 mL) and water (20 mL) were added to the reaction mixture. A saturated aqueous sodium hydrogen carbonate solution was added to the reaction mixture such that the pH was adjusted to 6.4. The organic layer was separated, washed with a saturated aqueous sodium chloride solution, and then dehydrated and dried over anhydrous sodium sulfate. The solvent was distilled off under reduced pressure, and the residue was purified by silica gel column chromatography [eluent; ethyl acetate:hexane = 70:30 → 100:0], thereby obtaining benzhydryl (3R,5R,6R)-3-(3-amino-2-oxoimidazolidin-1-yl)-6-((Z)-2-((1-((benzhydryloxy) carbonyl)cyclo butoxy)imino)-2-(2-((tert-butoxycarbonyl)amino)thiazol-4-yl)acetamido)-7-oxo-4-thia-1-azabi cyclo [3.2.0]heptane-3-carboxylate (104 mg) as light yellow solids.

NMR: 1.54 (9H, s), 1.92-2.02 (2H, m), 2.36-2.53 (2H, m), 2.56-2.67 (2H, m), 3.31-3.49 (4H, m), 3.52 (1H, d, J = 13.2 Hz), 3.81 (2H, s), 4.95 (1H, d, J = 13.2 Hz), 5.55 (1H, d, J = 4.0 Hz), 5.79 (1H, dd, J = 8.8, 3.6 Hz), 6.84 (1H, s), 6.90 (1H, s), 7.06 (1H, s), 7.09-7.15 (1H, m), 7.17-7.39 (20H, m), 8.13 (1H, s)

The compounds in Table 6 were obtained in the same manner as in Reference Example 35.

[Table 6]

| Ref. Ex. No. | Structural Formula | Name |
|---|---|---|
| 36 | 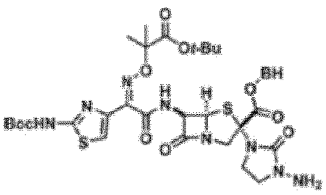 | Benzhydryl (3R,5R,6R)-3-(3-amino-2-oxoimidazolidin-1-yl)-6-((Z)-2-(((1-(tert-butoxy)-2-methyl-1-oxopropan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 37 | 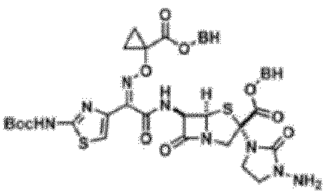 | Benzhydryl (3R,5R,6R)-3-(3-amino-2-oxoimidazolidin-1-yl)-6-((Z)-2-((1-((benzhydryloxy)carbonyl)cyclopropoxy)imino)-2-(2-((tert-butoxycarbonyl)amino)thiazol-4-yl)acetamido)-7-oxo-4-thia-1-azabicyclo[3.2.0]heptane-3-carboxylate |
| 38 | 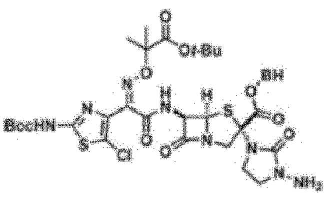 | Benzhydryl (3R,5R,6R)-3-(3-amino-2-oxoimidazolidin-1-yl)-6-((Z)-2-(((1-(tert-butoxy)-2-methyl-1-oxopropan-2-yl)oxy)imino)-2-(2-((tert-butoxycarbonyl)amino)-5-chlorothiazol-4-yl)acetamido)-7-oxo-4-thia-1-azabicyclo[3.2.0]heptane-3-carboxylate |
| 39 | 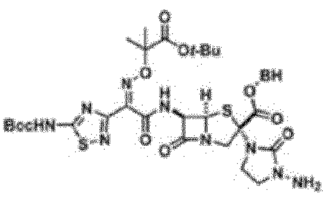 | Benzhydryl (3R,5R,6R)-3-(3-amino-2-oxoimidazolidin-1-yl)-6-((Z)-2-(((1-(tert-butoxy)-2-methyl-1-oxopropan-2-yl)oxy)imino)-2-(5-((tert-butoxycarbonyl)amino)-1,2,4-thiadiazol-3-yl)acetamido)-7-oxo-4-thia-1-azabicyclo[3.2.0]heptane-3-carboxylate |
| 40 | 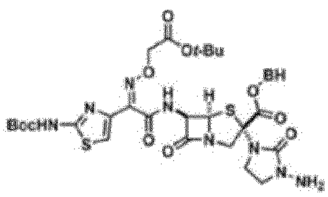 | Benzhydryl (3R,5R,6R)-3-(3-amino-2-oxoimidazolidin-1-yl)-6-((Z)-2-((2-(tert-butoxy)-2-oxoethoxy)imino)-2-(2-((tert-butoxycarbonyl)amino)thiazol-4-yl)acetamido)-7-oxo-4-thia-1-azabicyclo[3.2.0]heptane-3-carboxylate |

(continued)

| Ref. Ex. No. | Structural Formula | Name |
|---|---|---|
| 4 1 | 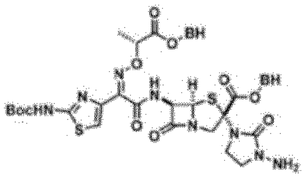 | Benzhydryl (3R,5R,6R)-3-(3-amino-2-oxoimidazolidin-1-yl)-6-((Z)-2-((((R)-1-(benzhydryloxy)-1-oxopropan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 4 2 | 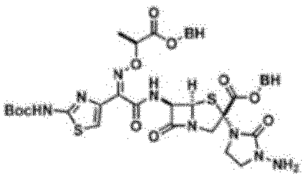 | Benzhydryl (3R,5R,6R)-3-(3-amino-2-oxoimidazolidin-1-yl)-6-((Z)-2-((((S)-1-(benzhydryloxy)-1-oxopropan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 4 3 | 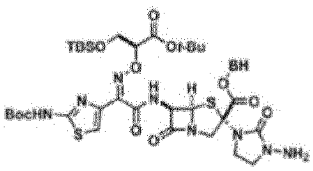 | Benzhydryl (3R,5R,6R)-3-(3-amino-2-oxoimidazolidin-1-yl)-6-((S,Z)-5-(tert-butoxycarbonyl)-2-(2 -((tert-butoxycarbonyl)amino) thiazol-4-yl)-8,8,9,9-tetramethyl-4,7-dioxa-3-aza-8-siladec-2-enamido)-7-oxo-4-thia-1-azabicyclo[3.2.0]heptane-3-carboxylate |
| 4 4 | 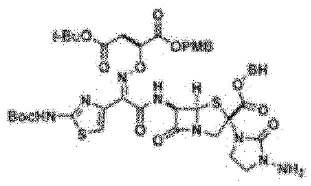 | 4-(tert-butyl)1-(4-methoxybenzyl)(S)-2-((((Z)-2-(((3R,5R,6R)-3-(3-amino-2-oxoimidazolidin-1-yl)-3-((benzhydryloxy)carbonyl)-7-oxo-4-thia-1-azabicyclo[3.2.0]heptan-6-yl)amino)-1-(2-{(tert-butoxycarbonyl)amino) thiazol-4-yl)-2-oxoethylidene)amino)oxy)succinic acid |
| 4 5 | 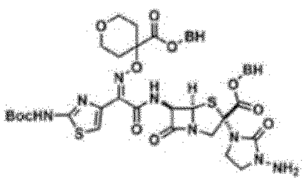 | Benzhydryl (3R,5R,6R)-3-(3-amino-2-oxoimidazolidin-1-yl)-6-((Z)-2-(((4-((benzhydryloxy)carbonyl)tetrahydro-2H-pyran-4-yl) oxy)imino)-2-(2-({tert-butoxycarbonyl)amino)thiazol-4-yl)acetamido)-7-oxo-4-thia-1-azabicyclo[3.2.0]heptane-3-carboxylate |
| 4 6 | 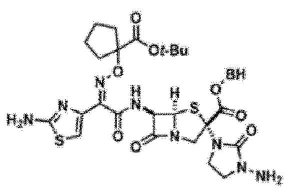 | Benzhydryl (3R,5R,6R)-3-(3-amino-2-oxoimidazolidin-1-yl)-6-((Z)-2-(2-aminothiazol-4-yl)-2-(((1-(tert-butoxycarbonyl)cyclopentyl)oxy)imino) acetamido)-7-oxo-4-thia-1-azabicyclo[3.2.0]heptane-3-carboxylate |

The measured values of NMR of the compounds in the table are as follows.

Reference Example 36

NMR: 1.39 (9H, s), 1.48-1.56 (15H, m), 3.37-3.57 (5H, m), 3.81 (2H, s), 4.95 (1H, d, J = 13.2 Hz), 5.58 (1H, d, J = 4.0 Hz), 5.79 (1H, dd, J = 8.0, 4.0 Hz), 6.87 (1H, s), 7.12-7.20 (1H, m), 7.21-7.41 (10H, m), 7.47 (1H, d, J = 8.0 Hz), 8.12 (1H, s)

Reference Example 37

NMR: 1.46-1.61 (4H, m), 1.54 (9H, s), 3.31-3.48 (4H, m), 3.50 (1H, d, J = 13.2 Hz), 3.82 (2H, s), 4.94 (1H, d, J = 13.2 Hz), 5.56 (1H, d, J = 3.6 Hz), 5.74 (1H, dd, J = 8.4, 3.6 Hz), 6.83 (1H, s), 6.88 (1H, s), 7.08-7.15 (1H, m), 7.16-7.43 (21H, m), 8.18 (1H, s)

Reference Example 38

NMR: 1.40 (9H, s), 1.50 (3H, s), 1.52 (3H, s), 1.52 (9H, s), 3.38-3.56 (5H, m), 3.81 (2H, s), 4.95 (1H, d, J = 13.2 Hz), 5.56 (1H, d, J = 4.0 Hz), 5.79 (1H, dd, J = 8.8, 3.2 Hz), 6.88 (1H, s), 7.18-7.39 (11H, m), 8.05 (1H, s)

Reference Example 39

NMR: 1.39 (9H, s), 1.55 (3H, s), 1.57 (3H, s), 1.57 (9H, s), 3.38-3.56 (5H, m), 3.83 (2H, s), 4.92 (1H, d, J = 13.2 Hz), 5.57 (1H, d, J = 3.6 Hz), 5.83 (1H, dd, J = 8.8, 3.6 Hz), 6.87 (1H, s), 7.12-7.18 (1H, m), 7.22-7.43 (10H, m), 8.58 (1H, s)

Reference Example 40

NMR: 1.41 (9H, s), 1.55 (9H, s), 3.37-3.58 (5H, m), 3.80 (2H, s), 4.57 (1H, d, J = 16.4 Hz), 4.63 (1H, d, J = 16.4 Hz), 5.00 (1H, d, J = 13.2 Hz), 5.60 (1H, d, J = 4.0 Hz), 5.66 (1H, dd, J = 7.2, 3.4 Hz), 6.87 (1H, s), 7.10-7.18 (1H, m), 7.20-7.41 (10H, m), 7.90 (1H, d, J = 7.2 Hz), 8.13 (1H, s)

Reference Example 41

NMR: 1.53 (3H, d, J = 7.2 Hz), 1.54 (9H, s), 3.25-3.49 (4H, m), 3.53 (1H, d, J = 13.2 Hz), 3.80 (2H, s), 4.96 (1H, d, J = 13.2 Hz), 5.02 (1H, dd, J = 14.0, 7.2 Hz), 5.55 (1H, d, J = 4.0 Hz), 5.78 (1H, dd, J = 9.2, 3.6 Hz), 6.82 (1H, s), 6.89 (1H, s), 7.05-7.12 (1H, m), 7.12-7.42 (21H, m), 8.12 (1H, s)

Reference Example 42

NMR: 1.51 (3H, d, J = 7.2 Hz), 1.55 (9H, s), 3.37-3.53 (5H, m), 3.83 (2H, s), 4.97-5.06 (2H, m), 5.53 (1H, d, J = 4.0 Hz), 5.69 (1H, dd, J = 7.6, 3.6 Hz), 6.86 (1H, s), 6.89 (1H, s), 7.06-7.12 (1H, m), 7.15-7.44 (21H, m), 8.08 (1H, s)

Reference Example 43

NMR: -0.01 (3H, s), 0.00 (3H, s), 0.82 (9H, s), 1.57 (9H, s), 3.25-3.60 (5H, m), 3.84 (2H, s), 4.06-4.18 (3H, m), 5.04-5.11 (2H, m), 5.46-5.53 (1H, m), 5.59 (1H, d, J = 4.0 Hz), 6.85 (1H, s), 6.92 (1H, s), 6.98-7.40 (20H, m), 7.72 (1H, d, J = 5.6 Hz), 8.10 (1H, s)

Reference Example 44

NMR: 1.40 (9H, s), 1.55 (9H, s), 2.83 (2H, d, J = 6.8 Hz), 3.34-3.59 (5H, m), 3.76 (3H, s), 3.81 (2H, s), 4.97 (1H, d, J = 12.0 Hz), 5.02 (1H, d, J = 13.2 Hz), 5.03 (1H, d, J = 12.0 Hz), 5.26 (1H, t, J = 6.4 Hz), 5.58 (1H, d, J = 3.6 Hz), 5.64 (1H, dd, J = 6.8, 3.6 Hz), 6.78 (1H, d, J = 8.8 Hz), 6.86 (1H, s), 7.12-7.40 (14H, m), 7.70 (1H, d, J = 7.2 Hz), 8.13 (1H, s)

Reference Example 45

NMR: 1.54 (9H, s), 2.09-2.29 (4H, m), 3.32-3.49 (4H, m), 3.52 (1H, dd, J = 13.2, 0.8 Hz), 3.67-3.85 (6H, m), 4.85 (1H, d, J = 13.2 Hz), 5.57 (1H, d, J = 4.0 Hz), 5.86 (1H, dd, J = 9.2, 3.6 Hz), 6.85 (1H, s), 6.90-6.93 (2H, m), 7.13-7.45 (21H, m), 8.15 (1H, s)

Reference Example 46

NMR: 1.38 (9H, s), 1.64-1.79 (4H, m), 2.02-2.25 (4H, m), 3.38-3.59 (5H, m), 3.82 (2H, s), 4.97 (1H, d, J = 13.2 Hz), 5.57 (1H, d, J = 3.6 Hz), 5.77 (1H, dd, J = 8.0, 3.6 Hz), 6.82 (1H, s), 6.87 (1H, s), 7.13-7.40 (11H, m)

Reference Example 47

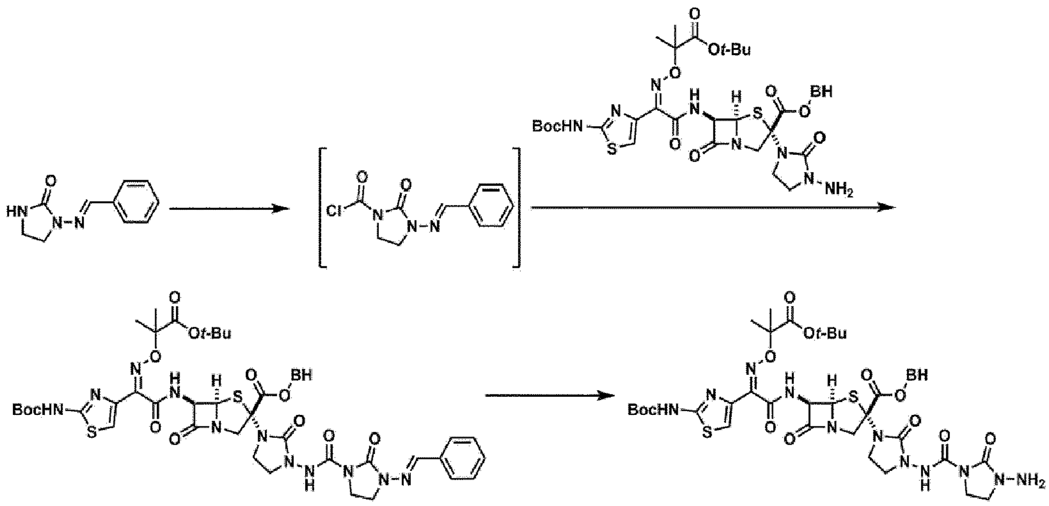

Reference Example 47 (1)

THF (2.3 mL) was added to (E)-1-(benzylideneamino)imidazolidin-2-one (230 mg), and the mixture was stirred under ice cooling. At the same temperature, triphosgene (180 mg) was added to the reaction mixture, and the reaction mixture was stirred at room temperature for 1 hour. Then, the reaction mixture was stirred at 60°C for 2 hours, thereby obtaining a THF mixture of (E)-3-(benzylideneamino)-2-oxoimidazolidine-1-carbonyl chloride.

THF (3 mL) and water (6 mL) were added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (300 mg), and the mixture was stirred under ice cooling. At the same temperature, sodium hydrogen carbonate (146 mg) and the prepared THF mixture of (E)-3-(benzylideneamino)-2-oxoimidazolidine-1-carbonyl chloride were sequentially added to the reaction mixture, and the reaction mixture was stirred for 1 hour. Ethyl acetate (15 mL) and water (15 mL) were added to the reaction mixture, and the organic layer was separated. The organic layer was washed with a 5% aqueous sodium chloride solution and then dehydrated and dried over anhydrous sodium sulfate. The solvent was distilled off under reduced pressure, and the residue was purified by silica gel column chromatography [eluent; chloroform:2-propanol = 100:0 → 90:10], thereby obtaining a target substance (66 mg) as a yellow oily substance.

Reference Example 47 (2)

By using the compound (66 mg) obtained in Reference Example 47 (1), benzhydryl (3R,5R,6R)-3-(3-(3-amino-2-oxoimidazolidine-1-carboxamido)-2-oxoimidazolidin-1-yl)-6-((Z )-2-(((1-(tert-butoxy)-2-methyl-1-oxopropan-2-yl)oxy) imino)-2-(2-((tert-butoxycarbonyl)amin o)thiazol-4-yl)acetamido)-7-oxo-4-thia-1-azabicyclo[3.2.0]heptane-3-carboxylate (20 mg) was obtained in the same manner as in Reference Example 27 (2) as a brown oily substance.

NMR: 1.39 (9H, s), 1.47-1.56 (15H, m), 3.48-3.63 (4H, m), 3.66-3.84 (7H, m), 5.03 (1H, d, J = 13.2 Hz), 5.59 (1H, d, J = 3.6 Hz), 5.78 (1H, dd, J = 8.8, 3.6 Hz), 6.86 (1H, s), 7.08-7.44 (11H, m), 9.43 (1H, s)

Reference Example 48

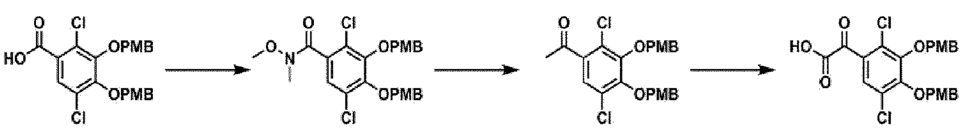

Reference Example 48 (1)

Dichloromethane (48 mL), N,O-dimethylhydroxylamine hydrochloride (1.64 g), and EDC (3.23 g) were sequentially added to 2,5-dichloro-3,4-bis((4-methoxybenzyl)oxy)benzoic acid (6.0 g). The reaction mixture was stirred at room temperature for 4 hours. Water (20 mL) was added to the reaction mixture, and the organic layer was separated. The organic layer was sequentially washed with water (20 mL), a 2% aqueous sodium chloride solution (20 mL), and a 10% aqueous sodium chloride solution (20 mL). The organic layer was dehydrated and dried over anhydrous magnesium sulfate, and then the solvent was distilled off under reduced pressure, thereby obtaining a target substance (3.7 g) as a yellow oily substance.

Reference Example 48 (2)

THF (40 mL) was added to the compound (3.7 g) obtained in Reference Example 48 (1), and the mixture was stirred under ice cooling. At the same temperature, a 3 mol/L methyl magnesium bromide/diethyl ether solution (5.5 mL) was added to the reaction mixture, and the reaction mixture was stirred at room temperature for 2 hours and 30 minutes. At the same temperature, a 3 mol/L methyl magnesium bromide/diethyl ether solution (2.7 mL) was added to the reaction mixture, and the reaction mixture was stirred at room temperature for 2 hours. A 3 mol/L methyl magnesium bromide/-diethyl ether solution (2.7 mL) was added to the reaction mixture, and the reaction mixture was stirred at room temperature for 1 hour. A saturated aqueous ammonium chloride solution, ethyl acetate, and 6 mol/L hydrochloric acid were sequentially added to the reaction mixture such that the pH was adjusted to 5.0. The organic layer was separated and washed with a saturated aqueous sodium chloride solution. The organic layer was dehydrated and dried over anhydrous magnesium sulfate, and then the solvent was distilled off under reduced pressure, thereby obtaining a target substance (1.62 g) as light yellow solids.

Reference Example 48 (3)

Pyridine (16 mL) and selenium dioxide (0.98 g) were sequentially added to the compound (1.62 g) obtained in Reference Example 48(2). The reaction mixture was stirred at a temperature of 90°C to 100°C for 5 hours and 30 minutes. The reaction mixture was filtered through celite, and the residue was sequentially washed with water and ethyl acetate. The organic layer was separated, 6 mol/L hydrochloric acid was added to the aqueous layer such that the pH was adjusted to a value lower than 2, and extraction was performed using ethyl acetate. The organic layers were combined and dehydrated and dried over anhydrous magnesium sulfate, and the solvent was distilled off under reduced pressure. The residue was purified by silica gel column chromatography [eluent; methanol:chloroform = 0:100 → 30:70]. The fraction containing a target substance was concentrated under reduced pressure, and ethyl acetate and IPE were added to the residue. Solids were collected by filtration, thereby obtaining 2-(2,5-dichloro-3,4-bis((4-methoxybenzyl)oxy)phenyl)-2-oxoacetic acid (200 mg) as light brown solids.

NMR (DMSO-$d_6$): 3.77 (3H, s), 3.78 (3H, s), 4.98 (2H, s), 5.07 (2H, s), 6.91-6.99 (5H, m), 7.34-7.43 (4H, m), 7.60 (1H, s)

Reference Example 49

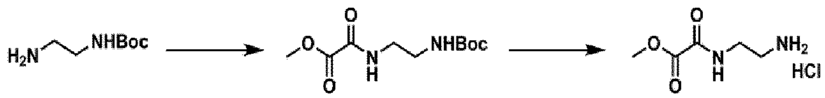

Reference Example 49 (1)

Dichloromethane (150 mL) and triethylamine (4.48 mL) were added to tert-butyl(2-aminoethyl)carbamate (5.0 g), and the mixture was stirred under ice cooling. At the same temperature, methyl chloroglyoxylate (2.95 mL) was added to the reaction mixture, and the reaction mixture was stirred at room temperature for 6 hours and 30 minutes. Water (100 mL) was added to the reaction mixture, and 1 mol/L hydrochloric acid was added thereto such that the pH was adjusted to 2.6. The organic layer was separated, and the aqueous layer was extracted twice by using dichloromethane (50 mL). The organic layers were combined and sequentially washed with water and a saturated aqueous sodium chloride solution. The organic layer was dehydrated and dried over anhydrous sodium sulfate, and then the solvent was distilled off under reduced pressure, thereby obtaining a target substance (7.1 g) as white solids.

Reference Example 49 (2)

Ethyl acetate (210 mL) was added to the compound (7.1 g) obtained in Reference Example 49 (1), and the mixture was stirred under ice cooling. At the same temperature, a 4 mol/L hydrochloric acid/ethyl acetate solution (71 mL) was added to the reaction mixture, and the reaction mixture was stirred at room temperature for 8 hours. Solids were collected by filtration and washed with ethyl acetate. The solids were dried, thereby obtaining methyl 2-((2-aminoethyl) amino)-2-oxoacetate hydrochloride (6.42g) as white solids.
NMR ($D_2O$): 3.23 (2H, t, J = 6.0 Hz), 3.64 (2H, t, J = 6.0 Hz), 3.91 (3H, s)
The compounds in Table 7 were obtained in the same manner as in Reference Example 49.

[Table 7]

| Ref. Ex. No. | Structural Formula | Name |
|---|---|---|
| 5 0 | 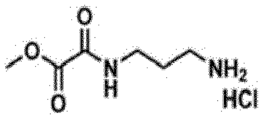 | Methyl 2-((3-aminopropyl)amino)-2-oxoacetate hydrochloride |
| 5 1 | 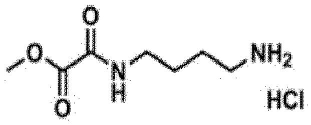 | Methyl 2-((4-aminobutyl)amino)-2-oxoacetate hydrochloride |

The measured values of NMR of the compounds in the table are as follows.

Reference Example 50

NMR ($D_2O$): 1.96 (2H, quintet, J = 7.2 Hz), 3.04 (2H, t, J = 7.8 Hz), 3.42 (2H, t, J = 6.8 Hz), 3.91 (3H, s)

Reference Example 51

NMR ($D_2O$): 1.61-1.77 (4H, m), 3.03 (2H, t, J = 7.2 Hz), 3.35 (2H, t, J = 6.4 Hz), 3.90 (3H, s)

Reference Example 52

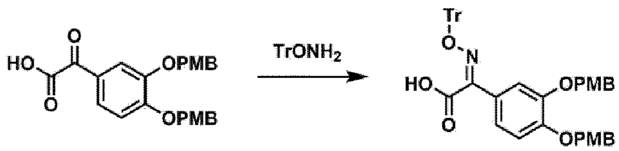


Methanol (25 mL) and O-tritylhydroxylamine (3.58 g) were added to 2-(3,4-bis((4-methoxybenzyl)oxy)phenyl)-2-oxoacetic acid (5.00 g), and the mixture was stirred at room temperature for 1 hour and 30 minutes. Water (125 mL) was added to the reaction mixture, the reaction mixture was stirred at room temperature for 30 minutes, and solids were collected by filtration. The solids were dried, thereby obtaining (Z)-2-(3,4-bis((4-methoxybenzyl)oxy)phenyl)-2-((trityloxy) imino)acetic acid (7.84 g) as yellow solids.
NMR (DMSO-$d_6$): 3.72 [3.74] (3H, s), 3.74 [3.76] (3H, s), 4.90 [4.92] (2H, s), 5.04 [5.11] (2H, s), 6.81-6.99 (5H, m), 7.05-7.11 (1H, m), 7.15-7.44 (20H, m)
The compounds in Table 8 were obtained in the same manner as in Reference Example 52.

[Table 8]

| Ref. Ex. No. | Structural Formula | Name |
|---|---|---|
| 5 3 | 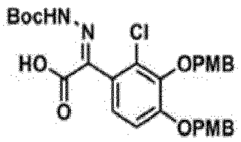 | (Z)-2-(2-(tert-butoxycarbonyl)hydradienylidene)-2-(2-chloro-3,4-bis((4-methoxybenzyl)oxy)phenyl) acetic acid |
| 5 4 | 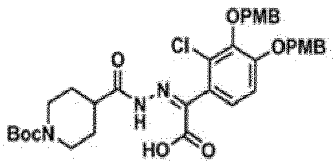 | (Z)-2-(2-(1-(tert-butoxycarbonyl)piperidine-4-carbonyl) hydradienylidene)-2-(2-chloro-3,4-bis((4-methoxybenzyl)oxy)phenyl) acetic acid |

The measured values of NMR of the compounds in the table are as follows.

Reference Example 53

NMR (DMSO-$d_6$): 1.44 [1.47] (9H, s), 3.75 [3.75] (3H, s), 3.78 [3.78] (3H, s), 4.89 (2H, s), 5.15 (2H, s), 6.83-6.91 (2H, m), 6.93-7.04 (2H, m), 7.07-7.25 (2H, m), 7.27-7.37 (2H, m), 7.40-7.49 (2H, m), 9.90 (1H, s), 12.40-12.44 (1H, brs)

Reference Example 54

NMR (DMSO-$d_6$): 1.16-1.46 (2H, m), 1.39 (9H, s), 1.47-1.74 (2H, m), 2.70-2.90 (1H, m), 3.72-4.05 (4H, m), 3.75 (3H, s), 3.78 (3H, s), 4.89 (2H, s), 5.16 (2H, s), 6.83-6.91 (2H, m), 6.95-7.09 (3H, m), 7.14-7.36 (3H, m), 7.40-7.50 (2H, m), 10.33-10.60 (1H, brs)

The compounds in Table 9 were obtained in the same manner as in Reference Example 3.

[Table 9]

| Ref.Ex. No. | Structural Formula | Name |
|---|---|---|
| 5 5 | 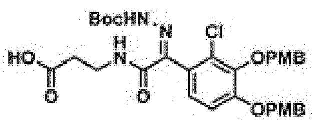 | (Z)-3-(2-(2-(tert-butoxycarbonyl)hydradienylidene)-2-(2-chloro-3,4-bis((4-methoxybenzyl)oxy)phenyl)acetamido)propionic acid |
| 5 6 | 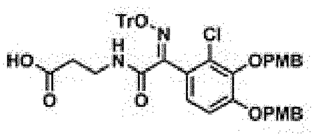 | (Z)-3-(2-(2-chloro-3,4-bis((4-methoxybenzyl)oxy)phenyl)-2-((trityloxy)imino)acetamido)propionic acid |
| 5 7 | 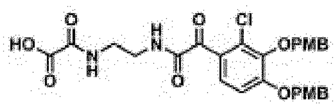 | 2-((2-(2-(2-chloro-3,4-bis((4-methoxybenzyl)oxy)phenyl)-2-oxoacetamido)ethyl)amino)-2-oxcacetic acid |
| 5 8 | 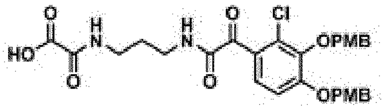 | 2-((3-(2-(2-chloro-3,4-bis((4-methoxybenzyl)oxy)phenyl)-2-oxcacetamido)propyl)amino)-2-oxoacetic acid |
| 5 9 | 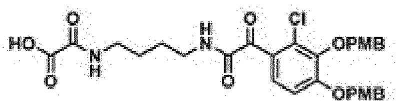 | 2-((4-(2-(2-chloro-3,4-bis((4-methoxybenzyl)oxy)phenyl)-2-oxoacetamido)butyl)amino)-2-oxoacetic acid |

(continued)

| Ref.Ex. No. | Structural Formula | Name |
|---|---|---|
| 6 0 | 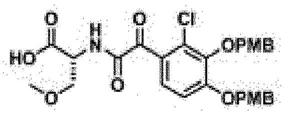 | N-(2-(2-chloro-3,4-bis((4-methoxybenzyl)oxy)phenyl)-2-oxoacetyl)-O-methyl-D-serine |

The measured values of NMR of the compounds in the table are as follows.

Reference Example 55

NMR (DMSO-$d_6$): 1.44 [1.46] (9H, s), 2.37-2.49 (2H, m), 3.25-3.35 (1H, m), 3.35-3.43 (1H, m), 3.75 [3.75] (3H, s), 3.78 (3H, s), 4.81-4.98 (2H, m), 5.15 [5.16] (2H, s), 6.84-6.93 (3H, m), 6.95-7.04 (2H, m), 7.15-7.37 (3H, m), 7.40-7.49 (2H, m), 7.83-7.93 (1H, m), 9.91 (1H, s), 12.26 [12.48] (1H, s)

Reference Example 56

NMR (DMSO-$d_6$): 1.97 [2.20] (2H, t, J = 6.2 Hz), 3.12-3.21 (2H, m), 3.66 [3.74] (3H, s),3.76 [3.78] (3H, s), 4.81 [4.93] (2H, s), 5.10 [5.20] (2H, s), 6.76-6.88 (2H, m), 6.93-7.03 (2H, m), 7.10-7.18 (4H, m), 7.18-7.37 (15H, m), 7.37-7.51 (2H, m), 8.03 (1H, t, J = 5.4 Hz)

Reference Example 57

NMR: 3.58-3.65 (4H, m), 3.79 (3H, s), 3.83 (3H, s), 4.94 (2H, s), 5.09 (2H, s), 6.78-6.84 (2H, m), 6.89-6.97 (3H, m), 7.29-7.37 (5H, m), 7.41 (1H, s), 7.60 (1H, d, J = 8.8 Hz), 7.86 (1H, s)

Reference Example 58

NMR: 1.83-1.91 (2H, m), 3.42-3.51 (4H, m), 3.80 (3H, s), 3.84 (3H, s), 5.00 (2H, s), 5.12 (2H, s), 6.80-6.86 (2H, m), 6.88-7.00 (3H, m), 7.22-7.39 (6H, m), 7.62 (1H, d, J = 8.8 Hz), 7.90 (1H, s)

Reference Example 59

NMR: 1.65-1.73 (4H, m), 3.40-3.47 (4H, m), 3.80 (3H, s), 3.84 (3H, s), 4.96 (2H, s), 5.12 (2H, s), 6.81-6.85 (2H, m), 6.90-6.97 (3H, m), 7.03-7.10 (1H, m), 7.23-7.39 (6H, m), 7.62 (1H, d, J = 8.4 Hz)

Reference Example 60

NMR: 3.42 (3H, s), 3.72 (1H, dd, J = 9.4, 3.8 Hz), 3.80 (3H, s), 3.83 (3H, s), 3.98 (1H, dd, J = 9.6, 3.2 Hz), 4.80 (1H, dt, J = 8.0, 3.6 Hz), 4.96 (2H, s), 5.11 (2H, s), 6.80-6.85 (2H, m), 6.90-6.97 (3H, m), 7.31-7.37 (5H, m), 7.61 (1H, d, J = 8.8 Hz), 7.68 (1H, d, J = 8.0 Hz)

Reference Example 61

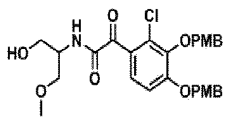

In the same manner as in Reference Example 3 (1), 2-(2-chloro-3,4-bis((4-methoxybenzyl)oxy)phenyl)-N-(1-hydroxy-3-methoxypropan-2-yl)-2-o xoacetamide was obtained as a light yellow oily substance.

NMR: 3.41 (3H, s), 3.63 (1H, dd, J = 9.6, 4.4 Hz), 3.70 (1H, dd, J = 9.6, 4.0 Hz), 3.76-3.83 (1H, m), 3.80 (3H, s), 3.84 (3H, s), 3.93 (1H, dd, J = 11.6, 4.0 Hz), 4.09-4.18 (1H, m), 4.96 (2H, s), 5.11 (2H, s), 6.80-6.86 (2H, m), 6.90-6.98 (3H, m), 7.31-7.38 (4H, m), 7.51 (1H, d, J = 8.4 Hz), 7.60 (1H, d, J = 8.4 Hz)

Reference Example 62

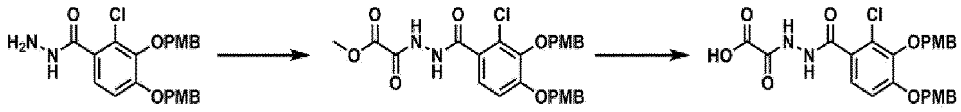

Reference Example 62 (1)

Dichloromethane (45 mL) and triethylamine (991 μL) were added to 2-chloro-3,4-bis ((4-methoxybenzyl)oxy) benzohydrazide (3.0 g), and the mixture was stirred under ice cooling. At the same temperature, methyl chloroglyoxylate (654 μL) was added to the reaction mixture, and the reaction mixture was stirred at room temperature overnight. Water (60 mL) was added to the reaction mixture, and solids were collected by filtration. The solids were washed twice with dichloromethane (5 mL), thereby obtaining a target substance (3.42 g) as white solids.

Reference Example 62 (2)

THF (68 mL), water (34 mL), and lithium hydroxide monohydrate (1.36 g) were sequentially added to the compound (3.42 g) obtained in Reference Example 62 (1), and the mixture was stirred at room temperature for 1 hour. Water (250 mL) and 2 mol/L hydrochloric acid were added to the reaction mixture under ice cooling such that the pH was adjusted to 1.9. Solids were collected by filtration, thereby obtaining 2-((2-(2-chloro-3,4-bis((4-methoxybenzyl)oxy) benzoyl)hydradienyl)-2-oxoacetic acid (1.43 g) as white solids.

NMR (DMSO-$d_6$): 3.75 (3H, s), 3.78 (3H, s), 4.90 (2H, s), 5.17 (2H, s), 6.85-6.90 (2H, m), 6.96-7.01 (2H, m), 7.20-7.28 (2H, m), 7.29-7.34 (2H, m), 7.42-7.47 (2H, m), 10.31 (1H, s), 10.78 (1H, s), 13.67-14.62 (1H, brs)

Reference Example 63

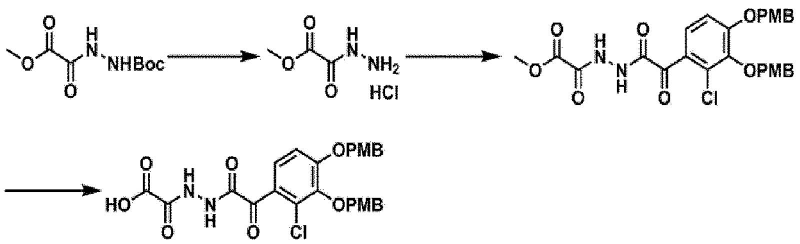

Reference Example 63 (1)

Ethyl acetate (64 mL) was added to tert-butyl 2-(2-methoxy-2-oxoacetyl)hydrazine-1-carboxylate (4.23 g), and the mixture was stirred under ice cooling. At the same temperature, a 4 mol/L hydrochloric acid/ethyl acetate solution (42 mL) was added to the reaction mixture, and the reaction mixture was stirred at room temperature for 6 hours. Solids were collected by filtration and washed with ethyl acetate. The solids were dried, thereby obtaining a target substance (2.65 g) as white solids.

Reference Example 63 (2)

2-(2-Chloro-3,4-bis((4-methoxybenzyl)oxy)phenyl)-2-oxoacetic acid (4.0 g), HOBt (1.42 g), EDC (2.01 g), DMAC (80 mL), and NMM (4.4 mL) were sequentially added to the compound (1.35 g) obtained in Reference Example 63 (1). The reaction mixture was stirred at room temperature overnight. Ethyl acetate (160 mL) and water (160 mL) were added to the reaction mixture. Hydrochloric acid (1 mol/L) was added to the reaction mixture such that the pH was adjusted to 3.5. The organic layer was separated, washed twice with water (100 mL), and sequentially washed with a saturated aqueous sodium hydrogen carbonate solution (100 mL) and a saturated aqueous sodium chloride solution (100 mL). The organic layer was dehydrated and dried over anhydrous sodium sulfate, and the solvent was distilled off under reduced pressure. Dichloromethane (15 mL) and hexane (15 mL) were added to the residue, and solids were collected by filtration,

thereby obtaining a target substance (1.60 g) as white solids.

Reference Example 63 (3)

By using the compound (1.60 g) obtained in Reference Example 63 (2), 2-(2-(2-(2-chloro-3,4-bis((4-methoxybenzyl)oxy)phenyl)-2-oxoacetyl)hydradienyl)-2-oxoaceti c acid (1.43 g) was obtained as white solids in the same manner as in Reference Example 62 (2).

NMR (DMSO-$d_6$): 3.74 (3H, s), 3.78 (3H, s), 4.91 (2H, s), 5.26 (2H, s), 6.83-6.88 (2H, m), 6.97-7.02 (2H, m), 7.26-7.32 (2H, m), 7.37 (1H, d, J = 8.8 Hz), 7.43-7.50 (3H, m), 7.74 (1H, d, J = 8.8 Hz), 10.70-10.92 (1H, brs), 10.95 (1H, s)

Reference Example 64

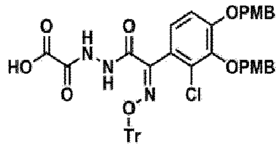

In the same manner as in Reference Example 63, (Z)-2-(2-(2-(2-chloro-3,4-bis((4-methoxybenzyl)oxy)phenyl)-2-((trityloxy)imino)acetyl)hydra dienyl)-2-oxoacetic acid was obtained as white solids.

NMR: 3.73 (3H, s), 3.84 (3H, s), 5.01 (2H, s), 5.11 (2H, s), 6.76-6.81 (2H, m), 6.90-7.05 (4H, m), 7.20-7.41 (20H, m), 8.47 (1H, s), 9.26-9.48 (1H, brs)

Reference Example 65

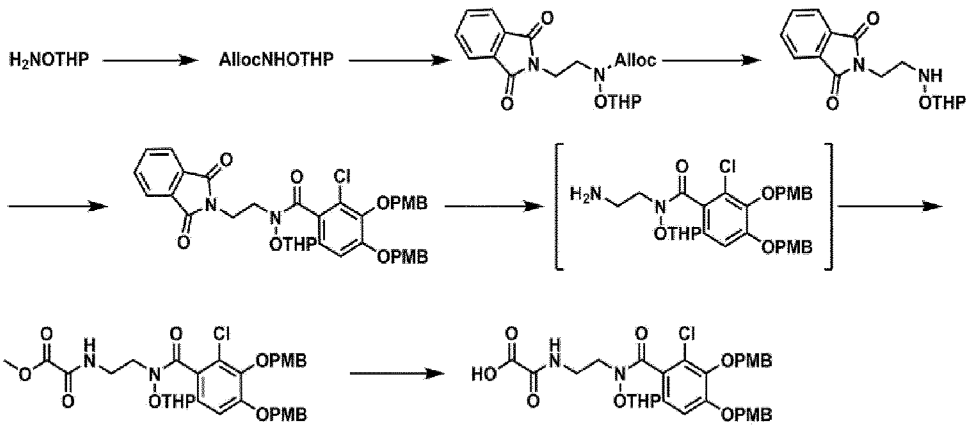

Reference Example 65 (1)

THF (70 mL), water (70 mL), and sodium hydrogen carbonate (9.8 g) were sequentially added to O-(tetrahydro-2H-pyran-2-yl)hydroxylamine (7.5 g), and the mixture was stirred under ice cooling. At the same temperature, allyl chloroformate (6.2 mL) was added to the reaction mixture, and the reaction mixture was stirred at room temperature for 1 hour. Ethyl acetate (50 mL) and water (50 mL) were added to the reaction mixture. Hydrochloric acid (2 mol/L) was added to the reaction mixture such that the pH was adjusted to 2.3. The organic layer was separated and washed with a saturated aqueous sodium chloride solution. The organic layer was dehydrated and dried over anhydrous sodium sulfate. The solvent was distilled off under reduced pressure, thereby obtaining a target substance (12.4 g) as a colorless oily substance.

Reference Example 65 (2)

2-(2-Hydroxyethyl)isoindoline-1,3-dione (3.2 g), triphenylphosphine (5.35 g), and toluene (30 mL) were added to

the compound (3.0 g) obtained in Reference Example 65 (1), and the mixture was stirred under ice cooling. At the same temperature, a 40% diisopropyl azodicarboxylate/toluene solution (9.1 mL) was added dropwise to the reaction mixture, and the reaction mixture was stirred at room temperature overnight. Magnesium chloride (3.54 g) was added to the reaction mixture, and the reaction mixture was stirred at 60°C for 1 hour. At the same temperature, hexane (30 mL) was added to the reaction mixture, and the reaction mixture was stirred for 1 hour at the same temperature. The reaction mixture was cooled to room temperature, and insoluble matters were filtered. The residue was washed with toluene (20 mL), and the solvent was distilled off under reduced pressure. The residue was purified by silica gel column chromatography [eluent; ethyl acetate:hexane = 10:90 → 40:60], and the fraction containing a target substance was concentrated under reduced pressure. The residue was purified by silica gel column chromatography [eluent; chloroform], thereby obtaining a target substance (4.02 g) as a colorless oily substance.

Reference Example 65 (3)

By using the compound (1.5 g) obtained in Reference Example 65 (2), a target substance (592 mg) was obtained as a colorless oily substance in the same manner as in Reference Example 10 (2).

Reference Example 65 (4)

By using the compound (592 mg) obtained in Reference Example 65 (3), a target substance (1.41 g) was obtained as white solids in the same manner as in Reference Example 2 (1).

Reference Example 65 (5)

THF (7 mL), methanol (7 mL), and hydrazine hydrate (485 μL) were added to the compound (700 mg) obtained in Reference Example 65 (4), and the mixture was stirred at 70°C for 1 hour. The reaction mixture was cooled to room temperature. The reaction mixture was filtered, and the residue was washed with dichloromethane (20 mL). Water was added to the filtrate, and the organic layer was separated. The aqueous layer was extracted twice by using dichloromethane (10 mL). The organic layers were combined and sequentially washed with water and a saturated aqueous sodium chloride solution. The organic layer was dehydrated and dried over anhydrous sodium sulfate, and the solvent was distilled off under reduced pressure until the amount of the solvent became about 50 mL. Triethylamine (143 μL) and methyl chloroglyoxylate (92 μL) were added to the reaction mixture under ice cooling, and the reaction mixture was stirred at room temperature overnight. Water (30 mL) was added to the reaction mixture, and the organic layer was separated. The aqueous layer was extracted twice by using dichloromethane (10 mL). The organic layers were combined and sequentially washed with water and a saturated aqueous sodium chloride solution. The organic layer was dehydrated and dried over anhydrous sodium sulfate, and the solvent was distilled off under reduced pressure. The residue was purified by silica gel column chromatography [eluent; ethyl acetate:hexane = 40:60 → 60:40], thereby obtaining a target substance (280 mg) as white solids.

Reference Example 65 (6)

By using the compound (280 mg) obtained in Reference Example 65 (5), 2-((2-(2-chloro-3,4-bis((4-methoxybenzyl)oxy)-N-((tetrahydro-2H-pyran-2-yl)oxy)benzamido )ethyl)amino)-2-oxoacetic acid (276 mg) was obtained as white solids in the same manner as in Reference Example 62 (2).

NMR: 1.30-1.88 (6H, m), 3.52-3.78 (3H, m), 3.80 (3H, s), 3.83 (3H, s), 3.85-4.21 (3H, m), 4.60-4.90 (1H, m), 4.96 (2H, s), 5.08 (2H, s), 6.81-6.86 (2H, m), 6.87-6.94 (3H, m), 6.95-7.01 (1H, m), 7.32-7.37 (5H, m), 8.20 (1H, s)

Reference Example 66

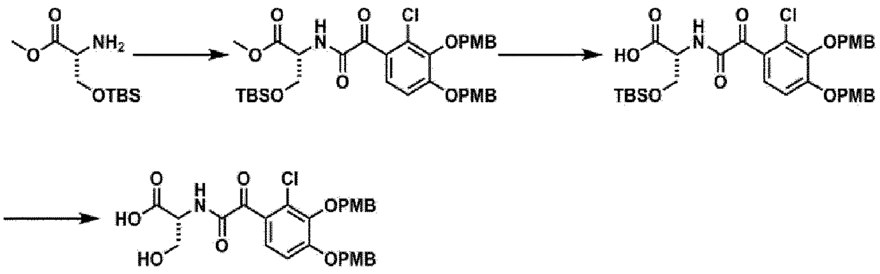

Reference Example 66 (1)

By using methyl O-(tert-butyldimethylsilyl)-D-serinate (2.3 g), a target substance (2.16 g) was obtained as a light yellow oily substance in the same manner as in reference Example 11.

Reference Example 66 (2)

By using the compound (2.16 g) obtained in Reference Example 66 (1), a target substance (1.92 g) was obtained as green solids in the same manner as in Reference Example 62 (2).

Reference Example 66 (3)

THF (8 mL) was added to the compound (400 mg) obtained in Reference Example 66 (2), and the mixture was stirred under ice cooling. At the same temperature, a 1 mol/L tetra-n-butylammonium fluoride/THF solution (912 μL) was added to the reaction mixture, and the reaction mixture was stirred at room temperature overnight. Ethyl acetate (10 mL) and water (10 mL) were added to the reaction mixture, and the organic layer was separated. The aqueous layer was extracted twice by using ethyl acetate (10 mL). The organic layer was washed with a saturated aqueous sodium chloride solution and dehydrated and dried over anhydrous sodium sulfate. The solvent was distilled off under reduced pressure, thereby obtaining (2-(2-chloro-3,4-bis((4-methoxybenzyl)oxy)phenyl)-2-oxoacetyl)-D-serine (313 mg) as green solids.

NMR: 3.74 (3H, s), 3.79 (3H, s), 3.83 (1H, s), 3.89-3.97 (1H, m), 4.17 (1H, dd, J = 11.6, 3.6 Hz), 4.60-4.66 (1H, m), 4.84 (2H, s), 4.96 (2H, s), 6.75 (2H, d, J = 8.8 Hz), 6.81-6.88 (3H, m), 7.20-7.38 (5H, m), 7.56 (1H, d, J = 8.4 Hz), 8.15 (1H, d, J = 7.2 Hz)

Reference Example 67

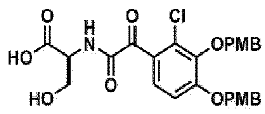

(2-(2-Chloro-3,4-bis((4-methoxybenzyl)oxy)phenyl)-2-oxoacetyl)-L-serine was obtained in the same manner as in Reference Example 66.

NMR: 3.75 (3H, s), 3.80 (3H, s), 3.83 (1H, s), 3.92-4.00 (1H, m), 4.15-4.22 (1H, m), 4.65-4.71 (1H, m), 4.86 (2H, s), 4.98 (2H, s), 6.74-6.78 (3H, m), 6.83-6.90 (4H, m), 7.22-7.38 (3H, m), 7.56 (1H, d, J = 8.8 Hz), 8.06 (1H, d, J = 6.8 Hz)

Reference Example 68

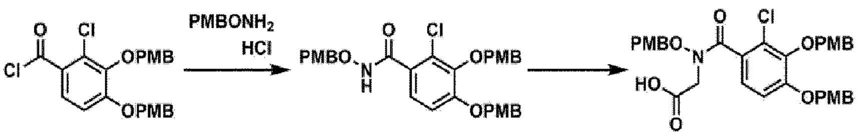

Reference Example 68 (1)

By using O-(4-methoxybenzyl)hydroxylamine hydrochloride (500 mg), a target substance (1.38 g) was obtained as white solids in the same manner as in Reference Example 2 (1).

Reference Example 68 (2)

Potassium carbonate (686 mg), acetone (3.5 mL), and 2-bromoacetic acid (345 mg) were sequentially added to the compound (700 mg) obtained in Reference Example 68 (1), and the mixture was stirred at room temperature overnight. DMF (7 mL), potassium carbonate (686 mg), and 2-bromoacetic acid (345 mg) were added to the reaction mixture, and the reaction mixture was stirred at room temperature for 2 days. Ethyl acetate (30 mL), water (30 mL), and 1 mol/L hydrochloric acid were added to the reaction mixture such that the pH was adjusted to 2.3. The organic layer was separated and washed with water and a saturated aqueous sodium chloride solution. The organic layer was dehydrated and dried over anhydrous sodium sulfate, and the solvent was distilled off under reduced pressure. The residue was purified by silica gel column chromatography [eluent; ethyl acetate:hexane = 34:66 → 66:34], thereby obtaining N-(2-chloro-3,4-bis((4-methoxybenzyl)oxy)benzoyl)-N-((4-methoxybenzyl)oxy)glycine (114 mg) as a colorless oily substance.

NMR: 3.78-3.82 (6H, m), 3.83 (3H, s), 4.91-5.00 (4H, m), 5.02-5.12 (4H, m), 6.88-7.00 (11H, m), 7.21-7.40 (4H, m)

Reference Example 69

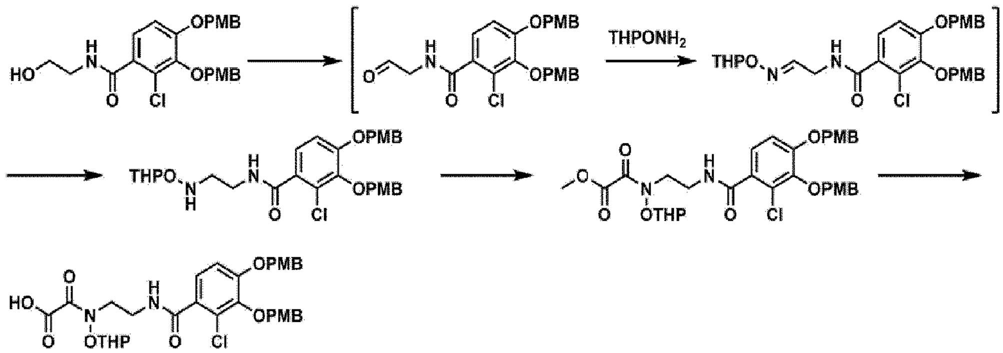


Reference Example 69 (1)

By using 2-chloro-N-(2-hydroxyethyl)-3,4-bis((4-methoxybenzyl)oxy)benzamide (2.0 g), a target substance (642 mg) was obtained as light yellow solids in the same manner as in Example 65 (1).

Reference Example 69 (2)

By using the compound (500 mg) obtained in Reference Example 69 (1), a target substance (607 mg) was obtained as light yellow solids in the same manner as in Reference Example 49 (1).

Reference Example 69 (3)

By using the compound (500 mg) obtained in Reference Example 69 (2), 2-((2-(2-chloro-3,4-bis((4-methoxybenzyl)oxy)benzamido)ethyl)((tetrahydro-2H-pyran-2-yl)o xy)amino)-2-oxoacetic acid (496 mg) was obtained as yellow solids in the same manner as in Reference Example 62 (2).

NMR: 1.45-1.57 (4H, m), 1.65-1.82 (2H, m), 3.15-3.21 (2H, m), 3.48-3.57 (1H, m), 3.63-3.70 (2H, m), 3.80 (3H, s), 3.83 (3H, s), 3.85-3.93 (1H, m), 4.73-4.78 (1H, m), 4.94 (2H, s), 5.08 (2H, s), 6.81-6.86 (2H, m), 6.89-6.94 (3H, m), 6.94-7.00 (1H, m), 7.30-7.38 (5H, m), 7.42 (1H, d, J = 8.8 Hz)

Reference Example 70

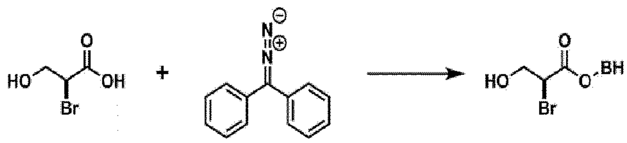

THF (14 mL) was added to (S)-2-bromo-3-hydroxypropionic acid (4.4 g), and the mixture was stirred under ice cooling. At the same temperature, diphenylmethyldiazomethane (4.6 g) in a THF (24 mL) solution was added dropwise to the reaction mixture for 40 minutes. The reaction mixture was stirred at room temperature overnight. Ethyl acetate (22 mL) and water (11 mL) were added to the reaction mixture. A saturated aqueous sodium hydrogen carbonate solution (11 mL) was added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining benzhydryl (S)-2-bromo-3-hydroxypropanoate (7.52 g) as a light yellow oily substance.

NMR: 2.35 (1H, t, J = 7.2 Hz), 3.92-4.01 (1H, m), 4.02-4.11 (1H, m), 4.43-4.49 (1H, m), 6.91 (1H, s), 7.28-7.40 (10H, m)

Reference Example 71

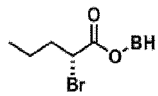

Benzhydryl (R)-2-bromopentanoate was obtained in the same manner as in Reference Example 70.

NMR: 0.91 (3H, t, J = 7.4 Hz), 1.22-1.52 (2H, m), 1.93-2.12 (2H, m), 4.33 (1H, dd, J = 8.0, 6.8 Hz), 6.89 (1H, s), 7.26-7.40 (10H, m)

Reference Example 72

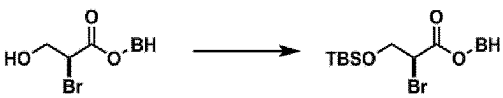

THF (75 mL) was added to benzhydryl (S)-2-bromo-3-hydroxypropanoate (7.52 g), and the mixture was stirred under ice cooling. At the same temperature, Imidazole (1.68 g), tert-butyl dimethylchlorosilane (3.83 g), and DMF (7.5 mL) were sequentially added to the reaction mixture, and the reaction mixture was stirred at room temperature overnight. Ethyl acetate (180 mL) and water (90 mL) were added to the reaction mixture, and the organic layer was separated. The organic layer was sequentially washed with water and a saturated aqueous sodium chloride solution and dehydrated and dried over anhydrous magnesium sulfate. The solvent was distilled off under reduced pressure. The residue was purified by silica gel column chromatography [eluent; ethyl acetate:hexane = 0:100 → 20:80], thereby obtaining benzhydryl (S)-2-bromo-3-((tert-butyldimethylsilyl)oxy)propanoate (7.0 g) as a light yellow oily substance.

NMR: -0.01 (3H, s), 0.03 (3H, s), 0.81 (9H, s), 3.88-3.96 (1H, m), 4.08-4.16 (1H, m), 4.28-4.35 (1H, m), 6.91 (1H, s), 7.27-7.38 (10H, m)

Reference Example 73

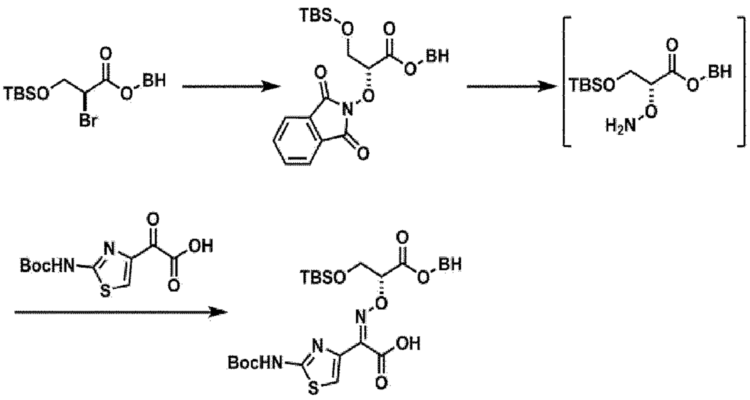

Reference Example 73 (1)

At room temperature, DMF (46mL), N-hydroxyphthalimide (4.73g), and triethylamine (3.24mL) were sequentially added to benzhydryl (S)-2-bromo-3-((tert-butyldimethylsilyl)oxy)propanoate (6.52g). The reaction mixture was stirred at the same temperature for 4 hours. Ethyl acetate (150 mL) and water (50 mL) were sequentially added to the reaction mixture, and the organic layer was separated. The organic layer was washed three times with a saturated aqueous sodium hydrogen carbonate solution and then washed with a saturated aqueous sodium chloride solution. The organic layer was dehydrated and dried over anhydrous sodium sulfate. The solvent was distilled off under reduced pressure, thereby obtaining a target substance (7.82 g) as a light yellow oily substance.

Reference Example 73 (2)

Dichloromethane (24 mL) was added to the compound (7.82 g) obtained in Reference Example 73 (1), and the mixture was stirred under ice cooling. At the same temperature, methylhydrazine (780 μL) was added to the reaction mixture, and the reaction mixture was stirred for 2 hours and 30 minutes at the same temperature. The reaction mixture was filtered, and the solvent was distilled off under reduced pressure. Methanol (47 mL) was added to the residue, and the mixture was stirred under ice cooling. At the same temperature, 2-(2-((tert-butoxycarbonyl)amino)thiazol-4-yl)-2-oxoacetic acid (4.0 g) was added to the reaction mixture, and the reaction mixture was stirred at room temperature for 2 hours and 30 minutes. Ethyl acetate (80 mL), water (50 mL), and 1 mol/L hydrochloric acid were added to the reaction mixture such that the pH was adjusted to 2.1, and the organic layer was separated. The organic layer was washed with a saturated aqueous sodium chloride solution and dehydrated and dried over anhydrous sodium sulfate. The solvent was distilled off under reduced pressure, hexane (200 mL) was added to the residue, and solids were collected by filtration. The solids were dried, thereby obtaining (R,Z)-5-((benzhydryloxy)carbonyl)-2-(2-((tert-butoxycarbonyl)amino)thiazol-4-yl)-8,8,9,9-tet ramethyl-4,7-dioxa-3-aza-8-siladec-2-enoic acid (8.73 g) as light yellow solids.

NMR: -0.02 (3H, s), 0.02 (3H, s), 0.81 (9H, s), 0.88 (9H, s), 4.09-4.18 (1H, m), 4.19-4.26 (1H, m), 5.11-5.15 (1H, m), 6.97 (1H, s), 7.27-7.34 (11H, m), 7.41 (1H, s), 8.22 (1H, s)

Reference Example 74

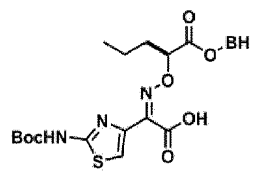

In the same manner as in Reference Example 73, (S,Z)-2-(((1-(benzhydryloxy)-1-oxopentan-2-yl)oxy)imino)-2-(2-((tert-butoxycarbonyl)amino) thiazol-4-yl)acetic acid was obtained.

NMR: 0.90 (3H, t, J = 7.4 Hz), 1.32-1.51 (2H, m), 1.53 (9H, s), 1.81-1.99 (2H, m), 5.09 (1H, dd, J = 8.0, 4.4 Hz), 6.93 (1H, s), 7.27-7.36 (11H, m), 7.37 (1H, s)

Reference Example 75

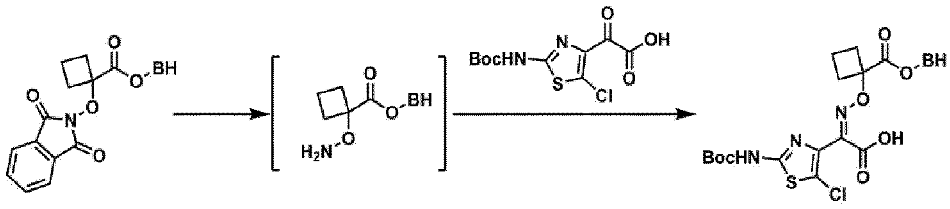

By using benzhydryl 1-((1,3-dioxoisoindoline-2-yl)oxy)cyclobutane-1-carboxylate, (Z)-2-((1-((benzhydryloxy) carbonyl)cyclobutoxy)imino)-2-(2-((tert-butoxycarbonyl)amino)-5-chlorothiazol-4-yl)acetic acid was obtained in the same manner as in Reference Example 73 (2).

NMR: 1.52 (9H, s), 2.07-2.19 (2H, m), 2.55-2.70 (4H, m), 6.94 (1H, s), 7.27-7.38 (12H, m)

The compounds in Table 10 were obtained in the same manner as in Reference Example 75.

[Table 10]

| Ref.Ex. No. | Structural Formula | Name |
|---|---|---|
| 76 | 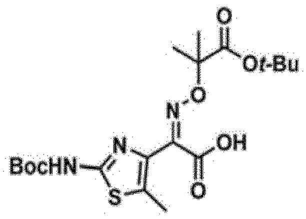 | (Z)-2-(((1-(tert-butoxy)-2-methyl-1-oxopropan-2-yl)oxy)imino)-2-(2-((tert-butoxycarbonyl)amino)-5-methylanisol-4-yl) acetic acid |
| 77 | 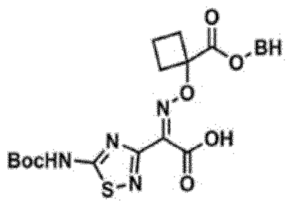 | (Z)-2-((1-((benzhydryloxy)carbonyl)cyclobutoxy)imino)-2-(5-((tert-butoxycarbonyl)amino)-1,2,4-thiadiazol-3-yl) acetic acid |

The measured values of NMR of the compounds in the table are as follows.

Reference Example 76

NMR (DMSO-$d_6$): 1.39 (9H, s), 1.42 (9H, s), 1.42 (3H, s), 1.46 (3H, s), 2.42 (3H, s), 11.54 (1H, s)

Reference Example 77

NMR: 1.57 (9H, s), 1.93-2.14 (2H, m), 2.42-2.72 (4H, m), 6.90 (1H, s), 7.20-7.40 (11H, m), 8.95-9.22 (1H, brs)

Reference Example 78

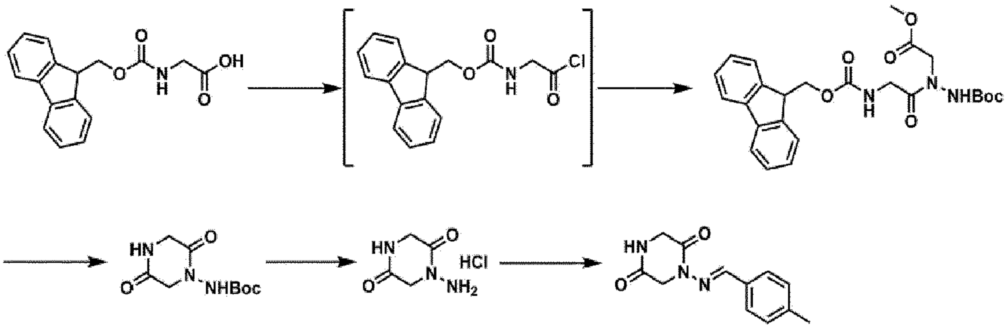

Reference Example 78 (1)

Dichloromethane (120 mL) was added to (((9H-fluoren-9-yl)methoxy)carbonyl)glycine (6.96 g), and the mixture was stirred under ice cooling. Oxalyl chloride (2.4 mL) and DMF (91 μL) were sequentially added to the reaction mixture. The reaction mixture was stirred at room temperature for 2 hours, and the solvent was distilled off under reduced pressure. Dichloromethane (50 mL) was added to the residue, thereby obtaining an acid chloride in a dichloromethane solution.

At room temperature, dichloromethane (25 mL), sodium hydrogen carbonate (5.90 g), and water (70 mL) were added to tert-butyl 2-(2-methoxy-2-oxoethyl)hydrazine-1-carboxylate (4.78 g). At the same temperature, the acid chloride in a dichloromethane solution was added dropwise to the reaction mixture. The reaction mixture was stirred at room temperature for 1 hour, and the organic layer was separated. The aqueous layer was extracted by using dichloromethane (20 mL), and the organic layer was dehydrated and dried over anhydrous sodium sulfate. The solvent was distilled off under reduced pressure. The residue was purified by silica gel column chromatography [eluent; ethyl acetate:hexane = 10:90 → 35:65], thereby obtaining a target substance (9.36 g) as white solids.

Reference Example 78 (2)

Dichloromethane (100 mL) was added to the compound (9.36 g) obtained in Reference Example 78 (1), and the mixture was stirred under ice cooling. At the same temperature, piperidine (5.7 mL) was added to the reaction mixture, and the reaction mixture was stirred at room temperature for 6 hours. The solvent was distilled off under reduced pressure, diethyl ether (20 mL) was added to the residue, and solids were collected by filtration. The residue was purified by silica gel column chromatography [eluent; methanol: chloroform = 0:100 → 10:90], thereby obtaining a target substance (3.03 g) as white solids.

Reference Example 78 (3)

Dichloromethane (65 mL) was added to the compound (3.0 g) obtained in Reference Example 78 (2), and the mixture was stirred under ice cooling. At the same temperature, a 4 mol/L hydrochloric acid/1,4-dioxane solution (33 mL) was added to the reaction mixture, and the reaction mixture was stirred at room temperature for 1 hour and 30 minutes. The solvent was distilled off under reduced pressure, thereby obtaining a target substance (2.2 g) as white solids.

Reference Example 78 (4)

Ethanol (22 mL) and p-tolualdehyde (1.6 mL) were added to the compound (2.2 g) obtained in Reference Example 78 (3), and the mixture was stirred at room temperature overnight. Solids were collected by filtration and washed with ethanol (10 mL). The solids were dried, thereby obtaining (E)-1-((4-methylbenzylidene)amino)piperazine-2,5-dione (2.72 g) as white solids.

NMR (DMSO-$d_6$): 2.35 (3H, s), 3.98 (2H, s), 4.32 (2H, s), 7.28 (2H, d, J = 8.0 Hz), 7.67 (2H, d, J = 8.0 Hz), 8.24 (1H, s), 8.31 (1H, s)

Reference Example 79

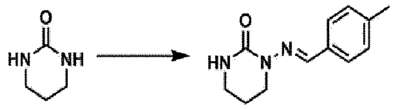

Concentrated sulfuric acid (69.1 g) was added dropwise to ice water (650 mL) under ice cooling. At the same temperature, tetrahydropyrimidin-2(1H)-one (25.0 g) was added to the reaction mixture. At the same temperature, a 34% aqueous sodium nitrite solution (50 mL) was added dropwise to the reaction mixture. At the same temperature, the reaction mixture was stirred for 1 hour. At the same temperature, zinc dust (37.5 g) was added to the reaction mixture by being divided into 5 portions. The reaction mixture was stirred at 20°C for 2 hours. Celpure (5 g) was added to the reaction mixture, and the reaction mixture was filtered. The residue was washed with water (50 mL). At room temperature, ethanol (100 mL) and p-tolualdehyde (27.0 g) were sequentially added to the filtrate, and the mixture was stirred for 3 hours at the same temperature. Solids were collected by filtration and sequentially washed with water (250 mL) and ethanol (25 mL). The solids were blast-dried at 40°C, thereby obtaining (E)-1-((4-methylbenzylidene)amino)tetrahydropyrimidin-2(1H)-one (7.3 g) as white solids.

Reference Example 80

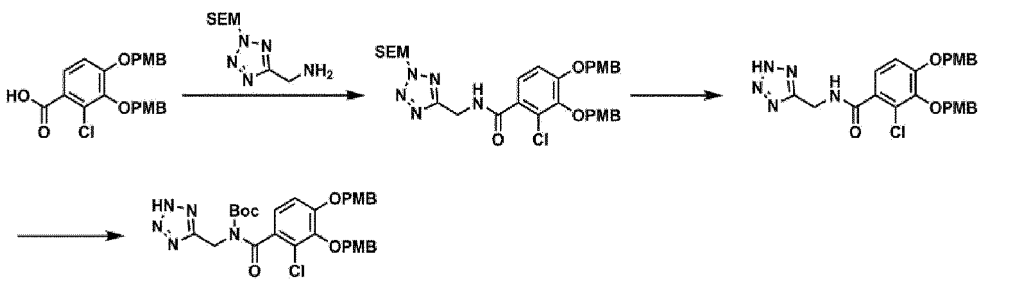

Reference Example 80 (1)

In the same manner as in Reference Example 3 (1), a target substance (10.8 g) was obtained as white solids from (2-((2-(trimethylsilyl)ethoxy)methyl)-2H-tetrazol-5-yl)methanamine (4.5 g).

Reference Example 80 (2)

In the same manner as in Reference Example 66 (3), a target substance (5.75 g) was obtained as white solids from the compound (10.8 g) obtained in Reference Example 80 (1).

Reference Example 80 (3)

THF (115 mL) was added to the compound (5.75 g) obtained in Reference Example 80 (2), and the mixture was stirred under ice cooling. At the same temperature, N,O-bis(trimethylsilyl)acetamide (2.78 mL) and 4-dimethylaminopyridine (1.38 g) were sequentially added to the reaction mixture, and the reaction mixture was stirred at room temperature for 1 hour. At the same temperature, di-tert-butyl dicarbonate (5.1 mL) was added dropwise to the reaction mixture, and the reaction mixture was stirred at room temperature for 1 hour. Ethyl acetate (200 mL) and water (200 mL) were added to the reaction mixture, and 1 mol/L hydrochloric acid was added thereto such that the pH was adjusted to 3.1. The organic layer was separated and washed with a saturated aqueous sodium chloride solution. The organic layer was dehydrated and dried over anhydrous magnesium sulfate, and the solvent was distilled off under reduced pressure. The residue was purified by silica gel column chromatography [eluent; methanol: chloroform = 0: 100 → 10:90], thereby obtaining tert-butyl((2H-tetrazol-5-yl)methyl)(2-chloro-3,4-bis((4-methoxybenzyl)oxy)benzoyl)carbama te (5.56 g) as white solids.

NMR (DMSO-$d_6$): 1.08 (9H, s), 3.75 (3H, s), 3.77 (3H, s), 4.89 (2H, s), 5.17-5.25 (4H, m), 6.84-6.91 (2H, m), 6.94-7.01 (2H, m), 7.20-7.35 (4H, m), 7.41-7.48 (2H, m)

The compounds in Table 11 were obtained in the same manner as in Reference Example 22.

[Table 11]

| Ref. Ex. No. | Structural Formula | Name |
|---|---|---|
| 8 1 | 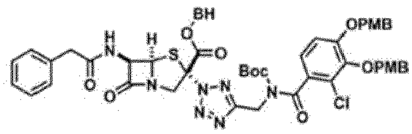 | Benzhydryl (3R,5R,6R)-3-(5-((N-(tert-butoxycarbonyl)-2-chloro-3,4-bis((4-methoxybenzyl)oxy)benzamido)methyl)-2H-tetrazol-2-vl)-7-oxo-6-(2-plienylacetaniido)-4-thia-1-azabicyclo [3.2.0]heptane-3-carboxylate |
| 8 2 | 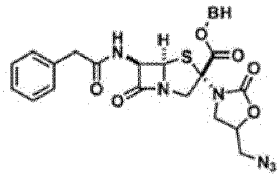 | Benzhydryl (3R,5R,6R)-3-(5-(azidomethyl)-2-oxooxazolidin-3-yl)-7-oxo-6-(2-phenylacetamido)-4-thia-1-azabicyclo[3.2.0]heptane-3-carboxylate |
| 8 3 | 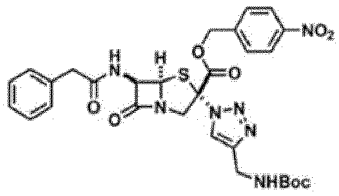 | 4-Nitrobenzyl (3R,5R,6R)-3-(4-(((tert-butoxycarbonyl)amino) methyl)-1H-1,2,3-triazol-1-yl)-7-oxo-6-(2-phenylacetamido)-4-thia-1-azabicyclo[3.2.0]heptane-3-carboxylate |

The measured values of NMR of the compounds in the table are as follows.

Reference Example 81

NMR: 1.13 (9H, s), 3.45-3.56 (3H, s), 3.80 (3H, s), 3.82-3.84 (5H, m), 4.95 (2H, s), 5.07 (1H, d, J = 13.6 Hz), 5.11 (2H, s), 5.66-5.72 (2H, m), 6.82-6.88 (4H, m), 6.88-6.96 (5H, m), 7.07-7.42 (18H, m)

Reference Example 82

NMR: 3.30-3.64 (6H, m), 3.74-3.89 (1H, m), 4.56-4.69 (1H, m), 4.90 [5.01] (1H, d, J = 13.4 Hz), 5.10-5.19 (2H, m), 6.15-6.31 (1H, m), 6.86 [6.87] (1H, s), 7.12-7.17 (2H, m), 7.20-7.44 (13H, m)

Reference Example 83

NMR: 1.46 (9H, s), 3.65 (2H, d, J = 2.4 Hz), 4.12 (1H, dd, J = 13.4, 1.0 Hz), 4.38 (2H, d, J = 5.6 Hz), 4.83 (1H, d, J = 13.2 Hz), 4.94-5.00 (1H, brs), 5.13 (1H, d, J = 13.2 Hz), 5.19 (1H, d, J = 13.6 Hz), 5.64 (1H, d, J = 4.0 Hz), 5.82 (1H, ddd, J = 9.3, 3.9, 0.9 Hz), 6.71 (1H, d, J = 9.6 Hz), 7.22-7.37 (7H, m), 7.65 (1H, s), 8.19-8.27 (2H, m)

Reference Example 84

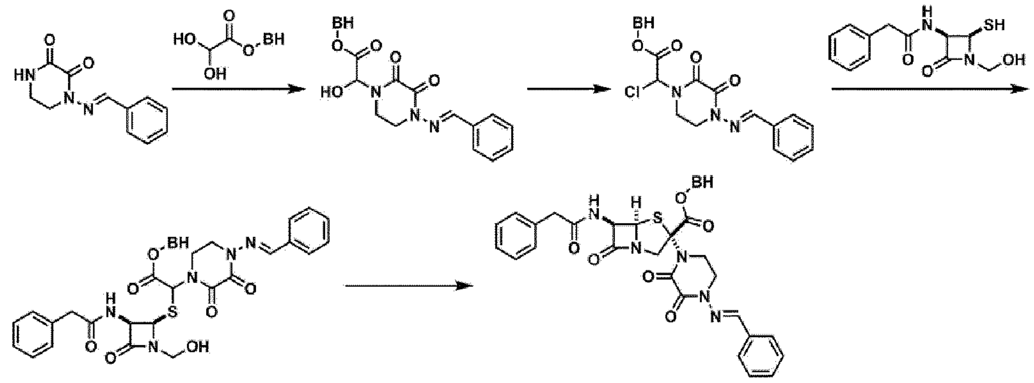

Reference Example 84 (1)

Dichloromethane (220 mL) and benzhydryl 2,2-dihydroxyacetate (13.7 g) were sequentially added to (E)-1-(benzylideneamino)piperazine-2,3-dione (11.0 g), and the mixture was stirred under ice cooling. At the same temperature, DBU (0.38 mL) was added to the reaction mixture, and the reaction mixture was stirred at room temperature for 3 hours. At the same temperature, benzhydryl 2,2-dihydroxyacetate (6.85 g) was added to the reaction mixture, and the reaction mixture was stirred overnight. IPE (110 mL) was added to the reaction mixture, and solids were collected by filtration and washed with IPE (50 mL). The solids were dried, thereby obtaining a target substance (21.4 g) as white solids.

Reference Example 84 (2)

THF (420 mL) was added to the compound (21.0 g) obtained in Reference Example 84 (1), and the mixture was stirred under ice cooling. At the same temperature, 2,6-lutidine (9.1 mL) and thionyl chloride (5.4 mL) were sequentially added to the reaction mixture. The reaction mixture was stirred at room temperature for 2 hours and 30 minutes, and insoluble matters were filtered. The solvent was distilled off under reduced pressure, thereby obtaining a mixture containing a target substance.

Reference Example 84 (3)

Dichloromethane (420 mL) and N-((2R,3R)-1-(hydroxymethyl)-2-mercapto-4-oxoazetidin-3-yl)-2-phenylacetamide (13.4 g) were sequentially added to the mixture obtained in Reference Example 84 (2), and the mixture was stirred under ice cooling. At the same temperature, triethylamine (7.0 mL) was added to the reaction mixture, and the reaction mixture was stirred for 2 hours. Water (420 mL) and 6 mol/L hydrochloric acid were added to the reaction mixture such that the pH was adjusted to 2.0. The organic layer was separated and sequentially washed with water and a saturated aqueous sodium chloride solution. The organic layer was dehydrated and dried over anhydrous sodium sulfate, and the solvent was distilled off under reduced pressure. The residue was purified by silica gel column chromatography [eluent; ethyl acetate:hexane = 50:50 → 100:0], thereby obtaining a target substance (16.1 g) as white solids.

Reference Example 84 (4)

THF (370 mL) was added to the compound (18.5 g) obtained in Reference Example 84 (3), and the mixture was stirred under ice cooling. At the same temperature, 2,6-lutidine (4.1 mL) and thionyl chloride (2.48 mL) were sequentially added to the reaction mixture. The reaction mixture was stirred at room temperature for 1 hour and 30 minutes. Insoluble matters were filtered, and the solvent was distilled off under reduced pressure. At room temperature, THF (370 mL) and N,O-bis(trimethylsilyl)acetamide (7.8 mL) were sequentially added to the residue, and the mixture was stirred for 30 minutes. At the same temperature, hexamethylphosphoric triamide (23 mL) was added to the reaction mixture, and the reaction mixture was cooled to -60°C. At the same temperature, a 1.3 mol/L lithium bis(trimethylsilyl)amide/tetrahydrofuran solution (24 mL) was added dropwise to the reaction mixture. The reaction mixture was stirred at -10°C for 1 hour. The reaction mixture was added to a mixture of ethyl acetate (750 mL), water (370 mL), and 1 mol/L hydrochloric acid (52 mL) under ice cooling, and the organic layer was separated. The organic layer was sequentially washed with water and a saturated aqueous sodium chloride solution. The organic layer was dehydrated and dried over anhydrous magnesium sulfate, and the solvent was distilled off under reduced pressure. The residue was purified by silica gel column chromatography [eluent; ethyl acetate:hexane = 40:60 → 80:20], thereby obtaining benzhydryl (3R,5R,6R)-3-(4-(((E)-benzylidene)amino)-2,3-dioxopiperazin-1-yl)-7-oxo-6-(2-phenylacetam ido)-4-thia-1-azabicyclo

[3.2.0]heptane-3-carboxylate (6.0 g) as light yellow solids.

NMR: 3.12 (1H, d, J = 13.6 Hz), 3.24 (1H, d, J = 15.2 Hz), 3.29 (1H, d, J = 14.8 Hz), 3.97-4.17 (4H, m), 5.39 (1H, d, J = 13.6 Hz), 5.42-5.49 (2H, m), 5.93 (1H, d, J = 6.8 Hz), 6.92 (1H, s), 7.06-7.14 (2H, m), 7.22-7.49 (16H, m), 7.69-7.78 (2H, m), 9.22 (1H, s)

The compounds in Table 12 were obtained in the same manner as in Reference Example 84.

[Table 12]

| Ref.Ex. No. | Structural Formula | Name |
|---|---|---|
| 8 5 | 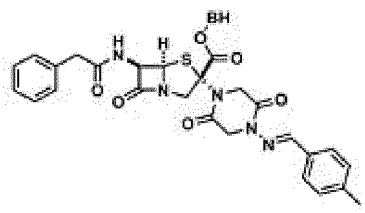 | Benzhydryl (3R,5R,6R)-3-(4-(((E)-4. methylbenzylidene) amino)-L5-dioxopiperazin-1-yl)-7-oxo-6-(2-phenylacetamido)-4-thia-1-azabicyclo[34.0]heptane-3-carboxylate |
| 8 6 | 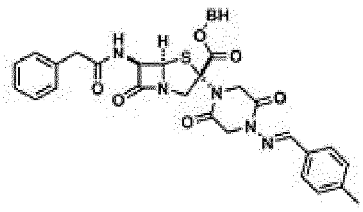 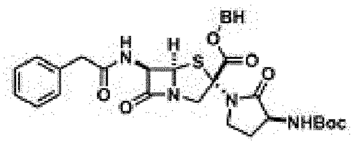 | (3R,5R,6R)-3-((S)-3-((tett-butoxycarbonyl)amino)-2-oxopyrrolidin-i-yl)-7-oxo-6-(2-phenylacetamidoF4-thia-1-azabicyclo[3.2.0]heptane-3-carboxylate |
| 8 7 | 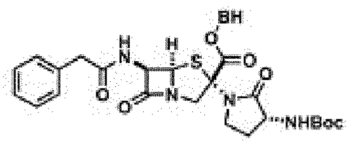 | Benzhydryl (3R,5R.6RH-((R)-3-{(tert-butoxycarbonyl) amine)-2-exopyrrolidin-1-yl). 7-oxo-6-(2-phenylacetamido)-4-thia-1-azabicyclo[3.2.0}heptane-3-carboxylate |
| 8 8 | 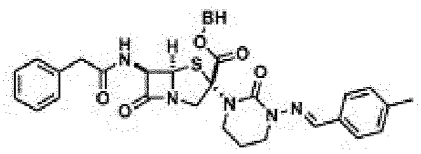 | Benzhydryl (3R,5R,6R)-3-(3-(((E)-4-methylbenzylidene) amino-2-exotetrahydropyrimidin- 1 (2H)-yl)-7-oxo-6-(2-phenylacetanudo)-4-thia-1-azabicyclo[3.2.0]heptane-3-carboxylate |

The measured values of NMR of the compounds in the table are as follows.

Reference Example 85

NMR: 2.40 (3H, s), 3.10 (1H, dd, J = 13.4, 1.0 Hz), 3.30 (2H, s), 4.19-4.41 (2H, m), 4.42-4.60 (2H, m), 5.29 (1H, d, J = 13.2 Hz), 5.46 (1H, d, J = 3.6 Hz), 5.52 (1H, dd, J = 8.0, 2.8 Hz), 6.05 (1H, d, J = 8.0 Hz), 6.92 (1H, s), 7.08-7.14 (2H, m), 7.20-7.43 (14H, m), 7.60-7.68 (3H, m), 8.59 (1H, s)

Reference Example 86

NMR: 1.45 (9H, s), 1.86-2.01 (1H, m), 2.53-2.66 (1H, m), 3.25 (1H, dd, J = 13.4, 1.0 Hz), 3.34 (2H, s), 3.46-3.56 (1H, m), 3.59-3.69 (1H, m), 3.97-4.09 (1H, m), 4.79-4.93 (1H, m), 5.01 (1H, d, J = 13.2 Hz), 5.44 (1H, d, J = 4.0 Hz), 5.54 (1H, dd, J = 8.6, 3.0 Hz), 6.29 (1H, d, J = 8.4 Hz), 6.85 (1H, s), 7.11-7.18 (2H, m), 7.19-7.43 (13H, m)

Reference Example 87

NMR: 1.46 (9H, s), 1.74-1.89 (1H, m), 2.55-2.72 (1H, m), 3.18 (1H, dd, J = 13.2, 0.8 Hz), 3.33 (2H, s), 3.50-3.63 (2H, m), 4.16-4.33 (1H, m), 4.63-4.78 (1H, m), 5.02 (1H, d, J = 13.2 Hz), 5.46 (1H, d, J = 3.6 Hz), 5.52 (1H, dd, J = 8.2, 3.4 Hz), 6.28 (1H, d, J = 8.4 Hz), 6.85 (1H, s), 7.10-7.16 (2H, m), 7.18-7.43 (13H, m)

Reference Example 88

NMR: 2.15-2.26 (2H, m), 2.38 (3H, s), 3.18 (1H, dd, J = 13.4, 1.0 Hz), 3.29 (2H, s), 3.53-3.77 (4H, m), 5.19 (1H, d, J = 13.6 Hz), 5.41 (1H, d, J = 3.6 Hz), 5.49 (1H, ddd, J = 8.4, 3.8, 0.8 Hz), 6.28 (1H, d, J = 8.4 Hz), 6.91 (1H, s), 7.11-7.42 (17H, m), 7.62 (2H, d, J = 8.4 Hz), 7.94 (1H, s)

The compounds in Table 13 were obtained in the same manner as in Reference Example 27.

[Table 13]

| Ref. Ex. No. | Structural Formula | Name |
|---|---|---|
| 89 | 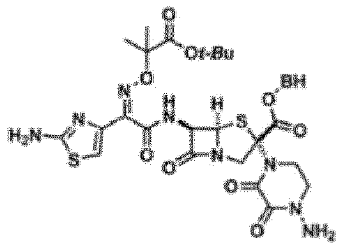 | Benzhydryl (3R,5R,6R)-3-(4-amino-2,3-dioxopiperazin-1-yl)-6-((Z)-2-(2-aminothiazol-4-yl)-2-(((1-(tert-butoxy)-2-methyl-1-oxopropan-2-yl)oxy)imino)acetamido)-7-oxo-4-thia-1-azabicyclo [3.2.0]heptane-3-carboxylate |
| 90 | 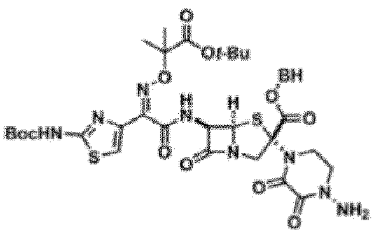 | Benzhydryl (3R,5R,6R)-3-(4-amino-2,3-dioxopiperazin-1-yl)-6-((Z)-2-(((1-(tert-butoxy)-2-methyl-1-oxopropan-2-yl)oxy)imino)-2-((tert-butoxycarbonyl)amino)thiazol-4-yl)acetamido)-7-oxo-4-thia-1-azabicyclo[3.2.0]heptane-3-carboxylate |
| 91 | 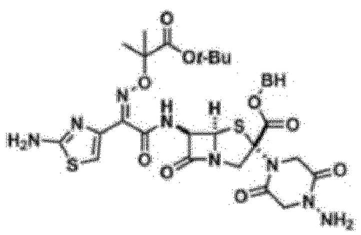 | Benzhydryl (3R,5R,6R)-3-(4-amino-2,5-dioxopiperazin-1-yl)-6-((Z)-2-((2-aminothiazol-4-yl)-2-(((1-(tert-butoxy)-2-methyl-1-oxopropan-2-yl)oxy)imino)acetamido)-7-oxo-4-thia-1-azabicyclo [3.2.0]heptane-3-carboxylate |
| 92 | 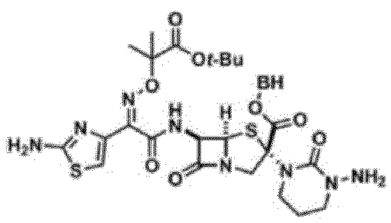 | Benzhydryl (3R,5R,6R)- 3-(3-amino-2-oxotetrahydropyrimidin-1 (2H)-yl)-6-((Z)-2-(2-aminothiazol-4-yl)-2-(((1-(tert-butoxy)-2-methyl-1-oxopropan-2-yl)oxy)imino)acetamido)-7-oxo-4-thia-1-azabicyclo[3.2.0]heptane-3-carboxylate |
| 93 | 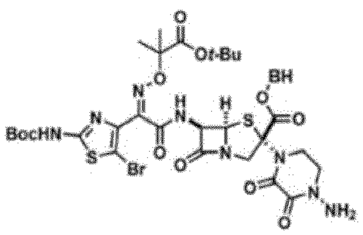 | Benzhydryl (3R,5R,6R)-3-(4-amino-2,3-dioxopiperazin-1-yl)-6-((Z)-2-(5-bromo-2-((tert-butoxycarbonyl)amino)thiazol-4-yl)-2-(((1-(tert-butoxy)-2-methyl-1-oxopropan-2-yl)oxy)imino)acetamido)-7-oxo-4-thia-1-azabicyclo[3.2.0]heptane-3-carboxylate |

(continued)

| Ref. Ex. No. | Structural Formula | Name |
|---|---|---|
| 9 4 | 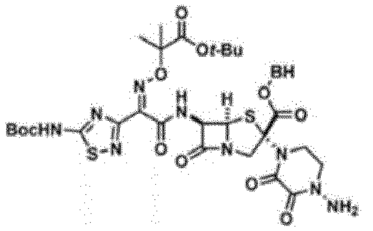 | Benzhydryl (3R,5R,6R)-3-(4-amino-2,3-dioxopiperazin-1-yl)-6-((Z)-2-(((1-(tert-butoxy)-2-methyl-1-oxopropan-2-yl)oxy)imino)-2-(5-((tert-butoxycarbonyl)amino)-1,2,4-thiadiazol-3-yl)acetamido)-7-oxo-4-thia-1-azabicyclo[3.2.0]heptane-3-carboxylate |
| 9 5 | 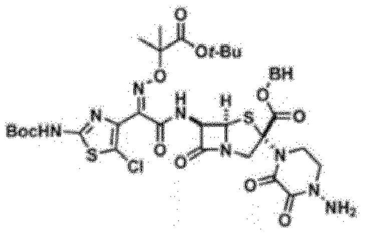 | Benzhydryl (3R,5R,6R)-3-(4-amino-2,3-dioxopiperazin- 1-yl)-6-((Z)-2-(((1-(tert-butoxy)-2-methyl-1-oxopropan-2-yl)oxy)imino)-2-(2-((tert-butoxycarbonyl)amino)-5-chlorothiazol-4-yl)acetamido)-7-oxo-4-thia-1-azabicyclo[3.2.0]heptane-3-carboxylate |

The measured values of NMR of the compounds in the table are as follows.

Reference Example 89

NMR: 1.40 (9H, s), 1.50 (6H, s), 3.12 (1H, d, J = 13.6 Hz), 3.71-3.85 (2H, m), 3.97-4.07 (2H, m), 5.42 (1H, d, J = 13.6 Hz), 5.53 (1H, d, J = 3.6 Hz), 5.66 (1H, dd, J = 7.2, 3.2 Hz), 6.10 (2H, s), 6.78 (1H, s), 6.84-6.92 (2H, m), 7.06-7.12 (1H, m), 7.15-7.44 (11H, m)

Reference Example 90

NMR: 1.40 (9H, s), 1.52 (3H, s), 1.52 (3H, s), 1.55 (9H, s), 3.11 (1H, dd, J = 13.6, 0.8 Hz), 3.69-3.86 (2H, m), 3.92-4.12 (2H, m), 4.59 (2H, s), 5.47 (1H, d, J = 13.6 Hz), 5.54 (1H, d, J = 3.6 Hz), 5.65 (1H, dd, J = 6.8, 3.6 Hz), 6.86 (1H, s), 7.00-7.08 (1H, m), 7.13-7.45 (11H, m), 8.14 (1H, s)

Reference Example 91

NMR: 1.40 (9H, s), 1.50 (3H, s), 1.51 (3H, s), 3.10 (1H, d, J = 13.6 Hz), 3.92-4.17 (2H, m), 4.30-4.46 (4H, m), 5.40 (1H, d, J = 13.6 Hz), 5.50 (1H, d, J = 3.6 Hz), 5.76 (1H, dd, J = 7.8, 3.4 Hz), 6.07 (2H, s), 6.80 (1H, s), 6.88 (1H, s), 7.14-7.33 (11H, m)

Reference Example 92

NMR: 1.39 (9H, s), 1.46 (3H, s), 1.48 (3H, s), 2.01-2.12 (2H, m), 3.17 (1H, dd, J = 13.4, 1.0 Hz), 3.36-3.48 (2H, m), 3.63 (2H, t, J = 6.0 Hz), 3.84-3.99 (2H, brs), 5.16 (1H, d, J = 13.6 Hz), 5.47 (1H, d, J = 4.0 Hz), 5.75 (1H, ddd, J = 8.6, 3.8, 0.8 Hz), 6.63 (2H, s), 6.77 (1H, s), 6.89 (1H, s), 7.01 (1H, d, J = 8.4 Hz), 7.14-7.43 (10H, m)

Reference Example 93

NMR: 1.42 (9H, s), 1.51-1.56 (15H, m), 3.08 (1H, dd, J = 13.8, 1.0 Hz), 3.70-3.86 (2H, m), 3.99-4.07 (2H, m), 4.58 (2H, s), 5.49 (1H, d, J = 13.6 Hz), 5.53 (1H, d, J = 3.6 Hz), 5.64 (1H, dd, J = 7.2, 3.6 Hz), 6.86 (1H, s), 6.93-7.44 (11H, m), 8.07 (1H, s)

Reference Example 94

NMR: 1.41 (9H, s), 1.55 (9H, s), 1.56 (3H, s), 1.58 (3H, s), 3.10 (1H, d, J = 12.8 Hz), 3.72-3.86 (2H, m), 4.00-4.09 (2H, m), 4.59 (2H, s), 5.47 (1H, d, J = 13.6 Hz), 5.54 (1H, d, J = 3.6 Hz), 5.66 (1H, d, J = 7.8, 3.0 Hz), 6.82 (1H, d, J = 7.2 Hz),

6.87 (1H, s), 6.98-7.06 (1H, m), 7.08-7.45 (9H, m), 8.41 (1H, s)

Reference Example 95

NMR: 1.42 (9H, s), 1.53 (9H, s), 1.54 (6H, s), 3.08 (1H, d, J = 12.4 Hz), 3.70-3.85 (2H, m), 3.97-4.08 (2H, m), 4.58 (2H, s), 5.49 (1H, d, J = 14.0 Hz), 5.53 (1H, d, J = 4.0 Hz), 5.64 (1H, d, J = 7.2, 3.2 Hz), 6.87 (1H, s), 6.97-7.01 (1H, m), 7.07-7.14 (1H, m), 7.17-7.43 (9H, m), 7.95 (1H, s)

The compounds in Table 14 were obtained in the same manner as in Reference Example 27 (1).

[Table 14]

| Ref.Ex. No. | Structural Formula | Name |
|---|---|---|
| 96 | 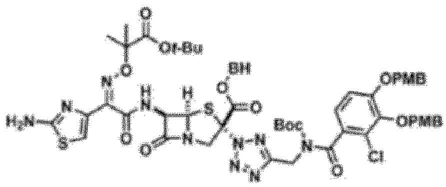 | Benzhydryl (3R,5R,6R)-6-((Z)-2-(2-aminothiazol-4-yl)-2-(((1-(tert-butoxy)-2-methyl-1-oxopropan-2-yl)oxy)imino)acetamido)-3-(5-((N-(tert-butoxycarbonyl)-2-chloro-3,4-bis((4-methoxybenzyl)oxy)benzamido)methyl)-2H-tetrazol-2-yl)-7-oxo-4-thia-1-azabicyclo[3.2.0]heptane-3-carboxylate |
| 97 | 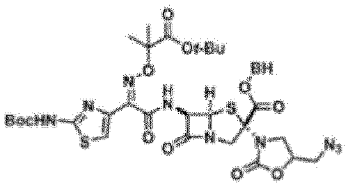 | Benzhydryl (3R,5R,6R)-3-(5-(azidomethyl)-2-oxooxazolidin-3-yl)-6-((Z)-2-(((1-(tert-butoxy)-2-methyl-1-oxopropan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 98 | 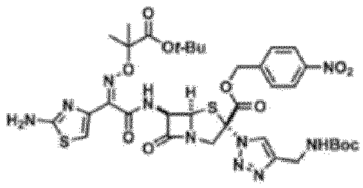 | 4-Nitrobenzyl (3R,5R,6R)-6-((Z)-2-(2-aminothiazol-4-yl)-2-(((1-(tert-butoxy)-2-methyl-1-oxopropan-2-yl)oxy)imino)acetamido)-3-(4-(((tert-butoxycarbonyl)amino)methyl)-1H-1,2.3-triazol-1-yl)-7-oxo-4-thia-1-azabicyclo[3.2.0]heptane-3-carboxylate |
| 99 | 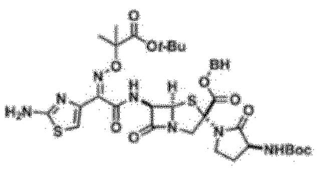 | Benzhydryl (3R,5R,6R)-6-((Z)-2-(2-aminothiazol-4-y1)-2-({(1-(tert-butoxy)-2-methyl-1-oxopropan-2-yl)oxy)imino)acetamido)-3-((S)-3-((tert-butoxycarbonyl)amino)-2-oxopyrrolidin-1-yl)-7-oxo-4-thia-1-azabicyclo[3.2.0]heptane-3-carboxylate |
| 100 | 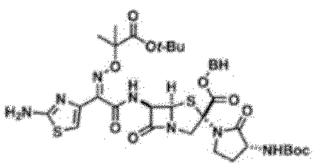 | Benzhydryl (3R,5R,6R)-6-((Z)-2-(2-aminothiazol-4-yl)-2-(((1-(tert-butoxy)-2-methyl-1-oxopropan-2-yl)oxy)imino)acetamido)-3-((R)-3-((tert-butoxycarbonyl)amino)-2-oxopyrrolidin-1-yl)-7-oxo-4-thia-1-azabicyclo[3.2.0]heptane-3-carboxylate |

The measured values of NMR of the compounds in the table are as follows.

Reference Example 96

NMR: 1.09-1.19 (9H, m), 1.36-1.60 (15H, m), 3.78-3.85 (6H, m), 4.94 (2H, s), 5.06-5.15 (3H, m), 5.30-5.38 (1H, m), 5.55-5.65 (1H, m), 5.72-5.76 (1H, m), 5.88-6.00 (1H, m), 6.81-6.87 (2H, m), 6.88-6.95 (4H, m), 7.10-7.40 (15H, m)

Reference Example 97

NMR: 1.39 (9H, s), 1.51 (3H, s), 1.53 (3H, s), 1.54 (9H, s), 3.33 (1H, d, J = 5.2 Hz), 3.43-3.66 (3H, m), 3.82 [3.91] (1H, t, J = 8.4 Hz), 4.58-4.70 (1H, m), 5.00 [5.06] (1H, d, J = 13.2 Hz), 5.61-5.66 (1H, m), 5.74-5.85 (1H, m), 6.87 (1H, s), 7.13-7.21 (1H, m), 7.22-7.56 (11H, m), 8.09 (1H, s)

Reference Example 98

NMR: 1.42 (9H, s), 1.46 (3H, s), 1.46 (3H, s), 1.46 (9H, s), 4.08 (1H, d, J = 13.6 Hz), 4.39 (2H, d, J = 5.6 Hz), 4.92 (1H, d, J = 13.2 Hz), 5.18-5.38 (2H, m), 5.75 (1H, d, J = 4.0 Hz), 5.95 (1H, dd, J = 8.4, 4.4 Hz), 6.27 (2H, s), 6.88 (1H, s), 6.94-7.01 (1H, m), 7.38-7.45 (2H, m), 7.61-7.73 (2H, m), 8.13-8.19 (2H, m)

Reference Example 99

NMR: 1.40 (9H, s), 1.44 (9H, s), 1.48 (3H, s), 1.50 (3H, s), 1.89-2.03 (1H, m), 2.50-2.68 (1H, m), 3.34 (1H, d, J = 13.2 Hz), 3.49-3.59 (1H, m), 3.68 (1H, t, J = 8.6 Hz), 3.99-4.09 (1H, m), 4.84-4.97 (1H, m), 5.09 (1H, d, J = 13.6 Hz), 5.54 (1H, d, J = 4.0 Hz), 5.78 (1H, dd, J = 8.6, 3.8 Hz), 6.66-6.76 (2H, brs), 6.76 (1H, s), 6.85 (1H, s), 6.86-6.94 (1H, m), 7.12-7.45 (10H, m)

Reference Example 100

NMR: 1.39 (9H, s), 1.44 (9H, s), 1.48 (3H, s), 1.49 (3H, s), 1.78-1.91 (1H, m), 2.51-2.71 (1H, m), 3.28 (1H, d, J = 13.2 Hz), 3.48-3.65 (2H, m), 4.17-4.35 (1H, m), 4.69-4.80 (1H, m), 5.10 (1H, d, J = 13.6 Hz), 5.57 (1H, d, J = 3.6 Hz), 5.78 (1H, dd, J = 8.2, 3.8 Hz), 6.39-6.67 (2H, brs), 6.79 (1H, s), 6.86 (1H, s), 7.05-7.14 (1H, m), 7.18-7.43 (10H, m)

Reference Example 101

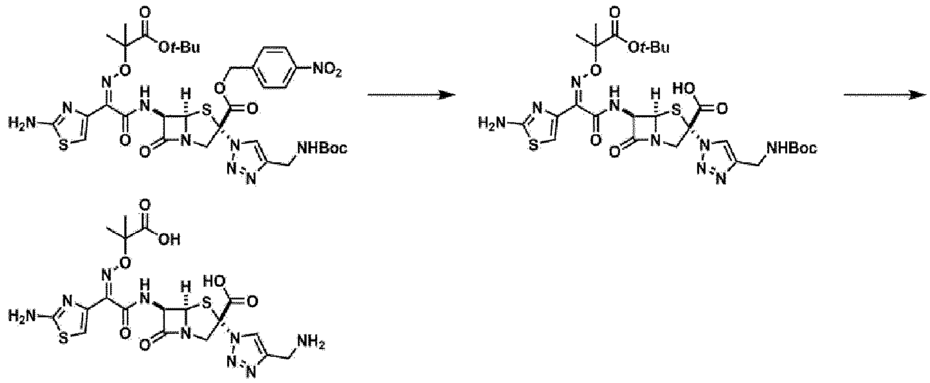

Reference Example 101 (1)

Ethyl acetate (3 mL) and 10% palladium-carbon (300 mg) were added to 4-nitrobenzyl(3R,5R,6R)-6-((Z)-2-(2-aminothiazol-4-yl)-2-(((1-(tert-butoxy)-2-methyl-1-oxop ropan-2-yl)oxy)imino)acetamido)-3-(4-(((tert-butoxycarbonyl)amino)methyl)-1H-1,2,3-triazol -1-yl)-7-oxo-4-thia-1-azabicyclo[3.2.0]heptane-3-carboxylate (300 mg), and the mixture was stirred at room temperature for 3 hours in a hydrogen atmosphere. The reaction mixture was filtered through celite, and the residue was washed with ethyl acetate. The solvent was distilled off under reduced pressure, thereby obtaining a target substance (233 mg) as yellow solids.

Reference Example 101 (2)

Dichloromethane (3.5 mL) and nitromethane (1.2 mL) were added to the compound (230 mg) obtained in Reference Example 101 (1), and the mixture was stirred at -20°C. At the same temperature, anisole (1.4 mL) and aluminum chloride (353 mg) were sequentially added to the reaction mixture. The reaction mixture was stirred at a temperature equal to or lower than -20°C for 1 hour and 30 minutes. At the same temperature, trifluoroacetic acid (0.13 mL) was added to the reaction mixture, and the reaction mixture was stirred at a temperature equal to or lower than -10°C for 1 hour 30 minutes. The reaction mixture was added to a mixture of acetonitrile (10 mL), water (10 mL), and trisodium citrate dihydrate (1.17 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was concentrated

under reduced pressure and lyophilized, thereby obtaining (3R,5R,6R)-3-(4-(aminomethyl)-1H-1,2,3-triazol-1-yl)-6-((Z)-2-(2-aminothiazol-4-yl)-2-(((2-carboxypropan-2-yl)oxy)imino)acetamido)-7-oxo-4-thia-1-azabicyclo[3.2.0] heptane-3-carbox ylic acid (40 mg) as white solids.

NMR ($D_2O$): 1.50 (3H, s), 1.52 (3H, s), 4.08 (1H, dd, J = 13.2, 1.2 Hz), 4.36 (2H, s), 4.74 (1H, d, J = 13.2 Hz), 5.64 (1H, d, J = 3.6 Hz), 5.81 (1H, dd, J = 3.6, 1.2 Hz), 7.04 (1H, s), 7.92 (1H, s)

Reference Example 102

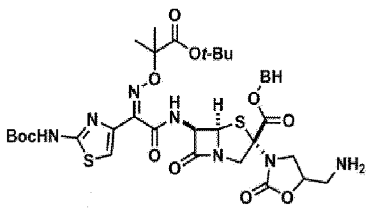

In the same manner as in Reference Example 101(1), benzhydryl(3R,5R,6R)-3-(5-(aminomethyl)-2-oxooxazolidin-3-yl)-6-((Z)-2-(((1-(tert-Butoxy)-2-methyl-1-oxopropan-2-yl)oxy)imino)-2-(2-((tert-butoxycarbonyl)amino)thiazol-4-yl)acetam ido)-7-Oxo-4-thia-1-azabicyclo[3.2.0]heptane-3-carboxylate was obtained.

NMR: 1.39 [1.39] (9H, s), 1.50 (3H, s), 1.52 (3H, s), 1.54 (9H, s), 2.73 [2.76] (1H, d, J = 5.8 Hz), 2.92-3.01 (1H, m), 3.55-3.63 (2H, m), 3.76 [3.86] (1H, t, J = 7.9 Hz), 4.54-4.61 (1H, m), 4.95 [5.05] (1H, d, J = 13.2 Hz), 5.61 [5.62] (1H, d, J = 3.8 Hz), 5.72-5.84 (1H, m), 6.88 (1H, s), 7.13-7.53 (15H, m)

The compounds of Table 15 were obtained in the same manner as in Reference Example 101 (2).

[Table 15]

| Ref. Ex. No. | Structural Formula | Name |
|---|---|---|
| 1 0 3 | 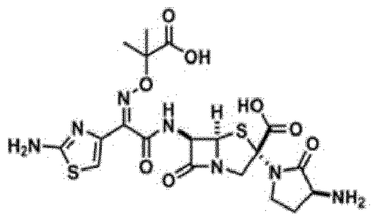 | (3R,5R,6R)-3-((S)-3-amino-2-oxopyrrolidin-1-yl)-6-((Z)-2-(2-aminothiazol-4-yl)-2-(((2-carboxypropan-2-yl)oxy)imino)acetamido)-7-oxo-4-thia-1-azabicyclo[3.2.0]heptane-3-carboxylate |
| 1 0 | 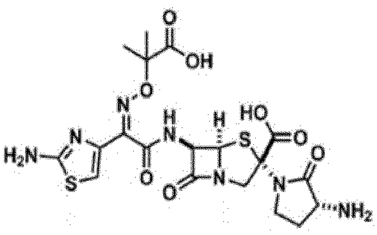 | (3R,5R,6R)-3-((R)-3-amino-2-oxopyrrolidin-1-yl)-6-((Z)-2-(2-aminothiazol-4-yl)-2-(((2-carboxypropan-2-yl)oxy)imino)acetamido)-7-oxo-4-thia-1-azabicyclo[3.2.0]heptane-3-carboxylate |

The measured values of NMR of the compounds in the table are as follows.

Reference Example 103

NMR ($D_2O$): 1.49 (3H, s), 1.51 (3H, s), 2.10-2.24 (1H, m), 2.59-2.72 (1H, m), 3.38 (1H, dd, J = 12.8, 1.2 Hz), 3.72-3.82 (1H, m), 3.86 (1H, t, J = 9.2 Hz), 4.25 (1H, dd, J = 10.4, 9.2 Hz), 4.82 (1H, d, J = 12.8 Hz), 5.68 (1H, d, J = 3.6 Hz), 5.72 (1H, dd, J = 3.6, 1.2 Hz), 7.05 (1H, s)

Reference Example 104

NMR ($D_2O$): 1.50 (3H, s), 1.51 (3H, s), 2.12-2.26 (1H, m), 2.61-2.73 (1H, m), 3.36 (1H, dd, J = 12.8, 1.2 Hz), 3.67-3.80 (1H, m), 3.80-3.93 (1H, m), 4.23 (1H, dd, J = 10.0, 9.2 Hz), 4.82 (1H, d, J = 12.8 Hz), 5.68 (1H, d, J = 3.6 Hz), 5.72 (1H, dd, J = 3.6, 1.2 Hz), 7.06 (1H, s)

The compounds in Table 16 were obtained in the same manner as in Reference Example 35.

[Table 16]

| Ref. Ex. No. | Structural Formula | Name |
|---|---|---|
| 105 | 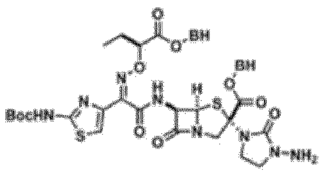 | Benzhydryl (3R,5R,6R)-3-(3-amino-2-oxoimidazolidin-1-yl)-6-((Z)-2-((((S)-1-(benzhydryloxy)-1-oxobutan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 106 | 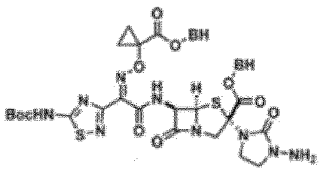 | Benzhydryl (3R,5R,6R)-3-(3-amino-2-oxoimidazolidin-1-yl)-6-((Z)-2-((1-((benzhydryloxy)carbonyl)cyclopropoxy)imino)-2-(5-((tert-butoxycarbonyl)amino)- 1,2,4-thiadiazol-3-yl)acetamido)-7-oxo-4-thia-1-azabicyclo[3.2.0]heptane-3-carboxylate |
| 107 | 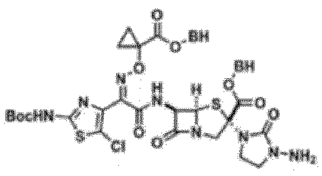 | Benzhydryl (3R,5R,6R)-3-(3-amino-2-oxoimidazolidin-1-yl)-6-((Z)-2-((1-((benzhydryloxy)carbonyl)cyclopropoxy)imino)-2-(2-((tert-butoxycarbonyl)amino)-5-chlorothiazol-4-yl)acetamido)-7-oxo-4-thia-1-azabicyclo[3.2.0]heptane-3-carboxylate |
| 108 | 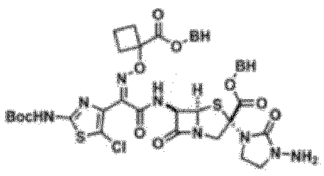 | Benzhydryl (3R,5R,6R)-3-(3-amino-2-oxoimidazolidin-1-yl)-6-((Z)-2-((1-((benzhydryloxy)carbonyl)cyclobutoxy}imino)-2-(2-((tert-butoxycarbonyl)amino)-5-chlorothiazol-4-yl)acetamido)-7-oxo-4-thia-1-azabicyclo[3.2.0]heptane-3-carboxylate |
| 109 | 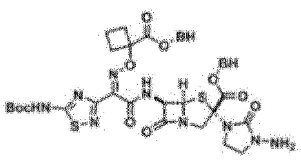 | Benzhydryl (3R,5R,6R)-3-(3-amino-2-oxoimidazolidin-1-yl)-6-((Z)-2-((1-((benzhydryloxy)carbonyl)cyclobutoxy)imino)-2-(5-((tert-butoxycarbonyl)amino)-1,2,4-thiadiazol-3-yl)acetamido)-7-oxo-4-thia-1-azabicyclo[3.2.0]heptane-3-carboxylate |
| 110 | 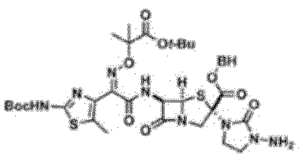 | Benzhydryl (3R,5R,6R)-3-(3-amino-2-oxoimidazolidin-1-yl)-6-((Z)-2-(((1-(tert-butoxy)-2-methyl-1-oxopropan-2-yl)oxy)imino)-2-(2-((tert-butoxycarbonyl)amino)-5-methylthiazol-4-yl)acetamido)-7-oxo-4-thia-1-azabicyclo[3.2.0]heptane-3-carboxylate |
| 111 | 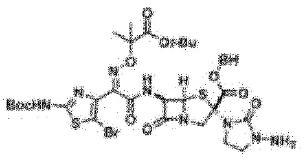 | Benzhydryl (3R,5R 6R)-3-(3-amino-2-oxoiniidazolidin-1-yl)-6-((Z)-2-(5-bromo-2-((ter-t-butoxycarbonyf)amino)thiazol-4-yl)-2-(((1-(tert-butoxy)-2-methyl-1-oxopropan-2-yl)oxy)imino)acetamido)-7-oxo-4-thia-1-azabicyclo[3.2.0]heptane-3-carboxylate |

(continued)

| Ref. Ex. No. | Structural Formula | Name |
|---|---|---|
| 112 | 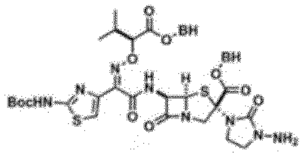 | Benzhydryl (3R,5R,6R)-3-(3-amino-2-oxoimidazolidin-1-yl)-6-((Z)-2-((((S)-1-(benzhydryloxy)-3-methyl-1 -oxobutan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate |
| 113 | 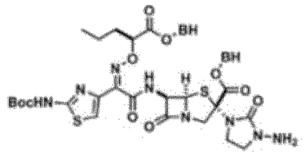 | Benzhydryl (3R,5R,6R)-3-(3-amino-2-oxoimidazolidin-1-yl)-6-((Z)-2-((((S)-1-(benzhydryloxy)-1-oxopentan-2-yl)oxy)imino)-2-(2-{{tert-butoxycarbonyl)amino)thiazol-4-vl)acetamido)-7-oxo-4-thia-1-azabicyclo[3.2.0]heptane-3-carboxylate |
| 114 | 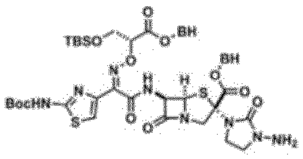 | Benzhydryl (3R,5R,6R)-3-(3-amino-2-oxoimidazolidin-1-yl)-6-((R,Z)-5-(benzhydryloxy)carbonyl-2-(2-((tert-butoxycarbonyl)amino)thiazol-4-yl)-8,8,9,9-tetramethyl-4,7-dioxa-3-aza-8-siladec-2-enamido)-7-oxo-4-thia-1-azabicyclo[3.2.0]heptane-3-carboxylate |

The measured values of NMR of the compounds in the table are as follows.

Reference Example 105

NMR: 0.94 (3H, t, J = 7.4 Hz), 1.55 (9H, s), 1.85-2.02 (2H, m), 3.30-3.57 (5H, m), 3.84 (2H, s), 4.91 (1H, dd, J = 7.8, 5.2 Hz), 5.03 (1H, d, J = 13.2 Hz), 5.56 (1H, d, J = 3.6 Hz), 5.64 (1H, dd, J = 6.6, 3.4 Hz), 6.86 (1H, s), 6.90 (1H, s), 7.01-7.09 (1H, m), 7.12-7.40 (20H, m), 7.58 (1H, d, J = 6.8 Hz), 8.11 (1H, s)

Reference Example 106

NMR: 1.51-1.64 (4H, m), 1.58 (9H, s), 3.33-3.55 (5H, m), 3.82 (2H, s), 4.91 (1H, d, J = 13.6 Hz), 5.56 (1H, d, J = 4.0 Hz), 5.78 (1H, dd, J = 8.6, 3.4 Hz), 6.83 (1H, s), 6.87 (1H, s), 7.07-7.14 (2H, m), 7.16-7.42 (19H, m), 8.60 (1H, s)

Reference Example 107

NMR: 1.46-1.59 (4H, m), 1.53 (9H, s), 3.32-3.54 (5H, m), 3.81 (2H, s), 4.92 (1H, d, J = 13.2 Hz), 5.53 (1H, d, J = 4.0 Hz), 5.75 (1H, dd, J = 8.6, 3.8 Hz), 6.82 (1H, s), 6.89 (1H, s), 7.14-7.43 (21H, m), 8.08 (1H, s)

Reference Example 108

NMR: 1.52 (9H, s), 1.90-2.02 (2H, m), 2.37-2.66 (4H, m), 3.35-3.51 (5H, m), 3.82 (2H, s), 4.95 (1H, d, J = 13.2 Hz), 5.52 (1H, d, J = 3.6 Hz), 5.77 (1H, dd, J = 9.4, 3.4 Hz), 6.83 (1H, s), 6.89 (1H, s), 7.13-7.42 (22H, m), 8.11 (1H, s)

Reference Example 109

NMR: 1.57 (9H, s), 1.97-2.09 (2H, m), 2.45-2.59 (2H, m), 2.60-2.71 (2H, m), 3.31-3.47 (4H, m), 3.50 (1H, dd, J = 13.2,0.8 Hz), 3.83 (2H, s), 4.93 (1H, d, J = 13.2 Hz), 5.54 (1H, d, J = 3.6 Hz), 5.80 (1H, dd, J = 8.6, 3.4 Hz), 6.83 (1H, s), 6.88 (1H, s), 7.02-7.13 (2H, m), 7.16-7.43 (19H, m), 8.63 (1H, s)

Reference Example 110

NMR: 1.39 (9H, s), 1.47-1.55 (15H, m), 2.48 (3H, s), 3.37-3.56 (5H, m), 3.82 (2H, s), 4.96 (1H, d, J = 13.2 Hz), 5.56 (1H, d, J = 4.0 Hz), 5.83 (1H, dd, J = 9.2, 4.0 Hz), 6.88 (1H, s), 7.13-7.21 (2H, m), 7.22-7.39 (9H, m), 7.94 (1H, s)

Reference Example 111

NMR: 1.40 (9H, s), 1.50-1.56 (15H, m), 3.40-3.56 (5H, m), 3.82 (2H, s), 4.95 (1H, d, J = 13.2 Hz), 5.56 (1H, d, J = 4.0 Hz), 5.79 (1H, dd, J = 8.4, 3.8 Hz), 6.88 (1H, s), 7.17-7.42 (11H, m), 8.14 (1H, s)

Reference Example 112

NMR: 0.90 (3H, d, J = 6.8 Hz), 1.00 (3H, d, J = 7.2 Hz), 1.55 (9H, s), 2.25-2.38 (1H, m), 3.39-3.58 (5H, m), 3.85 (2H, s), 4.84 (1H, d, J = 4.8 Hz), 5.06 (1H, d, J = 13.2 Hz), 5.52-5.57 (1H, m), 5.59 (1H, d, J = 3.6 Hz), 6.86 (1H, s), 6.91 (1H, s), 6.99-7.05 (1H, m), 7.09-7.41 (20H, m), 7.81 (1H, d, J = 5.6 Hz), 8.13 (1H, s)

Reference Example 113

NMR: 0.88 (3H, t, J = 7.4 Hz), 1.34-1.46 (2H, m), 1.55 (9H, s), 1.82-1.92 (2H, m), 3.48-3.56 (5H, m), 3.86 (2H, s), 4.97 (1H, t, J = 6.8 Hz), 5.05 (1H, d, J = 13.2 Hz), 5.57 (1H, d, J = 4.0 Hz), 5.64 (1H, dd, J = 6.6, 3.4 Hz), 6.86 (1H, s), 6.89 (1H, s), 7.01-7.08 (1H, m), 7.11-7.41 (20H, m), 7.53 (1H, d, J = 7.2 Hz), 8.12 (1H, s)

Reference Example 114

NMR: -0.04 (6H, s), 0.82 (9H, s), 1.55 (9H, s), 3.24-3.41 (4H, m), 3.50 (1H, d, J = 12.8 Hz), 3.82 (2H, s), 4.09-4.16 (2H, m), 5.01 (1H, d, J = 13.2 Hz), 5.05 (1H, t, J = 4.6 Hz), 5.50 (1H, d, J = 4.0 Hz), 5.77 (1H, dd, J = 8.8, 3.6 Hz), 6.83 (1H, s), 6.90 (1H, s), 6.99-7.08 (1H, m), 7.09-7.43 (21H, m), 8.15 (1H, s)

Reference Example 115

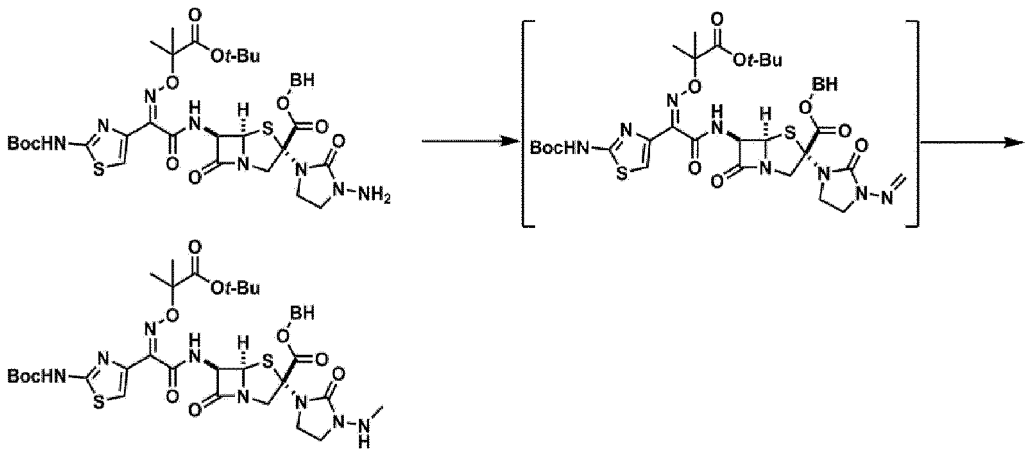

Paraformaldehyde (123 mg) and NMP (1 mL) were added to benzhydryl (3R,5R,6R)-3-(3-amino-2-oxoimidazolidin-1-yl)-6-((Z)-2-(((1-(tert-butoxy)-2-methyl 1-oxopropan-2-yl)oxy)imino)-2-(2-((tert-butoxycarbonyl)amino)thiazol-4-yl)acetamido)-7-ox o-4-thia-1-azabicyclo[3.2.0] heptane-3-carboxylate (100 mg), and the mixture was stirred at room temperature overnight. Acetic acid (14 μL) was added to the reaction mixture, and the mixture was stirred at room temperature for 8 hours. The reaction mixture was stirred at 50°C for 10 hours. The reaction mixture was cooled to room temperature, ethyl acetate (5 mL) and water (5 mL) were added to the reaction mixture, and the organic layer was separated. The organic layer was washed twice with a 5% aqueous sodium chloride solution. The organic layer was dehydrated and dried over anhydrous sodium sulfate, and the solvent was distilled off under reduced pressure. Dichloromethane (1 mL) was added to the residue, and a 85% borane-2-picoline complex (18 mg) and p-toluenesulfonic acid monohydrate (31 mg) were sequentially added thereto under ice cooling, and the reaction mixture was stirred at room temperature for 1 hour. At room temperature, ethyl acetate (5 mL) and water (5 mL) were added to the reaction mixture, and the organic layer was separated. The organic layer was washed twice with a 5% aqueous sodium chloride solution and then dehydrated and dried over anhydrous sodium sulfate. The solvent was distilled off under reduced pressure. The residue was purified by silica gel column chromatography [eluent; ethyl acetate:hexane = 70:30 → 100:0], thereby obtaining benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-methylamino)-2-oxoimidazolidin-1-yl)-7-ox o-4-thia-1-azabicyclo[3.2.0] heptane-3-carboxylate (52 mg) as a yellow oily substance.

NMR: 1.39 (9H, s), 1.51 (3H, s), 1.53 (3H, s), 1.54 (9H, s), 2.57 (3H, s), 3.39-3.57 (6H, m), 4.91 (1H, d, J = 13.2 Hz), 5.58 (1H, d, J = 4.0 Hz), 5.81 (1H, dd, J = 8.6, 3.4 Hz), 6.87 (1H, s), 7.15-7.41 (13H, m)

Reference Example 116

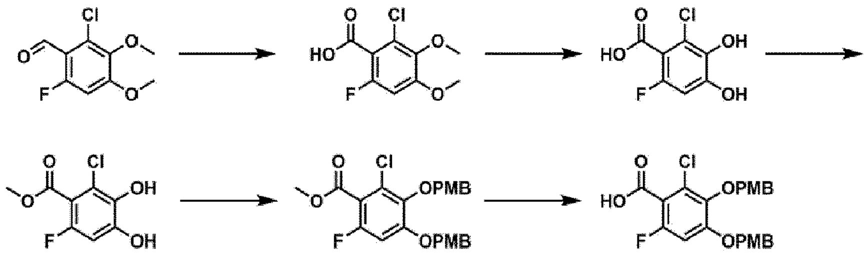

Reference Example 116 (1)

tert-Butanol (36 mL) and 2-methyl-2-butene (11 mL) were added to 2-chloro-6-fluoro-3,4-dimethoxybenzaldehyde (4.5 g). Sodium dihydrogen phosphate dihydrate (4.82 g) and water (9 mL) were added to the reaction mixture. Under ice cooling, an aqueous sodium chlorite solution (3.72 g of sodium chlorite and 9 mL of water) was added dropwise to the reaction mixture. The reaction mixture was stirred at room temperature for 4 hours and 30 minutes. Water (25 mL) was added to the reaction mixture. Under ice cooling, concentrated hydrochloric acid (3.9 mL) was added to the reaction mixture so that the pH was adjusted to 1.8. Solids were collected by filtration and washed with water. The solids were subjected to blast drying at 40°C, thereby obtaining a target substance (3.41 g) as yellow solids.

Reference Example 116 (2)

The compound (3.40 g) obtained in Reference Example 116 (1) was added to a 1 mol/L boron tribromide/dichloromethane solution (87 mL) at a temperature of 10°C or lower. The reaction mixture was stirred at room temperature for 6 hours and 30 minutes. Ice water (300 mL) was added to the reaction mixture, and extraction was performed using ethyl acetate (200 mL). The aqueous layer was extracted using ethyl acetate (200 mL). The organic layer was washed with a 5% aqueous sodium chloride solution (100 mL). The organic layer was dried over anhydrous magnesium sulfate, and the solvent was distilled off under reduced pressure. IPE was added to the residue, and solids were collected by filtration. The solids were dried, thereby obtaining a target substance (2.99 g) as gray solids.

Reference Example 116 (3)

Methanol (15 mL) and concentrated sulfuric acid (0.8 mL) were added to the compound (2.99 g) obtained in Reference Example 116 (2), and the mixture was stirred for 3 hours under reflux. Concentrated sulfuric acid (0.8 mL) was added to the reaction mixture, and the mixture was stirred at the same temperature for 4 hours. Concentrated sulfuric acid (0.8 mL) was added to the reaction mixture, and the mixture was stirred at the same temperature overnight. Concentrated sulfuric acid (0.4 mL) was added to the reaction mixture, and the mixture was stirred at the same temperature overnight. The reaction mixture was cooled to room temperature and added to ice water. The mixture was subjected to extraction using ethyl acetate. The solvent was distilled off under reduced pressure, thereby obtaining a target substance (3.2 g) as brown solids.

Reference Example 116 (4)

N,N-dimethylacetamide (22 mL), p-methoxybenzyl chloride (4.97 g), potassium carbonate (4.38 g), and potassium iodide (0.24 g) were added to the compound (3.18 g) obtained in Reference Example 116 (3). The reaction mixture was stirred at 55°C for 3 hours and 15 minutes. The reaction mixture was added to water (180 mL). Concentrated hydrochloric acid was added to the mixture so that the pH was adjusted to 7.5. Solids were collected by filtration and washed with water. The solids were subjected to blast drying at 40°C, thereby obtaining a target substance (4.83 g) as yellow solids.

Reference Example 116 (5)

THF (14 mL), 1 mol/L aqueous sodium hydroxide solution (31 mL), and methanol (7 mL) were added to the compound (4.8 g) obtained in Reference Example 116 (4). The reaction mixture was stirred for 3 hours under reflux. The reaction mixture was cooled to room temperature. At room temperature, concentrated hydrochloric acid (4.8 mL) and water (22 mL) were added to the reaction mixture. Solids were collected by filtration and washed with water. The solids were dried, thereby obtaining 2-chloro-6-fluoro-3,4-bis((4-methoxybenzyl)oxy)benzoic acid (4.12 g) as white solids.
NMR(DMSO-$d_6$): 3.74 (3H, s), 3.78 (3H, s), 4.85 (2H, s), 5.16 (2H, s), 6.85 (2H, d, J = 8.4 Hz), 6.99 (2H, d, J = 8.8 Hz), 7.22-7.31 (3H, m), 7.44 (2H, d, J = 8.4 Hz), 13.60-13.90 (1H, brs)
The compounds in Table 17 were obtained in the same manner as in Reference Example 48.

[Table 17]

| Ref. Ex. No. | Structural Formula | Name |
|---|---|---|
| 117 | 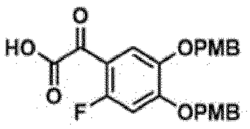 | 2-(2-Fluoro-4,5-bis((4-methoxybenzyl)oxy)phenyl)-2-oxoacetic acid |
| 118 | 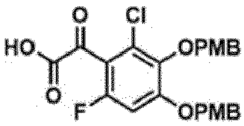 | 2-(2-Chloro-6-fluoro-3,4-bis((4-methoxybenzylyoxy)phenyl)-2-oxoacetic acid |
| 119 | 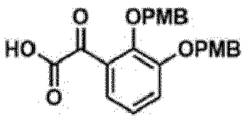 | 2-(2,3-Bis((4-methoxybenzyl)oxy)phenyl)-2-oxoacetic acid |

The measured values of NMR of the compounds in the table are as follows.

Reference Example 117

NMR(DMSO-$d_6$): 3.72-3.78 (6H, m), 4.98-5.34 (4H, m), 6.86-7.01 (4H, m), 7.09-7.46 (6H, m)

Reference Example 118

NMR(DMSO-$d_6$): 3.74 (3H, s), 3.78 (3H, s), 4.87 (2H, s), 5.21 (2H, s), 6.84 (2H, d, J = 8.4 Hz), 7.00 (2H, d, J = 8.8 Hz), 7.25 (2H, d, J = 8.8 Hz), 7.36 (1H, d, J = 12.0 Hz), 7.46 (2H, d, J = 8.8 Hz)

Reference Example 119

NMR(DMSO-$d_6$): 3.73 (3H, s), 3.78 (3H, s), 4.90 (2H, s), 5.15 (2H, s), 6.78 (2H, d, J = 8.8 Hz), 6.98 (2H, d, J = 8.8 Hz), 7.17 (2H, d, J = 8.8 Hz), 7.21-7.35 (2H, m), 7.46 (2H, d, J = 8.8 Hz), 7.55 (1H, dd, J =8.0, 1.6 Hz), 13.50-14.70 (1H, brs)
The compounds of Table 18 were obtained in the same manner as in Reference Example 16 (1) and Reference Example 80 (3).

[Table 18]

| Ref. Ex. No. | Structural Formula | Name |
|---|---|---|
| 120 | 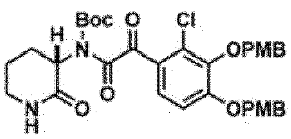 | teil-Btityl(S)-(2-(2-cliloro-3,4-bis((4-methoxybenzyloxy)phenyl)-2-oxoacety)(2-oxopiperidin-3-yl)carbamate |

(continued)

| Ref. Ex. No. | Structural Formula | Name |
|---|---|---|
| 121 | 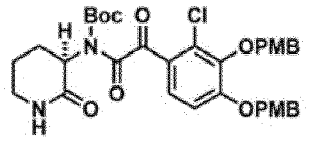 | tert-Butyl(R)-(2-(2-chloro-3,4-bis((4-methoxybenzyl)oxy)phenyl)-2-oxoacetyl)(2-oxopiperidin-3-yl)carbamate |
| 122 | 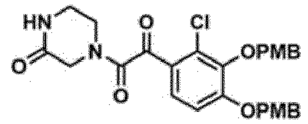 | 1-(2-cliloro-3,4-bis((4-methoxybenzyl)oxy)phenyl)-2-(3-oxopiperidin-1-yl)ethane-1,2-dione |

The measured values of NMR of the compounds in the table are as follows.

Reference Example 120

NMR: 1.31 (9H, s), 1.85-2.03 (2H, m), 2.13-2.34 (2H, m), 3.28-3.55 (2H, m), 3.79 (3H, s), 3.84 (3H, s), 4.94 (2H, s), 5.10-5.16 (3H, m), 6.01 (1H, s), 6.78-6.85 (2H, m), 6.90-6.96 (2H, m), 7.02 (1H, d, J = 8.8 Hz), 7.30-7.39 (4H, m), 7.87 (1H, d, J = 8.8 Hz)

Reference Example 121

NMR: 1.32 (9H, s), 1.83-2.02 (2H, m), 2.12-2.34 (2H, m), 3.27-3.53 (2H, m), 3.79 (3H, s), 3.83 (3H, s), 4.94 (2H, s), 4.98-5.23 (3H, m), 5.89 (1H, s), 6.78-6.85 (2H, m), 6.90-6.96 (2H, m), 7.02 (1H, d, J = 8.8 Hz), 7.30-7.39 (4H, m), 7.87 (1H, d, J = 8.8 Hz)

Reference Example 122

NMR: 3.44-3.56 (3H, m), 3.64-3.72 (1H, m), 3.76-3.87 (7H, m), 3.89-3.97 (1H, m), 4.92-4.98 (2H, m), 5.14 (2H, s), 6.10-6.25 (1H, m), 6.78-6.88 (2H, m), 6.90-6.97 (2H, m), 6.98-7.05 (1H, m), 7.28-7.40 (4H, m), 7.63-7.73 (1H, m)

Reference Example 123

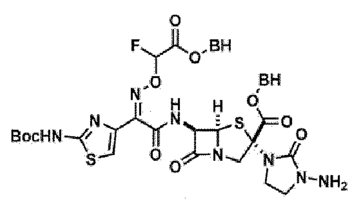

In the same manner as in Reference Example 35, benzhydryl(3R,5R,6R)-3-(3-amino-2-oxoimidazolidin-1-yl)-6-((Z)-2-((2-(benzhydryloxy)-1-fi uoro-2-oxoethoxy)imino)-2-(2-((tert-butoxycarbonyl)amino)thiazol-4-yl)acetamido)-7-oxo-4-t hia-1-azabicyclo[3.2.0]heptane-3-carboxylate was obtained.

NMR: 1.55 (9H, s), 3.30-3.52 (5H, m), 3.83 (2H, s), 5.00 (1H, d, J = 13.2 Hz), 5.28 [5.50] (1H, d, J = 3.8 Hz), 5.59 [5.64] (1H, dd, J = 8.4, 3.4 Hz), 6.16 [6.23] (1H, d, J = 56.8 Hz), 6.68 [6.76] (1H, d, J = 8.8 Hz), 6.83 (1H, d, J = 4.8 Hz), 6.96-7.06 (1H, m), 7.07-7.17 (1H, m), 7.18-7.41 (20H, m), 8.24-8.41 (1H, m)

Reference Example 124

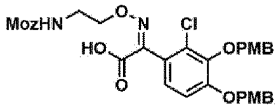

By using (4-methoxybenzyl)(2-bromoethyl)carbamate, (Z)-9-(2-chloro-3,4-bis((4-methoxybenzyl)oxy)phenyl)-1-(4-methoxyphenyl)-3-oxo-2,7-dioxa -4,8-diazadec-8-en-10-oic acid was obtained in the same manner as in Reference Example 73.

NMR(DMSO-$d_6$): 3.25-3.36 (2H, m), 3.74 (3H, s), 3.74 (3H, s), 3.78 (3H, s), 4.10-4.19 (2H, m), 4.89 (2H, s), 4.95 (2H, s), 5.16 (2H, s), 6.83-6.95 (4H, m), 6.99 (2H, d, J = 8.8 Hz), 7.10-7.34 (7H, m), 7.44 (2H, d, J = 8.4 Hz)

The compounds in Table 19 were obtained in the same manner as in Reference Example 84.

[Table 19]

| Ref. Ex. No. | Structural Formula | Name |
|---|---|---|
| 125 | 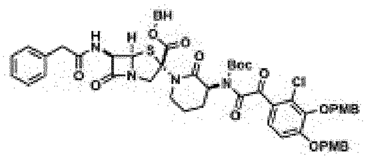 | Benzhydryl(3R,5R,6R)-3-((S)-3-(N-(tert-butoxycarbonyl)-2-(2-chloro-3,4-bis(4-methoxybenzyl)oxy)phenyl)-2-oxoacetamido)-2-oxopiperidin-1-yl)-7-oxo-6-(2-phenylacetamido)-4-thia-1-azabicyclo[3.2.0]heptane-3-carboxylate |
| 126 | 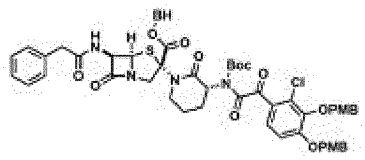 | Benzhydryl(3R,5R,6R)-3-((R)-3-(N-(tert-butaxycarbonyl)-2-(2-chloro-3,4-bis((4-methoxybenzyl)oxy)phenyl)-2-oxoacetamido)-2-oxopiperidin-1-yl)-7-oxo-6-(2-phenylacetamido)-4-thia-1-azabicyclo[3.2.0]heptane-3-carboxylate |
| 127 | 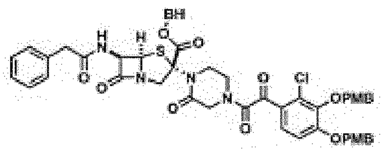 | Benzhydryl(3R,5R,6R)-3-((S)-3-(4-(2-(2-chloro-3,4-bas((4-methoxybenzyl)oxy)phenyl)-2-oxoacetyl)-2-oxopiperazin-1-yl)-7-oxo-6-(2-phenylacetamido)-4-thia-1-azabicyclo[3.2.0]heptane-3-carboxylate |

The measured values of NMR of the compounds in the table are as follows.

Reference Example 125

NMR: 1.20-1.35 (9H, m), 1.85-2.01 (1H, m), 2.09-2.21 (2H, m), 2.23-2.44 (1H, m), 3.32 (1H, d, J = 13.2 Hz), 3.49-3.65 (4H, m), 3.76 (3H, s), 3.84 (3H, s), 4.51 (1H, d, J = 12.4 Hz), 4.95 (2H, s), 5.02-5.22 (3H, m), 5.32 (1H, d, J = 4.0 Hz), 5.66 (1H, dd, J = 9.6, 3.6 Hz), 6.71-6.86 (4H, m), 6.86-7.10 (5H, m), 7.18-7.20 (1H, m), 7.20-7.41 (16H, m), 7.71-7.95 (1H, m)

Reference Example 126

NMR: 1.22-1.34 (9H, m), 1.81-2.01 (1H, m), 2.08-2.22 (2H, m), 2.23-2.47 (1H, m), 3.32 (1H, d, J = 13.2 Hz), 3.44-3.59 (4H, m), 3.76 (3H, s), 3.84 (3H, s), 4.52 (1H, d, J = 12.4 Hz), 4.83-5.26 (5H, m), 5.32 (1H, d, J = 4.0 Hz), 5.67 (1H, dd, J = 9.6, 4.0 Hz), 6.73-6.86 (3H, m), 6.88-7.46 (23H, m), 7.70-7.94 (1H, m)

Reference Example 127

NMR: 2.96-3.07 (1H, m), 3.25-3.33 (2H, m), 3.63-3.95 (12H, m), 4.88-4.97 (2H, m), 5.08-5.17 (2H, m), 5.19-5.29 (1H, m), 5.39-5.44 (1H, m), 5.44-5.53 (1H, m), 6.04-6.16 (1H, m), 6.72-7.03 (10H, m), 7.05-7.14 (3H, m), 7.15-7.46 (11H, m), 7.54-7.73 (2H, m)

Reference Example 128

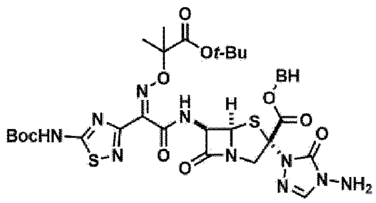

In the same manner as in Reference Example 27, benzhydryl(3R,5R,6R)-3-(4-amino-5-oxo-4,5-dihydro-1H-1,2,4-triazol-1-yl)-6-((Z)-2-(((1-(ter t-Butoxy)-2-methyl-1-oxopropan-2-yl)oxy)imino)-2-(2-((tert-butoxycarbonyl)amino)-1,2,4-thi adiazol-3-yl)acetamido)-7-oxo-4-thia-1-azabicyclo[3.2.0]heptane-3-carboxylate was obtained.
NMR: 1.38 (9H, s), 1.54-1.57 (9H, m), 1.59-1.62 (6H, m), 4.00(1H, dd, J = 13.2, 0.8 Hz), 4.20-4.33 (2H, brs), 4.70 (1H, d, J = 13.2 Hz), 5.65 (1H, d, J = 4.0 Hz), 5.96 (1H, dd, J = 9.2, 4.0 Hz), 6.80 (1H, s), 7.17-7.40 (10H, m), 7.46-7.55 (1H, m), 7.56 (1H, s), 8.63-8.73 (1H, brs)

Reference Example 129

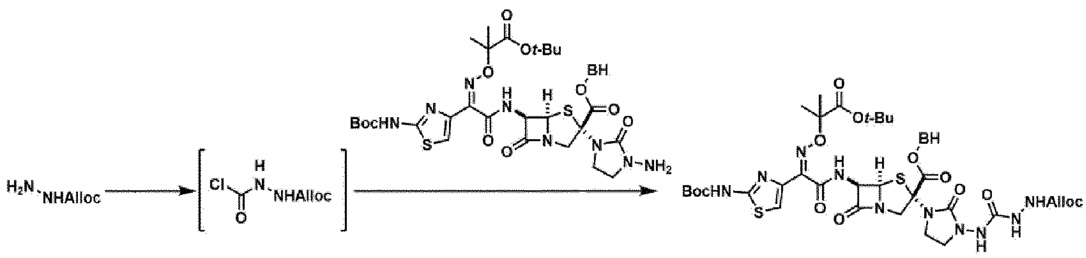

By using allylhydrazinecarboxylate (751 mg), benzhydryl (3R,5R,6R)-3-(3-(2-((allyloxy)carbonyl)hydrazine-1-carboxamido)-2-oxoimidazolidin-1-yl)-6 -((Z)-2-(((1-(tert-butoxy)-2-methyl-1-oxopropan-2-yl)oxy)imino)-2-(2-((tert-butoxycarbonyl)a mino)thiazol-4-yl)acetamido)-7-oxo-4-thia-1-azabicyclo[3.2.0]heptane-3-carboxylate (926 mg) was obtained in the same manner as in Reference Example 47 (1) as light yellow solids.
NMR: 1.37 (9H, s), 1.52 (3H, s), 1.53 (9H, s), 1.55 (3H, s), 2.76-3.72 (5H, m), 3.83-4.12 (1H, m), 4.63 (2H, d, J = 5.6 Hz), 4.66-4.79 (1H, m), 5.17-5.27 (1H, m), 5.27-5.37 (2H, m), 5.67-5.78 (1H, m), 5.83-5.97 (1H, m), 6.85 (1H, s), 7.15-7.42 (12H, m), 7.45-7.81 (3H, m)

Reference Example 130

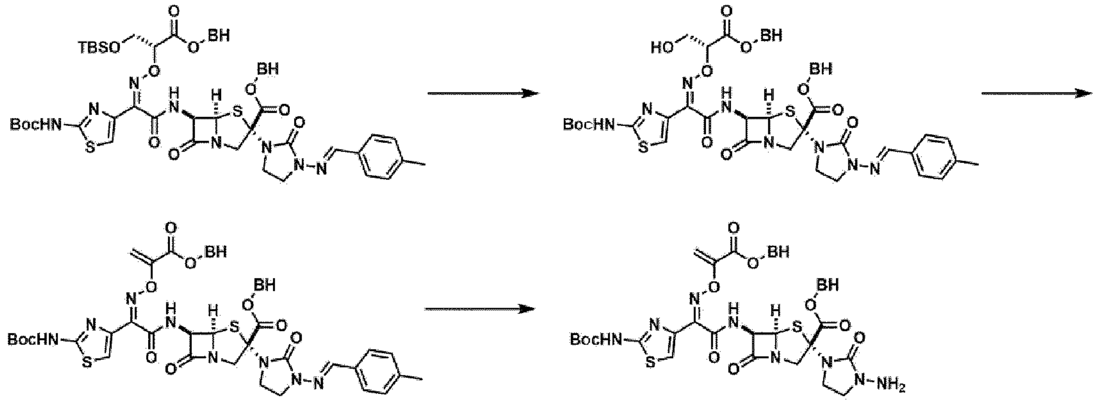

Reference Example 130 (1)

THF (25 mL) was added to benzhydryl (3R,5R,6R)-6-((R,Z)-5-((benzhydryloxy)carbonyl)-2-(2-((tert-butoxycarbonyl)amino)thiazol-4 -yl)-8,8,9,9-tetramethyl-4,7-dioxa-3-aza-8-siladec-2-enamido)-3-(3-(((E)-4-methylbenzylidene ) amino)-2-oxoimidazolidin-1-yl)-7-oxo-4-thia-1-azabicyclo[3.2.0]heptane-3-carboxylate (2.0 g), and the mixture was cooled to -10°C. Acetic acid (0.45 mL) and 1 mol/L tetra-n-butylammonium fluoride/THF solution (5.28 mL) was added to the reaction mixture. The reaction mixture was stirred for 1 hour under ice cooling, and then stirred for 5 hours at room temperature. Ethyl acetate (50 mL), a 5% aqueous citric acid solution (20 mL), and distilled water (20 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 50:50 → 100:0], thereby obtaining a target substance (2.34 g) as white solids.

Reference Example 130 (2)

THF (5 mL) was added to the compound (500 mg) obtained in Reference Example 130 (1). Under ice cooling, triphenylphosphine (150 mg), a 40% diisopropylazodicarboxylate/toluene solution (290 μL), and diphenylphosphoryl azide (124 μL) were added to the mixture. The reaction mixture was stirred for 1 hour under ice cooling, and then stirred for 5 hours at room temperature. The solvent was distilled off under reduced pressure, and the residue was purified by silica gel column chromatography [eluent; ethyl acetate:hexane = 20:80 → 70:30], thereby obtaining a target substance (368 mg) as brown solids.

Reference Example 130 (3)

By using the compound (320 mg) obtained in Reference Example 130 (2), benzhydryl (3R,5R,6R)-3-(3-amino-2-oxoimidazolidin-1-yl)-6-((Z)-2-(((3-(benzhydryloxy)-3-oxoprop-1-en-2-yl)oxy)imino)-2-(2-((tert-butoxycarbonyl)amino) thiazol-4-yl)acetamido)-7-oxo-4-thia-1-azabicyclo[3.2.0]heptane-3-carboxylate (280 mg) was obtained in the same manner as in Reference Example 35 (2) as yellow solids.

NMR: 1.55 (9H, s), 3.27-3.49 (5H, m), 3.83 (2H, s), 4.99 (1H, d, J = 13.6 Hz), 5.34 (1H, d, J = 3.6 Hz), 5.55 (1H, d, J = 2.0 Hz), 5.62 (1H, dd, J = 8.4, 3.2 Hz), 5.74 (1H, d, J = 2.0 Hz), 6.69 (1H, d, J = 8.4 Hz), 6.83 (1H, s), 6.92 (1H, s), 7.09-7.39 (21H, m), 8.38 (1H, s)

Example 1

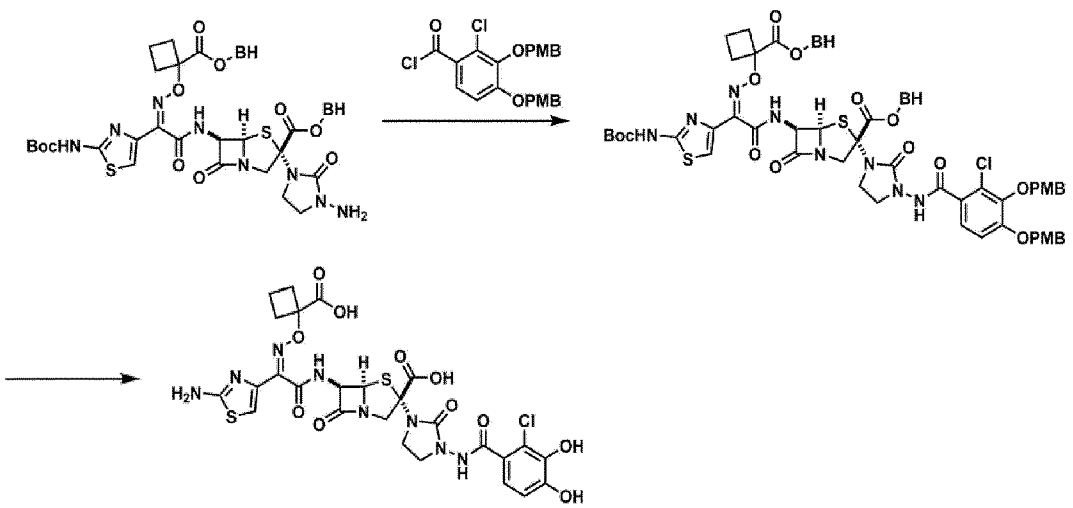

Example 1 (1)

THF (4.2 mL) and water (4.2 mL) were added to benzhydryl (3R,5R,6R)-3-(3-amino-2-oxoimidazolidin-1-yl)-6-((Z)-2-((1-((benzhydryloxy)carbonyl)cyclo butoxy)imino)-2-(2-((tert-butoxycarbonyl)amino)thiazol-4-yl)acetamido)-7-oxo-4-thia-1-azabi cyclo[3.2.0]heptane-3-carboxylate (104 mg), and the mixture was stirred under ice cooling.

At the same temperature, sodium hydrogen carbonate (11 mg) was added to the reaction mixture, and then 2-chloro-3,4-bis((4-methoxybenzyl)oxy)benzoyl chloride (52 mg) obtained in Reference Example 1 was sequentially added thereto. The reaction mixture was stirred at room temperature for 4 hours, ethyl acetate (15 mL) and water (15 mL) were then added thereto, and the organic layer was separated. The organic layer was washed with a saturated aqueous sodium chloride solution and dehydrated and dried over anhydrous sodium sulfate. The solvent was distilled off under reduced pressure, thereby obtaining a target substance (126 mg) as light yellow solids.

Example 1 (2)

Dichloromethane (1.9 mL) was added to the compound (126 mg) obtained in Example 1 (1), and the mixture was cooled to -20°C. At the same temperature, anisole (0.59 mL) and aluminum chloride (180 mg) were sequentially added to the reaction mixture, and the reaction mixture was stirred at the same temperature for 30 minutes. The reaction mixture was added to a mixture of acetonitrile (5 mL), water (5 mL), and trisodium citrate dihydrate (596 mg) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-((1-carboxycyclobutoxy)imino)acetamido)-3-(3-(2-chloro-3,4-dihydroxybenzamido)-2-oxoimidazolidin-1-yl)-7-oxo-4-thia-1-azabicyclo[3.2.0] heptane-3-carboxylic acid (15 mg) as white solids.

NMR: 1.80-2.09 (2H, m), 2.24-2.40 (2H, m), 2.40-2.62 (2H, m), 3.64 (1H, d, J = 12.4 Hz), 3.69-3.80 (4H, m,), 4.69 (1H, d, J = 12.4 Hz), 5.73 (1H, d, J = 4.0 Hz), 5.79 (1H, d, J = 2.8 Hz), 6.94 (1H, d, J = 8.0 Hz), 7.07 (1H, s), 7.10 (1H, d, J = 8.4 Hz)

MS: 725.00 $[M + H]^+$, 722.95 $[M - H]^-$

The compounds shown in Table 20 were obtained in the same manner as in Example 1.

[Table 20]

| Ex. No. | Structural Formula | Name |
|---|---|---|
| 2 | 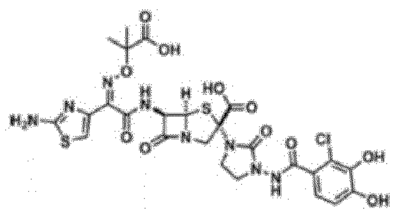 | (3R,5R,61t)-6-((Z)-2-(2-aminothiazol-4-yl)-2-(((2-carboxypropan-2-yl)oxy)imino)acetamido)-3-(3-(2-chloro-3,4-dihydroxybenzamido)-2-oxoimidazolidin-1-yl)-7-oxo-4-thia-1-azabicyclo[3.2.0] heptane-3-carboxylic acid |
| 3 | 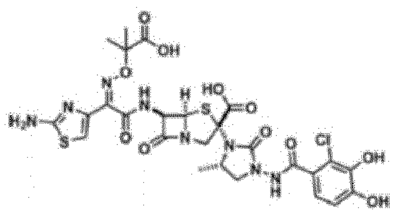 | (3R,5R,6R)-6-((Z)-2-(2-aminothiazol-4-yl)-2-(((2-carboxypropan-2-yl)oxy)imino)acetamido)-3-((S)-3-(2-chloro-3 ,4-dihydroxybenzamido)-5-methyl-2-oxoimidazolidin- 1-yl)-7-oxo-4-thia-1-azabicyclo[3.2.0]heptane-3-carboxylic acid |
| 4 | 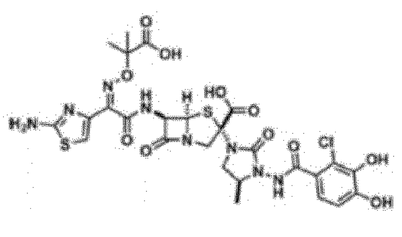 | (3R,5R,6R)-6-((Z)-2-(2-aminothiazol-4-yl)-2-(((.2-carboxypropan-2-yl)oxy)imino)acetamido)-3-((S)-3-(2-chloro-3,4-dihydroxybenzamido)-4-methyl-2-oxoimidazolidin-1-yl)-7-oxo-4-thia-1-azabicyclo[3.2.0]heptane-3-carboxylic acid |

(continued)

| Ex. No. | Structural Formula | Name |
|---|---|---|
| 5 | 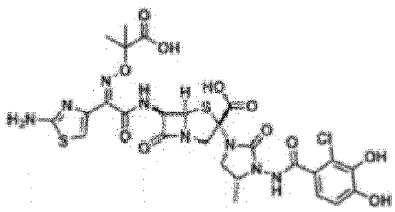 | (3R,5R,6R)-6-((.Z)-2-(2-aminothiazol-4-yl)-2-(((2-carboxypropan-2-yl)oxy)imino)acetamido)-3((R)-3-(2-chloro-3.4-dihydroxybenzamido)-4-methyl-2-oxoimidazolidin-1-yl)-7-oxo-4-thia-1-azabicyclo[3.2.0]heptane-3-carboxylic acid |
| 6 | 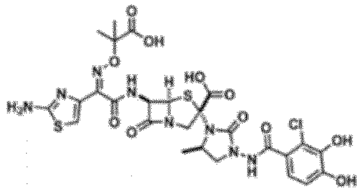 | (3R,5R,6R)-6-((Z)-2-(2-aminothiazol-4-yl)-2-(((2-carboxypropan-2-yl)oxy)imino)acetamido)-3-((R)-3-(2-chloro-3,4-dihydroxybenzamido)-5-methyl-2-oxoimidazolidin-1-yl)-7-oxo-4-thia-1-azabicyclo[3.2.0]heptane-3-carboxylic acid |
| 7 | 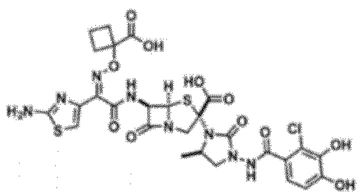 | (3R,-5R,6R)-6-((Z)-2-(2-ati-Anothiazol-4-yl)-2-(((1-carboxycyclobutoxy)imino)acetamido)-3-((R)-3-(2-chloro-3,4-dihydroxybenzamido)-5-methyl-2-oxoimidazolidin-1-yl)-7-oxo-4-thia-1-azabicyclo[3.2.0]heptane-3-carboxylic acid |
| 8 | 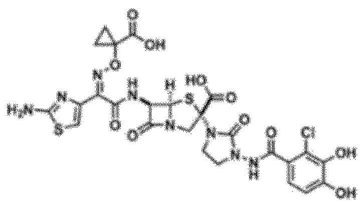 | (3R,5R,6R)-6-((Z)-2-(2-aminothiazol-4-yl)-2-((1-carboxycyclopropoxy)imino)acetamido)-3-(3-(2-chloro-3,4-dihydroxybenzamido)-2-oxoimidazolidin- 1-yl)-7-oxo-4-thia-1-azabicyclo[3.2.0]heptane-3-carboxylic acid |
| 9 | 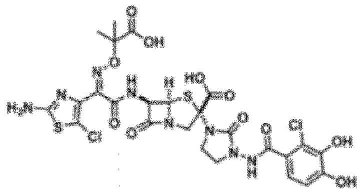 | (3R,3R,6R)-6-((Z)-2-(2-amino-5-chlorothiazol-4-yl)-2-(((2-carboxypropan-2-yl)oxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |
| 10 | 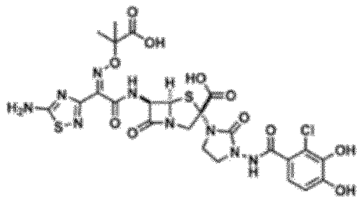 | (3R,5R,6R)-6-((Z)-2-(5-amino-1,2,4-thiadiazol-3-yl)-2-(((2-carboxypropan-2-yl)oxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |
| 11 | 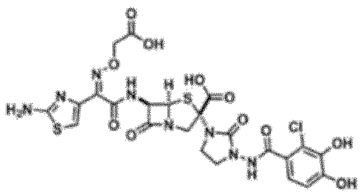 | (3R,5R,6R)-6-((Z)-2-(2-aminothiazol-4-yl)-2-((carboxymethoxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |

(continued)

| Ex. No. | Structural Formula | Name |
|---|---|---|
| 12 | 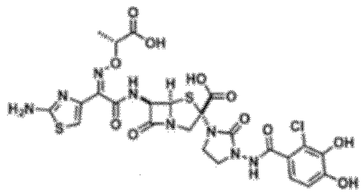 | (3R,5R,6R)-6-((Z)-2-(2-aminothiazol-4-yl)-2-((R)-1-carboxyethoxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |
| 13 | 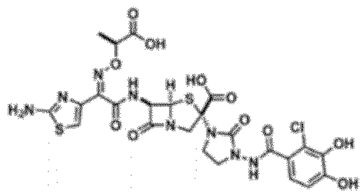 | (3R,5R,6R)-6-((Z)-2-(2-aminothiazol-4-yl)-2-(((S)-1-carboxyethoxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |
| 14 | 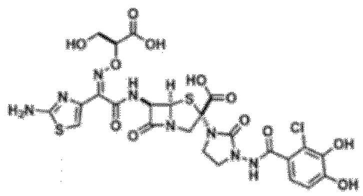 | (3R,5R,6R)-6-((Z)-2-(2-aminothiazol-4-yl)-2-(((S)-1-carboxy-2-hydroxyethoxy)imino)acetamido)-3-(3-(2-chloro-3,4-dihydroxybenzamido)-2-oxoimidazolidin- 1-yl)-7-oxo-4-thia-1-azabicyclo[3.2.0]heptane-3-carboxylic acid |
| 15 | 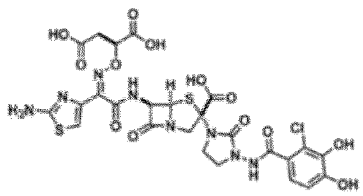 | (S)-2-((((Z)-1-(2-aminothiazol-4-yl)-2-(((3R,5R,6R)-3-carboxy-3-(3-(2-chloro-3,4-dihydroxybenzamido)-2-oxoimidazolidin-1-yl)-7-oxo-4-thia-1-azabicyclo[3.2.0]heptan-6-yl)amino)-2-oxoethylidene)amino)oxy)succinic acid |
| 16 | 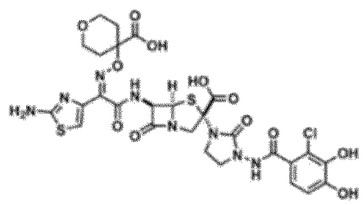 | (3R,5R,6R)-6-((Z)-2-(2-aminothiazol-4-yl)-2-((4-carboxytetrahydro-2H-pyran-4-yl)oxy)imino)acetamido)-3-(3-(2-chloro-3,4-dihydroxybenzamido)-2-oxoimidazolidin-1-yl)-7-oxo-4-thia-1-azabicyclo[3.2.0]heptane-3-carboxylic acid |
| 17 | 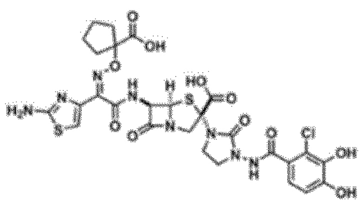 | (3R,5R,6R)-6-((Z)-2-(2-aminothiazol-4-yl)-2-(((1-carboxycyclopentyl)oxy)imino)acetamido)_3-(3-(2-chloro-3,4-dihydroxybenzamido)-2-oxoimidazolidin-1-yl)-7-oxo-4-thia-1-azabicyclo[3.2.0]heptane-3-carboxylic acid |
| 18 | 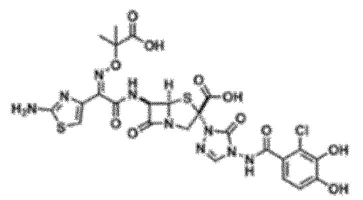 | (3R,3R,6R)-6-((Z)-2-(2-aminothiazol-4-yl)-2-(((2-carboxypropan-2-yl)oxy)imino)acetamido)-3-(4-(2-chloro-3,4-dihydroxybenzamido)-5-oxo-4,5-dihydro-1H-1,2,4-triazol-1-yl)-7-oxo-4-thia-1-azabicyclo[3.2.0]heptane-3-carboxylic acid |

The measured values of NMR and MS of the compounds in the table are as follows.

Example 2

NMR: 1.49 (3H, s), 1.51 (3H, s), 3.64 (1H, d, J = 12.4 Hz), 3.70-3.80 (4H, m), 4.68 (1H, d, J = 12.4 Hz), 5.71 (1H, d, J = 3.6 Hz), 5.76 (1H, d, J = 2.8 Hz), 6.94 (1H, d, J = 8.4 Hz), 7.04 (1H, s), 7.10 (1H, d, J = 8.4 Hz)
MS: 713.00 $[M + H]^+$, 710.90 $[M - H]^-$

Example 3

NMR: 1.28 (3H, d, J = 6.0 Hz), 1.42 (3H, s), 1.44 (3H, s), 3.28-3.40 (2H, m), 3.76-3.84 (1H, m), 4.21-4.31 (1H, m), 5.66 (2H, s), 6.87 (1H, d, J = 8.4 Hz), 6.97 (1H, s), 7.04 (1H, d, J = 8.4 Hz)
MS: 727.05 $[M + H]^+$, 725.00 $[M - H]^-$

Example 4

NMR: 1.28 (3H, d, J = 6.0 Hz), 1.40 (3H, s), 1.42 (3H, s), 3.17 (1H, t, J = 9.4 Hz), 3.57 (1H, d, J = 13.2 Hz), 3.73-3.81 (1H, m), 3.85-3.97 (1H, m), 4.54 (1H, d, J = 12.4 Hz), 5.60 (1H, d, J = 3.6 Hz), 5.67 (1H, d, J = 2.8 Hz), 6.85 (1H, d, J = 8.4 Hz), 6.95 (1H, s), 7.02 (1H, d, J = 8.4 Hz)
MS: 727.05 $[M + H]^+$, 725.10 $[M - H]^-$

Example 5

NMR: 1.37 (3H, d, J = 5.6 Hz), 1.49 (3H, s), 1.51 (3H, s), 3.35 (1H, t, J = 8.0 Hz), 3.61 (1H, dd, J = 12.6,1.0 Hz), 3.94-4.08 (2H, m), 4.71 (1H, d, J = 12.8 Hz), 5.72 (1H, d, J = 3.6 Hz), 5.77 (1H, dd, J = 3.8, 0.8 Hz), 6.94 (1H, d, J = 8.4 Hz), 7.03 (1H, s), 7.10 (1H, d, J = 8.4 Hz), 7.43 (1H, s)
MS: 727.10 $[M + H]^+$, 725.00 $[M - H]^-$

Example 6

NMR: 1.47 (3H, d, J = 6.4 Hz), 1.49 (3H, s), 1.51 (3H, s), 3.41 (1H, dd, J = 8.0, 2.0 Hz), 3.50 (1H, dd, J = 12.6, 1.4 Hz), 3.89 (1H, t, J = 8.2 Hz), 4.22-4.33 (1H, m), 4.83 (1H, d, J = 13.6 Hz), 5.75 (1H, dd, J = 3.8, 1.0 Hz), 5.78 (1H, d, J = 3.6 Hz), 6.93 (1H, d, J = 8.4 Hz), 7.04 (1H, s), 7.09 (1H, d, J = 8.4 Hz)
MS: 727.05 $[M + H]^+$, 725.05 [M - H]-

Example 7

NMR: 1.47 (3H, d, J = 6.4 Hz), 1.80-2.09 (2H, m), 2.26-2.79 (4H, m), 3.41 (1H, dd, J = 8.0, 2.4 Hz), 3.52 (1H, dd, J = 12.8, 1.2 Hz), 3.89 (1H, t, J = 8.2 Hz), 4.23-4.32 (1H, m), 4.83 (1H, d, J = 12.8 Hz), 5.78 (1H, d, J = 3.6 Hz), 5.81 (1H, d, J = 3.6 Hz), 6.94 (1H, d, J = 8.4 Hz), 7.08 (1H, s), 7.10 (1H, d, J = 8.4 Hz)
MS: 739.05 $[M + H]^+$, 737.05 $[M - H]^-$

Example 8

NMR: 1.22-1.43 (4H, m), 3.63 (1H, d, J = 12.4 Hz), 3.68-3.80 (4H, m), 4.67 (1H, d, J = 12.8 Hz), 5.69 (1H, d, J = 3.6 Hz), 5.74 (1H, d, J = 3.2 Hz), 6.93 (1H, d, J = 8.4 Hz), 7.10 (1H, d, J = 7.2 Hz), 7.11 (1H, s)
MS: 711.00 $[M + H]^+$, 708.95 $[M - H]^-$

Example 9

NMR: 1.51 (3H, s), 1.52 (3H, s), 3.67 (1H, d, J = 12.8 Hz), 3.70-3.79 (4H, m), 4.67 (1H, d, J = 12.8 Hz), 5.69 (1H, d, J = 4.0 Hz), 5.80 (1H, d, J = 2.8 Hz), 6.93 (1H, d, J = 8.4 Hz), 7.10 (1H, d, J = 8.4 Hz)
MS: 747.00 $[M + H]^+$, 744.90 $[M - H]^-$

Example 10

NMR: 1.53 (3H, s), 1.55 (3H, s), 3.65 (1H, d, J = 12.8 Hz), 3.70-3.80 (4H, m), 4.67 (1H, d, J = 12.8 Hz), 5.70 (1H, d, J = 3.6 Hz), 5.82 (1H, d, J = 4.0 Hz), 6.93 (1H, d, J = 8.4 Hz), 7.10 (1H, d, J = 8.4 Hz)
MS: 714.00 $[M + H]^+$, 711.95 $[M - H]^-$

Example 11

NMR: 3.62 (1H, d, J = 12.8 Hz), 3.69-3.80 (4H, m), 4.57 (2H, s), 4.68 (1H, d, J = 12.8 Hz), 5.70 (1H, d, J = 3.6 Hz), 5.77 (1H, d, J = 3.6 Hz), 6.94 (1H, d, J = 8.4 Hz), 7.10 (1H, s), 7.10 (1H, d, J = 8.4 Hz)
MS: 685.00 $[M + H]^+$,682.95 $[M - H]^-$

Example 12

NMR: 1.46 (3H, d, J = 7.2 Hz), 3.63 (1H, d, J = 12.8 Hz), 3.68-3.80 (4H, m), 4.63 (1H, d, J = 6.8 Hz), 4.68 (1H, d, J = 12.4 Hz), 5.71 (1H, d, J = 3.6 Hz), 5.76 (1H, d, J = 2.8 Hz), 6.94 (1H, d, J = 8.4 Hz), 7.07 (1H, s), 7.10 (1H, d, J = 8.4 Hz)
MS: 699.05 $[M + H]^+$,697.05 $[M - H]^-$

Example 13

NMR: 1.47 (3H, d, J = 7.2 Hz), 3.63 (1H, d, J = 12.8 Hz), 3.69-3.80 (4H, m), 4.63-4.69 (1H, m), 4.68 (1H, d, J = 13.2 Hz), 5.72 (1H, d, J = 3.6 Hz), 5.78 (1H, d, J = 2.8 Hz), 6.94 (1H, d, J = 8.4 Hz), 7.08 (1H, s), 7.10 (1H, d, J = 8.4 Hz)
MS: 699.05 $[M + H]^+$,696.95 $[M - H]^-$

Example 14

NMR: 3.63 (1H, d, J = 12.8 Hz), 3.69-3.80 (4H, m), 3.95 (1H, dd, J = 12.6, 7.0 Hz), 4.02 (1H, dd, J = 12.6, 3.0 Hz), 4.68 (1H, d, J = 12.8 Hz), 4.71 (1H, dd, J = 6.8, 3.2 Hz), 5.72 (1H, d, J = 3.6 Hz), 5.80 (1H, d, J = 3.6 Hz), 6.94 (1H, d, J = 8.4 Hz), 7.10 (1H, s), 7.10 (1H, d, J = 8.4 Hz)
MS: 715.00 $[M + H]^+$, 712.85 $[M - H]^-$

Example 15

NMR: 2.65 (1H, dd, J = 16.0, 10.0 Hz), 2.78 (1H, dd, J = 15.8,3.8 Hz), 3.62 (1H, d, J = 12.8 Hz), 3.68-3.81 (4H, m), 4.68 (1H, d, J = 12.8 Hz), 4.92 (1H, dd, J = 10.0, 3.6 Hz), 5.70 (1H, d, J = 4.0 Hz), 5.73 (1H, d, J = 2.8 Hz), 6.94 (1H, d, J = 8.4 Hz), 7.09 (1H, s), 7.11 (1H, d, J = 9.2 Hz)
MS: 743.00 $[M + H]^+$, 741.00 $[M - H]^-$

Example 16

NMR: 1.97-2.20 (4H, m), 3.62-3.93 (9H, m), 4.67 (1H, d, J = 12.8 Hz), 5.73 (1H, d, J = 4.0 Hz), 5.81 (1H, d, J = 3.6 Hz), 6.94 (1H, d, J = 8.4 Hz), 7.07 (1H, s), 7.10 (1H, d, J = 8.4 Hz)
MS: 755.10 $[M + H]^+$, 753.10 $[M - H]^-$

Example 17

NMR: 1.65-1.80 (4H, m), 1.97-2.17 (4H, m), 3.65 (1H, d, J = 12.4 Hz), 3.69-3.79 (4H, m), 4.68 (1H, d, J = 12.4 Hz), 5.71 (1H, d, J = 3.6 Hz), 5.75 (1H, d, J = 3.6 Hz), 6.93 (1H, d, J = 8.4 Hz), 7.03 (1H, s), 7.09 (1H, d, J = 8.4 Hz)
MS: 739.00 $[M + H]^+$, 736.90 $[M - H]^-$

Example 18

NMR: 1.49 (3H, s), 1.52 (3H, s), 4.00 (1H, d, J = 13.6 Hz), 4.57 (1H, d, J = 12.8 Hz), 5.68 (1H, d, J = 4.0 Hz), 5.83 (1H, d, J = 3.6 Hz), 6.81 (1H, d, J = 8.4 Hz), 7.03 (1H, s), 7.22 (1H, d, J = 8.4 Hz), 8.15 (1H, s)
MS: 711.90 $[M + H]^+$, 710.00 $[M - H]^-$

Example 19

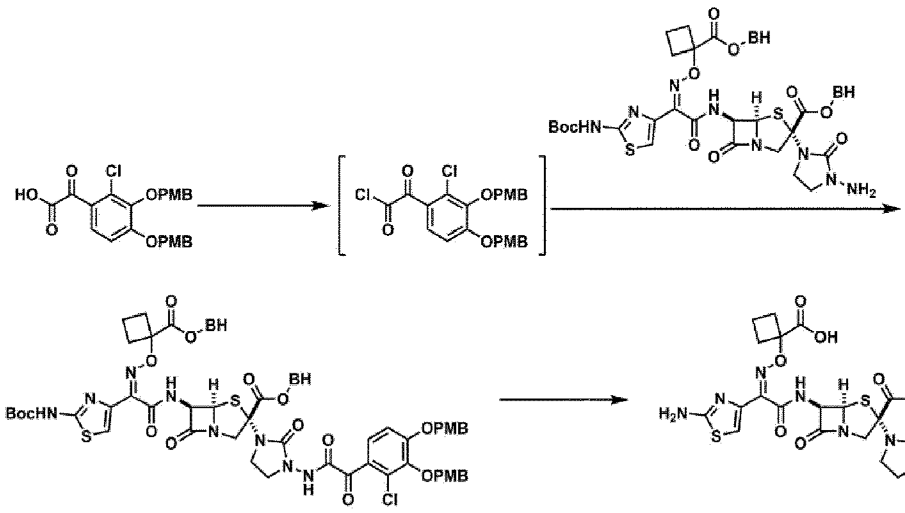

Example 19 (1)

Dichloromethane (1.0 mL) was added to 2-(2-chloro-3,4-bis((4-methoxybenzyl)oxy)phenyl)-2-oxoacetic acid (100 mg), and then oxalyl chloride (23 μL) and DMF (2 μL) were sequentially added thereto under ice cooling. The reaction mixture was stirred at room temperature for 1 hour, thereby obtaining 2-(2-chloro-3,4-bis((4-methoxybenzyl)oxy)phenyl)-2-oxoacetyl chloride in a dichloromethane solution.

Example 19 (2)

THF (2 mL) and water (2 mL) were added to benzhydryl (3R,5R,6R)-3-(3-amino-2-oxoimidazolidin-1-yl)-6-((Z)-2-((1-((benzhydryloxy)carbonyl)cyclo butoxy)imino)-2-(2-((tert-butoxycarbonyl)amino)thiazol-4-yl)acetamido)-7-oxo-4-thia-1-azabi cyclo[3.2.0]heptane-3-carboxylate (216 mg), and the mixture was stirred under ice cooling. At the same temperature, sodium hydrogen carbonate (55 mg) was added to the reaction mixture, and then 2-(2-chloro-3,4-bis((4-methoxybenzyl)oxy)phenyl)-2-oxoacetyl chloride obtained in Example 19 (1) in a dichloromethane solution was sequentially added thereto. The reaction mixture was stirred at room temperature for 1 hour, ethyl acetate (10 mL) and water (10 mL) were then added thereto, and the organic layer was separated. The organic layer was washed with a 5% aqueous sodium chloride solution and then dehydrated and dried over anhydrous sodium sulfate. The solvent was distilled off under reduced pressure, thereby obtaining a target substance (340 mg) as a yellow oily substance.

Example 19 (3)

Dichloromethane (6.2 mL) was added to the compound (312 mg) obtained in Example 19 (2), and the mixture was cooled to -20°C. At the same temperature, anisole (1.4 mL) and aluminum chloride (438 mg) were sequentially added to the reaction mixture. The reaction mixture was stirred at the same temperature for 1 hour. The reaction mixture was added to a mixture of acetonitrile (20 mL), water (10 mL), and trisodium citrate dihydrate (1.45 g) under ice cooling. A saturated aqueous sodium hydrogen carbonate solution was added to the reaction mixture such that the pH was adjusted to 5.2, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-((-1-carboxycyclobutoxy)imino)acetamido)-3-(3 -(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid (33.5 mg) as yellow solids.

NMR: 1.80-2.10 (2H, m), 2.25-2.60 (4H, m), 3.62 (1H, d, J = 12.2 Hz), 3.67-3.82 (4H, m), 4.69 (1H, d, J = 12.2 Hz), 5.70-5.82 (2H, m), 6.87-6.97 (1H, m), 7.07 (1H, s), 7.41-7.48 (1H, m)
MS: 753.05 $[M + H]^+$, 751.05 $[M - H]^-$

The compounds shown in Table 21 were obtained in the same manner as in Example 19.

[Table 21]

| Ex. No. | Structural Formula | Name |
|---|---|---|
| 2 0 | 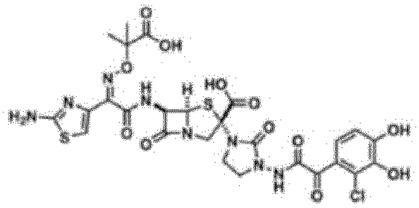 | (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 2 1 | 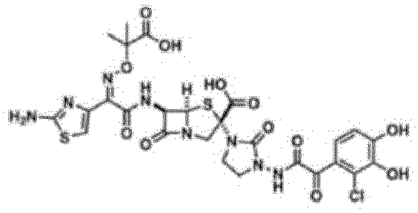 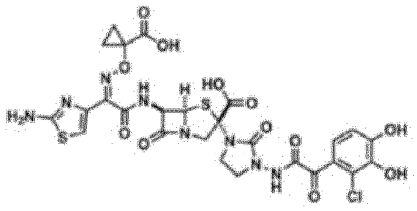 | (3R,5R,6R)-6-((Z)-2-(2-aminothiazol-4-yl)-2-((1-carboxycyclopropoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 2 2 | 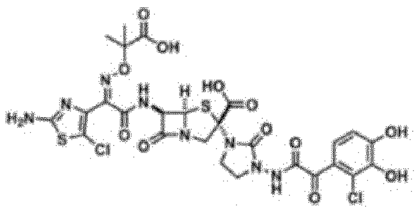 | (3R,5R,6R)-6-((Z)-2-(2-amino-5-chlorothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-l-yl)-7-oxo-4-thia-1-azabicyclo[3.2.0]heptane-3-carboxylic acid |
| 2 3 | 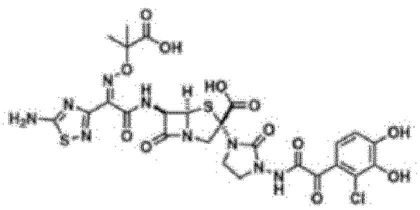 | (3R,5R,6R)-6-((Z)-2-(5-amino-1,2,4-thiadiazol-3-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 2 4 | 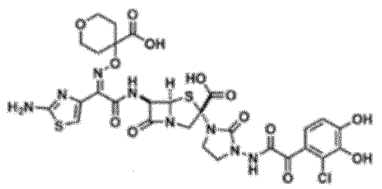 | (3R,5R,6R)-6-((Z)-2-(2-aminothiazol-4-yf)-2-(((4-carboxytetrahydro-2H-pyran-4-yl)oxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 2 5 | 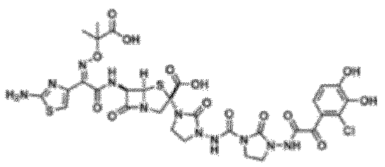 | (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl-carboxamido)-2-oxoimidazolidin-1-yl)-7-oxo-4-thia-1-azabicyclo[3.2.0]heptane-3-carboxylic acid |

(continued)

| Ex. No. | Structural Formula | Name |
|---|---|---|
| 2 6 | 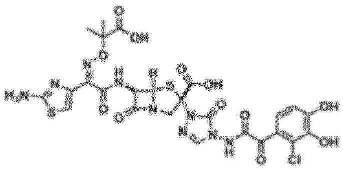 | (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetamido)-5-oxo-4,5-dihydro-1H-1,2,4-triazol-1-yl)-7-oxo-4-thia-1-azabicyclo[3.2.0]heptane-3-carboxylic acid |

The measured values of NMR and MS of the compounds in the table are as follows.

Example 20

NMR: 1.45-1.53 (6H, m), 3.62 (1H, d, J = 12.8 Hz), 3.66-3.82 (4H, m), 4.68 (1H, d, J = 12.8 Hz), 5.67-5.78 (2H, m), 6.89-6.98 (1H, m), 7.03 (1H, s), 7.41-7.48 (1H, m)
MS: 741.00 $[M + H]^+$, 739.00 $[M - H]^-$

Example 21

NMR: 1.22-1.43 (4H, m), 3.61 (1H, dd, J = 12.6, 1.0 Hz), 3.68-3.81 (4H, m), 4.29 [4.67] (1H, d, J = 12.4 Hz), 5.69 (1H, d, J = 3.6 Hz), 5.73 (1H, d, J = 4.4 Hz), 6.87[6.91] (1H, d, J = 8.6 Hz), 7.10 [7.03] (1H, s), 7.33[7.44] (1H, d, J = 8.8 Hz)
MS: 739.00 $[M + H]^+$, 736.90 $[M - H]^-$

Example 22

NMR: 1.46-1.55 (6H, m), 3.38-3.49 (1H, m), 3.55-3.81 (4H, m), 4.67 (1H, d, J = 12.4 Hz), 5.64-5.83 (2H, m), 6.88-6.97 (1H, m), 7.41-7.48 (1H, m)
MS: 774.95 $[M + H]^+$, 773.00 $[M - H]^-$

Example 23

NMR: 1.52 [1.53] (3H, s), 1.53 [1.55] (3H, s), 3.63 (1H, d, J = 13.2 Hz), 3.68-3.81 (4H, m), 4.31 [4.67] (1H, d, J = 12.6 Hz), 5.68 [5.70] (1H, d, J = 3.8 Hz), 5.77 [5.81] (1H, d, J = 3.4 Hz), 6.89 [6.93] (1H, d, J = 8.8 Hz), 7.44 (1H, d, J = 8.4 Hz)
MS: 742.00 $[M + H]^+$, 740.00 $[M - H]^-$

Example 24

NMR: 1.96-2.19 (4H, m), 3.35-3.50 (1H, m), 3.57-3.92 (8H, m), 4.27 [4.67] (1H, d, J = 12.6 Hz), 5.72 (1H, d, J = 3.6 Hz), 5.76 [5.81] (1H, d, J = 3.4 Hz), 6.91 [6.95] (1H, d, J = 8.8 Hz), 7.06 (1H, s), 7.44 [7.46] (1H, d, J = 8.4 Hz)
MS: 783.05 $[M + H]^+$, 780.90 $[M - H]^-$

Example 25

NMR: 1.49 (3H, s), 1.51 (3H, s), 3.58-3.76 (2H, m), 3.80-3.88 (2H, m), 3.94-4.02 (5H, m), 4.66 (1H, d, J = 12.4 Hz), 5.69 (1H, d, J = 3.6 Hz), 5.75 (1H, d, J = 3.6 Hz), 6.73 (1H, d, J = 8.8 Hz), 7.03 (1H, s), 7.45 (1H, d, J = 8.8 Hz)
MS: 868.00 $[M + H]^+$, 865.95 $[M - H]^-$

Example 26

NMR: 1.49 (3H, s), 1.52 (3H, s), 3.85 (1H, d, J = 12.8 Hz), 4.56 (1H, d, J = 12.4 Hz), 5.66 (1H, d, J = 4.0 Hz), 5.82 (1H, d, J = 3.6 Hz), 6.97 (1H, d, J = 8.8 Hz), 7.03 (1H, s), 7.49 (1H, d, J = 8.4 Hz), 8.09 (1H, s)
MS: 739.95 $[M + H]^+$, 737.90 $[M - H]^-$

Example 27

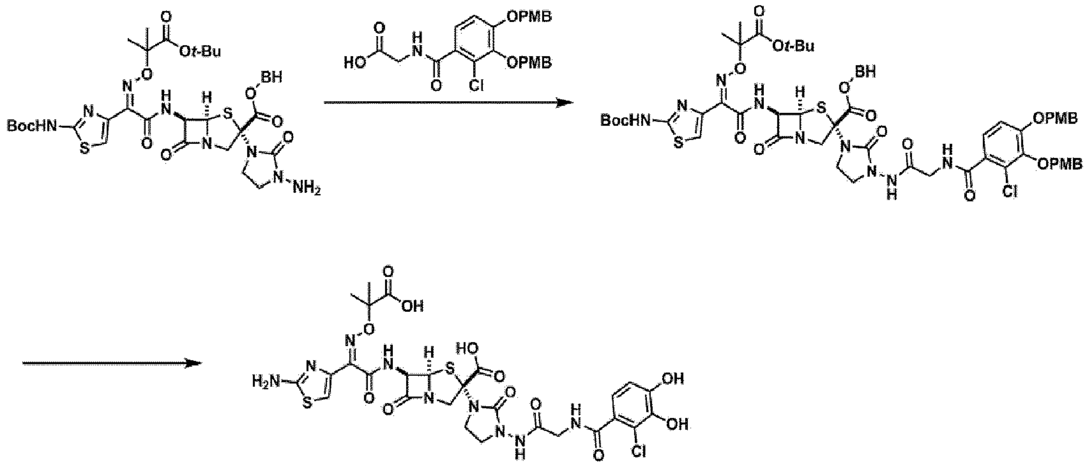

Example 27 (1)

(2-Chloro-3,4-bis((4-methoxybenzyl)oxy)benzoyl)glycine (112 mg), HoBt (34 mg), EDC (49 mg), DMF (2 mL), and NMM (31 μL) were sequentially added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (200 mg). The reaction mixture was stirred at room temperature overnight. Ethyl acetate (10 mL) and water (10 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, and the residue was purified by silica gel column chromatography [eluent; ethyl acetate:hexane = 40:60 → 100:0], thereby obtaining a target substance (188 mg) as light yellow solids.

Example 27 (2)

Dichloromethane (2.8 mL) was added to the compound (188 mg) obtained in Example 27 (1), and the mixture was stirred at -20°C. At the same temperature, anisole (0.92 mL) and aluminum chloride (282 mg) were sequentially added to the reaction mixture. The reaction mixture was stirred at a temperature equal to or lower than -20°C for 30 minutes. The reaction mixture was added to a mixture of acetonitrile (5 mL), water (5 mL), and trisodium citrate dihydrate (933 mg) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxybenzamido)acetamido)-2-oxoimidazolidin-1-yl)-7-oxo-4-thia-1-azabicyclo[3.2.0]heptane-3-carboxylic acid (42 mg) as light yellow solids.

NMR: 1.48 (3H, s), 1.50 (3H, s), 3.50-3.87 (5H, m), 4.17 (2H, s), 4.65 (1H, d, J = 12.8 Hz), 5.69 (1H, d, J = 4.0 Hz), 5.75 (1H, d, J = 3.6 Hz), 6.91 (1H, d, J = 8.4 Hz), 7.03 (1H, s), 7.07 (1H, d, J = 8.4 Hz)
MS: 777.00 $[M + H]^+$, 768.00 $[M - H]^-$

The compounds shown in Table 22 were obtained in the same manner as in Example 27.

[Table 22]

| Ex.No. | Structural Formula | Name |
|---|---|---|
| 2 8 | 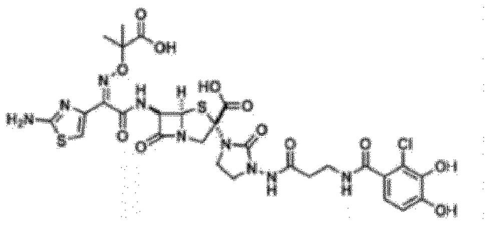 | (3R,5R,6R)-6-((Z)-2-(2-aminothiazol-4-yl)-2-(((2-carboxypropan-2-yl)oxy)imino)acetamido)-3-(3-(3-(2-chloro-3,4-dihydroxybenzamido)propanamido)-2-oxoimidazolidin-1-yl)-7-oxo-4-thia-1-azabicyclo[3.2.0]heptane-3-carboxylic acid |
| 2 9 | 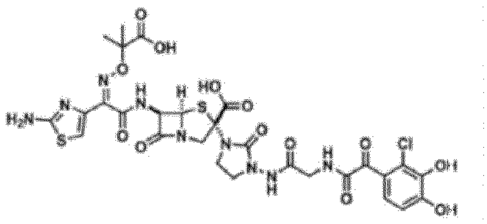 | (3R,5R,6R)-6-((Z)-2-(2-aminothiazol-4-yl)-2-(((2-carboxypropan-2-yl)oxy)imino)acetamido)-3-(3-(2-(2-(2-chloro-3,4-dihydroxyphenyl)-2-oxoacetamido)acetamido)-2-oxoimidazolidin-1-yl)-7-oxo-4-thia-1-azabicyclo[3.2.0]heptane-3-carboxylic acid |
| 3 0 | 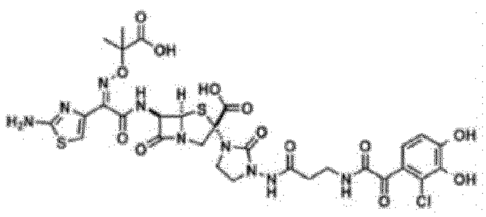 | (3R,5R,6R)-6-((Z)-2-(2-aminothiazol-4-yl)-2-(((2-carboxypropan-2-yl)oxy)imino)acetamido)-3-(3-(3-('2-(.2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid |
| 3 1 | 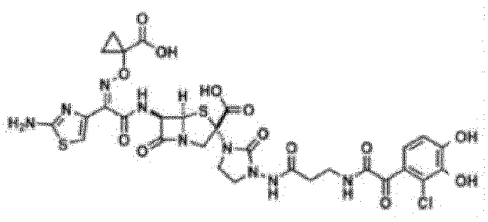 | (3R,5R,6R)-6-((Z)-2-(2-aminothiazol-4-yl)-2-((1-carboxycyclopropoxy)imino)acetamido)-3-(3-(3-(2-(2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid |

(continued)

| Ex.No. | Structural Formula | Name |
|---|---|---|
| 3 2 | 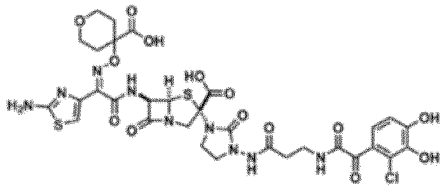 | (3R,5R,6R)-6-((Z)-2-(2-aminothiazol-4-yl)-2-(((4-carboxytetrahydro-2H-pyran-4-yl)oxy)imino)acetamido)-3-(3-(3-(2-(2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid |
| 3 3 | 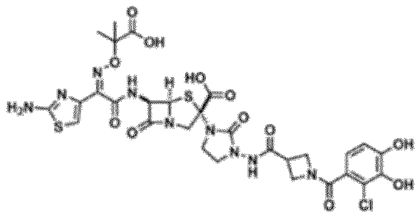 | (3R,5R,6R)-6-((Z)-2-(2-aminothiazol-4-yl)-2-(((2-carboxypropan-2-yl)oxy)imino)acetamido)-3-(3-(1-(2-chloro-3,4-dihydroxybenzoyl)azetidine)-3-carboxamido)-2-oxoimidazolidin-1-yl)-7-oxo-4-thia-1-azabicyclo [3.2.0]heptane-3-carboxylic acid |
| 3 4 | 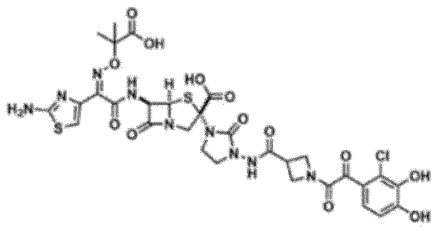 | (3R,5R,6R)-6-((Z)-2-(2-aminothiazol-4-yl)-2-(((2-carboxypropan-2-yl)oxy)imino)acetamido)-3-(3-(1-(2-chloro-3,4-dihydroxyphenyl)-2-oxoacetyl)azetidine-3-carboxamido)-2-oxoimidazolidin-1-yl)-7-oxo-4-thia-1-azabicyclo[3.2.0]heptane-3-carboxylic acid |

The measured values of NMR and MS of the compounds in the table are as follows.

Example 28

NMR: 1.48 (3H, s), 1.50 (3H, s), 2.65 (2H, t, J = 6.2 Hz), 3.54-3.72 (7H, m), 4.63 (1H, d, J = 12.8 Hz), 5.66 (1H, d, J = 3.6 Hz), 5.74 (1H, d, J = 3.2 Hz), 6.89 (1H, d, J = 8.4 Hz), 6.95 (1H, d, J = 8.4 Hz), 7.03 (1H, s)
MS: 784.05 $[M + H]^+$, 781.95 $[M - H]^-$.

Example 29

NMR: 1.49 (3H, s), 1.51 (3H, s), 3.50-3.77 (5H, m), 4.19 (2H, s), 4.66 (1H, d, J = 12.8 Hz), 5.69 (1H, d, J = 3.6 Hz), 5.75 (1H, d, J = 3.2 Hz), 6.93 (1H, d, J = 8.4Hz), 7.03 (1H, s), 7.41 (1H, d, J = 8.4 Hz)
MS: 798.00 $[M + H]^+$, 796.10 $[M - H]^-$

Example 30

NMR: 1.48 (3H, s), 1.50 (3H, s), 2.66 (2H, t, J = 6.2 Hz), 3.50-3.74 (7H, m), 4.61 (1H, d, J = 12.4 Hz), 5.64 (1H, d, J = 3.6 Hz), 5.72 (1H, d, J = 3.2 Hz), 6.94 (1H, d, J = 8.8 Hz), 7.03 (1H, s), 7.34 (1H, d, J = 8.8 Hz)

MS: 812.05 $[M + H]^+$, 810.15 $[M - H]^-$

Example 31

NMR: 1.21-1.43 (4H, m), 2.66 (2H, t, J = 6.0 Hz), 3.46-3.74 (7H, m), 4.60 (1H, d, J = 12.8 Hz), 5.62 (1H, d, J = 4.0 Hz), 5.70 (1H, d, J = 4.0 Hz), 6.92 (1H, d, J = 8.4 Hz), 7.10 (1H, s), 7.34 (1H, d, J = 8.8 Hz)
MS: 810.05 $[M + H]^+$, 807.90 $[M - H]^-$

Example 32

NMR: 1.95-2.18 (4H, m), 2.66 (2H, t, J = 6.2 Hz), 3.49-3.76 (9H, m), 3.77-3.90 (2H, m), 4.59 (1H, d, J = 12.4 Hz), 5.65 (1H, d, J = 3.6 Hz), 5.77 (1H, d, J = 3.2 Hz), 6.94 (1H, d, J = 8.8 Hz), 7.06 (1H, s), 7.34 (1H, d, J = 8.4 Hz)
MS: 854.10 $[M + H]^+$, 851.95 $[M - H]^-$

Example 33

NMR: 1.48 (3H, s), 1.50 (3H, s), 3.54-3.75 (6H, m), 4.13-4.34 (3H, m), 4.36-4.46 (1H, m), 4.65 (1H, dd, J = 12.4, 1.2 Hz), 5.69 (1H, d, J = 3.6 Hz), 5.75 (1H, d, J = 3.6 Hz), 6.87-6.97 (2H, m), 7.03 (1H, s)
MS: 796.05 $[M + H]^+$, 794.05 $[M - H]^-$

Example 34

NMR: 1.48 (3H, s), 1.50 (3H, s), 3.55-3.75 (6H, m), 4.30-4.53 (4H, m), 4.65 (1H, d, J = 12.4 Hz), 5.69 (1H, d, J = 3.6 Hz), 5.75 (1H, d, J = 3.6 Hz), 6.88 (1H, d, J = 8.8 Hz), 7.03 (1H, s), 7.40 (1H, d, J = 8.8 Hz)
MS: 824.05 $[M + H]^+$, 822.10 $[M - H]^-$

Example 35

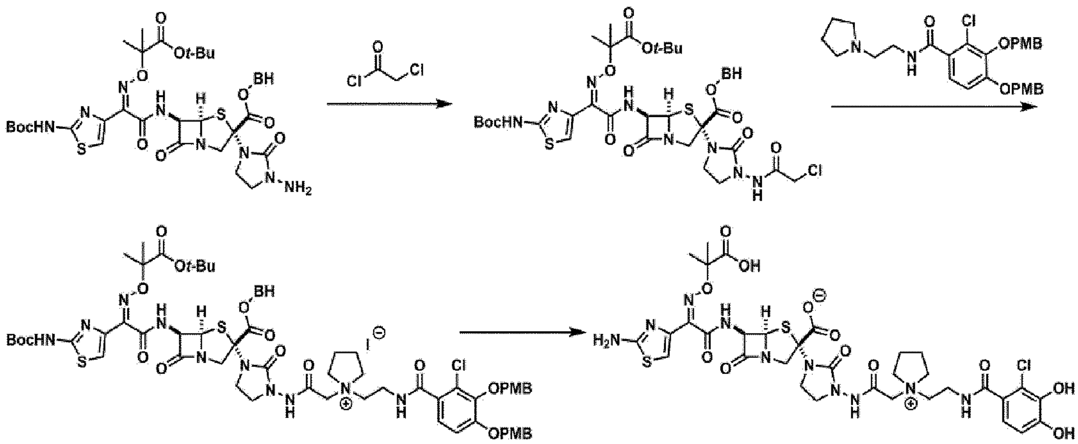

Example 35 (1)

Dichloromethane (3.9 mL) and pyridine (38 μL) were added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (390 mg), and the mixture was stirred under ice cooling. At the same temperature, chloroacetyl chloride (38 μL) was added to the reaction mixture, and the reaction mixture was stirred at room temperature for 2 hours. Water (10 mL) and 1 mol/L hydrochloric acid (2 mL) were added to the reaction mixture, and the organic layer was separated. The organic layer was dehydrated and dried over anhydrous sodium sulfate, and the solvent was distilled off under reduced pressure, thereby obtaining a target substance (424 mg) as a yellow oily substance.

Example 35 (2)

DMF (4.3 mL), 2-chloro-3,4-bis((4-methoxybenzyl)oxy)-N-(2-(pyrrolidin-1-yl)ethyl)benzamide (473 mg), and sodium iodide (68 mg) were sequentially added to the compound (424 mg) obtained in Example 35 (1), and the mixture was stirred at 40°C for 11 hours. Ethyl acetate (15 mL) and water (15 mL) were added to the reaction mixture. Hydrochloric acid (1 mol/L) was added to the reaction mixture such that the pH was adjusted to 2.5. The organic layer was separated, washed twice with a 5% aqueous sodium chloride solution, and then dehydrated and dried over anhydrous sodium sulfate. The solvent was distilled off under reduced pressure, thereby obtaining a target substance (702 mg) as a brown oily substance.

Example 35 (3)

Dichloromethane (15.0 mL) was added to the compound (702 mg) obtained in Example 35 (2), and the mixture was stirred at a temperature equal to or lower than -20°C. At the same temperature, anisole (3.0 mL) and aluminum chloride (1.50 g) were sequentially added to the reaction mixture. The reaction mixture was stirred at a temperature equal to or lower than -20°C for 1 hour. At a temperature equal to or lower than -20°C, aluminum chloride (421 mg) was added to the reaction mixture, and the reaction mixture was stirred at a temperature equal to or lower than -20°C for 1 hour. The reaction mixture was added to a mixture of acetonitrile (25 mL), water (15 mL), and trisodium citrate dihydrate (6.37 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(2-(1-(2-(2-chloro-3,4--dihydroxybenzamido)ethyl)pyrrolidin-1-ium-1-yl)acetamido)-2-o xoimidazolidin-1-yl)-7-oxo-4-thia-1-azabicyclo[3.2.0]heptane-3-carboxylate (33.5 mg) as white solids.

NMR: 1.48 (3H, s), 1.50 (3H, s), 2.17-2.34 (4H, m), 3.42-3.58 (4H, m), 3.61-3.71 (2H, m), 3.73-3.95(9H, m), 4.59 (1H, d, J = 12.8 Hz), 5.62 (1H, d, J = 4.0 Hz), 5.72 (1H, d, J = 4.0 Hz), 6.87-6.94 (1H, m), 6.96-7.04 (2H, m)
MS: 867.10 $[M + H]^+$, 865.05 $[M - H]^-$

Example 36

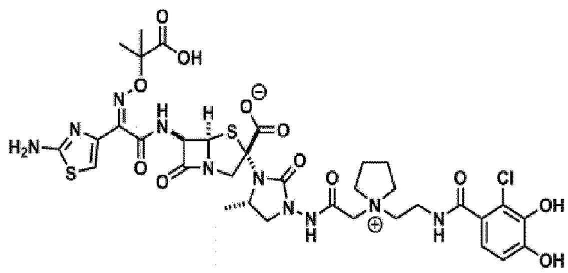

By using the compound obtained in Reference Example 29, (3R,5R,6R)-6-((Z)-2-(2-aminothiazol-4-yl)-2-(((2-carboxypropan-2-yl)oxy)imino)acetamido)-3-(S)-3-(2-(1-(2-(2-chloro-3,4-dihydroxybenzamido)ethyl)pyrrolidin-1-ium-1-yl)acetamido)-5 -methyl-2-oxoimidazolidin-1-yl)-7-oxo-4-thia-1-azabicyclo[3.2.0]heptane-3-carboxylate was obtained in the same manner as in Example 35.

NMR: 1.26 (3H, d, J = 4.8 Hz), 1.47 (3H, s), 1.50 (3H, s), 2.20-2.33 (4H, m), 3.06 (1H, dd, J = 4.0, 2.0 Hz), 3.26 (1H, d, J = 12.8 Hz), 3.58-3.67 (1H, m), 3.79-3.96 (9H, m), 4.17-4.27 (1H, m), 4.73 (1H, d, J = 12.8 Hz), 5.55 (1H, d, J = 3.6 Hz), 5.67 (1H, d, J = 3.6 Hz), 6.91 (1H, d, J = 8.4 Hz), 7.00 (1H, d, J = 8.4 Hz), 7.01 (1H, s)
MS: 881.15 $[M + H]^+$, 879.15 $[M - H]^-$

Example 37

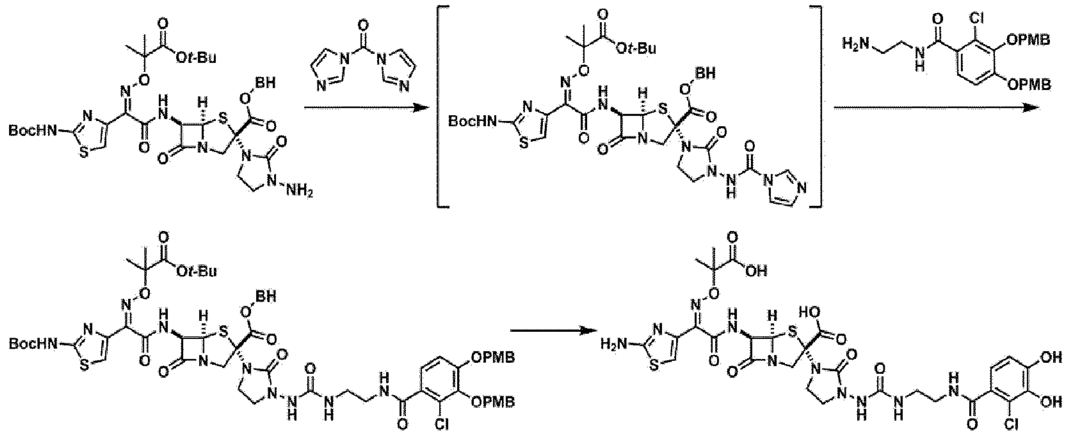

Example 37 (1)

Dichloromethane (7.0 mL) and 1,1'-carbonylimidazole (131 mg) were added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (350 mg), and the mixture was stirred at room temperature for 4 hours. At the same temperature, N-(2-aminoethyl)-2-chloro-3,4-bis((4-methoxybenzyl)oxy)benzamide (381 mg)was added to the reaction mixture,
and the reaction mixture was stirred at room temperature for 1 hour. The reaction mixture was added to a mixture of dichloromethane (35 mL) and water (35 mL) under ice cooling. Hydrochloric acid (1 mol/L) was added to the reaction mixture such that the pH was adjusted to 2.5. The organic layer was separated and sequentially washed with water and a saturated aqueous sodium chloride solution. The organic layer was dehydrated and dried over anhydrous sodium sulfate, and the solvent was distilled off under reduced pressure. The residue was purified by silica gel column chromatography [eluent; chloroform:2-propanol = 0:100 → 93:7], thereby obtaining a target substance (310 mg) as yellow solids.

Example 37 (2)

Dichloromethane (6.2 mL) was added to the compound (310 mg) obtained in Example 37 (1), and the mixture was stirred at a temperature equal to or lower than -20°C. At the same temperature, anisole (1.49 mL) and aluminum chloride (455 mg) were sequentially added to the reaction mixture. The reaction mixture was stirred at a temperature equal to or lower than -20°C for 30 minutes. The reaction mixture was added to a mixture of acetonitrile (20 mL), water (20 mL), and trisodium citrate dihydrate (1.51 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 84:16]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(3-(2-(2-chloro-3,4-dihydroxybenzamido)ethyl)ureido)-2-oxoimidazolidin-1-yl)-7-oxo-4-thia-1-azabicyclo[3.2.0]heptane-3-carboxylic acid (50 mg) as white solids.

NMR: 1.48 (3H, s), 1.50 (3H, s), 3.39-3.45 (2H, m), 3.48-3.56 (5H, m), 3.60-3.71 (2H, m), 4.59 (1H, d, J = 12.4 Hz), 5.63 (1H, d, J = 3.6 Hz), 5.72 (1H, d, J = 3.2 Hz), 6.90 (1H, d, J = 8.4 Hz), 6.97 (1H, d, J = 8.4 Hz), 7.03 (1H, s)
MS: 799.05 $[M + H]^+$, 797.15 $[M - H]^-$

The compounds shown in Table 23 were obtained in the same manner as in Example 37.

[Table 23]

| Ex.No. | Structural Formula | Name |
|---|---|---|
| 3 8 | 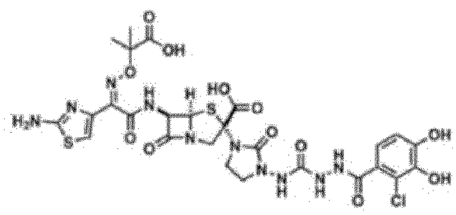 | (3R,5R,6R)-6-((Z)-2-(2-aminothiazol-4-yl)-2-((2-carboxypropan-2-yl)oxy)imino)acetamido)-3-(3-(2-(2-chloro-3,4-dihydroxybenzoyl)hydrazine-1-carboxamido)-2-oxoimidazolidin-1-yl)-7-oxo-4-thia-1-azabicyclo[3.2.0]heptane-3-carboxylic acid |

(continued)

| Ex.No. | Structural Formula | Name |
|---|---|---|
| 3 9 | 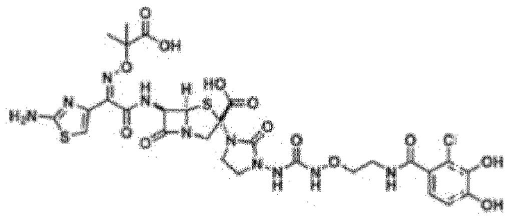 | (3R,5R,6R)-6-((Z)-2-(2-aminothiazol-4-yl)-2-(((2-carboxypropan-2-yl)oxy)imino)acetamido)-3-(3-(3-(2-(2-chloro-3,4-dihydroxybenzamido)ethoxy)ureido)-2-oxoimidazolidin-1-yl)-7-oxo-4-thia-1-azabicyclo[3.2.0]heptane-3-carboxylic acid |
| 4 0 | 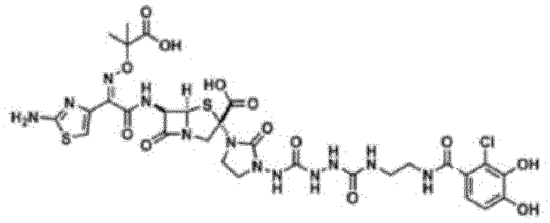 | (3R,5R,6R)-6-((Z)-2-(2-aininothiazol-4-yl)-2-(((2-carboxypropan-2-yl)oxy)imino)acetamido)-3-(3-((2-(2-chloro-3,4-dihydroxybenzamido) ethyl)carbamoy 1)hydrazine-1-carboxamido)-2-oxoimidazolidin-1-yl)-7-oxo-4-thia-1-azabicyclo [3.2.0]heptane-3-carboxylic acid |
| 4 1 | 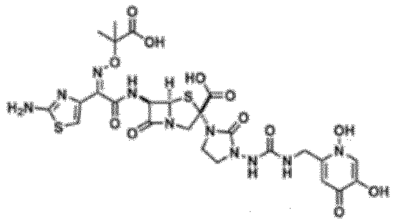 | (3R,5R,6R)-6-((Z)-2-(2-aminothiazol-4-yl)-2-(((2-carboxypropan-2-yl)oxy)imino)acetamido)-3-(3-(3-((1,5-dihydroxy-4-oxo-1,4-dihydropyridin-2-yl)methyl)ureido)-2-oxoimidazolidin-1-yl)-7-oxo-4-thia-1-azabicyclo[3.2.0]heptane-3-carboxylic acid |
| 4 2 | 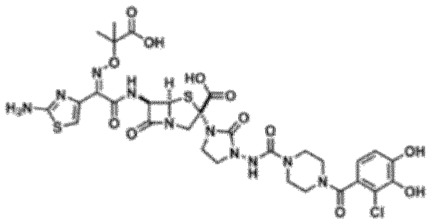 | (3R,5R,6R)-6-((Z2)-2-(2-aminothiazol-4-yl)-2-(((2-carboxypropan-2-yl)oxy)imino)acetamido)-3-(3-(4-(2-chloro-3,4-dihydroxybenzoyl)piperazine-1-carboxamido)-2-oxoimidazolidin-1-yl)-7-oxo-4-thia-1-azabicyclo[3.2.0]heptane-3-carboxylic acid |
| 4 3 | 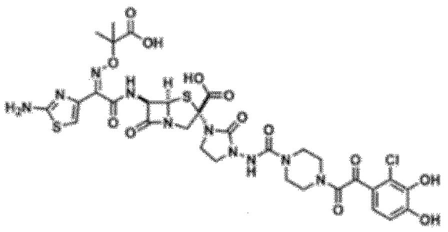 | (3R,5R,6R)-6-((Z)-2-(2-aminothiazol-4-y1)-2-((2-carboxypropan-2-yl)oxy)imino)acetamido)-3-(3-(4-(2-(2-chloro-3,4-dihydroxyphenyl)-2-oxoacetyl)piperazine-1-carboxamido)-2-oxoimidazolidin-1-yl)-7-oxo-4-thia-1-azabicyclo[3 2.0] heptane-3-carboxylic acid |

The measured values of NMR and MS of the compounds in the table are as follows.

Example 38

NMR: 1.49 (3H, s), 1.51 (3H, s), 3.52-3.80 (5H, m), 5.70 (1H, d, J = 3.6 Hz), 5.75 (1H, d, J = 3.6 Hz), 6.93 (1H, d, J = 8.4 Hz), 7.03 (1H, s), 7.11 (1H, d, J = 8.4 Hz)
MS: 771.00 $[M + H]^+$, 769.00 $[M - H]^-$

Example 39

NMR: 1.48 (3H, s), 1.50 (3H, s), 3.43-3.73 (7H, m), 4.06-4.13 (2H, m), 4.69 (1H, d, J = 12.8 Hz), 5.61 (1H, d, J = 3.6 Hz), 5.74 (1H, d, J = 3.6 Hz), 6.91 (1H, d, J = 8.4 Hz), 6.99 (1H, d, J = 8.4 Hz), 7.02 (1H, s)
MS: 815.10 $[M + H]^+$,812.95 $[M - H]^-$

Example 40

NMR: 1.48 (3H, s), 1.50 (3H, s), 3.36-3.69 (9H, m), 4.60 (1H, d, J = 12.0 Hz), 5.62 (1H, d, J = 3.6 Hz), 5.73 (1H, d, J = 3.6 Hz), 6.87-7.00 (2H, m), 7.02 (1H, s)
MS: 857.10 $[M + H]^+$, 854.90 $[M - H]^-$

Example 41

NMR: 1.47 (3H, s), 1.50 (3H, s), 3.50-3.80 (7H, m), 4.40-4.45 (1H, m), 5.70 (1H, d, J = 4.0 Hz), 5.75 (1H, d, J = 4.0 Hz), 6.72 (1H, s), 7.03 (1H, s), 7.44 (1H, s)
MS: 725.10 $[M + H]^+$, 723.05 $[M - H]^-$

Example 42

NMR: 1.48 (3H, s), 1.50 (3H, s), 3.40-3.50 (4H, m), 3.54-3.70 (6H, m), 3.66 (1H, d, J = 7.2 Hz), 3.72-3.82 (1H, m), 3.82-3.90 (1H, m), 4.63 (1H, d, J = 12.8 Hz), 5.68 (1H, d, J = 3.6 Hz), 5.75 (1H, dd, J = 4.0, 1.0 Hz), 6.82 (1H, d, J = 8.4 Hz), 6.95 (1H, d, J = 8.0 Hz), 7.02 (1H, s)
MS: 825.30 $[M + H]^+$, 823.20 $[M - H]^-$

Example 43

NMR: 1.48 (3H, s), 1.50 (3H, s), 3.58-3.70 (11H, m), 3.73-3.80 (2H, m), 4.63 (1H, d, J = 12.4 Hz), 5.68 (1H, d, J = 3.6 Hz), 5.74 (1H, d, J = 3.6 Hz), 6.90 (1H, d, J = 8.8 Hz), 7.03 (1H, s), 7.51 (1H, d, J = 8.8 Hz)
MS: 853.05 $[M + H]^+$, 851.15 $[M - H]^-$

Example 44

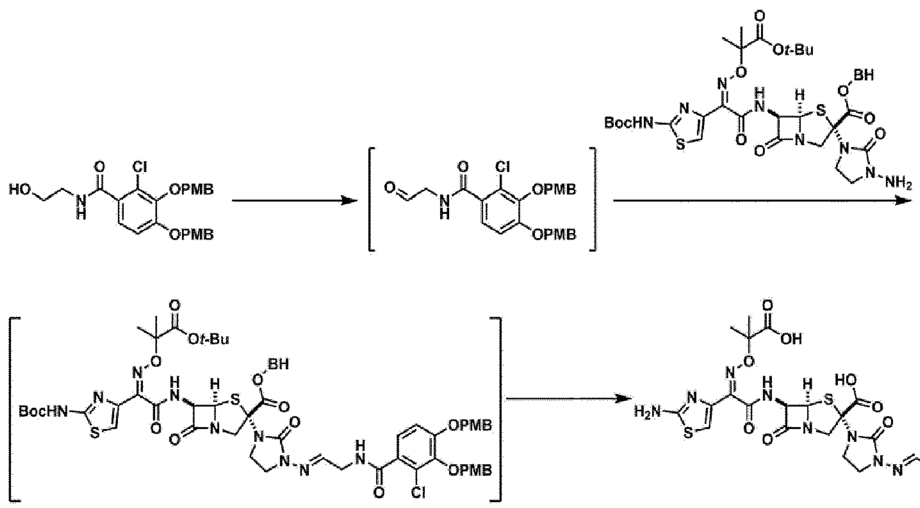

Dichloromethane (4.3 mL) was added to 2-chloro-N-(2-hydroxyethyl)-3,4-bis ((4-methoxybenzyl)oxy)benzamide (430 mg), and the mixture was stirred under ice cooling. Dess-Martin periodinane (773 mg) was added to the reaction mixture, and the reaction mixture was stirred at room temperature for 3 hours. Dichloromethane (10 mL), water (5 mL), and a 1 mol/L aqueous sodium thiosulfate solution (5 mL) were sequentially added to the reaction mixture, and the organic layer was separated. The organic layer was washed with a saturated aqueous sodium chloride solution and dehydrated and dried over anhydrous sodium sulfate. The solvent was distilled off under reduced pressure, dichloromethane (5 mL) benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (500 mg) were added to the residue, and the mixture was stirred at room temperature for 2 hours. Dichloromethane (15.2 mL) was added to the reaction mixture, and the reaction mixture was stirred at a temperature equal to or lower than -20°C. At the same temperature, anisole (3.8 mL) and aluminum chloride (1.16 g) were sequentially added to the reaction mixture. The reaction mixture was stirred at a temperature equal to or lower than -20°C for 30 minutes. The reaction mixture

was added to a mixture of acetonitrile (15 mL), water (15 mL), and trisodium citrate dihydrate (3.83 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(((E)-2-(2-chloro-3,4-dihydroxybenzamido)ethylidene)amino)-2-oxoimidazolidin-1-yl)-7 -oxo-4-thia-1-azabicyclo[3.2.0]heptane-3-carboxylic acid (22.7 mg) as white solids.

NMR: 1.40 (3H, s), 1.42 (3H, s), 3.49 (1H, d, J = 12.8 Hz), 3.59-3.78 (4H, m), 4.16 (2H, d, J = 4.0 Hz), 5.61 (1H, d, J = 3.6 Hz), 5.66 (1H, d, J = 3.6 Hz), 6.83 (1H, d, J = 8.4 Hz), 6.95 (1H, s), 6.96 (1H, d, J = 8.4 Hz), 6.99 (1H, t, J = 4.0Hz)
MS: 754.05 $[M + H]^+$, 752.10 $[M - H]^-$

Example 45

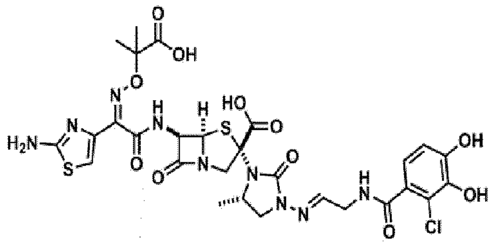

By using the compound obtained in Reference Example 29, (3R,5R,6R)-6-((Z)-2-(2-aminothiazol-4-yl)-2-(((2-carboxypropan-2-yl)oxy)imino)acetamido)-3-((S)-(3-(((E)-2-(2-chloro-3,4-dihydroxybenzamido)ethylidene)amino)-5-methyl-2-oxoimida zolidin-1-yl)-7-oxo-4-thia-1-azabicyclo[3.2.0]heptane-3-carboxylic acid was obtained in the same manner as in Example 44.

NMR: 1.21 (3H, d, J = 4.8 Hz), 1.39 (3H, s), 1.41 (3H, s), 3.29-3.41 (2H, m), 3.63-3.72 (1H, m), 4.15 (2H, d, J = 4.4 Hz), 4.24-4.35 (1H, m), 5.62-5.68 (2H, m), 6.82 (1H, d, J = 8.4 Hz), 6.92-6.99 (3H, m)
MS: 768.05 $[M + H]^+$, 766.10 $[M - H]^-$.

Example 46

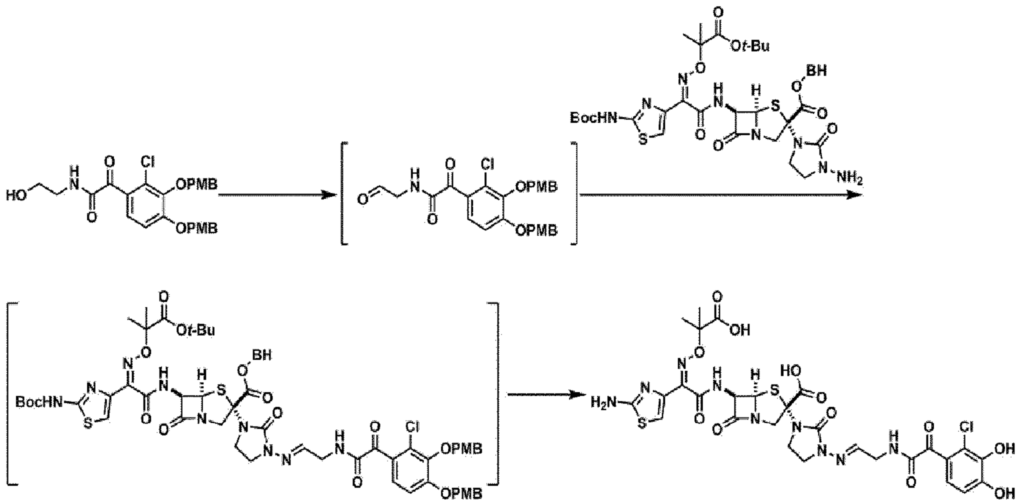

Dichloromethane (4.5 mL) was added to 2-(2-chloro-3,4-bis((4-methoxybenzyl)oxy)phenyl)-N-(2-hydroxyethyl)-2-oxoacetamide (450 mg), and the mixture was stirred under ice cooling. Dess-Martin periodinane (764 mg) was added to the reaction mixture, and the reaction mixture was stirred at room temperature for 3 hours. Dichloromethane (10 mL), water (5 mL), and a 1 mol/L aqueous sodium thiosulfate solution (5 mL) were sequentially added to the reaction

mixture, and the organic layer was separated. The organic layer was washed with a saturated aqueous sodium chloride solution and dehydrated and dried over anhydrous sodium sulfate. The solvent was distilled off under reduced pressure, dichloromethane (5 mL) and benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (500 mg) were added to the residue under ice cooling, and the mixture was stirred at room temperature for 2 hours. Dichloromethane (15.6 mL) was added to the reaction mixture, and the reaction mixture was stirred at a temperature equal to or lower than -20°C. At the same temperature, anisole (3.8 mL) and aluminum chloride (1.16 g) were sequentially added to the reaction mixture. The reaction mixture was stirred at a temperature equal to or lower than -20°C for 30 minutes. The reaction mixture was added to a mixture of acetonitrile (20 mL), water (20 mL), and trisodium citrate dihydrate (3.83 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(((E)-2-(2-(2-chloro-3,4-dihydroxyphenyl)-2-oxoacetamido)ethylidene)amino)-2-oxoimid azolidin-1-yl)-7-oxo-4-thia-1-azabicyclo[3.2.0]heptane-3-carboxylic acid (43.5 mg) as light yellow solids.

NMR: 1.40 (3H, s), 1.42 (3H, s), 3.49 (1H, d, J = 12.4 Hz), 3.58-3.80 (4H, m), 4.16 (2H, d, J = 4.0 Hz), 5.62 (1H, d, J = 3.6 Hz), 5.67 (1H, d, J = 3.6 Hz), 6.86 (1H, d, J = 8.8 Hz), 6.95 (1H, s), 6.97 (1H, t, J = 4.0 Hz), 7.34 (1H, d, J = 8.8 Hz)
MS: 782.05 $[M + H]^+$, 780.00 $[M - H]^-$

Example 47

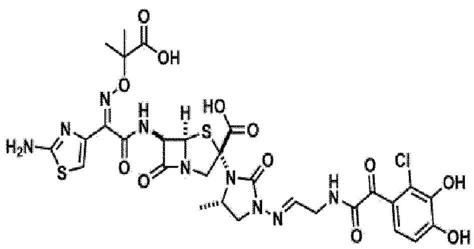

In the same manner as in Example 46, (3R,5R,6R)-6-((Z)-2-(2-aminothiazol-4-yl)-2-(((2-carboxypropan-2-yl)oxy)imino)acetamido)-3-((S)-(3-(((E)-2-(2-(2-chloro-3,4-dihydroxyphenyl)-2-oxoacetamido)ethylidene)amino)-5-methyl-2-oxoimidazolidin-1-yl)-7-oxo-4-thia-1-azabicyclo[3.2.0]heptane-3-carboxylic acid was obtained.

NMR: 1.24 (3H, d, J = 4.5 Hz), 1.40 (3H, s), 1.43 (3H, s), 3.28-3.43 (2H, m), 3.64-3.73 (1H, m), 4.17 (2H, d, J = 4.4 Hz), 4.25-4.35 (1H, m), 5.60-5.71 (2H, m), 6.89 (1H, d, J = 8.4 Hz), 6.93-7.01 (2H, m), 7.35 (1H, d, J = 8.4 Hz)
MS: 796.05 $[M + H]^+$, 793.90 $[M - H]^-$

Example 48

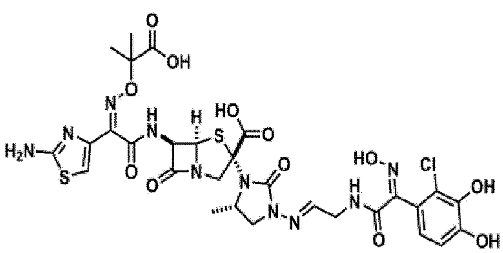

In the same manner as in Example 46, (3R,5R,6R)-6-((Z)-2-(2-aminothiazol-4-yl)-2-(((2-carboxypropan-2-yl)oxy)imino)acetamido)-3-((S)-(3-(((E)-2-((Z)-2-(2-chloro-3,4-dihydroxyphenyl)-2-(hydroxyimino)acetamido)ethylide ne)amino)-5-methyl-2-oxoimidazolidin-1-yl)-7-oxo-4-thia-1-azabicyclo[3.2.0]heptane-3-carbo xylic acid was obtained.

NMR: 1.22 (3H, d, J = 4.8 Hz), 1.40 (3H, s), 1.42 (3H, s), 3.30-3.38 (2H, m), 3.62-3.70 (1H, m), 4.08-4.13 (2H, m), 4.25-4.33 (1H, m), 5.61-5.70 (2H, m), 6.77 (1H, d, J = 8.4 Hz), 6.87-6.93 (2H, m), 6.96 (1H, s)

MS: 811.05 [M + H]+, 809.00 [M - H]-

Example 49

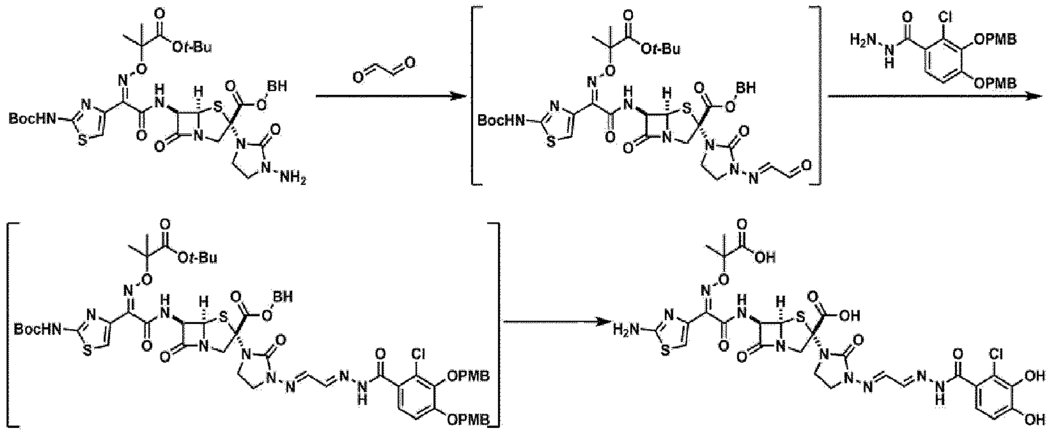

Example 49

THF (5 mL) was added to a 40% aqueous glyoxal solution (1.3 mL), and the mixture was stirred under ice cooling. Benzhydryl (3R,5R,6R)-3-(3-amino-2-oxoimidazolidin-1-yl)-6-((Z)-2-(((1-(tert-butoxy)-2-methyl-1-oxopr opan-2-yl)oxy) imino)-2-(2-((tert-butoxycarbonyl)amino)thiazol-4-yl)acetamido)-7-oxo-4-thia-1-azabicyclo[3.2.0]heptane-3-carboxylate (500 mg) and THF (10 mL) were added to the reaction mixture, and the reaction mixture was stirred at room temperature for 3 hours and 30 minutes. The reaction mixture was added to a mixture of water (15 mL), dichloromethane (15 mL), and 1 mol/L hydrochloric acid (1.5 mL), and the organic layer was separated. The organic layer was washed twice with a 5% aqueous sodium chloride solution and then dehydrated and dried over anhydrous sodium sulfate. The solvent was distilled off under reduced pressure, and dichloromethane (10 mL) and 2-chloro-3,4-bis ((4-methoxybenzyl)oxy) benzohydrazine (512 mg) were added to the residue, and the mixture was stirred at room temperature for 2 hours and 40 minutes. Dichloromethane (15.3 mL) was added to the reaction mixture, and the reaction mixture was stirred at a temperature equal to or lower than -20°C. At the same temperature, anisole (3.8 mL) and aluminum chloride (1.15 g) were sequentially added to the reaction mixture. The reaction mixture was stirred at a temperature equal to or lower than -20°C for 2 hours. At the same temperature, aluminum chloride (383 mg) was added to the reaction mixture, and the reaction mixture was stirred at the same temperature for 30 minutes. Then, aluminum chloride (383 mg) was added to the reaction mixture, and the reaction mixture was stirred at the same temperature for 30 minutes. The reaction mixture was added to a mixture of acetonitrile (25 mL), water (20 mL), and trisodium citrate dihydrate (3.80 g) under ice cooling. A saturated aqueous sodium hydrogen carbonate solution was added to the reaction mixture such that the pH was adjusted to 5.0, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(((1E,2E)-2-(2-(2-chloro-3,4-dihydroxybenzoyl)hydrazono)ethylidene)amino)-2-oxoimid azolidin-1-yl)-7-oxo-4-thia-1-azabicyclo[3.2.0]heptane-3-carboxylic acid (32.5 mg) as light yellow solids.

NMR: 1.41 (3H, s), 1.43 (3H, s), 3.48 (1H, d, J = 13.2 Hz), 3.75-3.86 (4H, m), 5.63 (1H, d, J = 3.4 Hz), 5.67 (1H, d, J = 3.4 Hz), 6.85 (1H, d, J = 8.4 Hz), 6.95 (1H, s), 7.00 (1H, d, J = 8.4 Hz), 7.38 (1H, d, J = 8.0 Hz), 7.91 (1H, d, J = 8.0 Hz)
MS: 767.05 [M + H]+, 765.10 [M - H]-

The compounds shown in Table 24 were obtained in the same manner as in Example 49.

[Table 24]

| Ex.No. | Structural Formula | Name |
|---|---|---|
| 5 0 | 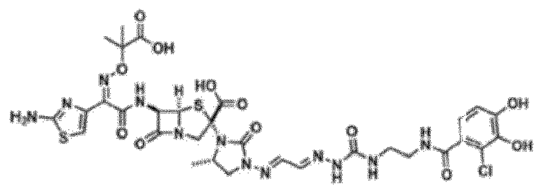 | (3R,5R,6R)-6-((Z)-2-(2-aminothiazol-4-yl)-2-(((2-carboxypropan-2-yl)oxy)imino)acetamido)-3-((S)-3-(((1E,2E)-2-(2-((2-(2-chloro-3,4-dihydroxybenzamido)ethyl)carbam oyl)hydrazono) ethylidene)amino)-5-methyl-2-oxoimidazolidin-1-yl)-7-oxo-4-thia-1-azabicyclo[3.2.0]heptane-3-carboxylic acid |
| 5 1 | 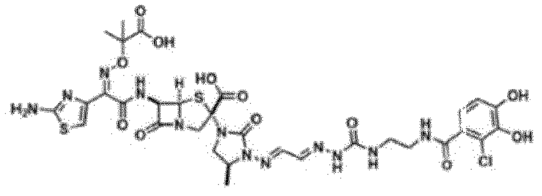 | (3R,5R,6R)-6-((Z)-2-(2-aminothiazol-4-yl)-2-(((2-carboxypropan-2-yl)oxy)imino)acetamido)-3-((S)-3-(((1E,2E)-2-(2-((2-(2-chloro-3,4-dihydroxybenzamido)ethyl)carbam oyl)hydrazono) ethylidene)amino)-4-methyl-2-oxoimidazolidin-1-yl)-7-oxo-4-thia-1-azabicyclo[3.2.0]heptane-3-carboxylic acid |
| 5 2 | 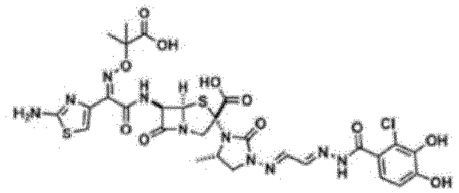 | (3R,5R,6R)-6-((Z)-2-(2-aminothiazol-4-yl)-2-(((2-carboxypropan-2-yl)oxy)imino)acetamido)-3-((S)-3-(((1E,2E)-2-(2-(2-chloro-3,4-dihydroxybenzoyl)hydrazono)ethyl idene)amino)-5-methyl-2-oxoimidazolidin-1-yl)-7-oxo-4-thia-1-azabicyclo[3.2.0]heptane-3-carboxylic acid |
| 5 3 | 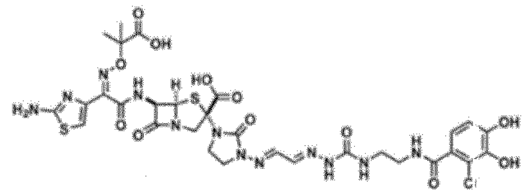 | (3R,5R,6R)-6-((Z)-2-(2-aminothiazol-4-yl)-2-(((2-carboxypropan-2-yl)oxy)imino)acetamido)-3-(3-(((1E,2E)-2-(2-((2-(2-chloro-3,4-dihydroxybenzamido)ethyl)carbam oyl)hydrazono) ethylidene)amino)-2-oxoimidazolidin-1-yl)-7-oxo-4-thia-1-azabicyclo[3.2.0]heptane-3-carboxylic acid |
| 5 4 | 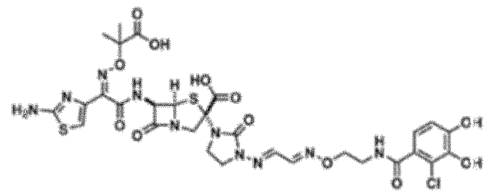 | (3R,5R,6R)-6-((Z)-2-(2-aminothiazol-4-yl)-2-(((2-carboxypropan-2-yl)oxy)imino)acetamido)-3-(3-(((1E,2E)-2-((2-(2-chloro-3,4-dihydroxybenzamido)ethoxy)imino )ethylidene)amino)-2-oxoimidazolidin- 1-yl)-7-oxo-4-thia-1-azabicyclo [3.2.0]heptane-3-carboxylic acid |

The measured values of NMR and MS of the compounds in the table are as follows.

Example 50

NMR: 1.26 (3H, d, J = 4.8 Hz), 1.42 (3H, s), 1.44 (3H, s), 3.35 (1H, d, J = 12.0 Hz), 3.39-3.52 (5H, m), 3.71-3.81 (1H, m), 4.30-4.42 (1H, m), 5.65-5.72 (2H, m), 6.82 (1H, d, J = 8.4 Hz), 6.90 (1H, d, J = 8.4 Hz), 6.98 (1H, s), 7.25 (1H, d, J = 8.0 Hz), 7.59 (1H, d, J = 8.0 Hz)
MS: 867.05 $[M + H]^+$, 865.00 $[M - H]^-$

Example 51

NMR: 1.31 (3H, d, J = 5.6 Hz), 1.50 (6H, s), 3.42-3.62 (7H, m), 3.85-4.00 (1H, t, J = 8.4 Hz), 4.37-4.48 (1H, m), 5.67-5.72 (1H, m), 5.72-5.78 (1H, m), 6.88 (1H, d, J = 8.0 Hz), 6.96 (1H, d, J = 8.0 Hz), 7.46-7.58 (2H, m), 7.62 (1H, d, J = 6.0 Hz)
MS: 867.10 $[M + H]^+$, 865.00 $[M - H]^-$

Example 52

NMR: 1.27 (3H, d, J = 4.8 Hz), 1.40 (3H, s), 1.43 (3H, s), 3.34 (1H, d, J = 12.8 Hz), 3.50-3.57 (1H, m), 3.79-3.88 (1H, m), 4.35-4.45 (1H, m), 5.63-5.71 (2H, m), 6.86 (1H, d, J = 8.4 Hz), 6.96 (1H, s), 7.01 (1H, d, J = 8.4 Hz), 7.37 (1H, d, J = 8.0 Hz), 7.92 (1H, d, J = 8.0 Hz)
MS: 781.05 $[M + H]^+$, 779.05 $[M - H]^-$

Example 53

NMR: 1.49 (3H, s), 1.51 (3H, s), 3.44-3.62 (5H, m), 3.73-3.91 (4H, m), 4.71 (1H, d, J = 12.4 Hz), 5.71 (1H, d, J = 3.6 Hz), 5.75 (1H, d, J = 3.6 Hz), 6.88 (1H, d, J = 8.4 Hz), 6.95 (1H, d, J = 8.4 Hz), 7.03 (1H, s), 7.33 (1H, d, J = 8.0 Hz), 7.65 (1H, d, J = 8.0 Hz)
MS: 853.10 $[M + H]^+$, 850.95 $[M - H]^-$

Example 54

NMR: 1.49 (3H, s), 1.51 (3H, s), 3.56 (1H, d, J = 12.4 Hz), 3.64-3.93 (6H, m), 4.34-4.42 (2H, m), 4.72 (1H, d, J = 12.4 Hz), 5.71 (1H, d, J = 3.6 Hz), 5.75 (1H, d, J = 3.6 Hz), 6.86-6.99 (2H, m), 7.03 (1H, s), 7.29 (1H, d, J = 8.4 Hz), 7.97 (1H, d, J = 8.4 Hz)
MS: 811.05 $[M + H]^+$, 808.95 $[M - H]^-$

Example 55

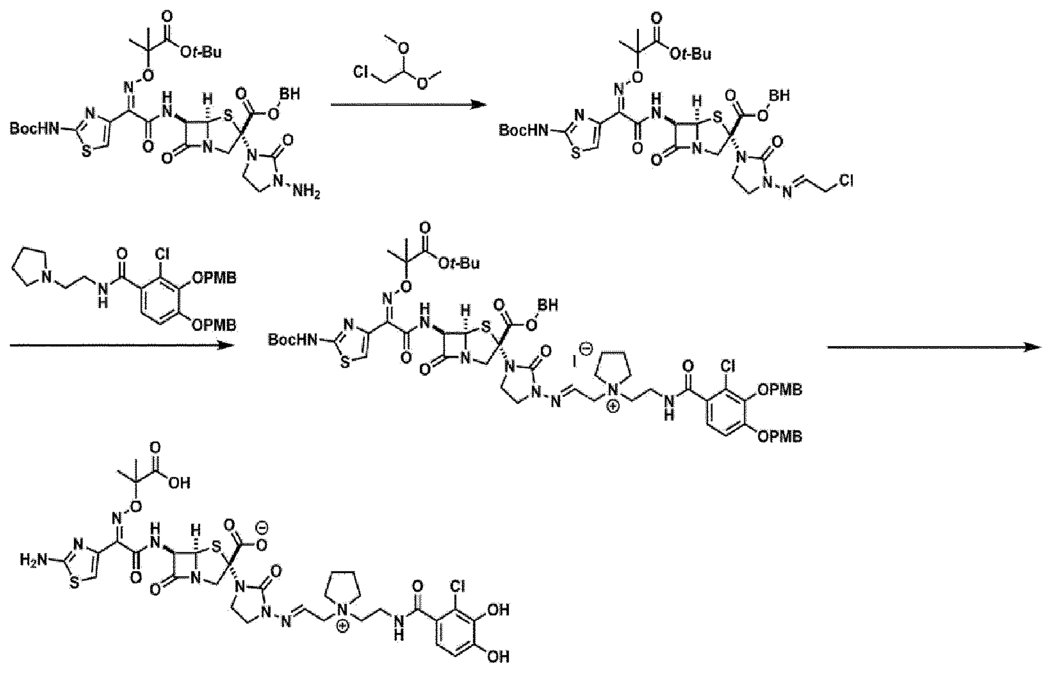

Example 55 (1)

Dichloromethane (10 mL) were added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (1.00 g), and the mixture was stirred under ice cooling. At the same temperature, 2-chloro-1,1-dimethoxyethane (2.0 mL) and p-toluenesulfonic acid monohydrate (66 mg) were sequentially added to the reaction mixture, and the reaction mixture was stirred at room temperature for 3 hours and 30 minutes. At the same temperature, 2-chloro-1,1-dimethoxyethane (0.66 mL) and p-toluenesulfonic acid monohydrate (44 mg) were sequentially added to the reaction mixture, and the reaction mixture was stirred at room temperature for 1 hour. The reaction mixture was added to a mixture of water (30 mL), ethyl acetate (30 mL), and 1 mol/L hydrochloric acid (1.5

mL), and the organic layer was separated. The organic layer was washed with a 5% aqueous sodium chloride solution and then dehydrated and dried over anhydrous sodium sulfate. The solvent was distilled off under reduced pressure, and the residue was purified by silica gel column chromatography [eluent; ethyl acetate:hexane = 40:60 → 70:30], thereby obtaining benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-((E)-2-chloroethylidene)amino)-2-oxoimidaz olidin-1-yl)-7-oxo-4-thia-1-azabicyclo[3.2.0]heptane-3-carboxylate (765 mg) as a yellow oily substance.

NMR($CDCl_3$): 1.39 (9H, s), 1.49-1.56 (15H, m), 3.48-3.74 (4H, m), 3.76-3.86 (1H, m), 4.28 (1H, d, J = 5.6 Hz), 5.10 (1H, d, J = 13.2 Hz), 5.60 (1H, d, J = 3.6 Hz), 5.76 (1H, dd, J = 8.0, 3.6 Hz), 6.86 (1H, s), 6.88-6.95 (1H, m), 7.08-7.15 (1H, m), 7.17-7.32 (11H, m), 7.40 (1H, d, J = 8.0 Hz), 8.08 (1H, s)

Example 55 (2)

DMF (7.0 mL) was added to benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-(((E)-2-chloroethylidene)amino)-2-oxoimida zolidin-1-yl)-7-oxo-4-thia-1-azabicyclo[3.2.0]heptane-3-carboxylate (705 mg), and the mixture was stirred under ice cooling. At the same temperature, 2-chloro-3,4-bis((4-methoxybenzyl)oxy)-N-(2-(pyrrolidin-1-yl)ethyl)benzamide (800 mg) and sodium iodide (57 mg) were sequentially added to the reaction mixture, and the reaction mixture was stirred at room temperature for 4 hours. Sodium iodide (57 mg) was added to the reaction mixture, and the reaction mixture was stirred at room temperature for 2 hours. Ethyl acetate (20 mL) and water (20 mL) were added to the reaction mixture. Hydrochloric acid (1 mol/L) was added to the reaction mixture such that the pH was adjusted to 2.6. The organic layer was separated, washed twice with a 5% aqueous sodium chloride solution, and then dehydrated and dried over anhydrous sodium sulfate. The solvent was distilled off under reduced pressure, thereby obtaining a target substance (1.175 g) as a light brown oily substance.

Example 55 (3)

Dichloromethane (10 mL) was added to the compound (1.175 g) obtained in Example 55 (2), and the mixture was stirred at a temperature equal to or lower than -20°C. At the same temperature, anisole (5.0 mL) and aluminum chloride (2.0 g) were sequentially added to the reaction mixture. The reaction mixture was stirred at a temperature equal to or lower than -20°C for 40 minutes. At the same temperature, aluminum chloride (1.0 g) was added to the reaction mixture, and the reaction mixture was stirred at the same temperature for 30 minutes. The reaction mixture was added to a mixture of acetonitrile (20 mL), water (20 mL), and trisodium citrate dihydrate (6.72 g) under ice cooling. A saturated aqueous sodium hydrogen carbonate solution was added to the reaction mixture such that the pH was adjusted to 5.7, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(((E)-2-(1-(2-(2-chloro-3,4--dihydroxybenzamido)ethyl) pyrrolidin-1-ium-1-yl)ethylidene )amino)-2-oxoimidazolidin-1-yl)-7-oxo-4-thia-1-azabicyclo[3.2.0]heptane-3-carboxylate (60.3 mg) as white solids.

NMR: 1.40 (3H, s), 1.42 (3H, s), 2.12-2.24 (4H, m), 3.41 (1H, d, J = 12.8 Hz), 3.50-3.88 (12H, m), 4.14-4.24 (2H, m), 5.59 (1H, d, J = 3.8 Hz), 5.67 (1H, d, J = 3.8 Hz), 6.74-7.07 (4H, m)
MS: 851.10 $[M + H]^+$, 849.00 $[M - H]^-$

The compounds shown in Table 25 were obtained in the same manner as in Example 55.

[Table 25]

| Ex.No. | Structural Formula | Name |
|---|---|---|
| 5 6 | 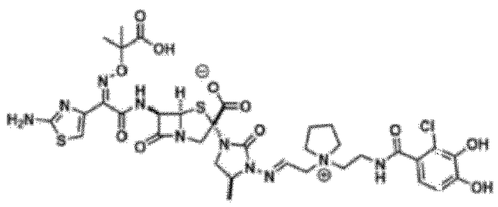 | (3R,5R,6R)-6-((Z)-2-(2-aminothiazol-4-yl)-2-(((2-carboxypropan-2-((S)-3-(((E)-2-(1-((2-(2-chloro-3,4-dihydroxybenzamido)ethyl)p yrrolidin-1-ium-1-yl)ethylidene)amino)-4-methyl-2-oxoimidazolidin-1-yl)-7-oxo-4-thia-1-azabicyclo [3.2.0]heptane-3-carboxylate |

(continued)

| Ex.No. | Structural Formula | Name |
|---|---|---|
| 5 7 | 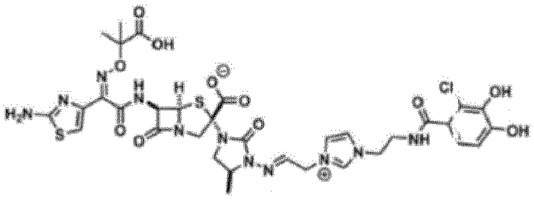 | (3R,5R,6R)-6-((Z)-2-(2-aminothiazol-4-yl)-2-(((2-carboxypropan-2-yl)oxy)imino)acetamido)-3-((S)-3-(((E)-2-(1-((2-(2-chloro-3,4-dihydroxybenzamido)ethyl)-1H-imidazol-3-ium-3-yl)ethylidene)amino)-4-methyl-2-oxoimidazolidin-1-yl)-7-oxo-4-thia-1-azabicyclo[3.2.0]heptane-3-carboxylate |
| 5 8 | 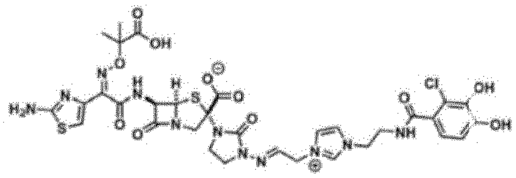 | (3R,5R,6R)-6-((Z)-2-(2-aminothiazol-4-yl)-2-(((2-carboxypropan-2-yl)oxy)imino)acetamido)-3-(3-(((E)-2-(1-(2-(2-chloro-3,4-dihydroxybenzamido)ethyl)-1H-imidazol-3-ium-3-yl)ethylidene)amino)-2-oxoimidazolidin-1-yl)-7-oxo-4-thia-1-azabicyclo[3.2.0]heptane-3-carboxylate |

The measured values of NMR and MS of the compounds in the table are as follows.

Example 56

NMR: 1.31 (3H, d, J = 6.0 Hz), 1.48 (3H, s), 1.50 (3H, s), 2.21-2.32 (4H, m), 3.51 (1H, d, J = 12.0 Hz), 3.60-3.72 (5H, m), 3.73-3.82 (2H, m), 3.87-3.97 (3H, m), 4.23-4.31 (2H, m), 4.37-4.47 (1H, m), 4.68 (1H, d, J = 12.8 Hz), 5.67 (1H, d, J = 3.6 Hz), 5.76 (1H, d, J = 3.2 Hz), 6.80-6.92 (1H, m), 7.02 (1H, s), 7.30 (1H, s), 7.34-7.40 (1H, m)
MS: 866.05 $[M + H]^+$, 864.15 $[M - H]^-$

Example 57

NMR: 1.16 (3H, d, J = 6.0 Hz), 1.48 (3H, s), 1.50 (3H, s), 3.12-3.15 (1H, m), 3.30 (1H, d, J = 12.8 Hz), 3.34 (1H, dd, J = 9.0,3.8 Hz), 3.78 (1H, t, J = 8.8 Hz), 4.47(2H,t,J=5.4 Hz), 4.52 (2H, t, J = 5.2 Hz), 4.59 (1H, d, J = 12.8 Hz), 5.13 (2H, d, J = 3.6 Hz), 5.60 (1H, d, J = 3.6 Hz), 5.74 (1H, d, J = 3.2 Hz), 6.81 (1H, d, J = 8.4 Hz), 6.84 (1H, d, J = 8.4 Hz), 6.87 (1H, s), 7.03 (1H, s), 7.44 (1H, s), 7.61 (1H, s), 8.99 (1H, s)
MS: 863.05 $[M + H]^+$, 861.35 $[M - H]^-$

Example 58

NMR: 1.49 (3H, s), 1.50 (3H, s), 3.27 (1H, d, J = 12.8 Hz), 3.27-3.42 (2H, m), 3.58-3.73 (2H, m), 3.79-3.89 (4H, m), 4.63 (1H, d, J = 13.2 Hz), 5.11 (2H, d, J = 4.0 Hz), 5.62 (1H, d, J = 3.6 Hz), 5.72 (1H, d, J = 3.6 Hz), 6.77-6.83 (2H, m), 6.97 (1H, t, J = 4.2 Hz), 7.04 (1H, s), 7.46 (1H, s), 7.67 (1H, s), 9.01 (1H, s)
MS: 849.10 $[M + H]^+$, 846.90 $[M - H]^-$

Example 59

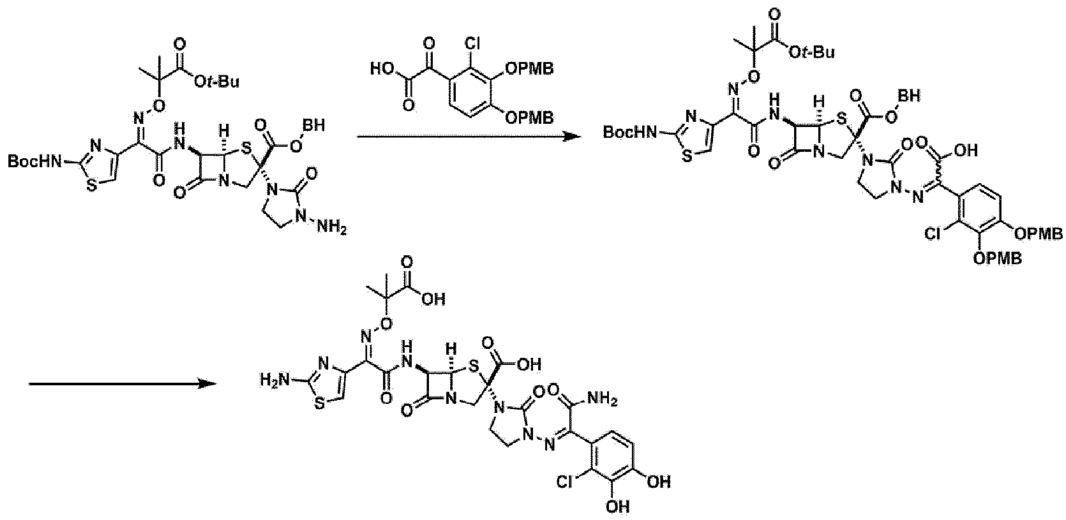

Example 59 (1)

Ethanol (4 mL) was added to 2-(2-chloro-3,4-bis((4-methoxybenzyl)oxy)phenyl)-2-oxoacetic acid (222 mg), and the mixture was stirred under ice cooling. At the same temperature, benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (400 mg) was added to the mixture, and the mixture was stirred at room temperature overnight. The reaction mixture was added to a mixture of ethyl acetate (40 mL) and water (40 mL). The organic layer was separated and washed twice with water (50 mL). The organic layer was washed with a saturated aqueous sodium chloride solution (10 mL) and then dehydrated and dried over anhydrous sodium sulfate. The solvent was distilled off under reduced pressure, thereby obtaining a target substance (500 mg) as light yellow solids.

Example 59 (2)

DMAC (5 mL) was added to the compound (500 mg) obtained in Example 59 (1), and the mixture was stirred under ice cooling. Ammonium chloride (45 mg), HOBt (106 mg), EDC (176 mg), and NMM (254 μL) were sequentially added to the reaction mixture under ice cooling. The reaction mixture was stirred at room temperature overnight. The reaction mixture was added to a mixture of ethyl acetate (30 mL) and water (30 mL), and 1 mol/L hydrochloric acid was added thereto such that the pH was adjusted to 5.4. The organic layer was separated, and the aqueous layer was extracted three times by using ethyl acetate (5 mL). The organic layers were combined, sequentially washed with water and a saturated aqueous sodium chloride solution, and then dehydrated and dried over anhydrous sodium sulfate. The solvent was distilled off under reduced pressure, thereby obtaining a target substance (499 mg) as yellow solids.

Example 59 (3)

Dichloromethane (10 mL) was added to the compound (499 mg) obtained in Example 59 (2), and the mixture was stirred at a temperature equal to or lower than -20°C. At the same temperature, anisole (2.5 mL) and aluminum chloride (766 mg) were sequentially added to the reaction mixture. The reaction mixture was stirred at a temperature equal to or lower than -20°C for 40 minutes. The reaction mixture was added to a mixture of acetonitrile (40 mL), water (40 mL), and trisodium citrate dihydrate (2.53 g) under ice cooling. A saturated aqueous sodium hydrogen carbonate solution was added to the reaction mixture such that the pH was adjusted to 5.3, and the aqueous layer was separated. The aqueous layer was concentrated under reduced pressure, and the residue was purified by medium-pressure reverse-phase silica gel column chromatography [eluent; water:acetonitrile = 100:0 → 75:25]. The aqueous solution containing a target substance was lyophilized, thereby obtaining (3R,5R,6R)-3-(3-(((Z)-2-amino-1-(2-chloro-3,4-dihydroxyphenyl)-2-oxoethylidene)amino)-2-oxoimidazolidin-1-yl)-6-((Z)-2-(2-aminothiazol-4-yl)-2-(((2-carboxypropan-2-yl)oxy)imino)a cetamido)-7-oxo-4-thia-1-azabicyclo[3.2.0]heptane-3-carboxylic acid (24.9 mg) as yellow solids.

NMR: 1.48 (3H, s), 1.50 (3H, s), 3.14-3.40 (2H, m), 3.50 (1H, d, J = 13.2 Hz), 3.53-3.65 (2H, m), 4.75 (1H, d, J = 8.8 Hz), 5.67 (1H, d, J = 3.6 Hz), 5.74 (1H, d, J = 3.6 Hz), 6.82 (1H, d, J = 8.0 Hz), 6.94 (1H, d, J = 8.0 Hz), 7.02 (1H, s)
MS: 740.05 $[M + H]^+$, 738.00 $[M - H]^-$

The compounds shown in Table 26 were obtained in the same manner as in Example 59.

[Table 26]

| Ex.No. | Structural Formula | Name |
|---|---|---|
| 60 | 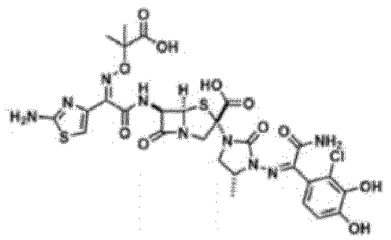 | (3R,5R,6R)-3-((R)-3-(((Z)-2-amino-1-(2-chloro-3,4-dihydroxyphenyl)-2-oxoethylidene)amino)-4-methyl-2-oxoimiazolidin-1-yl)-6-((Z)-2-(2-aminothiazol-4-yl)-2-(((2-carboxypropan-2-yl) oxy)imino)acetamido)-7-oxo-4-thia-1-azabicyclo[3.2.0]heptane-3-carboxylic acid |
| 61 | 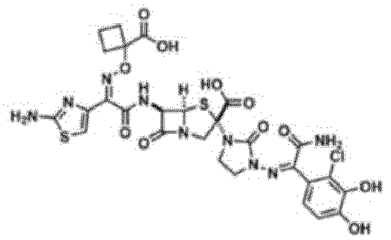 | (3R,5R,6R)-3-(3-(((Z)-2-amino-1-(2-chloro-3,4-dihydroxyphenyl)-2-oxoethylidene)amino)-2-oxoimidazolidin-1-yl)-6-((Z)-2-(2-aminothiazol-4-yl)-2-((1-carboxycyclobutoxy)imino)acetamido)-7-oxo-4-thia-1-azabicyclo[3.2.0]heptane-3-carboxylic acid |
| 62 | 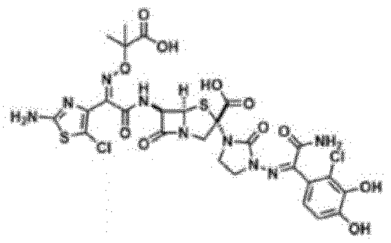 | (3R,5R,6R)-3-(3-(((Z)-2-amino-1-(2-chloro-3,4-dihydroxyphenyl)-2-oxoethylidene)amino)-2-oxoimidazolin-1-yl)-6-((Z)-2-(2-amino-5-chlorothiazol-4-yl)-2-(((2-carboxypropan-2-yl)oxy)imino)acetamido)-7-oxo-4-thia-1-azabicyclo[3.2.0]heptane-3-carboxylic acid |

The measured values of NMR and MS of the compounds in the table are as follows.

Example 60

NMR: 0.98 (3H, d, J = 6.0Hz), 1.49 (3H, s), 1.51 (3H, s), 3.10-3.11 (1H, m), 3.29 (1H, d, J = 8.0Hz), 3.58-3.64 (2H, m), 4.61 (1H, d, J = 12.4 Hz), 5.65 (1H, d, J = 3.6 Hz), 5.75 (1H, d, J = 3.6 Hz), 6.88-6.94 (1H, m), 6.97 (1H, d, J = 8.0Hz), 7.04 (1H, s)
MS: 754.15 $[M + H]^+$, 752.10 $[M - H]^-$

Example 61

NMR: 1.80-1.93 (1H, m), 1.94-2.07 (1H, m), 2.24-2.38 (2H, m), 2.39-2.49 (1H, m), 2.49-2.60 (1H, m), 3.13-3.37 (2H, m), 3.50 (1H, d, J = 13.2 Hz), 3.55-3.66 (2H, m), 4.70 (1H, d, J = 2.0Hz), 5.69 (1H, d, J = 3.6 Hz), 5.76 (1H, d, J = 3.6 Hz), 6.82 (1H, d, J = 8.4 Hz), 6.94 (1H, d, J = 8.4 Hz), 7.06 (1H, s)
MS: 752.00 $[M + H]^+$, 750.15 $[M - H]^-$

Example 62

NMR: 1.50 (3H, s), 1.51 (3H, s), 3.15-3.37 (2H, m), 3.52 (1H, d, J = 12.8 Hz), 3.55-3.65 (2H, m), 4.75 (1H, d, J = 5.2 Hz), 5.65 (1H, d, J = 4.0 Hz), 5.78 (1H, d, J = 3.2 Hz), 6.82 (1H, d, J = 8.4 Hz), 6.94 (1H, d, J = 8.4 Hz)
MS: 774.20 $[M + H]^+$, 772.10 $[M - H]^-$

Example 63

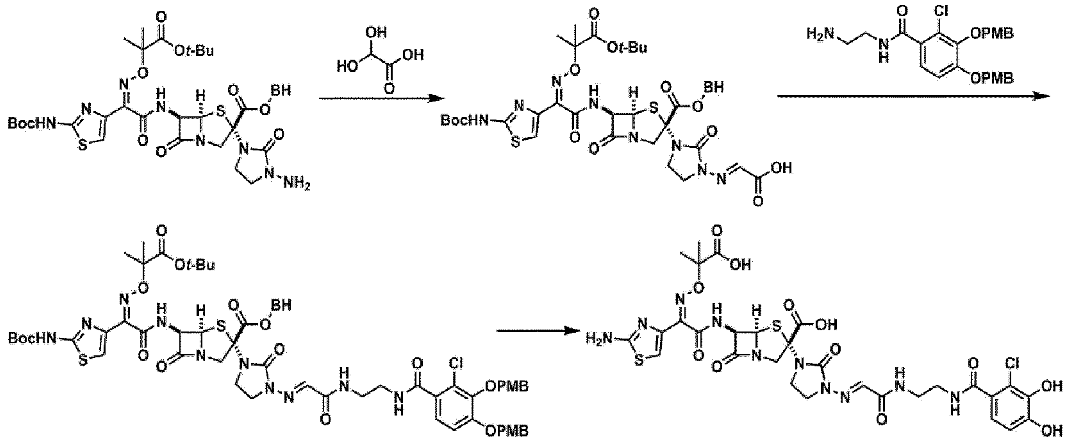

Example 63 (1)

THF (3 mL) was added to glyoxylic acid monohydrate (160 mg), and the mixture was stirred under ice cooling. At the same temperature, benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (300 mg) was added to the reaction mixture, and the reaction mixture was stirred at room temperature for 3 hours. The reaction mixture was added to a mixture of ethyl acetate (30 mL) and water (30 mL). The organic layer was separated and dehydrated and dried over anhydrous sodium sulfate. The solvent was distilled off under reduced pressure, thereby obtaining a target substance (340 mg) as yellow solids.

Example 63 (2)

DMAC (3.1 mL) was added to the compound (309 mg) obtained in Example 63 (1), and the mixture was stirred under ice cooling. At the same temperature, N-(2-aminoethyl)-2-chloro-3,4-bis((4-methoxybenzyl)oxy)benzamide (158 mg), HOBt (51 mg), EDC (71 mg), and NMM (81 μL) were sequentially added to the reaction mixture. The reaction mixture was stirred at room temperature overnight. At room temperature, the reaction mixture was added to a mixture of ethyl acetate (20 mL) and water (20 mL), and 1 mol/L hydrochloric acid was added thereto such that the pH was adjusted to 5.4. The organic layer was separated, and the aqueous layer was extracted three times by using ethyl acetate (5 mL). The organic layers were combined, sequentially washed with water and a saturated aqueous sodium chloride solution, and then dehydrated and dried over anhydrous sodium sulfate. The solvent was distilled off under reduced pressure, thereby obtaining benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-(((E)-2-((2-(2-chloro-3,4-bis((4-methoxyben zyl)oxy)benzamido)ethyl)amino)-2-oxoethylidene)amino)-2-oxoimidazolidin-1-yl)-7-oxo-4-th ia-1-azabicyclo[3.2.0]heptane-3-carboxylate (336 mg) as light yellow solids.

NMR ($CDCl_3$): 1.39 (9H, s), 1.51 (3H, s), 1.52 (3H, s), 1.54 (9H, s), 3.50 (1H, d, J = 13.6 Hz), 3.55-3.69 (8H, m), 3.80 (3H, s), 3.83 (3H, s), 4.93 (2H, s), 5.06 (2H, s), 5.14 (1H, d, J = 13.6 Hz), 5.60 (1H, d, J = 4.0 Hz), 5.75 (1H, dd, J = 7.8, 3.8 Hz), 6.77-6.85 (7H, m), 6.86 (1H, s), 6.86-6.94 (6H, m), 7.04-7.10 (1H, m), 7.21-7.45 (12H, m)

Example 63 (3)

Dichloromethane (3.4 mL) was added to the compound (168 mg) obtained in Example 63 (2), and the mixture was stirred at a temperature equal to or lower than -20°C. At the same temperature, anisole (799 μL) and aluminum chloride (244 mg) were sequentially added to the reaction mixture. The reaction mixture was stirred at a temperature equal to or lower than -20°C for 30 minutes. The reaction mixture was added to a mixture of acetonitrile (10 mL), water (10 mL), and trisodium citrate dihydrate (809 mg) under ice cooling. A saturated aqueous sodium hydrogen carbonate solution was added to the reaction mixture such that the pH was adjusted to 5.2, and the aqueous layer was separated. The aqueous layer was concentrated under reduced pressure, and the residue was purified by medium-pressure reverse-phase silica gel column chromatography [eluent; water:acetonitrile = 100:0 → 75:25]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(((E)-2-((2-(2-chloro-3,4-dihydroxybenzamido)ethyl)amino)-2-oxoethylidene)amino)-2-o xoimidazolidin-1-yl)-7-oxo-4-thia-1-azabicyclo[3.2.0]heptane-3-carboxylic acid (20.5 mg) as white solids.

NMR: 1.49 (3H, s), 1.51 (3H, s), 3.55 (1H, d, J = 13.6 Hz), 3.54-3.61 (4H, m), 3.76-3.84 (2H, m), 3.86-3.95 (2H, m),

4.73 (1H, d, J = 12.8 Hz), 5.70 (1H, d, J = 3.6 Hz), 5.75 (1H, d, J = 3.6 Hz), 6.86-6.95 (3H, m), 7.04 (1H, s)
MS: 811.05 $[M + H]^+$, 809.30 $[M - H]^-$

Example 64

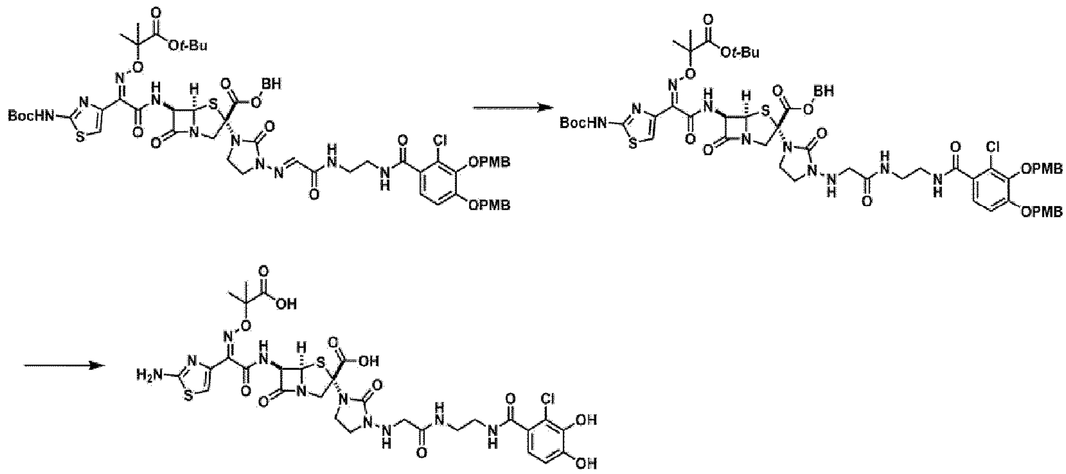

Example 64 (1)

Dichloromethane (3.4 mL) was added to benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-(((E)-2-((2-(2-chloro-3,4-bis((4-methoxyben zyl)oxy)benzamido)ethyl)amino)-2-oxoethylidene)amino)-2-oxoimidazolidin-1-yl)-7-oxo-4-th ia-1-azabicyclo[3.2.0]heptane-3-carboxylate (168 mg), and the mixture was stirred under ice cooling. At the same temperature, a 85% borane-2-picoline complex (19 mg) and p-toluenesulfonic acid monohydrate (47 mg) were sequentially added to the reaction mixture, and the reaction mixture was stirred at room temperature for 3 hours and 30 minutes. The reaction mixture was ice-cooled, and a 85% borane-2-picoline complex (12 mg) and p-toluenesulfonic acid monohydrate (23 mg) were sequentially added thereto, and the reaction mixture was stirred at room temperature overnight. At room temperature, the reaction mixture was added to a mixture of ethyl acetate (15 mL) and water (15 mL), and a saturated aqueous sodium hydrogen carbonate solution was added thereto such that the pH was adjusted to 4.6. The organic layer was separated, and the aqueous layer was extracted three times by using ethyl acetate. The organic layers were combined, sequentially washed with water and a saturated aqueous sodium chloride solution, and then dehydrated and dried over anhydrous sodium sulfate. The solvent was distilled off under reduced pressure, and the residue was purified by silica gel column chromatography [eluent; chloroform:2-propanol = 100:0 → 93:7], thereby obtaining a target substance (101 mg) as yellow solids.

Example 64 (2)

Dichloromethane (2.0 mL) was added to the compound (101 mg) obtained in Example 64 (1), and the mixture was stirred at a temperature equal to or lower than -20°C. At the same temperature, anisole (480 μL) and aluminum chloride (147 mg) were sequentially added to the reaction mixture. The reaction mixture was stirred at a temperature equal to or lower than -20°C for 30 minutes. The reaction mixture was added to a mixture of acetonitrile (6 mL), water (6 mL), and trisodium citrate dihydrate (486 mg) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 80:20]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-((-2-((2-(2-chloro-3,4-dihydroxybenzamido)ethyl)amino)-2-oxoethyl)amino)-2-oxoimida zolidin-1-yl)-7-oxo-4-thia-1-azabicyclo[3.2.0]heptane-3-carboxylic acid (1.7 mg) as light yellow solids.

NMR: 1.47 (3H, s), 1.49 (3H, s), 3.14 (1H, d, J = 13.2 Hz), 3.58 (2H, s), 3.34-3.65(8H, m), 4.40 (1H, d, J = 12.8 Hz), 5.38 (1H, d, J = 3.6 Hz), 5.68 (1H, d, J = 3.6 Hz), 6.90 (1H, d, J = 8.0 Hz), 6.93 (1H, d, J = 8.0 Hz), 6.99 (1H, s)
MS: 813.10 $[M + H]^+$, 810.90 $[M - H]^-$

Example 65

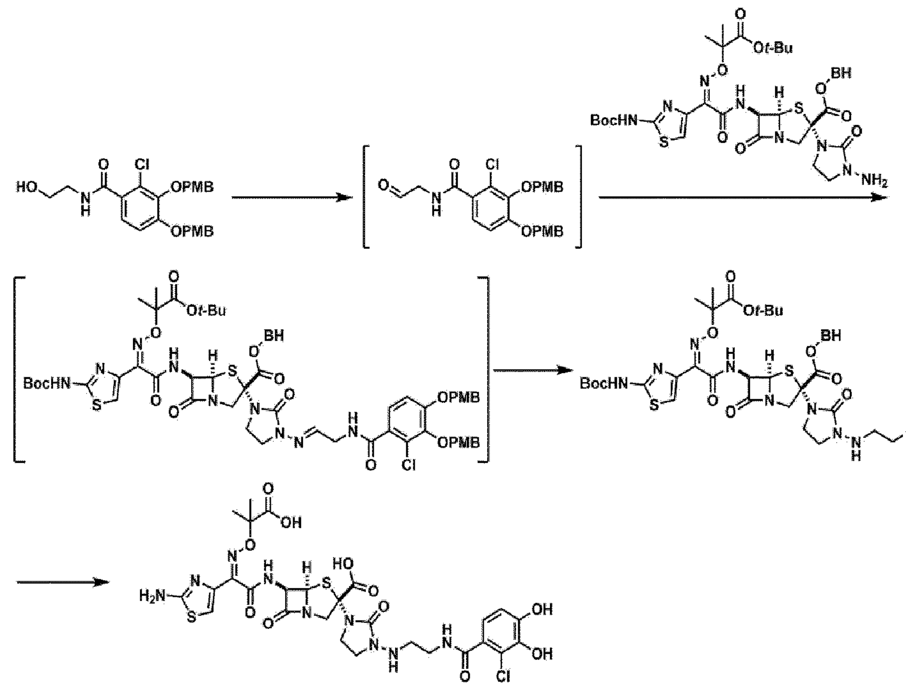

Example 65 (1)

Dichloromethane (3.6 mL) was added to 2-chloro-N-(2-hydroxyethyl)-3,4-bis ((4-methoxybenzyl)oxy)benzamide (0.36 g), and the mixture was stirred under ice cooling. Dess-Martin periodinane (0.65 g) was added to the reaction mixture, and the reaction mixture was stirred at room temperature for 1 hour. Dichloromethane (10 mL), water (5 mL), and a 1 mol/L aqueous sodium thiosulfate solution (5 mL) were sequentially added to the reaction mixture, and the organic layer was separated. The organic layer was washed with a saturated aqueous sodium chloride solution and dehydrated and dried over anhydrous sodium sulfate. The solvent was distilled off under reduced pressure, dichloromethane (10 mL) and benzhydryl (3R,5R,6R)-3-(3-amino-2-oxoimidazolidin-1-yl)-6-((Z)-2-(((1-(tert-butoxy)-2-methyl-1-oxopr opan-2-yl)oxy) imino)-2-(2-((tert-butoxycarbonyl)amino)thiazol-4-yl)acetamido)-7-oxo-4-thia-1-azabicyclo[3.2.0]heptane-3-carboxylate (0.50 g) were added to the residue, and the mixture was stirred at room temperature for 1 hour. The reaction mixture was ice-cooled, and a 85% borane-2-picoline complex (91 mg) and p-toluenesulfonic acid monohydrate (220 mg) were sequentially added to the reaction mixture, and the reaction mixture was stirred at room temperature for 1 hour. At room temperature, dichloromethane (5 mL) and water (10 mL) were added to the reaction mixture, and a saturated aqueous sodium hydrogen carbonate solution was added thereto such that the pH was adjusted to 3.5. The organic layer was separated, washed with a saturated aqueous sodium chloride solution, and then dehydrated and dried over anhydrous sodium sulfate. The solvent was distilled off under reduced pressure, and the residue was purified by silica gel column chromatography [eluent; ethyl acetate:hexane = 70:30 → 100:0], thereby obtaining benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-((2-(2-chloro-3,4-bis((4-methoxybenzyl)oxy )benzamido)ethyl)amino)-2-oxoimidazolidin-1-yl)-7-oxo-4-thia-1-azabicyclo[3.2.0]heptane-3-carboxylate (625 mg) as a yellow oily substance.

NMR ($CDCl_3$): 1.37 (9H, s), 1.44-1.56 (15H, m), 2.86-3.04 (2H, m), 3.15-3.29 (1H, m), 3.48-3.52 (6H, m), 3.53-3.62 (1H, m), 3.80 (3H, s), 3.83 (3H, s), 4.23-4.33 (1H, m), 4.92 (1H, d, J = 13.2 Hz), 4.95 (2H, s), 5.08 (2H, s), 5.56 (1H, d, J = 3.6 Hz), 5.79 (1H, dd, J = 8.4, 3.6 Hz), 6.80-6.87 (4H, m), 6.89-6.96 (4H, m), 7.13-7.29 (13H, m), 7.49 (1H, d, J = 8.4 Hz), 8.17 (1H, s)

Example 65 (2)

Dichloromethane (12.5 mL) was added to benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-((2-(2-chloro-3,4-bis((4-methoxybenzyl)oxy )benzamido)ethyl)amino)-2-oxoimidazolidin-1-yl)-7-oxo-4-thia-1-azabicyclo[3.2.0]heptane-3-carboxylate

(625 mg), and the mixture was stirred at a temperature equal to or lower than -20°C. At the same temperature, anisole (3.1 mL) and aluminum chloride (0.95 g) were sequentially added to the reaction mixture. The reaction mixture was stirred at a temperature equal to or lower than -20°C for 1 hour. The reaction mixture was added to a mixture of acetonitrile (30 mL), water (20 mL), and trisodium citrate dihydrate (3.14 g) under ice cooling. A saturated aqueous sodium hydrogen carbonate solution was added to the reaction mixture such that the pH was adjusted to 5.2, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-((2-(2-chloro-3,4-dihydroxybenzamido)ethyl)amino)-2-oxoimidazolidin-1-yl)-7-oxo-4-thia-1-azabicyclo[3.2.0]heptane-3-carboxylic acid (112.5 mg) as white solids.

NMR: 1.47 (3H, s), 1.49 (3H, s), 3.05-3.19 (2H, m), 3.30 (1H, d, J = 12.8 Hz), 3.43-3.66 (6H, m), 4.47 (1H, d, J = 12.8 Hz), 5.52 (1H, d, J = 3.6 Hz), 5.72 (1H, d, J = 3.6 Hz), 6.90 (1H, d, J = 8.0 Hz), 7.01 (1H, d, J = 8.0 Hz), 7.02 (1H, s)
MS: 756.05 $[M + H]^+$, 754.10 $[M - H]^-$

Example 66

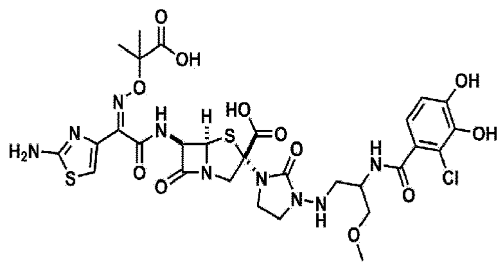

In the same manner as in Example 65, (3R,5R,6R)-6-((Z)-2-(2-aminothiazol-4-yl)-2-(((2-carboxypropan-2-yl)oxy)imino)acetamido)-3-(3-((2-(2-chloro-3,4-dihydroxybenzamido)-3-methoxypropyl)amino)-2-oxoimidazolidin-1-y l)-7-oxo-4-thia-1-azabicyclo[3.2.0]heptane-3-carboxylic acid was obtained.

NMR: 1.47 (3H, s), 1.49 (3H, s), 3.06-3.20 (2H, m), 3.24 (1H, d, J = 11.6 Hz), 3.38-3.75 (6H, m), 3.43 (3H, s), 4.26-4.38 (1H, m), 4.46 [4.48] (1H, d, J = 12.8 Hz), 5.52 [5.55] (1H, d, J = 3.6 Hz), 5.69-5.76 (1H, m), 6.91 (1H, d, J = 8.4 Hz), 6.99-7.06 (2H, m)
MS: 800.10 $[M + H]^+$, 797.95 $[M - H]^-$

Example 67

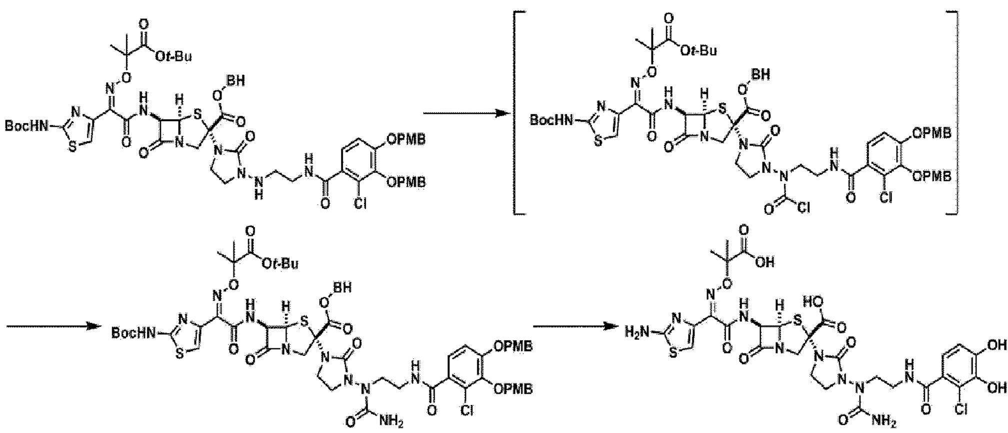

Example 67 (1)

Dichloromethane (2.2 mL) was added to benzhydryl (3R,5R,6R)-6-((Z)-2-(((1-(tert-butoxy)-2-methyl-1-oxopropan-2-yl)oxy)imino)-2-(2-((tert-but oxycarbonyl)amino)thiazol-4-yl)acetamido)-3-(3-((2-(2-chloro-3,4-bis((4-methoxybenzyl)oxy )benzamido)ethyl)amino)-2-oxoimidazolidin-1-yl)-7-oxo-4-thia-1-azabicyclo[3.2.0]heptane-3-carboxylate (0.22 g), and the mixture was stirred under ice cooling. At the same temperature, triphosgene (20 mg) and N,N-diisopropylethylamine (35 μL) were sequentially added to the reaction mixture, and the reaction mixture was stirred at room temperature for 1 hour 30 minutes. At room temperature, ammonium chloride (13 mg) and triethylamine (46 μL) were added to the reaction mixture, and the reaction mixture was stirred for 1 hour. The reaction mixture was added to a mixture of ethyl acetate (10 mL), water (10 mL), and 1 mol/L hydrochloric acid (2 mL). The organic layer was separated, washed with a 5% aqueous sodium chloride solution, and then dehydrated and dried over anhydrous sodium sulfate. The solvent was distilled off under reduced pressure, and the residue was purified by silica gel column chromatography [eluent; ethyl acetate:hexane = 100:0 → chloroform:2-propanol = 90:10], thereby obtaining a target substance (99 mg) as a light brown oily substance.

Example 67 (2)

Dichloromethane (2 mL) was added to the compound (100 mg) obtained in Example 67 (1), and the mixture was stirred at a temperature equal to or lower than -20°C. At the same temperature, anisole (480 μL) and aluminum chloride (147 mg) were sequentially added to the reaction mixture. The reaction mixture was stirred at a temperature equal to or lower than -20°C for 1 hour. The reaction mixture was added to a mixture of acetonitrile (10 mL), water (5 mL), and trisodium citrate dihydrate (486 mg) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(1-(2-(2-chloro-3,4-dihydroxybenzamido)ethyl)ureido)-2-oxoimidazolidin-1-yl)-7-oxo-4-thia-1-azabicyclo [3.2.0]heptane-3-carboxylic acid (2.8 mg) as white solids.

NMR: 1.44-1.52 (6H, m), 3.32 (1H, d, J = 12.4 Hz), 3.39-3.99 (8H, m), 4.41-4.51 (1H, m), 5.50 (1H, dd, J = 6.0, 3.6 Hz), 5.68-5.73 (1H, m), 6.88-6.94 (1H, m), 6.97-7.06 (2H, m)
MS: 799.05 $[M + H]^+$, 796.90 $[M - H]^-$

Example 68

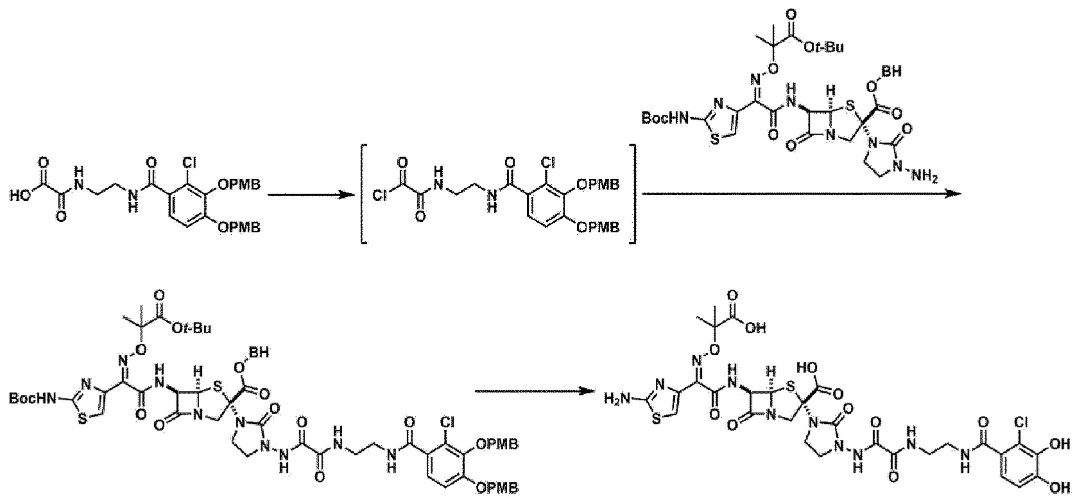

Example 68 (1)

Dichloromethane (1.3 mL) was added to the compound (70 mg) obtained in Reference Example 17, and the mixture was stirred under ice cooling. At the same temperature, oxalyl dichloride (13 μL) and DMF (7 μL) were sequentially added to the reaction mixture, and the reaction mixture was stirred for 1 hour. At the same temperature, oxalyl dichloride (7 μL) and DMF (3 μL) were sequentially added to the reaction mixture, and the reaction mixture was stirred for 1 hour, thereby obtaining a dichloromethane mixture containing the corresponding acid chloride.

THF (2.8 mL) and water (2.8 mL) were added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (111 mg), and the mixture was stirred under ice cooling. At the same temperature, sodium hydrogen carbonate (33 mg) was added to the reaction mixture. Then, at the same temperature, the prepared dichloromethane mixture containing the acid chloride was added dropwise to the reaction mixture. The reaction mixture was stirred at the same temperature for 15 minutes. Ethyl acetate (10 mL) and water (10 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining a target substance (179 mg) as yellow solids.

Example 68 (2)

Dichloromethane (3.6 mL) was added to the compound (179 mg) obtained in Example 68 (1), and the mixture was stirred at a temperature equal to or lower than -20°C. At the same temperature, anisole (841 μL) and aluminum chloride (258 mg) were sequentially added to the reaction mixture. The reaction mixture was stirred at a temperature equal to or lower than -20°C for 30 minutes. The reaction mixture was added to a mixture of acetonitrile (11 mL), water (11 mL), and trisodium citrate dihydrate (852 mg) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water: acetonitrile = 100:0 → 90:10]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(2-((2-(2-chloro-3,4-dihydroxybenzamido)ethyl)amino)-2-oxoacetamido)-2-oxoimidazoli din-1-yl)-7-oxo-4-thia-1-azabicyclo[3.2.0]heptane-3-carboxylic acid (29.1 mg) as white solids.

NMR: 1.48 (3H, s), 1.50 (3H, s), 3.54-3.62 (5H, m), 3.62-3.67 (2H, m), 3.69-3.79 (2H, m), 4.65 (1H, d, J = 12.4 Hz), 5.69 (1H, d, J = 3.6 Hz), 5.75 (1H, d, J = 3.2 Hz), 6.90 (1H, d, J = 8.4 Hz), 6.94 (1H, d, J = 8.0 Hz), 7.03 (1H, s)
MS: 827.10 $[M + H]^+$, 825.30 $[M - H]^-$

Example 69

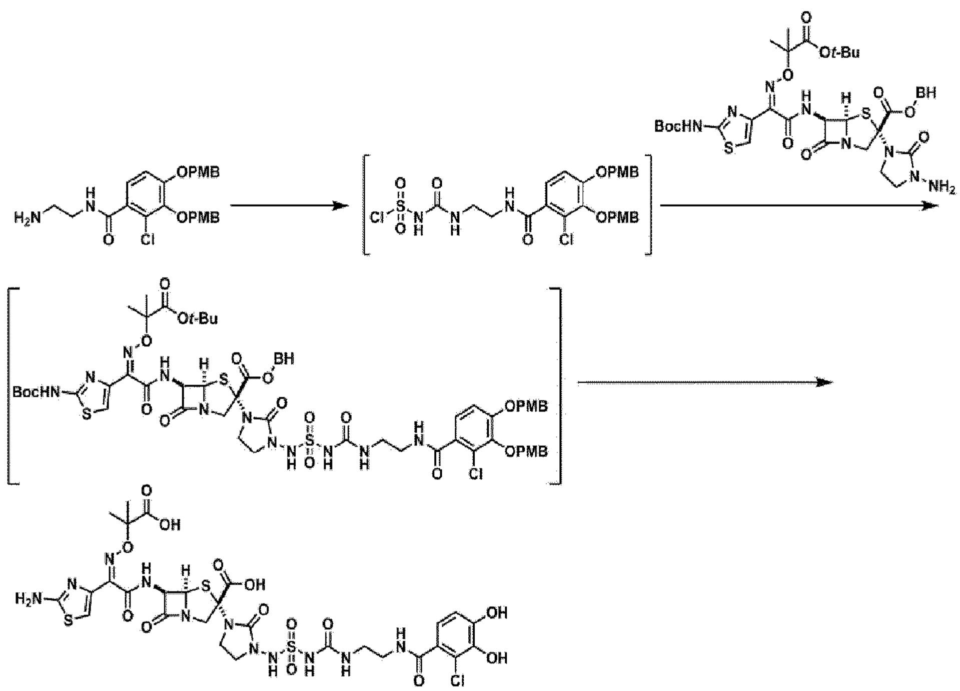

Example 69

Dichloromethane (1.9 mL) was added to N-(2-aminoethyl)-2-chloro-3,4-bis ((4-methoxybenzyl)oxy)benzamide (90 mg), and the mixture was stirred under ice cooling. At the same temperature, chlorosulfonyl isocyanate (17 μL) was added to the reaction mixture, and the reaction mixture was stirred at the same temperature for 40 minutes. At the same

temperature, benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (150 mg) and N,N-diisopropylethylamine (36 μL) were sequentially added to the reaction mixture, and the reaction mixture was stirred for 2 hours. At the same temperature, anisole (1.1 mL) and aluminum chloride (347 mg) were sequentially added to the reaction mixture, and the reaction mixture was stirred for 30 minutes. At the same temperature, the reaction mixture was added to a mixture of acetonitrile (20 mL), water (15 mL), and trisodium citrate dihydrate (1.15 g). A saturated aqueous sodium hydrogen carbonate solution was added to the reaction mixture such that the pH was adjusted to 5.3, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-((N-((2-(2-chloro-3,4-dihydroxybenzamido)ethyl)carbamoyl)sulfamoyl)amino)-2-oxoimi dazolidin-1-yl)-7-oxo-4-thia-1-azabicyclo [3.2.0]heptane-3-carboxylic acid as white solids.

MS: 878.00 $[M + H]^+$, 875.90 $[M - H]^-$

Example 70

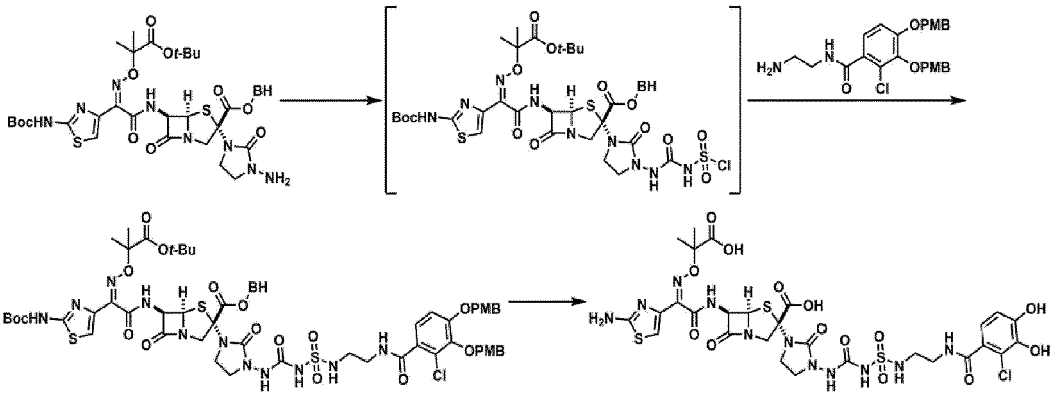

Example 70

Dichloromethane (1.7 mL) was added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (150 mg), and the mixture was stirred under ice cooling. At the same temperature, chlorosulfonyl isocyanate (15 μL) was added to the reaction mixture, and the reaction mixture was stirred at the same temperature for 40 minutes. At the same temperature, N-(2-aminoethyl)-2-chloro-3,4-bis((4-methoxybenzyl)oxy)benzamide (82 mg) and N,N-diisopropylethylamine (36 μL) were sequentially added to the reaction mixture, and the reaction mixture was stirred for 2 hours. At the same temperature, anisole (1.1 mL) and aluminum chloride (347 mg) were sequentially added to the reaction mixture, and the reaction mixture was stirred for 30 minutes. At the same temperature, the reaction mixture was added to a mixture of acetonitrile (20 mL), water (15 mL), and trisodium citrate dihydrate (1.15 g). A saturated aqueous sodium hydrogen carbonate solution was added to the reaction mixture such that the pH was adjusted to 5.3, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-(3-(N-(2-(2-chloro-3,4-dihydroxybenzamido)ethyl)sulfamoyl)ureido)-2-oxoimidazolidin-1-yl)-7-oxo-4-thia-1-azabicyclo[3.2.0]heptane-3-carboxylic acid as white solids.

NMR: 1.48 (3H, s), 1.49 (3H, s), 3.23 (2H, t, J = 6.2 Hz), 3.32-3.44 (1H, m), 3.49-3.60 (6H, m), 4.51 (1H, d, J = 12.8 Hz), 5.58 (1H, d, J = 3.6 Hz), 5.71 (1H, d, J = 3.2 Hz), 6.92 (1H, d, J = 8.0 Hz), 7.02 (1H, s), 7.07 (1H, d, J = 8.4 Hz)
MS: 878.05 $[M + H]^+$, 875.90 $[M - H]^-$

Example 71

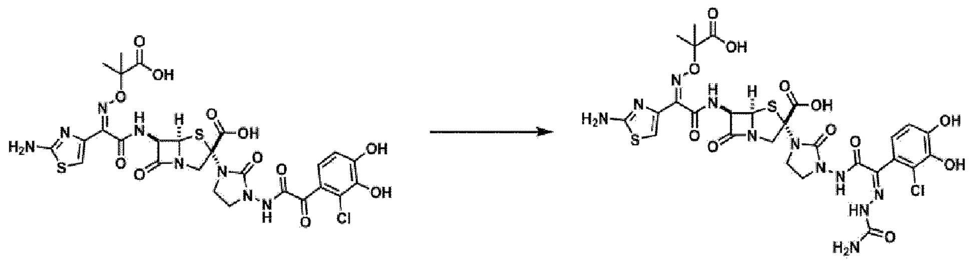

Water (1.0 mL) and semicarbazide hydrochloride (4.5 mg) were added to (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thi a-1-azabicyclo[3.2.0]heptane-3-carboxylic acid (30 mg), and the mixture was stirred. A saturated aqueous sodium hydrogen carbonate solution was added to the reaction mixture such that the pH was adjusted to 4.6, and the mixture was stirred at room temperature for 6 days. The reaction mixture was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-((Z)-2-(2-carbamoylhydrozono)-2-(2-chloro-3,4-dihydroxyphenyl)acetamido)-2-oxoimid azolidin-1-yl)-7-oxo-4-thia-1-azabicyclo[3.2.0]heptane-3-carboxylic acid (3.3 mg) as white solids.

NMR: 1.44-1.53 (6H, m), 3.52-3.80 (5H, m), 4.65 (1H, d, J = 12.4 Hz), 5.69 (1H, d, J = 3.6 Hz), 5.75 (1H, d, J = 3.6 Hz), 6.98 (1H, d, J = 8.4 Hz), 7.02 (1H, s), 7.07 (1H, d, J = 8.4 Hz)
MS: 798.05 $[M + H]^+$, 795.90 $[M - H]^-$

The compounds shown in Table 27 were obtained in the same manner as in Example 1.

[Table 27]

| Ex.No. | Structural Formula | Name |
|---|---|---|
| 72 | 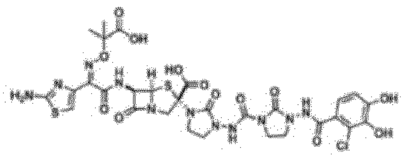 | (3R,5R,6R)-6-((Z)-2-(2-aminothiazol-4-yl)-2-(((2-carboxypropan-2-yl)oxy)imino)acetamido)-3-(3-(3-(2-chloro-3,4-dihydroxybenzamido)-2-oxoimidazolidine-1-carboxamido)-2-oxoimidazolidin-1-yl)-7-oxo-4-thia-1-azabicyclo[3.2.0]heptane-3-carboxylic acid |
| 73 | 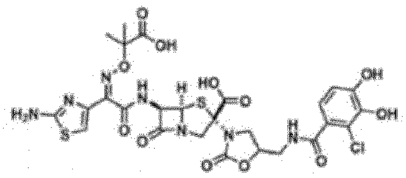 | (3R,5R,6R)-6-((Z)-2-(2-aminothiazol-4-yl)-2-(((2-carboxypropan-2-yl)oxy)imino)acetamido)-3-(5-((2-chloro-3,4-dihydroxybenzamido)methyl)-2-oxoimidazolidine-1-carboxamido)-2-oxoimidazolidin-3-yl)-7-oxo-4-thia-1-azabicyclo[3.2.0]heptane-3-carboxylic acid |
| 74 | 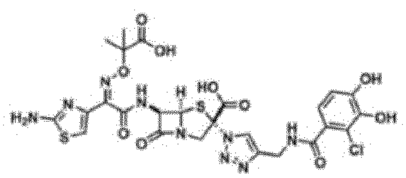 | (3R,5R,6R)-6-((Z)-2-(2-aminothiazol-4-yl)-2-(((2-carboxypropan-2-yl)oxy)imino)acetamido)-3-(4-((2-chloro-3,4-dihydroxybenzamido)methyl)-1H-1,2,3-triazol-1-yl)-7-oxo-4-thia-1-azabicyclo[3.2.0]heptane-3-carboxylic acid |

The measured values of NMR and MS of the compounds in the table are as follows.

Example 72

NMR: 1.49 (3H, s), 1.51 (3H, s), 3.57-3.75 (6H, m), 3.77-3.89 (2H, m), 3.93-4.02 (1H, m), 4.65 (1H, d, J = 12.0 Hz), 5.70 (1H, d, J = 3.6 Hz), 5.73-5.77 (1H, m), 6.95 (1H, d, J = 8.4 Hz), 7.03 (1H, s), 7.15 (1H, d, J = 8.4 Hz)
MS: 840.00 $[M + H]^+$, 837.85 $[M - H]^-$

Example 73

NMR: 1.43 (3H, s), 1.44 (3H, s), 3.72-3.89 (4H, m), 3.95-4.03 (1H, m), 4.60 (1H, d, J = 12.8 Hz), 4.91-5.00 (1H, m), 5.64 (1H, d, J = 3.6 Hz), 5.73 (1H, d, J = 3.6 Hz), 6.90 (1H, d, J = 8.4 Hz), 6.93 (1H, s), 7.00 (1H, d, J = 8.8 Hz)

MS: 728.00 [M + H]+, 726.00 [M - H]-

Example 74

NMR: 1.47 (3H, s), 1.50 (3H, s), 4.10 (1H, dd, J = 13.2, 1.2 Hz), 4.65-4.73 (3H, m), 5.59 (1H, d, J = 3.6 Hz), 5.81 (1H, dd, J = 3.8, 1.0 Hz), 6.91 (1H, d, J = 8.4 Hz), 7.01 (1H, s), 7.01 (1H, d, J = 8.4 Hz), 7.87 (1H, s)
MS: 710.05 [M + H]+, 708.00 [M - H]-

The compounds shown in Table 28 were obtained in the same manner as in Example 19.

[Table 28]

| Ex.No. | Structural Formula | Name |
|---|---|---|
| 75 | 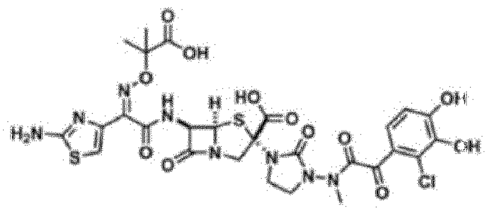 | (3R,5R,6R)-6-((Z)-2-(2-aminothiazol-4-yl)-2-(((2-carboxypropan-2-yl)oxy)imino)acetamido)-3-(3-(2-(2-chloro-3,4-diydroxyphenyl)-N-methyl-2-oxoacetamido)-2-oxoimidazolidin-1-yl)-7-oxo-4-thia-1-azabicyclo[3.2.0]heptane-3-carboxylic acid |
| 76 | 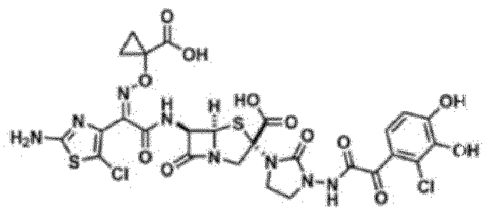 | (3R,5R,6R)-6-((Z)-2-(2-amino-5-chlorothiazol-4-yl)-2-((1-carboxycyclopropoxy)imino)acetamide)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 77 | 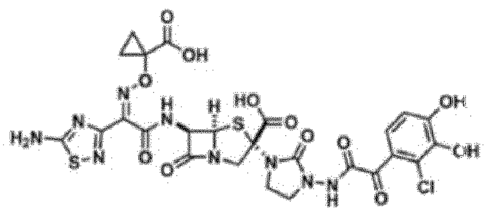 | (3R,5R,6R)-6-(Z)-2-(5-amino-1,2,4-thiadiazol-3-yl)-2-((1-carboxycyclopropoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 78 | 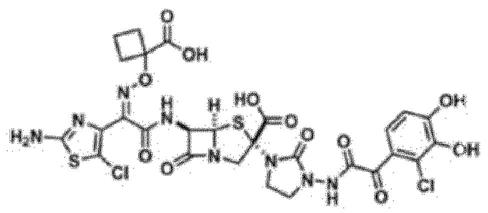 | (3R,5R,6R)-6-((Z)-2-(2-amino-5-chlorothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 79 | 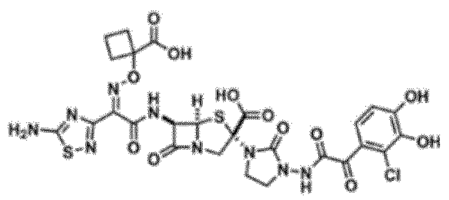 | (3R,5R,6R)-6-((Z)-2-(5-amino-1,2,4-thiadiazol-3-yl)-2-((1-carboxycyclobutyxo)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 80 | 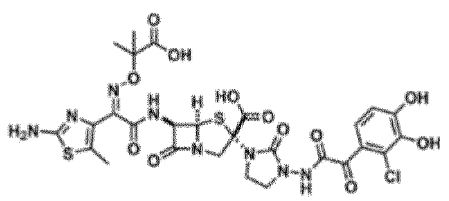 | (3R,5R,6R)-6-((Z)-2-(2-amino-5-methylthiazol-4-yl)-2-((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |

(continued)

| Ex.No. | Structural Formula | Name |
|---|---|---|
| 81 | 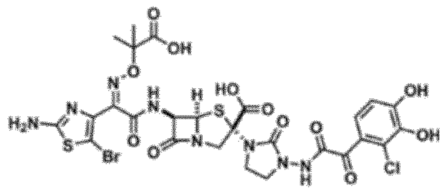 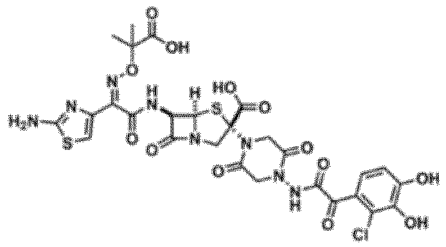 | (3R,5R,6R)-6-((Z)-2-(2-amino-5-bromothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 82 | 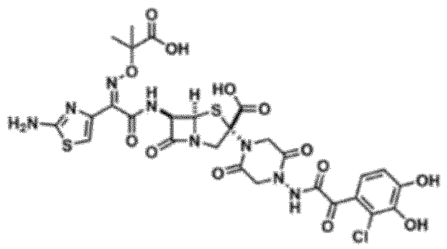 | (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)_2-oxoacetamido)-2,5-dioxopiperazin-1-yl)-7-oxo-4-thia-1-azabicyclo[3.2.0]heptane-3-carboxylic acid |
| 83 | 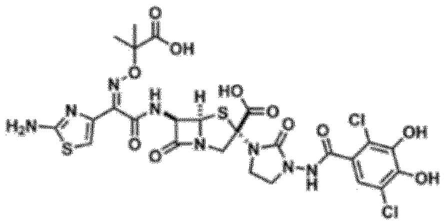 | (3R,5R,6R)-6-((Z)-2-(2-aminothiazol-4-yl)-2-(((2-carboxypropan-2-yl)oxy)imino)acetamido)-3-(3-(2,5-dichloro-3,4-dihydroxybenzamido)-2-oxoimidazolidin-1-yl)-7-oxo-4-thia-1-azabicyclo[3.2.0]heptane-3-carboxylic acid |
| 84 | 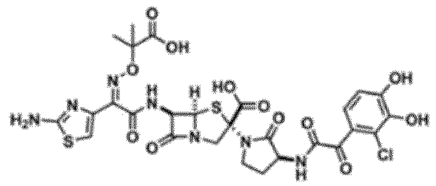 | (3R,5R,6R)-6-((Z)-2-(2-aminothiazol-4-yl)-2-(((2-carboxypropan-2-yl)oxy)imino)acetamido)-3-((S)-3-(2-(2-chloro-3,4-dihydroxyphenyl)-2-oxoacetamido)-2-oxopyrrolidin-1-yl)-7-oxo-4-thia-1-azabicyclo[3.2.0]heptane-3-carboxylic acid |
| 85 | 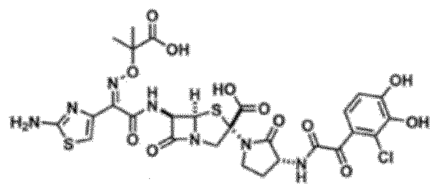 | (3R,5R,6R)-6-((Z)-2-(2-aminothiazol-4-yl)-2-(((2-carboxypropan-2-yl)oxy)imino)acetamido)-3-((R)-3-(2-(2-chloro-3,4-dihydroxyphenyl)-2-oxoacetamido)-2-oxopyrrolidin-1-yl)-7-oxo-4-thia-1-azabicyclo[3.2.0]heptane-3-carboxylic acid |

(continued)

| Ex.No. | Structural Formula | Name |
|---|---|---|
| 86 | 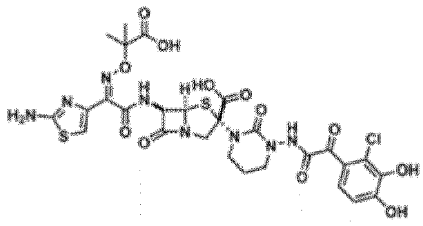 | (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxotetrahydropyrrolidin-1(2H)-yl)-7-oxo-4-thia-1-azabicyclo[3.2.0]heptane-3-carboxylic acid |

The measured values of NMR and MS of the compounds in the table are as follows.

Example 75

NMR: 1.49 (3H, s), 1.50 (3H, s), 3.18 (3H, s), 3.20-3.37 (2H, m), 3.44-3.52 (1H, m), 3.59-3.73 (2H, m), 4.29 (1H, d, J = 12.8 Hz), 4.99 (1H, d, J = 3.6 Hz), 5.71 (1H, d, J = 3.2 Hz), 6.94 (1H, d, J = 8.8 Hz), 7.04 (1H, s), 7.39 (1H, d, J = 8.4 Hz)
MS: 754.95 $[M + H]^+$, 752.95 $[M - H]^-$

Example 76

NMR: 1.22-1.46 (4H, m), 3.64 (1H, d, J = 12.8 Hz), 3.67-3.82 (4H, m), 4.66 (1H, d, J = 12.8 Hz), 5.67 (1H, d, J = 3.6 Hz), 5.79 (1H, d, J = 2.8 Hz), 6.87 (1H, d, J = 8.8 Hz), 7.44 (1H, d, J = 8.4 Hz)
MS: 772.90 $[M + H]^+$, 771.00 $[M - H]^-$

Example 77

NMR: 1.24-1.47 (4H, m), 3.62 (1H, dd, J = 13.0, 1.0 Hz), 3.67-3.82 (4H, m), 4.66 (1H, d, J = 12.8 Hz), 5.68 (1H, d, J = 3.6 Hz), 5.80 (1H, d, J = 2.8 Hz), 6.90 (1H, d, J = 8.8 Hz), 7.44 (1H, d, J = 8.4 Hz)
MS: 739.90 $[M + H]^+$, 737.90 $[M - H]^-$

Example 78

NMR: 1.81-1.93 (1H, m), 2.05-2.10 (1H, m), 2.26-2.40 (2H, m), 2.40-2.50 (1H, m), 2.51-2.61 (1H, m), 3.41-3.47 (1H, m), 3.65 (1H, d, J = 12.8 Hz), 3.65-3.81 (3H, m), 4.68 (1H, d, J = 12.4 Hz), 5.71 (1H, d, J = 2.8 Hz), 5.83 (1H, d, J = 3.6 Hz), 6.93 (1H, d, J = 8.8 Hz), 7.44 (1H, d, J = 8.4 Hz)
MS: 786.95 $[M + H]^+$, 785.15 $[M - H]^-$

Example 79

NMR: 1.85-1.95 (1H, m), 1.98-2.12 (1H, m), 2.28-2.43 (2H, m), 2.44-2.64 (2H, m), 3.41-3.46 (1H, m), 3.63 (1H, d, J = 12.8 Hz), 3.67-3.81 (3H, m), 4.68 (1H, d, J = 12.8 Hz), 5.72 (1H, d, J = 3.6 Hz), 5.84 (1H, d, J = 2.8 Hz), 6.95 (1H, d, J = 8.4 Hz), 7.44 (1H, d, J = 8.4 Hz)
MS: 754.25 $[M + H]^+$, 752.00 $[M - H]^-$

Example 80

NMR: 1.49 (3H, s), 1.50 (3H, s), 2.36 (3H, s), 3.58-3.62 (1H, m), 3.62-3.65 (1H, m), 3.68-3.80 (3H, m), 4.67 (1H, d, J = 12.4 Hz), 5.69 (1H, d, J = 3.6 Hz), 5.79 (1H, d, J = 4.0 Hz), 6.93 (1H, d, J = 8.4 Hz), 7.44 (1H, d, J = 8.4 Hz)
MS: 755.00 $[M + H]^+$, 753.05 $[M - H]^-$

Example 81

NMR: 1.52 (3H, s), 1.53 (3H, s), 3.64 (1H, d, J = 12.4 Hz), 3.69-3.79 (4H, m), 4.67 (1H, d, J = 12.4 Hz), 5.69 (1H, d, J = 4.0 Hz), 5.80 (1H, d, J = 2.8 Hz), 6.94 (1H, d, J = 8.4 Hz), 7.44 (1H, d, J = 8.8 Hz)
MS: 818.90 $[M + H]^+$, 816.90 $[M - H]^-$

Example 82

NMR: 1.49 (3H, s), 1.50 (3H, s), 3.26 (1H, d, J = 11.8 Hz), 4.32-4.62 (4H, m), 5.13 (1H, d, J = 12.8 Hz), 5.71 (1H, d, J = 3.6 Hz), 5.74 (1H, dd, J = 3.6, 0.8 Hz), 6.81 (1H, d, J = 8.8 Hz), 7.02 (1H, s), 7.48 (1H, d, J = 8.8 Hz)
MS: 769.05 $[M + H]^+$, 767.00 $[M - H]^-$

Example 83

NMR: 1.49 (3H, s), 1.51 (3H, s), 3.64 (1H, d, J = 12.8 Hz), 3.70-3.77 (4H, m), 4.68 (1H, d, J = 12.8 Hz), 5.71 (1H, d, J = 3.6 Hz), 5.76 (1H, d, J = 2.8 Hz), 6.97 (1H, s), 7.05 (1H, s)
MS: 746.95 $[M + H]^+$, 745.05 $[M - H]^-$

Example 84

NMR: 1.49 (3H, s), 1.50 (3H, s), 2.14-2.28 (1H, m), 2.56-2.68 (1H, m), 3.40 (1H, dd, J = 12.8, 1.2 Hz), 3.74-3.84 (1H, m), 3.84-3.93 (1H, m), 4.69 (1H, t, J = 9.4 Hz), 4.87 (1H, d, J = 12.8 Hz), 5.69 (1H, d, J = 3.6 Hz), 5.72 (1H, dd, J = 3.6, 0.8 Hz), 6.92 (1H, d, J = 8.4 Hz), 7.03 (1H, s), 7.38 (1H, d, J = 8.4 Hz)
MS: 740.05 $[M + H]^+$, 738.10 $[M - H]^-$

Example 85

NMR: 1.48 (3H, s), 1.51 (3H, s), 2.09-2.24 (1H, m), 2.58-2.71 (1H, m), 3.44 (1H, d, J = 12.8 Hz), 3.67-3.78 (1H, m), 3.80-3.90 (1H, m), 4.78-4.82 (2H, m),5.70 (1H, d, J = 3.6 Hz), 5.74 (1H, d, J = 3.6 Hz), 6.90 (1H, d, J = 8.4 Hz), 7.04 (1H, s), 7.39 (1H, d, J = 8.8 Hz)
MS: 740.05 $[M + H]^+$, 738.00 $[M - H]^-$

Example 86

NMR: 1.48 (3H, s), 1.50 (3H, s), 2.15-2.34 (2H, m), 3.25 (1H, d, J = 12.4 Hz), 3.44-3.53 (1H, m), 3.59-3.71 (2H, m), 3.84-3.96 (1H, m), 4.96 (1H, d, J = 12.8 Hz), 5.65 (1H, d, J = 3.2 Hz), 5.72 (1H, d, J = 2.8 Hz), 6.62 (1H, d, J = 8.4 Hz), 7.04 (1H, s), 7.32 (1H, d, J = 8.4 Hz)
MS: 755.05 $[M + H]^+$, 752.95 $[M - H]^-$
The compounds shown in Table 29 were obtained in the same manner as in Example 27.

[Table 29]

| Ex.No. | Structural Formula | Name |
|---|---|---|
| 87 | 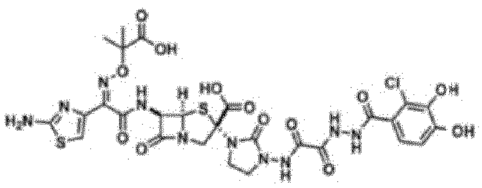 | (3R,5R,6R)-6-((Z)-2-(2-aminothiazol-4-yl)-2-(((2-carboxypropan-2-yl)oxy)imino)acetamido)-3-(3-(2-(2-(2-chloro-3,4-dihydroxybenzoyl)hydrazinyl)-2-oxoacetamido)-2-oxoimidazolidin-1-yl)-7-oxo-4-thia-1-azabicyclo[3.2.0]heptane-3-carboxylic acid |
| 88 | 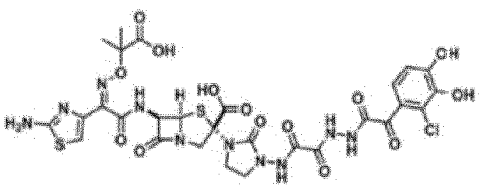 | (3R,5R,6R)-6-((Z)-2-(2-aminothiazol-4-yl)-2-(((2-carboxypropan-2-yl)oxy)imino)acetamido)-3-(3-(2-(2-(2-(2-chloro-3,4-dihydroxyphenyl)-2-oxoacetyl)hydrazinyl)-2-oxoacetamido)-2-oxoimidazolidin-1-yl)-7-oxo-4-thia-1-azabicyclo[3.2.0]heptane-3-carboxylic acid |
| 89 | 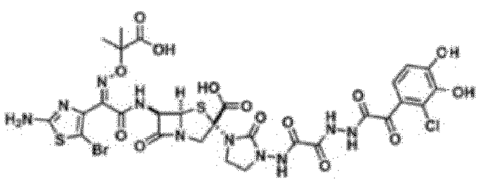 | (3R,5R,6R)-6-((Z)-2-(2-amino-5-bromothiazol-4-yl)-2-(((2-carboxypropan-2-yl)oxy)imino)acetamido)-3-(3-(2-(2-(2-(2-chloro-3,4-dihydroxyphenyl)-2-oxoacetyl)hydrazinyl)-2-oxoacetamido)-2-oxoimidazolidin-1-yl)-7-oxo-4-thia-1-azabicyclo[3.2.0]heptane-3-carboxylic acid |

(continued)

| Ex.No. | Structural Formula | Name |
|---|---|---|
| 90 | 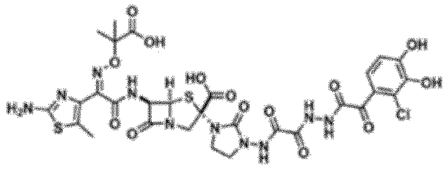 | (3R,5R,6R)-6-((Z)-2-(2-amino-5-methylthiazol-4-yl)-2-(((2-carboxypropan-2-yl)oxy)imino)acetamido)-3-(3-(2-(2-(2-(2-chloro-3,4-dihydroxyphenyl)-2-oxoacetyl)hydrazinyl)-2-oxoacetamido)-2-oxoimidazolidin-1-yl)-7-oxo-4-thia-1-azabicyclo[3.2.0]heptane-3-carboxylic acid |
| 91 | 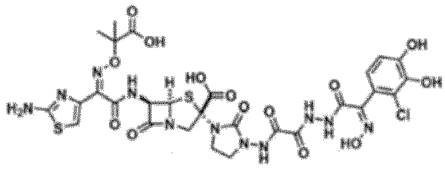 | (3R,5R,6R)-6-((Z)-2-(2-aminothiazol-4-yl)-2-(((2-carboxypropan-2-yl)oxy)imino)acetamido)-3-(3-(2-(2-((Z)-2-(2-chloro-3,4-dihydroxyphenyl)-2-hydroxyimino)acetyl)hydrazinyl)-2-oxoacetamido)-2-oxoimidazolidin-1-yl)-7-oxo-4-thia-1-azabicyclo [3.2.0]heptane-3-carboxylic acid |
| 92 | 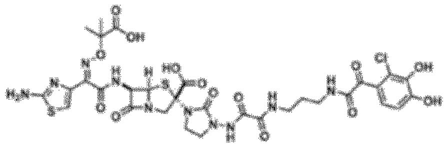 | (3R,5R,6R)-6-((Z)-2-(2-aminothiazol-4-yl)-2-(((2-carboxypropan-2-yl)oxy)imino)acetamido)-3-(3-(2-((3-(2-(2-chloro-3,4-dihydroxyphenyl)-2-oxoacetamido)propyl)amino)-2-oxoacetamido)-2-oxoimidazolidin-1-yl)-7-oxo-4-thia-1-azabicyclo[3.2.0]heptane-3-carboxylic acid |
| 93 | 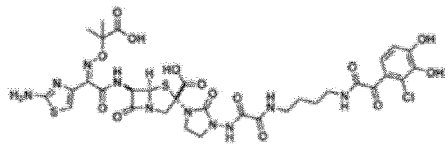 | (3R,5R,6R)-6-((Z)-2-(2-aminothiazol-4-yl)-2-(((2-carboxypropan-2-yl)oxy)imino)acetamido)-3-(3-(2-((4-(2-(2-chloro-3,4-dihydroxyphenyl)-2-oxoacetamido)butyl)amino)-2-oxoacetamido)-2-oxoimidazolidin-1-yl)-7-oxo-4-thia-1-azabicyclo[3.2.0]heptane-3-carboxylic acid |
| 94 | 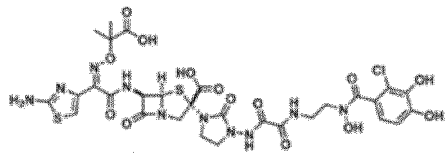 | (3R,5R,6R)-6-((Z)-2-(2-aminothiazol-4-yl)-2-(((2-carboxypropan-2-yl)oxy)imino)acetamido)-3-(3-(2-((2-(2-chloro-N,3,4-trihydroxybenzamido)ethyl)amino)-2-oxoacetamido)-2-oxoimidazolidin-1-yl)-7-oxo-4-thia-1-azabicyclo[3.2.0]heptane-3-carboxylic acid |

The measured values of NMR and MS of the compounds in the table are as follows.

Example 87

NMR: 1.49 (3H, s), 1.51 (3H, s), 3.61 (1H, d, J = 12.8 Hz), 3.64-3.79 (4H, m), 4.67 (1H, d, J = 12.4 Hz), 5.70 (1H, d, J = 3.6 Hz), 5.75 (1H, d, J = 2.8 Hz), 6.95 (1H, d, J = 8.4 Hz), 7.04 (1H, s), 7.15 (1H, d, J = 8.4 Hz)
MS: 799.05 $[M + H]^+$, 797.00 $[M - H]^-$

Example 88

NMR: 1.49 (3H, s), 1.51 (3H, s), 3.61 (1H, d, J = 12.8 Hz), 3.64-3.79 (4H, m), 4.67 (1H, d, J = 12.4 Hz), 5.70 (1H, d, J = 3.6 Hz), 5.75 (1H, d, J = 2.8 Hz), 6.92 (1H, d, J = 8.4 Hz), 7.03 (1H, s), 7.42 (1H, d, J = 8.4 Hz)
MS: 827.05 $[M + H]^+$, 824.90 $[M - H]^-$

Example 89

NMR: 1.52 (3H, s), 1.53 (3H, s), 3.60-3.70 (3H, m), 3.70-3.78 (2H, m), 4.66 (1H, d, J = 13.6 Hz), 5.68 (1H, d, J = 4.0 Hz), 5.80 (1H, d, J = 3.6 Hz), 6.95 (1H, d, J = 8.4 Hz), 7.43 (1H, d, J = 8.8 Hz)
MS: 904.90 $[M + H]^+$, 902.60 $[M - H]^-$

Example 90

NMR: 1.50 (3H, s), 1.52 (3H, s), 2.34 (3H, s), 3.48-3.80 (5H, m), 4.65 (1H, d, J = 12.8 Hz), 5.68 (1H, d, J = 3.6 Hz),

5.78 (1H, d, J = 2.8 Hz), 6.97 (1H, d, J = 8.8 Hz), 7.44 (1H, d, J = 8.4 Hz)
MS: 841.00 $[M + H]^+$, 838.90 $[M - H]^-$

Example 91

NMR: 1.49 (3H, s), 1.51 (3H, s), 3.59 (1H, d, J = 12.4 Hz), 3.62-3.78 (4H, m), 4.66 (1H, d, J = 12.8 Hz), 5.69 (1H, d, J = 3.6 Hz), 5.75 (1H, d, J = 4.0 Hz), 6.88 (1H, d, J = 8.4 Hz), 6.98 (1H, d, J = 8.4 Hz), 7.03 (1H, s)
MS: 842.05 $[M + H]^+$, 839.90 $[M - H]^-$

Example 92

NMR: 1.49 (3H, s), 1.50 (3H, s), 1.92 (2H, t, J = 6.4 Hz), 3.38-3.46 (4H, m), 3.54-3.65 (3H, m), 3.68-3.76 (2H, m), 4.64 (1H, d, J = 12.4 Hz), 5.68 (1H, d, J = 4.0 Hz), 5.74 (1H, d, J = 2.8 Hz), 6.96 (1H, d, J = 8.4 Hz), 7.03 (1H, s), 7.33 (1H, d, J = 8.8 Hz)
MS: 869.10 $[M + H]^+$, 867.00 $[M - H]^-$

Example 93

NMR: 1.49 (3H, s), 1.50 (3H, s), 1.62-1.70 (4H, m), 3.31-3.42 (4H, m), 3.53-3.66 (3H, m), 3.66-3.76 (2H, m), 4.65 (1H, d, J = 12.8 Hz), 5.68 (1H, d, J = 4.0 Hz), 5.74 (1H, d, J = 3.2 Hz), 6.96 (1H, d, J = 8.4 Hz), 7.04 (1H, s), 7.31 (1H, d, J = 8.4 Hz)
MS: 883.05 $[M + H]^+$, 880.95 $[M - H]^-$

Example 94

NMR: 1.49 (3H, s), 1.51 (3H, s), 3.48-3.56 (2H, m), 3.60 (1H, d, J = 13.2 Hz), 3.63-3.83 (6H, m), 4.67 (1H, d, J = 12.8 Hz), 5.71 (1H, d, J = 4.0 Hz), 5.75 (1H, d, J = 2.8 Hz), 6.77 (1H, d, J = 8.4 Hz), 6.93 (1H, d, J = 8.4 Hz), 7.04 (1H, s)
MS: 843.10 $[M + H]^+$, 840.90 $[M - H]^-$

The compounds shown in Table 30 were obtained in the same manner as in Example 49.

[Table 30]

| Ex.No. | Structural Formula | Name |
|---|---|---|
| 95 | 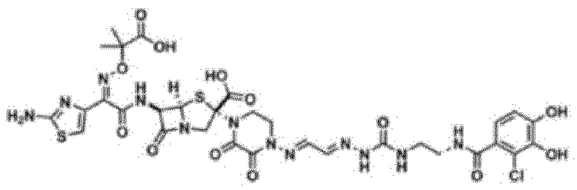 | (3R,5R,6R)-6-((Z)-2-(2-aminothiazol-4-yl)-2-(((2-carboxypropan-2-yl)oxy)imino)acetamido)-3-(4-(((1E,2E)-2-(2-((2-(2-chloro-3,4-dihydroxybenzamido)ethyl)carba moyl)-2-oxoacetamido)-2-oxoimihydrazinylidene)ethyliden e)amino)-2,3-dioxopiperazin-1-yl)-7-oxo-4-thia-1-azabicyclo[3.2.0]heptane-3-carboxylic acid |

The measured values of NMR and MS of the compounds in the table are as follows.

Example 95

NMR: 1.49 (3H, s), 1.51 (3H, s), 3.26 (1H, d, J = 12.8 Hz), 3.49-3.60 (4H, m), 4.05-4.35 (4H, m), 5.08 (1H, d, J = 13.2 Hz), 5.71 (1H, d, J = 4.0 Hz), 5.75 (1H, d, J = 2.8 Hz), 6.90 (1H, d, J = 8.4 Hz), 6.97 (1H, d, J = 8.4 Hz), 7.03 (1H, s), 7.70 (1H, d, J = 8.0 Hz), 7.95 (1H, d, J = 8.0 Hz)
MS: 881.15 $[M + H]^+$, 878.90 $[M - H]^-$

The compounds shown in Table 31 were obtained in the same manner as in Example 59.

[Table 31]

| Ex.No. | Structural Formula | Name |
|---|---|---|
| 96 | 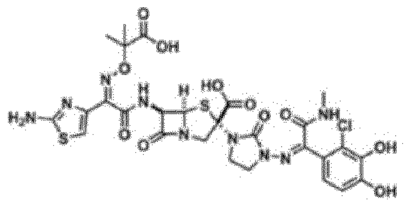 | (3R,5R,6R)-6-((Z)-2-(2-aminothiazol-4-yl)-2-(((2-carboxypropan-2-yl)oxy)imino)acetamido)-3-(3-(((Z)-1-(2-chloro-3,4-dihydroxyphenyl)-2-(methylamino)-2-oxoethylidene)amino)-2-oxoimidazolidin-1-yl)-7-oxo-4-thia-1-azabicyclo[3.2.0]heptane-3-carboxylic acid |
| 97 | 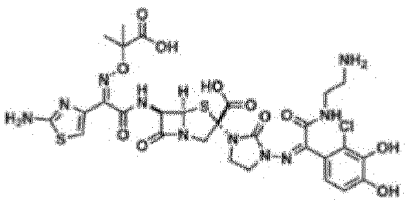 | (3R,5R,6R)-3-(3-(((Z)-2-((2-aminoethyl)amino)-1-(2-chloro-3,4-dihydroxyphenyl)-2-oxoethylidene)amino)-2-oxoimidazolidin-1-yl)-6-((Z)-2-(2-aminothiazol-4-yl)-2-(((2-carboxypropan-2-yl)oxy)imino)acetamido)-7-oxo-4-thia-1-azabicyclo[3.2.0]heptane-3-carboxylic acid |
| 98 | 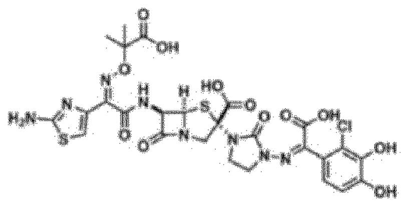 | (3R,5R,6R)-6-((Z)-2-(2-aminothiazol-4-yl)-2-(((2-carboxypropan-2-yl)oxy)imino)acetamido)-3-(3-(((Z)-carboxy(2-chloro-3,4-dihydroxyphenyl)methylene)amino)-2-oxoimidazolin-1-yl)-7-oxo-4-thia-1-azabicyclo[3.2.0]heptane-3-carboxylic acid |

The measured values of NMR and MS of the compounds in the table are as follows.

Example 96

NMR: 1.48 (3H, s), 1.50 (3H, s), 2.86 (3H, s), 3.11-3.35 (2H, m), 3.49 (1H, d, J = 12.4 Hz), 3.53-3.65 (2H, m), 5.67 (1H, d, J = 3.6 Hz), 5.74 (1H, d, J = 3.2 Hz), 6.81 (1H, d, J = 8.0 Hz), 6.94 (1H, d, J = 8.0 Hz), 7.02 (1H, s)
MS: 754.05 $[M + H]^+$, 752.05 $[M - H]^-$

Example 97

NMR: 1.57 (3H, s), 1.61 (3H, s), 3.18-3.25 (4H, m), 3.55-3.69 (5H, m), 4.93-5.09 (1H, m), 5.42-5.49 (1H, m), 5.69-5.73 (1H, m), 6.81 (1H, d, J = 8.4 Hz), 6.93 (1H, d, J = 8.4 Hz), 7.20-7.24 (1H, m)
MS: 783.10 $[M + H]^+$, 781.05 $[M - H]^-$

Example 98

NMR: 1.48 (3H, s), 1.49 (3H, s), 3.12-3.24 (2H, m), 3.51-3.60 (3H, m), 4.67 (1H, d, J = 12.8 Hz), 5.64 (1H, d, J = 4.0 Hz), 5.73 (1H, d, J = 3.2 Hz), 6.79 (1H, d, J = 8.4 Hz), 6.93 (1H, d, J = 8.0 Hz), 7.02 (1H, s)
MS: 741.05 $[M + H]^+$, 739.10 $[M - H]^-$

Example 99

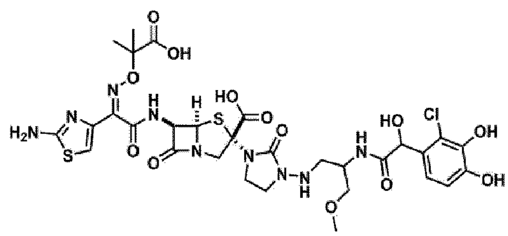

In the same manner as in Example 65, (3R,5R,6R)-6-((Z)-2-(2-aminothiazol-4-yl)-2-(((2-carboxypropan-2-yl) oxy)imino)acetamido)-3-(3-((2-(2-(2-chloro-3,4-dihydroxyphenyl)-2-hydroxyacetamido)-3-methoxypropyl)amino)-2-oxoimidazolidin-1-yl)-7-oxo-4-thia-1-azabicyclo[3.2.0]heptane-3-carboxylic acid was obtained.

NMR: 1.42-1.56 (6H, m), 3.03-3.13 (1H, m), 3.16-3.99 (11H, m), 4.12-4.34 (1H, m), 4.45-4.56 (1H, m), 5.10-5.39 (1H, m), 5.45-5.63 (1H, m), 5.64-5.78 (1H, m), 6.82-6.97 (2H, m), 6.98-7.06 (1H, m)
MS: 830.10 [M + H]$^+$, 827.90 [M - H]$^-$

The compounds shown in Table 32 were obtained in the same manner as in Example 68.

[Table 32]

| Ex.No. | Structural formula | Name |
|---|---|---|
| 100 | 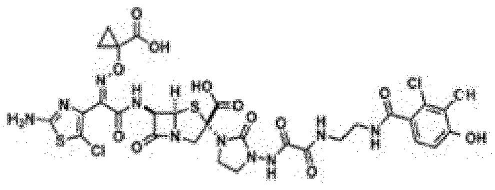 | (3R,5R,6R)-6-((Z)-2-(2-amino-5-chlorothiazol-4-yl)-2-((1-carboxycyclopropoxy)imino)acetamido)-3-(3-(2-((2-(2-chloro-3,4-dihydroxybenzamido) ethyl)amino)-2-oxoacetamido)-2-oxoimidazolidin-1-yl)-7-oxo-4-thia-1-azabicyclo[3.2.0]heptane-3-carboxylic acid |
| 101 | 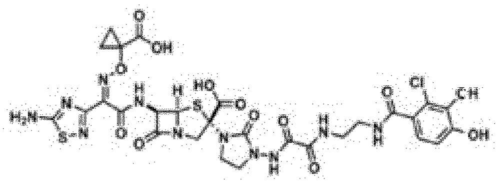 | (3R,5R,6R)-6-((Z)-2-(5-amino-1,2,4-thiadiazol-3-yl)-2-((1-carboxycyclopropoxy)imino)acetamido)-3-(3-(2-((2-(2-chloro-3,4-dihydroxybenzamido) ethyl)amino)-2-oxoacetamido)-2-oxoimidazolin-1-yl)-7-oxo-4-thia-1-azabicyclo[3.2.0]heptane-3-carboxylic acid |
| 102 | 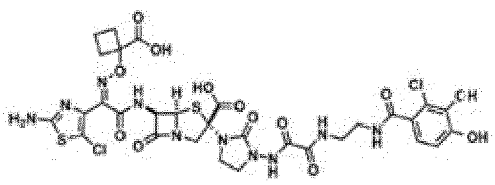 | (3R,5R,6R)-6-((Z)-2-(2-amino-5-chlorothiazol-4-yl)-2-((1-carboxycyclobutoxy)imino)acetamido)-3-(3-(2-((2-(2-chloro-3,4-dihydroxybenzamido) ethyl)amino)-2-oxcacetamido)-2-oxoimidazolidin-1-yl)-7 oxo-4-thia-1-azabicyclo[3.2.0]heptane-3-carboxylic acid |
| 103 | 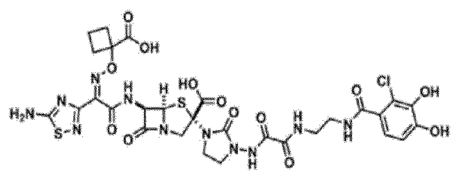 | (3R,5R,6R)-6-((Z)-2-(5-amino-1,2,4-thiadiazol-3-yl)-2-((1-carboxycyclobutoxy)imino)acetamido)-3-(3-(2-((2-(2-chloro-3,4-dihydroxybenzamido) ethyl)amino)-2-oxoacetamido)-2-oxoimidazolidin-1-yl)-7-oxo-4-thia-1-azabicyclo[3.2.0]heptane-3-carboxylic acid |
| 104 | 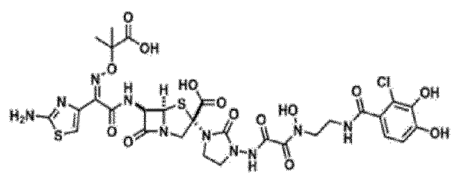 | (3R,5R,6R)-6-((Z)-2-(2-aminothiazol-4-yl)-2-(((2-carboxypropan-2-yl)oxy)imino)acetamido)-3-(3-(2-((2-(2-chloro-3,4-dihydroxybenzamido)ethyl)(hydroxy)amino )-2-oxoacetamido)-2-oxoimidazolidin-1-yl)-7-oxo-4-thia-1-azabicyclo[3.2.0]heptane-3-carboxylic acid |

The measured values of NMR and MS of the compounds in the table are as follows.

Example 100

NMR: 1.23-1.44 (4H, m), 3.53-3.67 (8H, m), 3.72 (1H, d, J = 6.8 Hz), 4.63 (1H, d, J = 12.8 Hz), 5.65 (1H, d, J = 4.0 Hz), 5.78 (1H, d, J = 3.2 Hz), 6.89 (1H, d, J = 8.0 Hz), 6.94 (1H, d, J = 8.4 Hz)
MS: 858.95 [M + H]$^+$, 856.95 [M - H]$^-$

Example 101

NMR: 1.27-1.46 (4H, m), 3.50-3.69 (7H, m), 3.69-3.77 (2H, m), 4.63 (1H, d, J = 12.8 Hz), 5.66 (1H, d, J = 3.6 Hz), 5.79 (1H, d, J = 3.6 Hz), 6.90 (1H, d, J = 8.4 Hz), 6.94 (1H, d, J = 8.4 Hz)
MS: 826.15[M + H]$^+$, 824.10[M - H]$^-$

Example 102

NMR: 1.82-1.95 (1H, m), 1.96-2.09 (1H, m), 2.27-2.40 (2H, m), 2.40-2.50 (1H, m), 2.51-2.60 (1H, m), 3.54-3.67 (7H, m), 3.70-3.75 (2H, m), 4.65 (1H, d, J = 12.8 Hz), 5.69 (1H, d, J = 3.6 Hz), 5.82 (1H, d, J = 3.6 Hz), 6.90 (1H, d, J = 8.4 Hz), 6.94 (1H, d, J = 8.4 Hz)
MS: 873.00 $[M + H]^+$, 871.00 $[M - H]^-$

Example 103

NMR: 1.84-1.97 (1H, m), 1.98-2.11 (1H, m), 2.28-2.44 (2H, m), 2.44-2.53 (1H, m), 2.53-2.63 (1H, m), 3.54-3.68 (7H, m), 3.70-3.77 (2H, m), 4.66 (1H, d, J = 12.4 Hz), 5.70 (1H, d, J = 3.6 Hz), 5.83 (1H, dd, J = 3.6,1.2 Hz), 6.89 (1H, d, J = 8.4 Hz), 6.94 (1H, d, J = 8.4 Hz)
MS: 840.25 $[M + H]^+$, 838.25 $[M - H]^-$

Example 104

NMR: 1.45 (3H, s), 1.46 (3H, s), 3.42-3.73 (9H, m), 4.55 (1H, d, J = 12.4 Hz), 5.65 (1H, d, J = 3.6 Hz), 5.70 (1H, dd, J = 10.2, 3.0 Hz), 6.88 (1H, s), 6.93 (1H, d, J = 8.8 Hz), 6.99 (1H, d, J = 6.4 Hz)
MS: 843.05 $[M + H]^+$, 841.05 $[M - H]^-$

Example 105

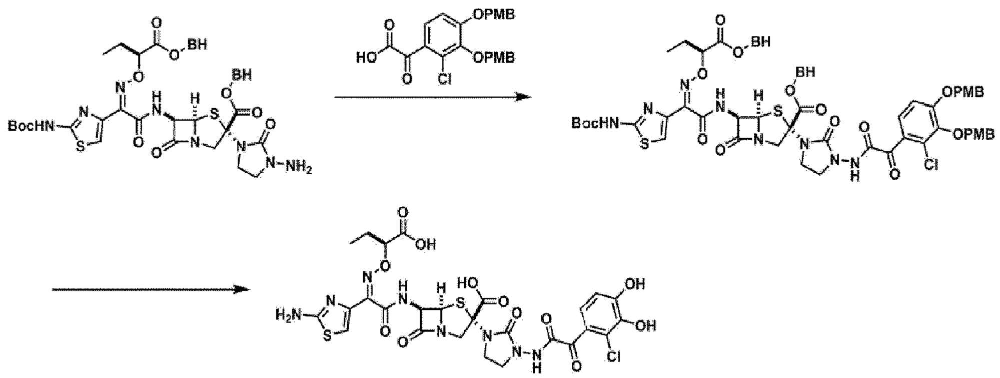

Example 105 (1)

2-(2-Chloro-3,4-bis((4-methoxybenzyl)oxy)phenyl)-2-oxoacetic acid (155 mg), HATU (129 mg), DMAC (3 mL), and NMM (75 μL) were sequentially added to benzhydryl (3R,5R,6R)-3-(3-amino-2-oxoimidazolidin-1-yl)-6-((Z)-2-((((S)-1-(benzhydryloxy)-1-oxobuta n-2-yl)oxy)imino)-2-(2-((tert-butoxycarbonyl)amino)thiazol-4-yl)acetamido)-7-oxo-4-thia-1-a zabicyclo[3.2.0]heptane-3-carboxylate (300 mg). The reaction mixture was stirred at room temperature for 2 hours. Ethyl acetate (10 mL) and water (10 mL) were added to the reaction mixture, and the organic layer was separated. The organic layer was washed with a saturated aqueous sodium chloride solution and dehydrated and dried over anhydrous sodium sulfate. The solvent was distilled off under reduced pressure. The residue was purified by silica gel column chromatography [eluent; ethyl acetate:hexane = 30:70 → 70:30], thereby obtaining a target substance (374 mg) as light yellow solids.

Example 105 (2)

Dichloromethane (5.6 mL) was added to the compound (374 mg) obtained in Example 105 (1), and the mixture was stirred at -20°C. At the same temperature, anisole (1.7 mL) and aluminum chloride (529 mg) were sequentially added to the reaction mixture. The reaction mixture was stirred at a temperature equal to or lower than -20°C for 30 minutes. The reaction mixture was added to a mixture of acetonitrile (15 mL), water (15 mL), and trisodium citrate dihydrate (1.75 g) under ice cooling. A saturated aqueous sodium hydrogen carbonate solution was added to the reaction mixture such that the pH was adjusted to 5.3, and the aqueous layer was separated. The aqueous layer was concentrated under reduced pressure, and the residue was purified by medium-pressure reverse-phase silica gel column chromatography [eluent;

water: acetonitrile = 100:0 → 90:10]. The aqueous solution containing a target substance was lyophilized, thereby obtaining (3R,5R,6R)-6-((Z)-2-(2-aminothiazol-4-yl)-2-(((S)-1-carboxypropoxy)imino)acetamido)-3-(3-(2-(2-chloro-3,4-dihydroxyphenyl)-2-oxoacetamido)-2-oxoimidazolidin-1-yl)-7-oxo-4-thia-1-a zabicyclo[3.2.0]heptane-3-carboxylic acid (90 mg) as yellow solids.

NMR: 0.97 (3H, t, J = 7.4 Hz), 1.77-1.94 (2H, m), 3.63 (1H,d, J=12.8 Hz), 3.67-3.87 (4H, m), 4.49-4.56 (1H, m), 4.68 (1H, d, J = 12.8 Hz), 5.71 (1H, d, J = 4.0 Hz), 5.79 (1H, d, J = 3.6 Hz), 6.83 (1H, d, J = 8.4 Hz), 7.05-7.08 (1H, m), 7.44 (1H, d, J = 8.8 Hz)

MS: 741.05 $[M + H]^+$, 738.95 $[M - H]^-$

The compounds shown in Table 33 were obtained in the same manner as in Example 105.

[Table 33]

| Ex. No. | Structural formula | Name |
|---|---|---|
| 106 | 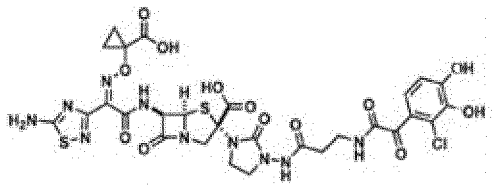 | (3R,5R,6R)-6-((Z)-2-(5-amino-1,2,4-thiadiazol-3-yl)-2-((1-carboxycyclopropoxy)imino)acetamido)-3-(3-(3-(2-(2-chloro-3,4-dihydroxyphenyl)-2-oxoacetamido)proanamido)-2-oxoimidazolidin-1-yl)-7-oxo-4-thia-1-azabicyclo[3.2.0]heptane-3-carboxylic acid |
| 107 | 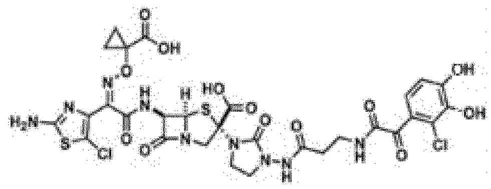 | (3R,5R,6R)-6-((Z)-2-(2-amino-5-chlorothiazol-4-yl)-2-((1-carboxycyclopropoxy)imino)acetamido)-3-(3-(3-(2-(2-chloro-3,4-dihydroxyphenyl)-2-oxoacetamido)proanamido)-2-oxoimidazolidin-1-yl)-7-oxo-4-thia-1-azabicyclo[3.2.0]heptane-3-carboxylic acid |
| 108 | 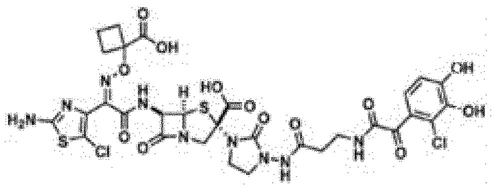 | (3R,5R,6R)-6-((Z)-2-(2-amino-5-chlorothiazol-4-yl)-2-((1-carboxycyclobutoxy)imino)acetamido)-3-(3-(3-(2-(2-chloro-3,4-dihydroxyphenyl)-2-oxoacetamido)proanamido)-2-oxoimidazolidin-1-yl)-7-oxo-4-thia-1-azabicyclo[3.2.0]heptane-3-carboxylic acid |
| 109 | 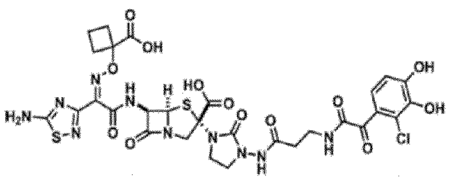 | (3R,5R,6R)-6-((Z)-2-(5-amino-1,2,4-thiadiazol-3-yl)-2-((1-carboxycyclobutoxy)imino)acetamido)-3-(3-(3-(2-(2-chloro-3,4-dihydroxyphenyl)-2-oxoacetamido)proanamido)-2-oxoimidazolidin-1-yl)-7-oxo-4-thia-1-azabicyclo[3.2.0]heptane-3-carboxylic acid |
| 110 | 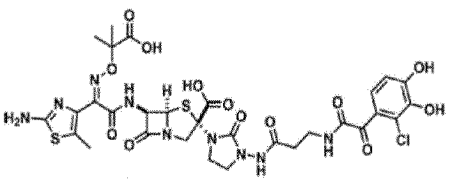 | (3R,5R,6R)-6-((Z)-2-(2-amino-5-methylthiazol-4-yl)-2-(((2-carboxypropan-2-yl)oxy)imino)acetamido)-3-(3-(3-(2-(2-chloro-3,4-dihydroxyphenyl)-2-oxoacetamido)propanamido)-2-oxoimidazolidin-1-yl)-7-oxo-4-thia-1-azabicyclo[3.2.0]heptane-3-carboxylic acid |
| 111 | 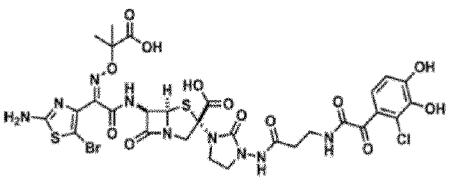 | (3R,5R,6R)-6-((Z)-2-(2-amino-5-bromothiazol-4-yl)-2-(((2-carboxypropan-2-yl)oxy)imino)acetamido)-3-(3-(3-(2-(2-chloro-3,4-dihydroxyphenyl)-2-oxoacetamido)proanamido)-2-oxoimidazolidin-1-yl)-7-oxo-4-thia-1-azabicyclo[3.2.0]heptane-3-carboxylic acid |

(continued)

| Ex. No. | Structural formula | Name |
|---|---|---|
| 112 | 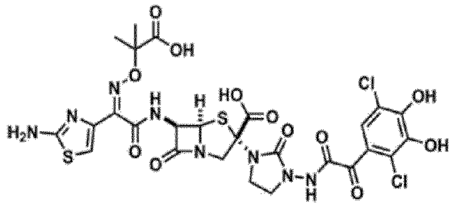 | (3R,5R,6R)-6-((Z)-2-(2-aminothiazol-4-yl)-2-(((2-carboxypropan-2-yl)oxy)imino)acetamido)-3-(3-(2-(2,5-dichloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 113 | 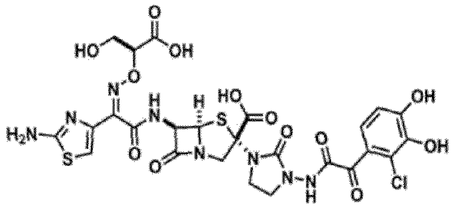 | (3R,5R,6R)-6-((Z)-2-(2-aminothiazol-4-yl)-2-(((S)-1-carboxy-2-hydroxyethoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 114 | 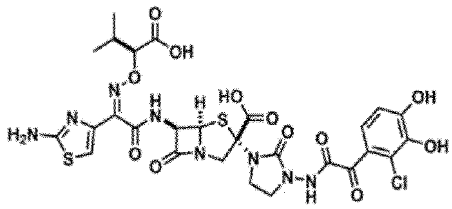 | (3R,5R,6R)-6-((Z)-2-(2-aminothiazol-4-yl)-2-(((S)-1-carboxy-2-methylpropoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 115 | 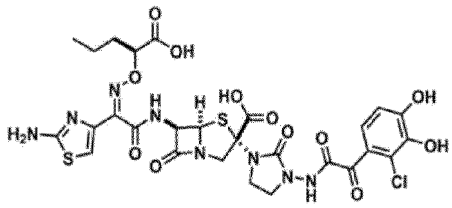 | (3R,5R,6R)-6-((Z)-2-(2-aminothiazol-4-yl)-2-(((S)-1-carboxybutoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 1 1 6 | 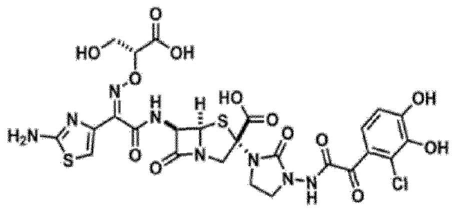 | (3R,5R,6R)-6-((Z)-2-(2-aminothiazol-4-yl)-2-(((R)-1-carboxy-2-hydroxyethoxy)imino)acetamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 117 | 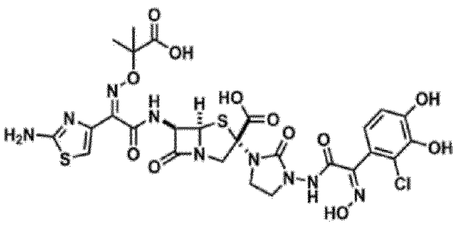 | (3R,5R,6R)-6-((Z)-2-(2-aminothiazol-4-yl)-2-(((2-carboxypropan-2-yl)oxy)imino)acetamido)-3-(3-((Z)-2-(2-chloro-3,4-dihydroxyphenyl)-2-hydroxyimino)acetamido)-2-oxoimidazolidin-1-yl)-7-oxo-4-thia-1-azabicyclo [3.2.0]heptane-3-carboxylic acid |
| 118 | 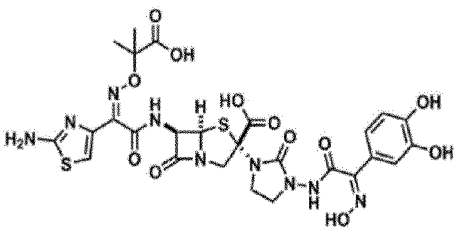 | (3R,5R,6R)-6-((Z)-2-(2-aminothiazol-4-yl)-2-(((2-carboxypropan-2-yl)oxy)imino)acetamido)-3-(3-((Z)-2-(3,4-dihydroxyphenyl)-2-hydroxyimino) acetamido)-2-oxoimidazolidin-1-yl)-7-oxo-4-thia-1-azabicyclo[3.2.0]heptane-3-carboxylic acid |

(continued)

| Ex. No. | Structural formula | Name |
|---|---|---|
| 119 | 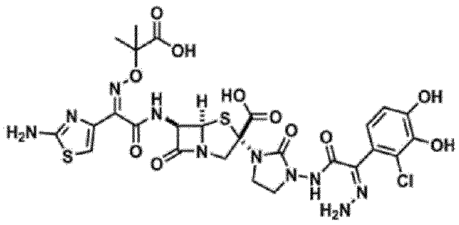 | (3R,5R,6R)-6-((Z)-2-(2-aminothiazol-4-yl)-2-(((2-carboxypropan-2-yl)oxy)imino)acetamido)-3-(3-((Z)-2-(2-chloro-3,4-dihydroxyphenyl)-2-hydrazinylideneacetamido)-2-oxoimidazolidin-1-yl)-7-oxo-4-thia-1-azabicyclo [3.2.0]heptane-3-carboxylic acid |
| 120 | 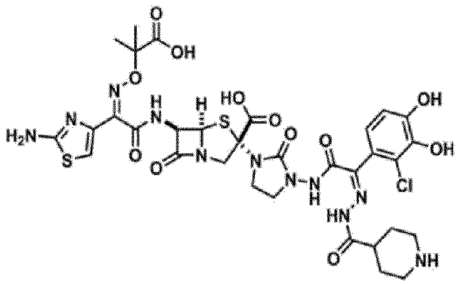 | (3R,5R,6R)-6-((Z)-2-(2-aminothiazol-4-yl)-2-(((2-carboxypropan-2-yl)oxy)imino)acetamido)-3-(3-((Z)-2-(2-chloro-3,4-dihydroxyphenyl)-2-(2-piperidine-4-carbonyl)hydrazinylidene)acetamido)-2-oxoimidazolidin-1-yl)-7-oxo-4-thia-1-azabicyclo[3.2.0]heptane-3-carboxylic acid |
| 121 | 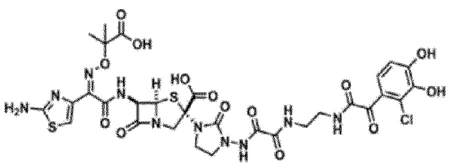 | (3R,5R,6R)-6-((Z)-2-(2-aminothiazol-4-yl)-2-(((2-carboxypropan-2-yl)oxy)imino)acetamido)-3-(3-(2-((2-(2-(2-chloro-3,4-dihydroxyphenyl)-2-oxoacetamido)ethyl)amino)-2-oxoacetamido)-2-oxoimidazolidin-1-yl)-7-oxo-4-thia-1-azabicyclo[3.2.0]heptane-3-carboxylic acid |
| 122 | 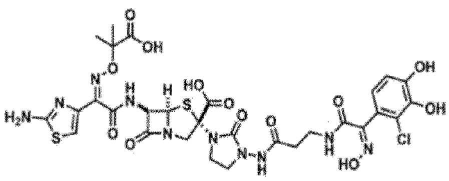 | (3R,5R,6R)-6-((Z)-2-(2-aminothiazol-4-yl)-2-(((2-carboxypropan-2-yl)oxy)imino)acetamido)-3-(3-(3-((Z)-2-(2-chloro-3,4-dihydroxyphenyl)-2-(hydroxyamino)acetamido)propanamido)-2-oxoimidazolidin-1-yl)-7-oxo-4-thia-1-azabicyclo[3.2.0]heptane-3-carboxylic acid |
| 123 | 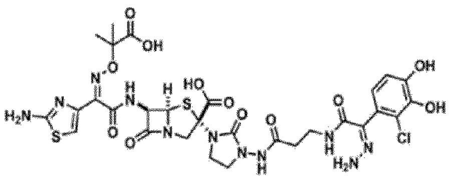 | (3R,5R,6R)-6-((Z)-2-(2-aminothiazol-4-yl)-2-(((2-carboxypropan-2-yl)oxy)imino)acetamido)-3-(3-(3-((Z)-2-(2-chloro-3,4-dihydroxyphenyl)-2-hydrazinylideneacetamido)propanamido)-2-oxoimidazolidin-1-yl)-7-oxo-4-thia-1-azabicyclo[3.2.0]heptane-3-carboxylic acid |
| 124 | 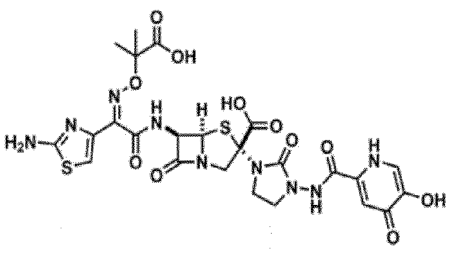 | (3R,5R,6R)-6-((Z)-2-(2-aminothiazol-4-yl)-2-(((2-carboxypropan-2-yl)oxy)imino)acetamido)-3-(3-(5-hydroxy-4-oxo-1,4-dihydropyridine-2-carboxamido)-2-oxoimidazolidin-1-yl)-7-oxo-4-thia-1-azabicyclo[3.2.0]heptane-3-carboxylic acid |
| 125 | 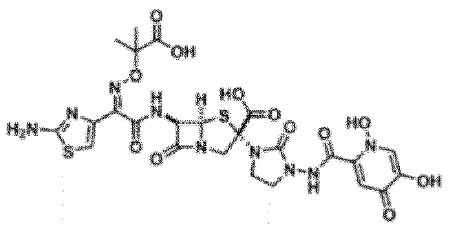 | (3R,5R,6R)-6-((Z)-2-(2-aminothiazol-4-yl)-2-(((2-carboxypropan-2-yl)oxy)imino)acetamido)-3-(3-(1,5-dihydroxy-4-oxo-1,4-dihydropyridine-2-carboxamido)-2-oxoimidazolidin-1-yl)-7-oxo-4-thia-1-azabicyclo[3.2.0]heptane-3-carboxylic acid |

(continued)

| Ex. No. | Structural formula | Name |
|---|---|---|
| 126 | 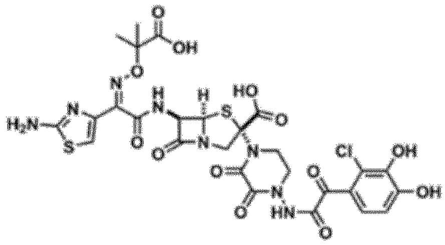 | (3R,5R,6R)-6-((Z)-2-(2-aminothiazol-4-yl)-2-(((2-carboxypropan-2-yl)oxy)imino)acetamido)-3-(4-(2-(2-choro-3,4-dihydroxyphenyl)-2-oxoacetamido)-2,3-dioXopiperazin-1-yl)-7-oxo-4-thia-1-azabicyclo[3.2.0]heptane-3-carboxylic acid |
| 127 | 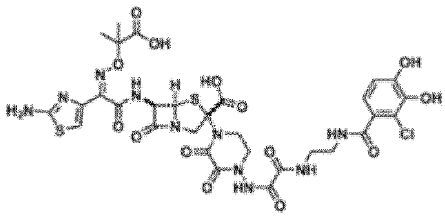 | (3R,5R,6R)-6-((Z)-2-(2-aminothiazol-4-yl)-2-(((2-carboxypropan-2-yl)oxy)imino)acetamido)-3-(4-(2-((2-(2-chloro-3,4-dihydroxybenzamido)ethyl)amino)-2-oxoacetamido)-2,3-dioxopiperazin-1-yl)-7-oxo-4-thia-1-azabicyclo[3.2.0]heptane-3-carboxylic acid |
| 128 | 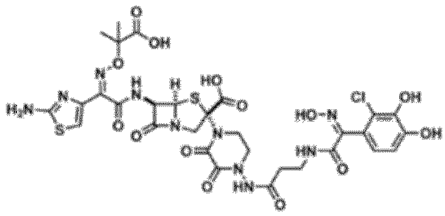 | (3R,5R,6R)-6-((Z)-2-(2-aminothiazol-4-yl)-2-(((2-carboxypropan-2-yl)oxy)imino)acetamidon)-3-(4-(3-((Z)-2-(2-chloro-3,4-dihydroxyphenyl)-2-(hydroxyimino)acetamido)propana mido)-2,3-dioxopiperazin-1-yl)-7-oxo-4-thia-1-azabicyclo[3.2.0]heptane-3-carboxylic acid |
| 129 | 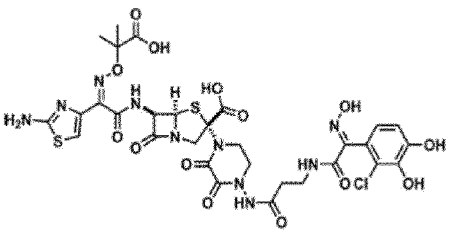 | (3R,5R,6R)-6-((Z)-2-(2-aminothiazol-4-yl)-2-(((2-carboxypropan-2-yl)oxy)imino)acetamido)-3-(4-(3-((E)-2-(2-chloro-3,4-dihydroxyphenyl)-2-(hydroxyimino)acetamido)propanamido)-2,3-dioxopiperazin-1-yl)-7-oxo-4-thia-1-azabicyclo[3.2.0]heptane-3-carboxylic acid |
| 130 | 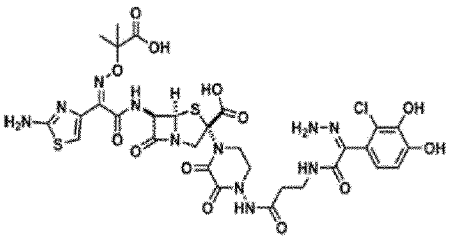 | (3R,5R,6R)-6-((Z)-2-(2-aminothiazol-4-yl)-2-(((2-carboxypropan-2-yl)oxy)imino)acetamido)-3-(4-(3-((Z)-2-(2-chloro-3,4-dihydroxyphenyl)-2-hydrazinylideneacetamido)propanamido)-2,3-dioxopiperazin-1-yl)-7-oxo-4-thia-1-azabicyclo[3.2.0]heptane-3-carboxylic acid |
| 131 | 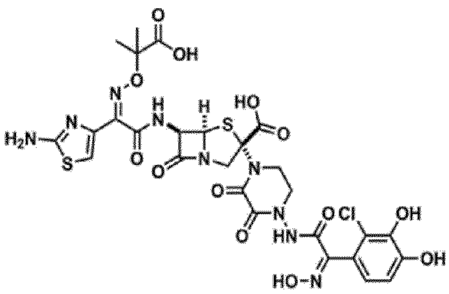 | (3R,5R,6R)-6-((Z)-2-(2-aminothiazol-4-yl)-2-(((2-carboxypropan-2-yl)oxy)imino)acetamido)-3-(4-(3-((Z)-2-(2-chloro-3,4-dihydroxyphenyl)-2-(hydroxyamino)acetamido)-2,3-dioxopiperazin-1-yl)-7-oxo-4-thia-1-azabicyclo[3.2.0]heptane-3-carboxylic acid |

(continued)

| Ex. No. | Structural formula | Name |
|---|---|---|
| 13 2 | 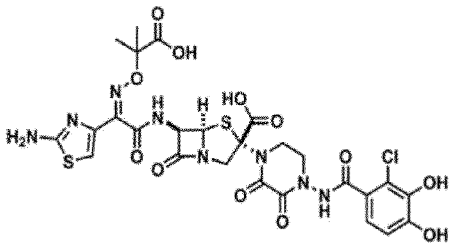 | (3R,5R,6R)-6-((Z)-2-(2-aminothiazol-4-yl)-2-(((2-carboxypropan-2-yl)oxy)imino)acetamido)-3-(4-(2-chloro-3,4-dihydroxybenzamido)-2,3-dioxopiperazin-1-yl)-7-oxo-4-thia-1-azabicyclo[3.2.0]heptane-3-carboxylic acid |
| 133 | 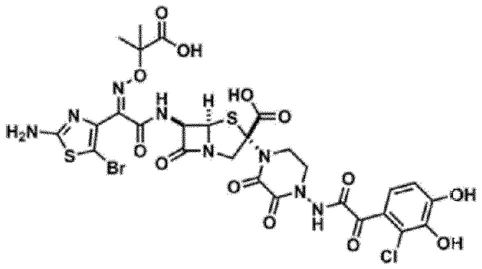 | (3R,5R,6R)-6-((Z)-2-(2-amino-5-bromothiazol-4-yl)-2-(((2-carboxypropan-2-yl)oxy)imino)acetamido)-3-(4-(2-(2-chloro-3,4-dihydroxyphenyl)-2-oxoacetamido)-2,3-dioxopiperazin-1-yl)-7-oxo-4-thia-1-azabicyclo[3.2.0]heptane-3-carboxylic acid |
| 134 | 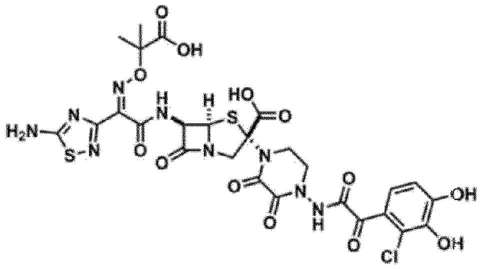 | (3R,5R,6R)-6-((Z)-2-(5-amino-1,2,4-thiadiazol-3-yl)-2-(((2-carboxypropan-2-yl)oxy)imino)acetamido)-3-(4-(2-(2-chloro-3,4-dihydroxyphenyl)-2-oxoacetamido) 2,3-dioxopiperazin-1-yl)-7-oxo-4-thia-1-azabicyclo[3.2.0]heptane-3-carboxylic acid |
| 135 | 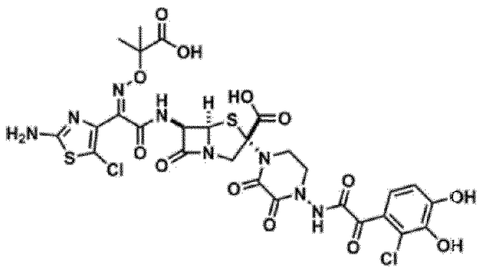 | (3R,5R,6R)-6-((Z)-2-(2-amino-5-chlorothiazol-4-yl)-2-(((2-carboxypropan-1-yl)oxy)imino)acetamido)-3-(4-(2-(2-chloro-3,4-dihydroxyphenyl)-2-oxoacetamido)-2,3-dioxopiperazin-1-yl)-7-oxo-4-thia-1-azabicyclo[3.2.0]heptane-3-carboxylic acid |
| 136 | 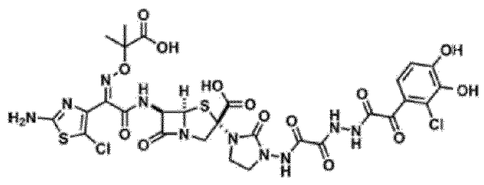 | (3R,5R,6R)-6-((Z)-2-(2-amino-5-chlorothiazol-4-yl)-2-(((2-carboxypropan-2-yl)oxy)imino)acetamido)-3-(3-(2-(2-(2-(2-chloro-3,4-dihydroxyphenyl)-2-oxoacetyl)hydrizinyl)-2-oxoacetamido)-2-oxoimidazolidin-1-yl)-7-oxo-4-thia-1-azabicyclo[3.2.0]heptane-3-carboxylic acid |

The measured values of NMR and MS of the compounds in the table are as follows.

Example 106

NMR: 1.23-1.46 (4H, m), 2.65 (2H, t, J = 6.2 Hz), 3.52-3.62 (3H, m), 3.62-3.74 (4H, m), 4.59 (1H, d, J = 12.4 Hz), 5.39 (1H, d, J = 3.6 Hz), 5.77 (1H, d, J = 4.0 Hz), 6.95 (1H, d, J = 8.4 Hz), 7.34 (1H, d, J = 8.8 Hz)
MS: 810.95 $[M + H]^+$, 809.10 $[M - H]^-$

Example 107

NMR: 1.22-1.45 (4H, m), 2.66 (2H, t, J = 6.4 Hz), 3.53-3.72 (7H, m), 4.58 (1H, d, J = 12.8 Hz), 5.60 (1H, d, J = 4.0 Hz), 5.75 (1H, d, J = 4.0 Hz), 6.95 (1H, d, J = 8.8 Hz), 7.34 (1H, d, J = 8.8 Hz)
MS: 843.95 $[M + H]^+$, 842.05 $[M - H]^-$

Example 108

NMR: 1.81-1.95 (1H, m), 1.95-2.08 (1H, m), 2.27-2.39 (2H, m), 2.39-2.49 (1H, m), 2.50-2.60 (1H, m), 2.66 (2H, t, J = 6.4 Hz), 3.54-3.72 (7H, m), 4.61 (1H, d, J = 12.8 Hz), 5.64 (1H, d, J = 4.0 Hz), 5.79 (1H, d, J = 3.6 Hz), 6.95 (1H, d, J = 8.8 Hz), 7.34 (1H, d, J = 8.4 Hz)
MS: 858.00 $[M + H]^+$, 855.95 $[M - H]^-$

Example 109

NMR: 1.83-1.96 (1H, m), 1.96-2.10 (1H, m), 2.27-2.41 (2H, m), 2.43-2.53 (1H, m), 2.53-2.62 (1H, m), 2.65 (2H, t, J = 6.2 Hz), 3.53-3.73 (7H, m), 4.61 (1H, d, J = 12.4 Hz), 5.66 (1H, d, J = 3.6 Hz), 5.81 (1H, d, J = 3.6 Hz), 6.95 (1H, d, J = 8.4 Hz), 7.34 (1H, d, J = 8.4 Hz)
MS: 825.20 $[M + H]^+$, 822.95 $[M - H]^-$

Example 110

NMR: 1.49 (3H, s), 1.50 (3H, s), 2.34 (3H, s), 2.66 (2H, t, J = 6.2 Hz), 3.52-3.62 (3H, m), 3.64-3.71 (4H, m), 4.59 (1H, d, J = 12.4 Hz), 5.63 (1H, d, J = 4.0 Hz), 5.75 (1H, d, J = 3.2 Hz), 6.96 (1H, d, J = 8.4 Hz), 7.34 (1H, d, J = 8.4 Hz)
MS: 826.00 $[M + H]^+$, 824.00 $[M - H]^-$

Example 111

NMR: 1.50 (3H, s), 1.52 (3H, s), 2.66 (2H, t, J = 6.2 Hz), 3.54-3.63 (3H, m), 3.63-3.71 (4H, m), 4.59 (1H, d, J = 12.4 Hz), 5.62 (1H, d, J = 4.0 Hz), 5.76 (1H, d, J = 2.8 Hz), 6.96 (1H, d, J = 8.4 Hz), 7.34 (1H, d, J = 8.8 Hz)
MS: 889.90 $[M + H]^+$, 887.75 $[M - H]^-$

Example 112

NMR: 1.49 (3H, s), 1.51 (3H, s), 3.54-3.68 (2H, m), 3.68-3.80 (3H, m), 4.68 (1H, d, J = 13.2 Hz), 5.71 (1H, d, J = 4.0 Hz), 5.75 (1H, d, J = 3.6 Hz), 7.05 (1H, s), 7.40 (1H, s)
MS: 774.95 $[M + H]^+$, 773.00 $[M - H]^-$

Example 113

NMR: 3.61 (1H, d, J = 12.8 Hz), 3.68-3.80 (4H, m), 3.93-4.02 (2H, m), 4.64-4.73 (2H, m), 5.71 (1H, d, J = 4.0 Hz), 5.80 (1H, d, J = 2.8 Hz), 6.96 (1H, d, J = 8.8 Hz), 7.10 (1H, s), 7.44 (1H, d, J = 8.4 Hz)
MS: 743.00 $[M + H]^+$, 741.00 $[M - H]^-$

Example 114

NMR: 0.98 (3H, d, J = 6.8 Hz), 1.01 (3H, d, J = 7.2 Hz), 2.15-2.22 (1H, m), 3.64 (1H, d, J = 12.8 Hz), 3.68-3.87 (4H, m), 4.30-4.36 (1H, m), 4.67 (1H, dd, J = 12.6, 4.6 Hz), 5.71 (1H, d, J = 3.6 Hz), 5.77-5.82 (1H, m), 6.84 (1H, d, J = 8.4 Hz), 7.06 (1H, s), 7.44 (1H, d, J = 8.8 Hz)
MS: 755.05 $[M + H]^+$, 752.95 $[M - H]^-$

Example 115

NMR: 0.94 (3H, t, J = 7.4 Hz), 1.35-1.49 (2H, m), 1.75-1.86 (2H, m), 3.66 (1H, d, J = 12.4 Hz), 3.70-3.87 (4H, m), 4.55-4.61 (1H, m), 4.66 (1H, d, J = 12.8 Hz), 5.70 (1H, d, J = 3.6 Hz), 5.80 (1H, d, J = 3.6 Hz), 6.81[6.88] (1H, d, J = 8.8 Hz), 7.06 [7.06] (1H, s),7.44 [7.45] (1H, d, J = 8.6 Hz)
MS: 755.05 $[M + H]^+$, 752.95 $[M - H]^-$

Example 116

NMR: 3.61 (1H, d, J = 12.8 Hz), 3.68-3.80 (4H, m), 3.91-4.02 (2H, m), 4.64-4.73 (2H, m), 5.71 (1H, d, J = 4.0 Hz), 5.80 (1H, d, J = 3.6 Hz), 6.92 (1H, d, J = 8.4 Hz), 7.08 (1H, s), 7.44 (1H, d, J = 8.4 Hz)
MS: 743.00 $[M + H]^+$, 740.90 $[M - H]^-$

Example 117

NMR: 1.48 (3H, s), 1.50 (3H, s), 3.60 (1H, d, J = 12.8 Hz), 3.61-3.78 (4H, m), 4.65[4.66] (1H, d, J = 12.6 Hz), 5.69 [5.69] (1H, d, J = 3.6 Hz), 5.75 (1H, d, J = 3.6 Hz), 6.87 (1H, d, J = 8.0 Hz), 6.98 (1H, d, J = 8.4 Hz), 7.03[7.03] (1H, s)
MS: 756.05 $[M + H]^+$, 753.95 $[M - H]^-$

Example 118

NMR: 1.49 (3H, s), 1.51 (3H, s), 3.61 (1H, d, J = 12.8 Hz), 3.63-3.81 (4H, m), 4.66 (1H, d, J = 12.8 Hz), 5.70 (1H, d, J = 3.6 Hz), 5.75 (1H, d, J = 4.0 Hz), 6.94-7.07 (3H, m), 7.12 [7.12] (1H, s)
MS: 722.10 $[M + H]^+$, 720.00 $[M - H]^-$

Example 119

NMR: 1.48 (3H, s), 1.50 (3H, s), 3.54-3.77 (5H, m), 4.64 (1H, d, J = 12.8 Hz), 5.68 (1H, d, J = 3.6 Hz), 5.74 (1H, dd, J = 3.6, 0.8 Hz), 6.79 (1H, d, J = 8.0 Hz), 7.01 (1H, d, J = 8.4 Hz), 7.02 (1H, s)
MS: 755.10 $[M + H]^+$, 752.95 $[M - H]^-$

Example 120

NMR: 1.48 (3H, s), 1.50 (3H, s), 1.70-2.18 (4H, m), 2.90-3.18 (1H, m), 3.18-3.41 (2H, m), 3.41-3.62 (2H, m), 3.62-3.81 (4H, m), 3.81-3.92 (1H, m), 4.64 (1H, d, J = 12.8 Hz), 5.67-5.78 (2H, m), 6.76-6.90 (1H, m), 6.92-7.09 (2H, m)
MS: 866.15 $[M + H]^+$, 864.10 $[M - H]^-$

Example 121

NMR: 1.49 (3H, s), 1.50 (3H, s), 3.51-3.68 (8H, m), 3.70-3.77 (1H, m), 4.63 (1H, d, J = 12.8 Hz), 5.69 (1H, d, J = 3.6 Hz), 5.74 (1H, d, J = 3.2 Hz), 6.95 (1H, d, J = 8.8 Hz), 7.05 (1H, s), 7.28 (1H, d, J = 8.4 Hz)
MS: 855.00 $[M + H]^+$, 853.25 $[M - H]^-$

Example 122

NMR: 1.48 (3H, s), 1.50 (3H, s), 2.58 (2H, t, J = 6.2 Hz), 3.62-3.73 (7H, m), 4.63 (1H, d, J = 12.8 Hz), 5.68 (1H, d, J = 3.6 Hz), 5.75 (1H, dd, J = 3.6, 1.2 Hz), 6.81 (1H, d, J = 8.4 Hz), 6.97 (1H, d, J = 8.4 Hz), 7.03 (1H, s)
MS: 827.10 $[M + H]^+$, 824.95 $[M - H]^-$

Example 123

NMR: 1.49 (3H, s), 1.50 (3H, s), 2.56 (2H, t, J = 6.4 Hz), 3.50-3.73 (7H, m), 4.63 (1H, d, J = 12.8 Hz), 5.68 (1H, d, J = 3.6 Hz), 5.75 (1H, dd, J = 3.8, 1.0Hz), 6.74 (1H, d, J = 8.4 Hz), 7.00 (1H, d, J = 8.4 Hz), 7.03 (1H, s)
MS: 826.10 $[M + H]^+$, 824.00 $[M - H]^-$

Example 124

NMR: 1.49 (3H, s), 1.51 (3H, s), 3.62 (1H, dd, J = 12.8, 1.2 Hz), 3.66-3.82 (4H, m), 4.67 (1H, d, J = 12.4 Hz), 5.71 (1H, d, J = 4.0 Hz), 5.76 (1H, dd, J = 3.6, 0.8 Hz), 7.03 (1H, s), 7.16 (1H, s), 7.82 (1H, s)
MS: 680.10 $[M + H]^+$, 678.00 $[M - H]^-$

Example 125

NMR: 1.49 (3H, s), 1.51 (3H, s), 3.65 (1H, dd, J = 12.8, 1.2 Hz), 3.68-3.79 (4H, m), 4.66 (1H, d, J = 12.8 Hz), 5.71 (1H, d, J = 3.6 Hz), 5.76 (1H, dd, J = 3.6, 1.2 Hz), 7.03 (1H, s), 7.42 (1H, s), 7.59 (1H, s)

MS: 696.10 [M + H]+, 694.00 [M - H]-

Example 126

NMR: 1.49 (3H, s), 1.50 (3H, s), 3.28 (1H, d, J = 13.2 Hz), 4.01-4.13 (2H, m), 4.18-4.36 (2H, m), 5.09 (1H, d, J = 13.2 Hz), 5.71 (1H, d, J = 3.6 Hz), 5.74 (1H, d, J = 3.6 Hz), 6.81 (1H, d, J = 8.8 Hz), 7.03 (1H, s), 7.47 (1H, d, J = 8.4 Hz)
MS: 769.05 [M + H]+, 766.90 [M - H]-

Example 127

NMR: 1.49 (3H, s), 1.50 (3H, s), 3.18 [3.26] (1H, dd, J = 12.8, 1.2 Hz), 3.53-3.67 (4H, m), 3.76-4.33 (4H, m), 5.05 [5.08] (1H, d, J = 13.0Hz), 5.67[5.69] (1H, d, J = 3.6 Hz), 5.74 (1H, dd, J = 3.6, 1.2 Hz), 6.77-6.87 (2H, m), 7.02 [7.03] (1H, s)
MS: 855.20 [M + H]+, 852.95 [M - H]-

Example 128

NMR: 1.49 (3H, s), 1.50 (3H, s), 2.64 (2H, t, J = 6.2 Hz), 3.25 (1H, dd, J = 13.0, 1.0 Hz), 3.66 (2H, t, J = 6.4 Hz), 3.82-3.95 (2H, m), 4.09-4.27 (2H, m), 5.07 (1H, d, J = 12.8 Hz), 5.69 (1H, d, J = 3.6 Hz), 5.74 (1H, dd, J = 3.2, 0.8 Hz), 6.80 (1H, d, J = 8.0 Hz), 6.97 (1H, d, J = 8.0 Hz), 7.02 (1H, s)
MS: 855.10 [M + H]+, 852.90 [M - H]-

Example 129

NMR: 1.49 (3H, s), 1.50 (3H, s), 2.61-2.73 (2H, m), 3.25 (1H, d, J = 12.8 Hz), 3.62-3.92 (4H, m), 4.07-4.27 (2H, m), 5.07 (1H, d, J = 12.8 Hz), 5.70 (1H, d, J = 3.2 Hz), 5.74 (1H, d, J = 3.2 Hz), 6.93 (1H, d, J = 8.4 Hz), 6.97 (1H, d, J = 8.0 Hz), 7.02 (1H, s)
MS: 855.10 [M + H]+, 852.90 [M - H]-

Example 130

NMR: 1.49 (3H, s), 1.50 (3H, s), 2.62 (2H, t, J = 6.4 Hz), 3.25 (1H, dd, J = 12.8,1.2 Hz), 3.58-3.94 (2H, m), 3.83-3.94 (2H, m), 4.10-4.28 (2H, m), 5.07 (1H, d, J = 13.2 Hz), 5.70 (1H, d, J = 3.6 Hz), 5.73 (1H, dd, J = 3.6, 1.2 Hz), 6.74 (1H, d, J = 8.4 Hz), 7.01 (1H, d, J = 8.4 Hz), 7.02 (1H, s)
MS: 854.20 [M + H]+, 852.00 [M - H]-

Example 131

NMR: 1.48 (3H, s), 1.50 (3H, s), 3.26 (1H, dd, J = 12.8, 0.8 Hz), 3.97-4.08 (2H, m), 4.16-4.33 (2H, m), 5.08 (1H, d, J = 13.2 Hz), 5.69 (1H, d, J = 3.6 Hz), 5.74 (1H, dd, J = 3.6, 1.2 Hz),6.87 (1H, d, J = 8.4 Hz), 6.98 (1H, d, J = 8.4 Hz), 7.02 (1H, s)
MS: 784.05 [M + H]+, 781.85 [M - H]-

Example 132

NMR: 1.49 (3H, s),1.51 (3H, s), 3.29 (1H, dd, J = 13.0, 1.0 Hz), 4.05-4.15 (2H, m), 4.20-4.36 (2H, m), 5.10 (1H, d, J = 12.8 Hz), 5.71 (1H, d, J = 3.6 Hz), 5.75 (1H, dd, J = 3.6, 1.2 Hz), 6.95 (1H, d, J = 8.8 Hz), 7.03 (1H, s), 7.17 (1H, d, J = 8.4 Hz)
MS: 741.05 [M + H]+, 739.05 [M - H]-

Example 133

NMR: 1.52 (3H, s), 1.53 (3H, s), 3.28 (1H, d, J = 12.4 Hz), 4.01-4.13 (2H, m), 4.17-4.37 (2H, m), 5.09 (1H, d, J = 13.2 Hz), 5.67 (1H, d, J = 3.2 Hz), 5.76-5.82 (1H, m), 6.90-7.07 (1H, m), 7.42-7.51 (1H, m)
MS: 846.90 [M + H]+, 844.90 [M - H]-

Example 134

NMR: 1.53 (3H, s), 1.54 (3H, s), 3.28 (1H, d, J = 12.8 Hz), 4.02-4.15 (2H, m), 4.16-4.37 (2H, m), 5.08 (1H, d, J = 12.8 Hz), 5.68 (1H, d, J = 2.8 Hz), 5.80 (1H, d, J = 2.8 Hz), 6.98 (1H, d, J = 8.4 Hz), 7.46 (1H, d, J = 7.6 Hz)
MS: 770.05 [M + H]+, 767.95 [M - H]-

Example 135

NMR: 1.50 (3H, s), 1.52 (3H, s), 3.29 (1H, d, J = 13.2 Hz), 4.01-4.15 (2H, m), 4.15-4.37 (2H, m), 5.09 (1H, d, J = 12.8 Hz), 5.67 (1H, d, J = 3.6 Hz), 5.78 (1H, d, J = 2.0Hz), 6.98 (1H, d, J = 8.4 Hz), 7.46 (1H, d, J = 8.4 Hz)
MS: 802.95 $[M + H]^+$, 800.90 $[M - H]^-$

Example 136

NMR: 1.51 (3H, s), 1.52 (3H, s), 3.57-3.80 (5H, m), 4.65 (1H, d, J = 12.8 Hz), 5.68 (1H, d, J = 4.0 Hz), 5.79 (1H, d, J = 3.6 Hz), 6.96 (1H, d, J = 8.4 Hz), 7.43 (1H, d, J = 8.4 Hz)
MS: 861.00 $[M + H]^+$, 858.95 $[M - H]^-$

Example 137

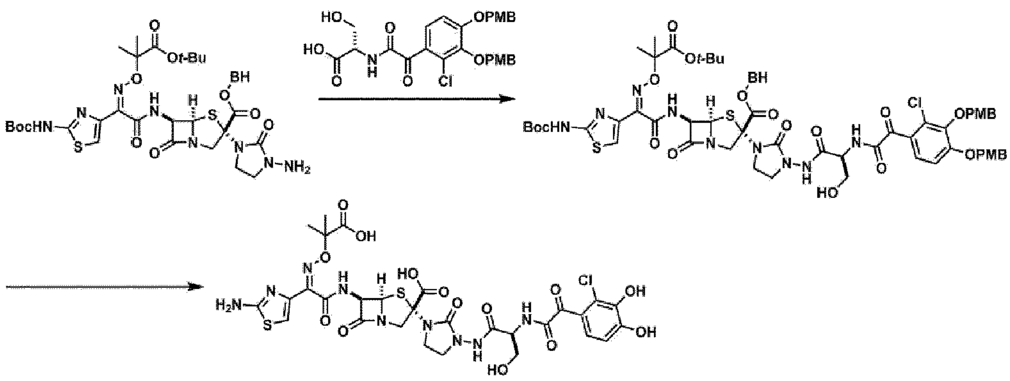

Example 137 (1)

(2-(2-Chloro-3,4-bis((4-methoxybenzyl)oxy)phenyl)-2-oxoacetyl)-L-serine (166 mg), 4-(4,6-dimethoxy-1,3,5-triazin-2-yl)-4-methylmorpholinium chloride (137 mg), and THF (3.6 mL) were sequentially added to benzhydryl (3R,5R,6R)-3-(3-amino-2-oxoimidazolidin-1-yl)-6-((Z)-2-(((1-(tert-butoxy)-2-methyl-1-oxopr opan-2-yl)oxy)imino)-2-(2-((tert-butoxycarbonyl)amino)thiazol-4-yl)acetamido)-7-oxo-4-thia-1-azabicyclo[3.2.0]heptane-3-carboxylate (120 mg). The reaction mixture was stirred at room temperature overnight. Ethyl acetate (6 mL) and water (6 mL) were added to the reaction mixture, and the organic layer was separated. The aqueous layer was extracted twice by using ethyl acetate (10 mL). The organic layer was washed a saturated aqueous sodium chloride solution (20 mL) and dehydrated and dried over anhydrous sodium sulfate. The solvent was distilled off under reduced pressure. The residue was purified by silica gel column chromatography [eluent; ethyl acetate:hexane = 50:50 → 100:0], thereby obtaining a target substance (200 mg) as brown solids.

Example 137 (2)

Dichloromethane (4 mL) was added to the compound (200 mg) obtained in Example 137 (1), and the mixture was stirred at -20°C. At the same temperature, anisole (940 μL) and aluminum chloride (288 mg) were sequentially added to the reaction mixture. The reaction mixture was stirred at a temperature equal to or lower than -20°C for 30 minutes. The reaction mixture was added to a mixture of acetonitrile (15 mL), water (15 mL), and trisodium citrate dihydrate (952 mg) under ice cooling. A saturated aqueous sodium hydrogen carbonate solution was added to the reaction mixture such that the pH was adjusted to 5.2, and the aqueous layer was separated. The aqueous layer was concentrated under reduced pressure, and the residue was purified by medium-pressure reverse-phase silica gel column chromatography [eluent; water:acetonitrile = 100:0 → 85:15]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(3-((S)-2-(2-(2-chloro-3,4-dihydroxyphenyl)-2-oxoacetamido)-3-hydroxypropanamido)-2-o xoimidazolidin-1-yl)-7-oxo-4-thia-1-azabicyclo[3.2.0]heptane-3-carboxylic acid (35 mg) as yellow solids.
NMR: 1.49 (3H, s), 1.50 (3H, s), 3.54-3.68 (3H, m), 3.68-3.76 (2H, m), 3.99 (2H, d, J = 5.6 Hz), 4.66 (1H, d, J = 13.2 Hz), 5.69 (1H, d, J = 3.6 Hz), 5.75 (1H, d, J = 4.0 Hz), 6.95 (1H, d, J = 8.4 Hz), 7.04 (1H, s), 7.40 (1H, d, J = 8.8 Hz)
MS: 828.00 $[M + H]^+$, 826.15 $[M - H]^-$

The compounds shown in Table 34 were obtained in the same manner as in Example 137.

[Table 34]

| Ex.No. | Structural Formula | Name |
|---|---|---|
| 138 | 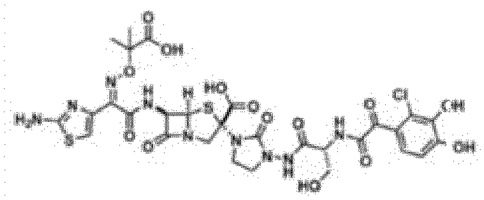 | (3R,5R,6R)-6-((Z)-2-(2-aminothiazol-4-yl)-2-(((2-carboxypropan-2-yl)oxy)imino)acetamido)-3-(3-((R-2-(2-(2-chloro-3,4-dihydroxyphenyl)-2-oxoacetamido)-3-hydroxypropanamido)-2-oxoimidazolidin-1-yl)-7-oxo thia-1-azabicyclo[3.2.0]heptane-3-carboxylic acid |
| 139 | 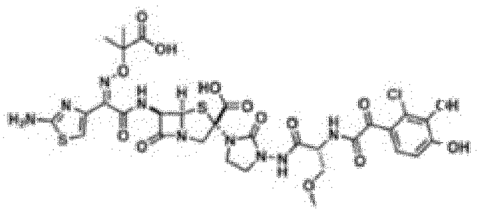 | (3R,5R,6R)-6-((Z)-2-(2-aminothiazol-4-yl)-2-(((2-carboxypropan-2-yl)oxy)imino)acetamido)-3-(3-((R)-2-(2-(2-chloro-3,4-dihydroxyphenyl)-2-oxoacetamido)-3-methoxypropanamido)-2-oxoimidazolidin-1-yl)-7-oxo-4-thia-1-azabicyclo[3.2.0]heptane-3-carboxylic acid |
| 140 | 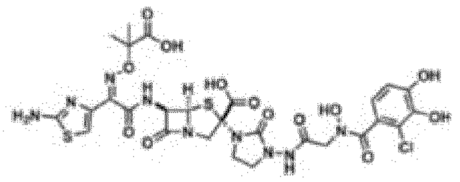 | (3R,5R,6R)-6-((Z)-2-(2-ammothiazol-4-yl)-2-(((2-carboxypropan-2-yl)oxy)imino)acetamido)-3-(3-(2-(2-chloro-N,3,4-trihydroxybenzamido)acetamido )-2-oxoimidazolidin-1-yl)-7-oxo-4-thia-1-azabicyclo[3.2.0] heptane-3-carboxylic acid |

The measured values of NMR and MS of the compounds in the table are as follows.

Example 138

NMR: 1.48 (3H, s), 1.50 (3H, s), 3.53-3.67 (3H, m), 3.67-3.75 (2H, m), 3.99 (2H, d, J = 6.0 Hz), 4.67 (1H, d, J = 5.6 Hz), 5.69 (1H, d, J = 4.0 Hz), 5.74 (1H, d, J = 3.2 Hz), 6.94 (1H, d, J = 8.8 Hz), 7.03 (1H, s), 7.39 (1H, d, J = 8.8 Hz)
MS: 828.00 $[M + H]^+$, 826.15 $[M - H]^-$

Example 139

NMR: 1.46 (3H, s), 1.47 (3H, s), 3.40 (3H, s), 3.42-3.47 (1H, m), 3.51-3.64 (3H, m), 3.64-3.71 (2H, m), 3.84 (2H, d, J = 5.6 Hz), 4.62 (1H, d, J = 12.8 Hz), 5.65 (1H, d, J = 4.0 Hz), 5.71 (1H, d, J = 3.2 Hz), 6.94 (1H, d, J = 8.4 Hz), 7.01 (1H, s), 7.36 (1H, d, J = 8.4 Hz)
MS: 841.95 $[M + H]^+$, 840.05 [M - H]-

Example 140

NMR: 1.49 (3H, s), 1.51 (3H, s), 3.54-3.86 (7H, m), 4.66 (1H, dd, J = 12.6, 3.0 Hz), 5.69 (1H, d, J = 3.6 Hz), 5.75 (1H, d, J = 3.2 Hz), 6.93 (1H, d, J = 8.4 Hz), 7.03 (1H, s), 7.07 (1H, d, J = 8.4 Hz)
MS: 786.05 $[M + H]^+$, 783.95 $[M - H]^-$

Example 141

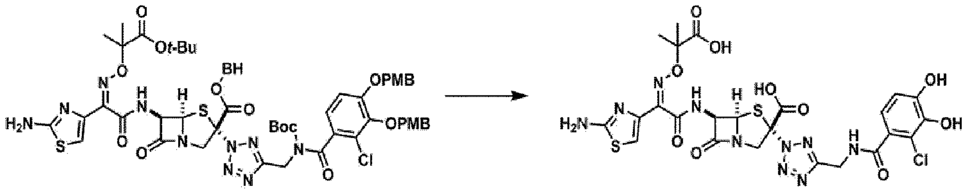

Dichloromethane (1.1 mL) was added to benzhydryl (3R,5R,6R)-6-((Z)-2-(2-aminothiazol-4-yl)-2-(((1-(tert-butoxy)-2-methyl-1-oxopropan-2-yl)o xy)imino)acetamido)-3-(5-((N-(tert-butoxycarbonyl)-2-chloro-3,4-bis((4-methoxybenzyl)oxy) benzamido)methyl)-2H-tetrazol-2-yl)-7-oxo-4-thia-1-azabicyclo[3.2.0]heptane-3-carboxylate (71 mg), and the mixture was stirred at -20°C. At the same temperature, anisole (0.36 mL) and aluminum chloride (110 mg) were sequentially added to the reaction mixture. The reaction mixture was stirred at a temperature equal to or lower than -20°C for 50 minutes. The reaction mixture was added to a mixture of acetonitrile (5 mL), water (5 mL), and trisodium citrate dihydrate (0.37 g) under ice cooling. A saturated aqueous sodium hydrogen carbonate solution was added to the reaction mixture such that the pH was adjusted to 5.1, and the aqueous layer was separated. The aqueous layer was concentrated under reduced pressure, and the residue was purified by medium-pressure reverse-phase silica gel column chromatography [eluent; water:acetonitrile = 100:0 → 75:25]. The aqueous solution containing a target substance was lyophilized, thereby obtaining (3R,5R,6R)-6-((Z)-2-(2-aminothiazol-4-yl)-2-(((2-carboxypropan-2-yl)oxy)imino)acetamido)-3-(5-((2-chloro-3,4-dihydroxybenzamido)methyl)-2H-tetrazol-2-yl)-7-oxo-4-thia-1-azabicyclo [3.2.0]heptane-3-carboxylic acid (7.6 mg) as white solids.

NMR: 1.45 (3H, s), 1.46 (3H, s), 4.16 (1H, d, J = 12.8 Hz), 4.67 (1H, dd, J = 16.4, 5.6 Hz), 4.78 (1H, dd, J = 16.4, 5.6 Hz), 4.90 (1H, d, J = 12.8 Hz), 5.70 (1H, d, J = 4.0 Hz), 5.77 (1H, dd, J = 8.0,4.0Hz), 6.78 (1H, d, J = 8.0 Hz), 6.84 (1H, s), 6.86 (1H, d, J = 8.0 Hz), 7.70 (1H, d, J = 1.2 Hz), 8.86 (1H, t, J = 5.6 Hz), 9.34 (1H, d, J = 8.0 Hz), 10.08-10.09 (1H, brs)
MS: 711.05 $[M + H]^+$, 708.90 $[M - H]^-$

The compounds shown in Table 35 were obtained in the same manner as in Example 19.

[Table 35]

| Ex. No. | Structural Formula | Name |
|---|---|---|
| 142 | 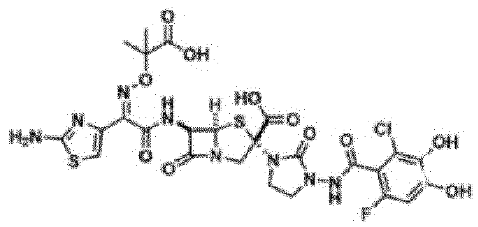 | (3R,5R,6R)-6-((Z)-2-(2-ammothiazol-4-)-2-(((2-carboxypropan-2-yl)oxy)imino)acetamido)-3-(3-(2-chloro-6-fluoro-3,4-dihydroxybenzamido)-2-oxoimidazolidin-1-yl)-7-oxo-4-thia-1-azabicyclo[3.2.0]heptane-3-carboxylic acid |
| 143 | 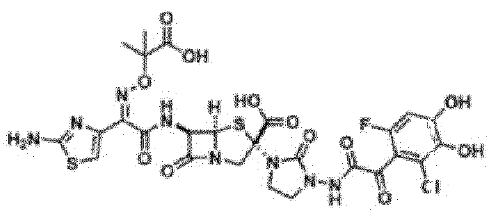 | (3R,5R,6R)-6-((Z)-2-(2-aminothiazol-4-yl)-2-(((2-carboxypropan-2-yl)oxy)imino)acetamido)-3-(3-(2-chloro-6-fluoro-3,4-dihydroxyphenyl)-2-oxoacetamido)-2-oxoimidazolidin-1-yl)-7-oxo-4-thia-1-azabicyclo[3.2.0]heptane-3-carboxylic acid |
| 144 | 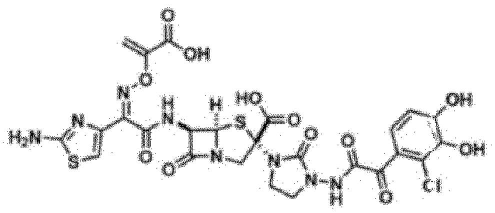 | (3R,5R,6R)-6-((Z)-2-(2-aminothiazol-4-yl)-2-(((1-carboxyvinyl)oxy))imino)acetami do)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |

The measured values of NMR and MS of the compounds in the table are as follows.

Example 142

NMR: 1.49 (3H, s), 1.51 (3H, s), 3.65 (1H, d, J = 12.8 Hz), 3.72-3.78 (4H, m), 4.68 (1H, d, J = 12.8 Hz), 5.71 (1H, d, J = 3.6 Hz), 5.76 (1H, d, J = 2.8 Hz), 6.75 (1H, d, J = 10.8Hz), 7.03 (1H, s)
MS: 731.00 $[M + H]^+$, 729.00 $[M - H]^-$

Example 143

NMR: 1.49 (3H, s), 1.51 (3H, s), 3.61 (1H, d, J = 12.4 Hz), 3.65-3.81 (4H, m), 4.67 (1H, d, J = 12.8 Hz), 5.70 (1H, d, J = 3.6 Hz), 5.75 (1H, d, J = 3.6 Hz), 6.58 (1H, d, J = 12.8 Hz), 7.03 (1H, s)
MS: 759.00 $[M + H]^+$, 757.00 $[M - H]^-$

Example 144

NMR: 3.35-3.82 (4H, m), 3.58 (1H, d, J = 12.8 Hz), 4.70 (1H, d, J = 12.8 Hz), 5.21-5.25 (1H, m), 5.33-5.39 (1H, m), 5.70 (1H, d, J = 3.6 Hz), 5.79 (1H, d, J = 3.2 Hz), 6.61 (1H, d, J = 8.4 Hz), 7.28 (1H, s), 7.32 (1H, d, J = 8.4 Hz)
MS: 725.05 $[M + H]^+$, 722.90 $[M - H]^-$

The compounds shown in Table 36 were obtained in the same manner as in Example 105.

[Table 36]

| Ex.No. | Structural Formula | Name |
|---|---|---|
| 145 | 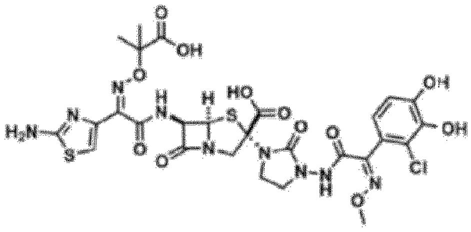 | (3R,5R,6R)-6-((Z)-2-(2-aminothiazol-4-yl)-2-(((2-carboxypropan-2-yl)oxy)imino)acetamido)-3-(3-((Z)-2-(2-chloro-3,4-dihydroxyphenyl)-2-(methoxyimino)acetamido)-2-oxoimidazolidin-1-yl)-7-oxo-4-thia-1-azabicyclo [3.2.0]heptane-3-carboxylic acid |
| 14 6 | 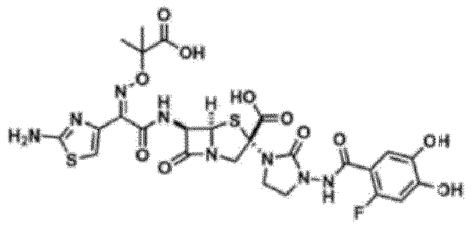 | (3R,5R,6R)-6-((Z)-2-(2 aminothiazol-4-yl)-2-(((2-carboxypropan-2-yl)oxy)imino)acetamido)-3-(3-(2-fluoro-4,5-dihydroxybenzamido)-2-oxoimidazolidin-1-yl)-7-oxo-4-thia-1-azabicyclo[3.2.0]heptane-3-carboxylic acid |
| 147 | 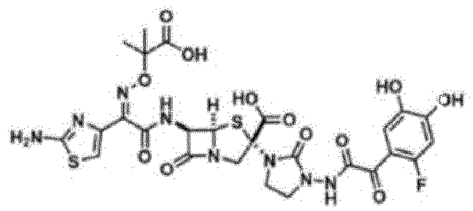 | (3R,5R,6R)-6-((Z)-2-(2-aminothiazol-4-yl)-2-(((2-carboxypropan-2-yl)oxy)imino)acetamido)-3-(3-(2-(2-fluoro-4,5-dihydroxyphenyl)-2-oxoacetamido)-2-oxoimidazolidin-1-yl)-7-oxo-4-thia-1-azbicyclo[3.2.0]heptane-3-carboxylic acid |
| 148 | 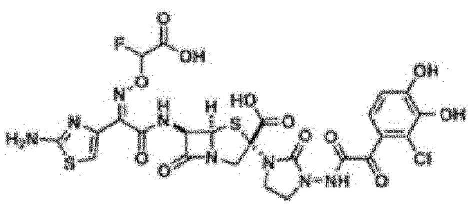 | (3R,5R,6R)-6-((Z)-2-(2-aminothiazol-4-yl)-2-((carboxyfluoromethoxy)imino)ac etamido)-3-(3-(2-(2-chloro-3,4-dihydroxyphenyl)-2-oxoacetamido)-2-oxoimidazolidin-1-yl)-7-oxo-4-thia-1-azabicyclo[3.2.0]heptane-3-carboxylic acid |

(continued)

| Ex.No. | Structural Formula | Name |
|---|---|---|
| 149 | 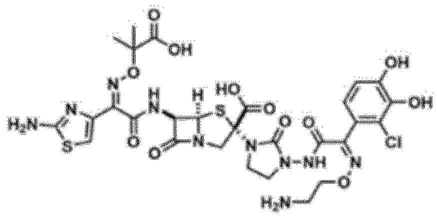 | (3R,5R,6R)-3-(3-((Z)-2-((2-aminoethoxy)imino)-2-(2-chloro-3,4-dihydroxyphenyl)acetamido)-2-oxoimidazolidin-1-yl)-6-((Z)-2-(2-aminothiazol-4-yl)-2-(((2-carboxypropan-2-yl)oxy)imino)acetamido)-7-oxo-4-thia-1-azabicyclo[3.2.0]heptane-3-carboxylic acid |
| 150 | 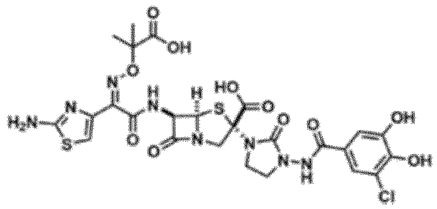 | (3R,5R,6R)-6-((Z)-2-(2-aminothiazol-4-yl)-2-(((2-carboxypropan-2-yl)oxy)imino)acetamido)-3-(3-(3-chloro-4,5-dihydroxybenzamido)-2-oxoimidazolidin-1-yl)-7-oxo-4-thia-1-azabicyclo[3.2.0]heptane-3-carboxylic acid |
| 151 | 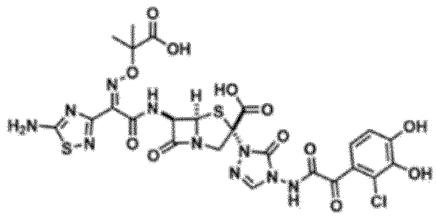 | (3R,5R,6R)-6-((Z)-2-(5-amino-1,2,4-thiadiazol-3-yl)-2-(((2-carboxypropan-2-yl)oxy)imino)acetamido)-3-(4-(2-2-chloro-3,4-dihydroxyphenyl)-2-oxoacetamido)-5-oxo-4,5-dihydro-1H-1,2,4-triazol-1-yl)-7-oxo-4-thia-1-azabicyclo[3.2.0]heptane-3-carboxylic acid |
| 152 | 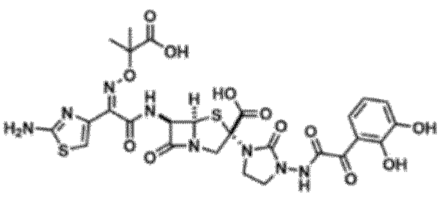 | (3R,5R,6R)-6-((Z)-2-(2-aminothiazol-4-yl)-2-(((2-carboxypropan-2-yl)oxy)imino)acetamido)-3-(3-(2-(2,3-dihydroxyphenyl)-2-oxoacetamido)-2-oxoimidazolidin-1-yl)-7-oxo-4-thia-1-azadicyclo[3.2.0]heptane-3-carboxylic acid |

The measured values of NMR and MS of the compounds in the table are as follows.

Example 145

NMR: 1.48 (3H, s), 1.50 (3H, s), 3.31-3.77 (5H, m), 3.96-4.08 (3H, m), 4.65 (1H, d, J = 12.4 Hz), 5.68 (1H, d, J = 3.6 Hz), 5.74(1H, d, J = 3.6 Hz), 6.57 (1H, s), 6.84-7.08 (2H, m)
MS: 770.05 $[M + H]^+$, 768.00 $[M - H]^-$

Example 146

NMR: 1.44 (3H, s), 1.46 (3H, s), 3.56 (1H, dd, J = 12.8, 0.8 Hz), 3.60-3.72 (4H, m), 4.60 (1H, d, J = 12.8 Hz), 5.65 (1H, d, J = 3.6 Hz), 5.69 (1H, dd, J = 3.6, 0.8 Hz), 6.74 (1H, d, J = 12.0 Hz), 7.00 (1H, s), 7.23 (1H, d, J = 7.2 Hz)
MS: 697.05 $[M + H]^+$, 695.15 $[M - H]^-$

Example 147

NMR: 1.49 (3H, s), 1.51 (3H, s), 3.38-3.82 (5H, m), 4.68 (1H, d, J = 12.8 Hz), 5.71 (1H, d, J = 4.0 Hz), 5.76 (1H, d, J = 3.6 Hz), 6.70 (1H, d, J = 12.4 Hz), 7.04 (1H, s), 7.36 (1H, d, J = 6.8 Hz)
MS: 725.00 $[M + H]^+$, 722.95 $[M - H]^-$

Example 148

NMR: 3.60 (1H, d, J = 12.8 Hz), 3.66-3.82 (4H, m), 4.66-4.72 (1H, m), 5.71 (1H, d, J = 3.6 Hz), 5.76 (1H, d, J = 5.6 Hz), 6.00 [6.01] (1H, d, J = 59.6 Hz), 6.58-6.67 (1H, m), 7.22-7.27 (1H, m), 7.29-7.36 (1H, m)

MS: 731.20 $[M + H]^+$, 728.80 $[M - H]^-$

Example 149

NMR: 1.49 (3H, s), 1.50 (3H, s), 3.27-3.60 (4H, m), 3.61-3.88 (3H, m), 4.44-4.59 (2H, m), 4.67 (1H, d, J = 12.8 Hz), 5.67-5.71 (1H, m), 5.73-5.87 (1H, m), 6.89-7.13 (3H, m)
MS: 799.25 $[M + H]^+$, 797.00 $[M - H]^-$

Example 150

NMR: 1.49 (3H, s), 1.51 (3H, s), 3.58-3.80 (5H, m), 4.67 (1H, d, J = 12.8 Hz), 5.70 (1H, d, J = 3.6 Hz), 5.75 (1H, d, J = 2.8 Hz), 7.04 (1H, s), 7.29 (1H, d, J = 2.0 Hz), 7.46 (1H, d, J = 2.0 Hz)
MS: 713.00 $[M + H]^+$, 710.95 $[M - H]^-$

Example 151

NMR: 1.53 (3H, s), 1.55 (3H, s), 4.00 (1H, dd, J = 12.8, 1.2 Hz), 4.56 (1H, d, J = 12.8 Hz), 5.66 (1H, d, J = 3.6 Hz), 5.88 (1H, d, J = 2.8 Hz), 6.63 (1H, d, J = 8.4 Hz), 7.40 (1H, d, J = 8.4 Hz), 8.15 (1H, s)
MS: 741.05 $[M + H]^+$, 739.00 $[M - H]^-$

Example 152

NMR: 1.49 (3H, s), 1.51 (3H, s), 3.45-3.55 (1H, m), 3.63 (1H, dd, J = 12.8, 1.2 Hz), 3.68-3.82 (3H, m), 4.68 (1H, d, J = 12.4 Hz), 5.64-5.73 (1H, m), 5.73-5.78 (1H, m), 6.94-7.02 (1H, m), 7.04 (1H, s), 7.20-7.34 (1H, m), 7.53 (1H, dd, J = 8.2, 1.4 Hz)
MS: 707.10 $[M + H]^+$, 705.05 $[M - H]^-$

Example 153

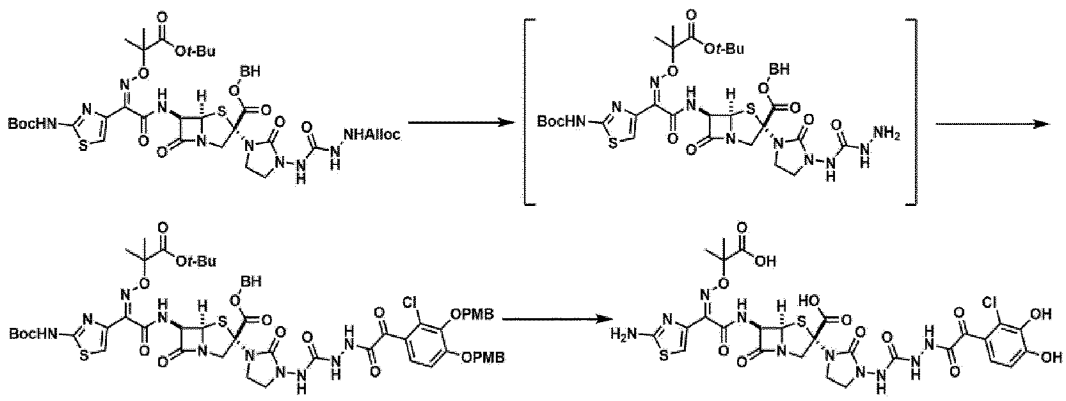

Example 153 (1)

THF (14 mL), 1,3-dimethylbarbituric acid (187 mg), and tetrakis(triphenylphosphine)palladium (0) (159 mg) were sequentially added to the compound (926 mg) obtained in Reference Example 129, and the mixture was stirred at room temperature for 1 hour. Ethyl acetate (14 mL), water (14 mL), and 1 mol/L hydrochloric acid were added to the reaction mixture, and the organic layer was separated. The organic layer was sequentially washed with water and a saturated aqueous sodium chloride solution and dehydrated and dried over anhydrous sodium sulfate. The mixture was filtered, and 2-(2-chloro-3,4-bis((4-methoxybenzyl)oxy)phenyl)-2-oxoacetic acid (420 mg), HATU (420 mg), DMAC (9.3 mL), and NMM (609 μL) were added to the filtrate. The solvent (about 15 mL) was distilled off under reduced pressure, and the obtained mixture was stirred at room temperature for 1 hour. HATU (105 mg) and NMM (30 μL) were added to the reaction mixture, and the mixture was stirred at room temperature for 2 hours and 30 minutes. Ethyl acetate (15 mL), water (15 mL), and 1 mol/L hydrochloric acid were added to the reaction mixture, and the organic layer was separated. The organic layer was sequentially washed with water and a saturated aqueous sodium chloride solution and dehydrated and dried over

anhydrous sodium sulfate. The solvent was distilled off under reduced pressure, and the residue was purified by silica gel column chromatography [eluent; acetone:chloroform = 10:90 → 30:70], thereby obtaining a target substance (530 mg) as light yellow solids.

Example 153 (2)

By using the compound (530 mg) obtained in Example 153 (1), (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetyl)hydrazine-1-carboxamido)-2-oxoimidazo lidin-1-yl)-7-oxo-4-thia-1-azabicyclo[3.2.0]heptane-3-carboxylic acid was obtained in the same manner as in Example 1 (2) as yellow solids (94.4 mg).

NMR: 1.49 (3H, s), 1.51 (3H, s), 3.48-3.81 (5H, m), 4.62-4.71 (1H, m), 5.70 (1H, d, J = 3.6 Hz), 5.75 (1H, d, J = 3.2 Hz), 6.95 (1H, d, J = 8.8 Hz), 7.04 (1H, s), 7.44 (1H, d, J = 8.4 Hz)
MS: 799.05 $[M + H]^+$, 797.00 $[M - H]^-$

Example 154

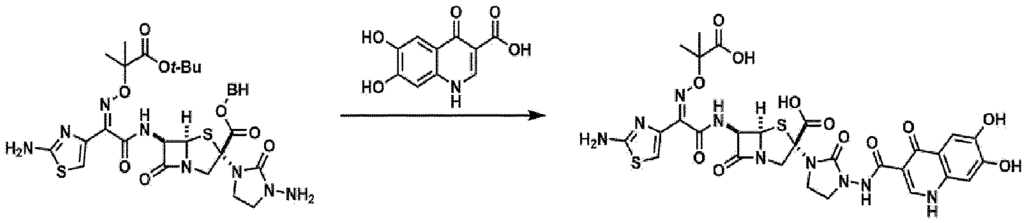

Dichloromethane (2.3 mL) was added to 6,7-dihydroxy-4-oxo-1,4-dihydroquinoline-3-carboxylic acid (100 mg), and then trimethylsilyl chloride (236 μL) and N,N-diisopropylethylamine (315 μL) were sequentially added thereto under ice cooling. The reaction mixture was stirred at the same temperature for 30 minutes. At the same temperature, oxalyl chloride (47 μL) and DMF (10 μL) were sequentially added to the reaction mixture. The reaction mixture was stirred at the same temperature for 1 hour and 30 minutes. At the same temperature, oxalyl chloride (47 μL) was added to the reaction mixture, and the reaction mixture was stirred at the same temperature for 1 hour, thereby obtaining a mixture of acid chlorides.

A mixture of THF (4.5 mL), water (4.5 mL), sodium hydrogen carbonate (171 mg), and the obtained mixture of acid chlorides was added to the compound (311 mg) obtained in Reference Example 28, and the mixture was stirred at room temperature for 1 hour. Ethyl acetate (30 mL) and water (20 mL) were added to the reaction mixture, and the organic layer was separated. The organic layer was washed with a saturated aqueous sodium chloride solution and dehydrated and dried over anhydrous sodium sulfate. The solvent was distilled off under reduced pressure, thereby obtaining yellow solids. By using the obtained yellow solids, (3R,5R,6R)-6-((Z)-2-(2-aminothiazol-4-yl)-2-(((2-carboxypropan-2-yl)oxy)imino)acetamido)-3-(3-(6,7-dihydroxy-4-oxo-1,4-dihydroquinoline-3-carboxamido)-2-oxoimidazolidin-1-yl)-7-o xo-4-thia-1-azabicyclo[3.2.0]heptane-3-carboxylic acid (9.5 mg) was obtained in the same manner as in Example 1 (2) as light yellow solids.

NMR($DMSO-d_6$): 1.36 (3H, s), 1.41 (3H, s), 3.00-3.80 (5H, m), 4.41 (1H, d, J = 12.8 Hz), 5.50-5.63 (2H, m), 6.78 (1H, s), 7.28 (2H, s), 9.17-9.32 (1H, m)
MS: 746.05 $[M + H]^+$, 744.15 $[M - H]^-$

Example 155

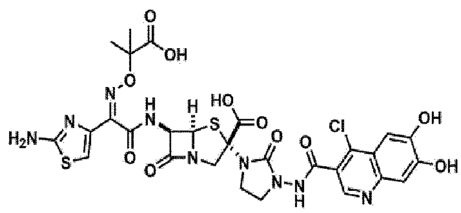

In the same manner as in Example 154, (3R,5R,6R)-6-((Z)-2-(2-aminothiazol-4-yl)-2-(((2-carboxypropan-2-yl)oxy)imino)acetamido)-3-(3-(4-chloro-6,7-dihydroxyquinoline-3-carboxamido)-2-oxoimidazolidin-1-yl)-7-oxo-4-thia-1-azabicyclo[3.2.0]heptane-3-carboxylic acid (28.1 mg) was obtained as yellow solids.

NMR: 1.50 (3H, s), 1.51 (3H, s), 3.64 (1H, d, J = 12.8 Hz), 3.72-3.84 (4H, m), 4.70 (1H, d, J = 12.8 Hz), 5.72 (1H, d, J = 3.6 Hz), 5.76(1H, d, J = 3.6 Hz), 6.96 (1H, s), 7.04 (1H, s), 7.30 (1H, s), 8.51 (1H, s)
MS: 764.10 $[M + H]^+$, 761.90 $[M - H]^-$

Example 156

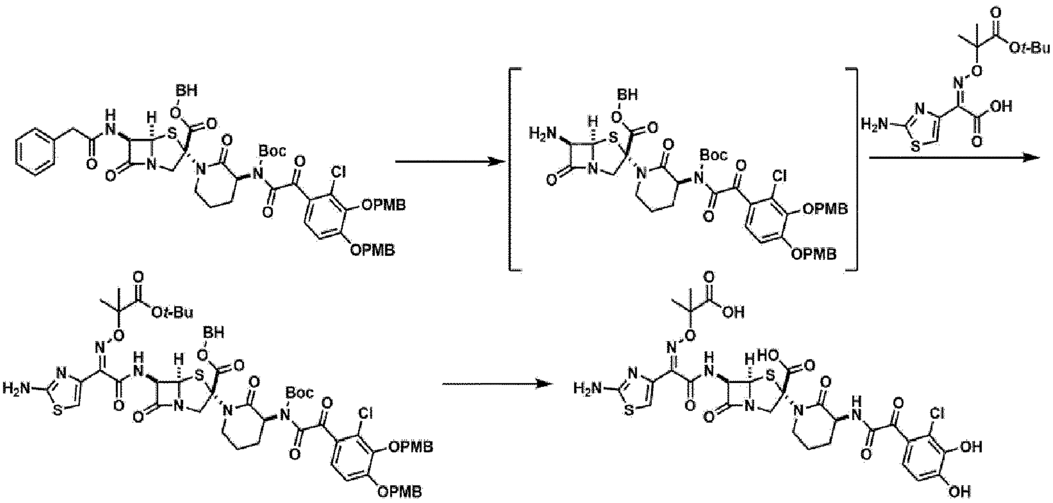

Example 156 (1)

By using the compound (400 mg) obtained in Reference Example 125, a target substance (138 mg) was obtained as light brown solids in the same manner as in Reference Example 27 (1).

Example 156 (2)

By using the compound (138 mg) obtained in Example 156 (1), (3R,5R,6R)-6-((Z)-2-(2-aminothiazol-4-yl)-2-(((2-carboxypropan-2-yl)oxy)imino)acetamido)-3-(3-((S)-3-(2-(2-chloro-3,4-dihydroxyphenyl)-2-oxoacetamido)-2-oxopiperidin-1-yl)-7-oxo-4 -thia-1-azabicyclo[3.2.0]heptane-3-carboxylic acid (45 mg) was obtained in the same manner as in Example 1 (2) as yellow solids.

NMR: 1.48 (3H, s), 1.50 (3H, s), 1.86-2.32 (4H, m), 3.21 (1H, d, J = 12.8 Hz), 3.50-4.10 (2H, m), 4.42-4.68 (1H, m), 4.95-5.11 (1H, m), 5.59-5.67 (1H, m), 5.68-5.76 (1H, m), 6.96 (1H, d, J = 8.8 Hz), 7.03 (1H, s), 7.39 (1H, d, J = 8.4 Hz)
MS: 754.05 $[M + H]^+$, 752.10 $[M - H]^-$

The compounds shown in Table 37 were obtained in the same manner as in Example 156.

[Table 37]

| Ex.No. | Structural formula | Name |
|---|---|---|
| 157 | 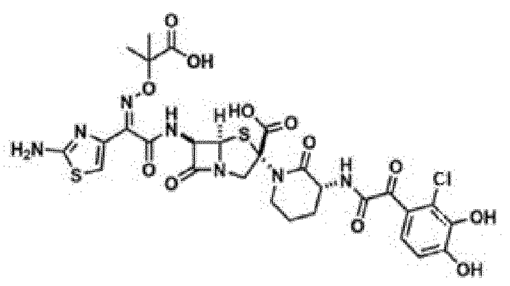 | (3R,5R,6R)-6-((Z)-2-(2-aminothiazol4-yl)-2-(((2-carboxypropan-2-yl)oxy)imino)acetamido)-3-((R)-3-(2-(2-chloro-3,4-dihydroxyphenyl)-2-oxoacetamido)-2-oxopiperidin-1-yl)-7-oxo-4-thia-1-azabicyclo[3.2.0]heptane-3-carboxylic acid |

(continued)

| Ex.No. | Structural formula | Name |
|---|---|---|
| 158 | 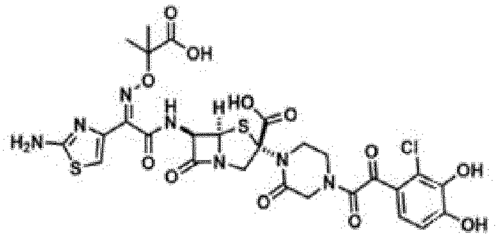 | (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetyl)-2-oxopiperazin-1-yl)-7-oxo-4-thia-1-azabicyclo[3.2.0]heptane-3-carboxylic acid |

The measured values of NMR and MS of the compounds in the table are as follows.

Example 157

NMR: 1.48 (3H, s), 1.50 (3H, s), 1.77-2.34 (4H, m), 3.21 (1H, d, J = 12.8 Hz), 3.60-3.88 (2H, m), 4.54-4.67 (1H, m), 4.95-5.07 (1H, m), 5.64 (1H, d, J = 3.6 Hz), 5.72 (1H, d, J = 3.6 Hz), 6.94 (1H, d, J = 8.8 Hz), 7.03 (1H, s), 7.39 (1H, d, J = 8.8 Hz)
MS: 754.05 $[M + H]^+$, 751.95 $[M - H]^-$

Example 158

NMR: 1.43-1.54 (6H, m), 3.20 (1H, d, J = 13.2 Hz), 3.65-4.12 (4H, m), 4.13-4.46 (2H, m), 4.98-5.11 (1H, m), 5.59-5.76 (2H, m), 6.53-6.96 (1H, m), 6.97-7.07 (1H, m), 7.23-7.59 (1H, m)
MS: 740.05 $[M + H]^+$, 737.95 $[M - H]^-$

Preparation examples and test examples of the present invention will be described, but the present invention is not limited thereto. The invention is as described in the claims.

The abbreviation for each of the preparation examples and test examples has the following meaning.

PIPC: piperacillin Sodium
CAZ: ceftazidime hydrate
IPM: imipenem hydrate·cilastatin sodium
MPC: mecillinam
LVFX: levofloxacin hydrate
AMK: amikacin sulfate
TC: tetracycline hydrochloride
VCM: vancomycin hydrochloride
PL-B: polymyxin B sulfate
TMP: trimethoprim
RFP: rifampicin
SBT: sulbactam sodium
TAZ: tazobactam sodium
CVA: potassium clavulanate
AVI: avibactam sodium
NAC: nacubactam
ZID: zidebactam
VAB: vaborbactam
MEPM: meropenem hydrate
CTX: cefotaxime sodium
CAZ: ceftazidime hydrate
Compound A: Compound obtained in Example 20
Compound B: Compound obtained in Example 19
Compound C: Compound obtained in Example 126
Compound D: Compound obtained in Example 22
Compound E: Compound obtained in Example 21
Compound F: Compound obtained in Example 143

Compound G: Compound obtained in Example 144

Preparation Example 1

(1) Equimolar sodium hydrogen carbonate and water are added to 2 g of the compound A and dissolved. The obtained solution is subjected to sterilizing filtration and lyophilized, thereby obtaining lyophilized powder.
(2) One chamber of a plastic double bag (double-chamber container) is filled with the lyophilized powder obtained in (1) and ceftazidime hydrate (1 g as ceftazidime), and sealed by heat seal welding. The other chamber is filled with 100 mL of physiological saline and sealed, thereby obtaining an injection kit.

Preparation Example 2

Equimolar sodium hydrogen carbonate and water are added to 4 g of the compound A and 0.5 g of tazobactam and dissolved. The obtained solution is subjected to sterilizing filtration, and a glass vial is filled with the solution, followed by lyophilization. Then, the vial is sealed with a rubber stopper under reduced pressure and further fastened with an aluminum cap, thereby obtaining an injection preparation.

Preparation Example 3

A preparation is prepared in the same manner as in Preparation Example 2 by using 4 g of the compound B and 0.5 g of tazobactam and then lyophilized, thereby obtaining an injection preparation.

Preparation Example 4

One chamber of a plastic double bag (double-chamber container) is filled with lyophilized powder containing approximately 2 g of the compound A obtained in the same manner as in Preparation Example 1 (1) and sulbactam sodium (1 g as sulbactam), and sealed by heat seal welding. The other chamber is filled with 100 mL of physiological saline and sealed, thereby obtaining an injection kit.

Preparation Example 5

A glass vial is filled with lyophilized powder containing approximately 2 g of the compound A obtained in the same manner as in Preparation Example 1 (1) and lyophilized powder of piperacillin sodium (2 g as piperacillin). Then, the vial is sealed with a rubber stopper and further fastened with an aluminum cap, thereby obtaining an injection preparation.

Preparation Example 6

One chamber of a plastic double bag (double-chamber container) is filled with lyophilized powder containing approximately 4 g of the compound A obtained in the same manner as in Preparation Example 1 (1) and avibactam sodium (1 g as avibactam), and sealed by heat seal welding. The other chamber is filled with 100 mL of physiological saline and sealed, thereby obtaining an injection kit preparation.

Test Example 1 Antibacterial activity evaluation test

The minimum inhibitory concentration (MIC) was measured according to the Clinical and Laboratory Standards Institute (CLSI) standard method by using the following broth microdilution method.
As bacteria, a Pseudomonas aeruginosa strain ATCC27853, an AmpC-derepressed Pseudomonas aeruginosa mutant strain (S-3028), an IMP-1-containing Pseudomonas aeruginosa strain (S-2838), a VIM-2-containing Pseudomonas aeruginosa strain (S-3779 ), a GES-19 and GES-20-containing Pseudomonas aeruginosa strain (S-3759), a CTX-M-15-containing Escherichia coli strain (TK-1747), a KPC-2-containing Klebsiella pneumoniae strain (Y-995), an OXA-48-containing Klebsiella pneumoniae strain (Y-1062), and an NDM-1-containing Klebsiella pneumoniae strain (Y-1007) were used. The test bacterial cells that had been cultured overnight on a Mueller Hinton agar medium were scraped off, suspended at a density equivalent to 0.5 McFarland standard, and diluted 10-fold, thereby obtaining an inoculum. A cation-adjusted Mueller Hinton medium containing a test compound was inoculated with 0.005 mL of the inoculum, and the cells were cultured at 35°C for 16 to 20 hours. The minimum drug concentration at which the growth of bacteria was not visually observed was defined as MIC (μg/mL).

As test compounds, the compounds obtained in Examples 2, 8, 19, 20, 21, 22, 23, 28, 29, 30, 31, 45, 47, 53, and 68 were used.

The results are shown in Table 38.

[Table 38]

| Example No. | Pseudo monas aeruginosa ATCC 27853 | Pseudo monas aeruginosa S-3028 | Pseudo monas aeruginosa S-2838 | Pseudo monas aeruginosa S-3779 | Pseudo monas aeruginosa S-3759 | Escherichia coli TK-1747 | Klebsiella pneumoniae Y-1007 | Klebsiella pneumoniae Y-995 | Klebsiella pneumoniae Y-1062 |
|---|---|---|---|---|---|---|---|---|---|
| 2 | 1 | 1 | 0.5 | 0.25 | 4 | 0.25 | < 0.06 | 0.12 | 2 |
| 8 | 0.25 | 0.5 | 4 | 1 | 2 | 0.12 | < 0.06 | 0.06 | 16 |
| 19 | 0.25 | 2 | 0.25 | 0.25 | 0.25 | 0.5 | < 0.06 | 0.25 | 0.12 |
| 20 | 0.5 | 0.25 | 0.12 | 0.25 | 0.5 | < 0.06 | < 0.06 | 0.12 | 0.5 |
| 21 | 0.25 | 4 | 2 | 0.5 | 1 | 0.12 | < 0.06 | < 0.06 | 2 |
| 22 | 0.5 | 0.5 | 0.5 | 0.25 | 0.5 | < 0.06 | < 0.06 | 0.06 | 0.06 |
| 23 | 0.25 | 0.5 | 1 | 0.25 | 1 | 0.25 | < 0.06 | 0.25 | 0.5 |
| 28 | 0.5 | 0.25 | 0.25 | 0.5 | 2 | 2 | < 0.06 | 1 | 0.25 |
| 29 | 0.25 | < 0.06 | 1 | 0.5 | 0.5 | 2 | < 0.06 | 0.5 | 1 |
| 30 | 0.25 | < 0.06 | 0.5 | 0.5 | 0.5 | 0.25 | < 0.06 | 0.25 | 0.5 |
| 31 | 0.25 | < 0.06 | 1 | 0.25 | 0.5 | 1 | 0.12 | 0.12 | 1 |
| 45 | 1 | 2 | 2 | 1 | 2 | 0.12 | < 0.06 | 0.25 | 1 |
| 47 | 0.5 | 0.12 | 4 | 1 | 1 | < 0.06 | < 0.06 | 0.5 | 0.25 |
| 53 | 0.5 | 1 | 4 | 0.5 | 2 | 2 | 0.12 | 2 | 0.5 |
| 68 | 1 | 0.25 | 1 | 2 | 4 | 1 | < 0.06 | 0.5 | 1 |

Test Example 2 Test for protection against systemic infection in mouse with multidrug-resistant Pseudomonas aeruginosa

As mice, ICR male SPF mice (4 weeks old: 10 mice per group) were used. Clinically isolated multidrug-resistant Pseudomonas aeruginosa strain (S-2838 strain) cultured overnight on a Mueller-Hinton agar plate at 37°C was cultured on a cation-adjusted Mueller Hinton medium for 5 hours, and then diluted 20-fold with a 10% mucin/phosphate buffer, thereby preparing an inoculum. An infection was induced by intraperitoneally inoculating the mice with the inoculum at 0.5 mL (about $10^6$ CFU/mouse). Each test compound was dissolved in physiological saline, and 1 hour after the infection, the compound was subcutaneously administered once to the mice at 40 mg/kg. The control group was administered with the same amount of physiological saline used as a vehicle. The number of surviving mice was recorded 3 days after the infection.

As test compounds, the compounds obtained in Examples 19, 20, 21, 22, 23, and 31 were used.

As a result, it has been revealed that while all the control groups not being administered with the test compounds die, the mice in the groups administered with the test compounds of Examples 19, 20, 21, 22, 23, and 31 show a survival rate equal to or higher than 90% 3 days after the inoculation with bacteria, which tells that the test compounds have in-vivo antibacterial activity against multidrug-resistant Pseudomonas aeruginosa.

Test example 3

An antibacterial activity evaluation test was performed in the same manner as in Test Example 1.

As test compounds, the compounds obtained in Examples 26, 73, 74, 76, 78, 82, 83, 84, 85, 86, 88, 104, 105, 107, 113, 114, 115, 117, 121, 122, 126, 136, 139, and 141 were used.

The results are shown in Table 39.

[Table 39]

| Example No. | Pseudo monas aeruginosa ATCC 27853 | Pseudo monas aeruginosa S-3028 | Pseudo monas aeruginosa S-2838 | Pseudo monas aeruginosa S-3779 | Pseudo monas aeruginosa S-3759 | Escherichia coli TK-1747 | Klebsiella pneumoniae Y-1007 | Klebsiella pneumoniae Y-995 | Klebsiella pneumoniae Y-1062 |
|---|---|---|---|---|---|---|---|---|---|
| 26 | 1 | 0.12 | 0.25 | 0.25 | 0.25 | 0.12 | < 0.06 | 0.12 | 0.5 |
| 73 | 0.5 | 0.25 | 0.5 | 1 | 0.5 | 0.25 | 0.12 | 16 | 1 |
| 74 | 0.5 | 0.5 | 0.25 | 1 | 1 | 1 | 0.25 | 2 | 4 |
| 76 | 0.5 | 0.12 | 0.5 | 0.25 | 0.25 | 0.06 | 0.25 | 0.25 | 0.25 |
| 78 | 0.5 | < 0.06 | 1 | 0.25 | 1 | 0.25 | < 0.06 | 0.25 | 0.25 |
| 82 | 0.5 | 4 | 4 | 1 | 2 | 1 | 0.5 | 4 | 2 |
| 83 | 0.25 | 0.5 | 0.5 | 0.5 | 1 | 0.5 | < 0.06 | 0.25 | 2 |
| 84 | 0.5 | 0.5 | 0.5 | 0.5 | 1 | < 0.06 | < 0.06 | 0.5 | < 0.06 |
| 85 | 0.5 | 0.12 | 0.25 | 0.25 | 0.25 | < 0.06 | < 0.06 | < 0.06 | 0.12 |
| 86 | 4 | 1 | 1 | 2 | 2 | 1 | < 0.06 | 0.5 | 1 |
| 88 | 0.5 | 0.5 | 0.5 | 0.5 | 0.5 | 2 | < 0.06 | 0.25 | 0.5 |
| 104 | 2 | 2 | 0.5 | 1 | 4 | 1 | < 0.06 | 2 | 2 |
| 105 | 0.25 | < 0.06 | 0.25 | 0.25 | 0.5 | 0.5 | < 0.06 | 0.12 | 0.5 |
| 107 | 0.25 | 2 | 0.5 | 0.25 | 0.5 | 0.25 | < 0.06 | 2 | 0.25 |
| 113 | 4 | 0.25 | 0.5 | 0.5 | 1 | < 0.06 | < 0.06 | 0.12 | 0.25 |
| 114 | 2 | 1 | 4 | 1 | 2 | 0.25 | < 0.06 | 0.25 | 0.25 |
| 115 | 0.5 | 0.12 | 1 | 0.5 | 0.5 | 0.25 | < 0.06 | 0.25 | 1 |
| 117 | 2 | 0.25 | 2 | 0.5 | 2 | 0.12 | < 0.06 | 0.25 | 0.5 |
| 121 | 2 | 2 | 4 | 1 | 2 | < 0.06 | 0.12 | 0.25 | 0.5 |
| 122 | 1 | 2 | 2 | 2 | 2 | 0.5 | < 0.06 | 0.5 | 1 |
| 126 | 1 | 0.5 | 2 | 1 | 0.5 | < 0.06 | < 0.06 | 0.12 | 1 |
| 136 | 0.5 | 0.12 | 0.12 | 0.5 | 0.5 | < 0.06 | 0.12 | 0.25 | 0.12 |
| 139 | 1 | 0.5 | 0.5 | 1 | 2 | 2 | 0.12 | 1 | 4 |
| 141 | 1 | 0.5 | 0.25 | 1 | 4 | 0.5 | 0.25 | > 32 | 1 |

Test example 4

A test for protection against systemic infection in a mouse with multidrug-resistant Pseudomonas aeruginosa was performed in the same manner as in Test Example 2.

As test compounds, the compounds obtained in Examples 83, 105, 114, 117, 122, 126, and 139 were used.

As a result, it has been revealed that while all the control groups not being administered with the test compounds die, the mice in the groups administered with the test compounds of Examples 83, 105, 114, 117, 122, 126, and 139 show a survival rate equal to or higher than 90% 3 days after the inoculation with bacteria, which tells that the test compounds have in-vivo antibacterial activity against multidrug-resistant Pseudomonas aeruginosa.

Test Example 5 Evaluation test for combinational effect of test compound and antibacterial compound

By using a broth microdilution method, the combinational effect of antibacterial activity of a test compound and an antibacterial compound was evaluated.

As test compounds, the compounds A, B, and C were used.

As antibacterial compounds, PIPC (commercially available product), CAZ (commercially available product), IPM (commercially available product), MPC (commercially available product), LVFX (manufactured by Chem-Impex International, Inc.), AMK (commercially available product), PL-B (commercially available product), TMP (commercially available product), and RFP (commercially available product) were used.

As bacterial strains, an Escherichia coli ATCC25922 strain (hereinafter, called Escherichia coli A), a Klebsiella pneumoniae ATCC13883 strain (hereinafter, called Klebsiella pneumoniae A), and a Pseudomonas aeruginosa ATCC27853 strain (hereinafter, called Pseudomonas aeruginosa A) were used.

The minimum inhibitory concentration (hereinafter, called MIC) was measured according to the Clinical and Laboratory Standards Institute (CLSI) standard method by using the following broth microdilution method.

The bacterial cells that had been cultured overnight on a Mueller Hinton agar medium were scraped off, suspended at a density equivalent to 0.5 McFarland standard. The obtained suspension was diluted at 10 fold, thereby obtaining an inoculum. Cation-adjusted Mueller Hinton media containing only a test compound, containing only an antibacterial compound, or containing a test compound and an antibacterial compound were inoculated with 0.005 mL of the inoculum, and the cells were cultured at 35°C for 18 to 20 hours. The minimum drug concentration at which the growth of bacteria was not visually observed was defined as MIC (μg/mL).

The antibacterial activity exhibited in a case where only a test compound is used, in a case where only an antibacterial compound is used, or in a case where a test compound and an antibacterial compound are used in combination was measured by a checkerboard method, and an FIC index (Fractional inhibitory concentration index) was determined.

The FIC Index is the minimum value calculated by the following formula.

FIC Index = (MIC value obtained in a case where test compound is used in combination/MIC value obtained in a case where test compound is used alone) + (MIC value obtained in a case where antibacterial compound is used in combination/MIC value obtained in a case where antibacterial compound is used alone)

In a case where the FIC Index was 0.5 or less, it was decided that the use of two drugs in combination brings about a synergistic effect.

In a case where the FIC Index was more than 0.5 and 1 or less, it was decided that the use of two drugs in combination brings about an additive effect. (Diagnostic Microbiology and Infectious Disease, 2004, Vol. 49, p. 197)

Tables 40 to 42 show the FIC indices of the combinations of the compounds A, B and C and the antibacterial compounds.

[Table 40]

| Test compound | Antibacterial substance | Strain | FIC index |
|---|---|---|---|
| **Compound A** | **PIPC** | **Escherichia coli A** | **0.5625** |
| **Compound A** | **CAZ** | **Escherichia coli A** | **0.625** |
| **Compound A** | **IPM** | **Escherichia coli A** | **0.5156** |
| **Compound A** | **MPC** | **Escherichia coli A** | **0.5078** |
| **Compound A** | **LVFX** | **Escherichia coli A** | **0.75** |
| **Compound A** | **AMK** | **Escherichia coli A** | **0.5625** |
| **Compound A** | **TC** | **Escherichia coli A** | **0.75** |

(continued)

| Test compound | Antibacterial substance | Strain | FIC index |
|---|---|---|---|
| Compound A | TMP | Escherichia coli A | 0.625 |
| Compound A | RFP | Escherichia coli A | 0.625 |
| Compound A | PIPC | Klebsiella pneumoniae A | 0.375 |
| Compound A | CAZ | Klebsiella pneumoniae A | 0.625 |
| Compound A | IPM | Klebsiella pneumoniae A | 0.375 |
| Compound A | MPC | Klebsiella pneumoniae A | < 0.5 |
| Compound A | LVFX | Klebsiella pneumoniae A | 0.75 |
| Compound A | PL-B | Klebsiella pneumoniae A | 0.125 |
| Compound A | TMP | Klebsiella pneumoniae A | 0.375 |
| Compound A | RFP | Klebsiella pneumoniae A | 0.502 |
| Compound A | PIPC | Pseudomonas aeruginosa A | 0.5 |
| Compound A | CAZ | Pseudomonas aeruginosa A | 0.5625 |
| Compound A | IPM | Pseudomonas aeruginosa A | 0.252 |
| Compound A | MPC | Pseudomonas aeruginosa A | 0.5 >, ≤ 1 |
| Compound A | LVFX | Pseudomonas aeruginosa A | 0.5625 |
| Compound A | AMK | Pseudomonas aeruginosa A | 0.5 |
| Compound A | TC | Pseudomonas aeruginosa A | 0.375 |
| Compound A | PL-B | Pseudomonas aeruginosa A | 0.625 |
| Compound A | RFP | Pseudomonas aeruginosa A | 0.281 |

[Table 41]

| Test compound | Antibacterial substance | Strain | FIC index |
|---|---|---|---|
| Compound B | PIPC | Escherichia coli A | 0.53 |
| Compound B | CAZ | Escherichia coli A | 0.625 |
| Compound B | MPC | Escherichia coli A | 0.508 |
| Compound B | TMP | Escherichia coli A | 0.531 |
| Compound B | RFP | Escherichia coli A | 0.531 |
| Compound B | PIPC | Klebsiella pneumoniae A | < 0.5 |
| Compound B | CAZ | Klebsiella pneumoniae A | 0.625 |
| Compound B | IPM | Klebsiella pneumoniae A | 0.375 |
| Compound B | MPC | Klebsiella pneumoniae A | < 0.375 |
| Compound B | LVFX | Klebsiella pneumoniae A | 0.563 |
| Compound B | AMK | Klebsiella pneumoniae A | 0.75 |
| Compound B | PL-B | Klebsiella pneumoniae A | 0.156 |
| Compound B | TMP | Klebsiella pneumoniae A | 0.375 |
| Compound B | RFP | Klebsiella pneumoniae A | 0.281 |
| Compound B | PIPC | Pseudomonas aeruginosa A | 0.375 |
| Compound B | CAZ | Pseudomonas aeruginosa A | 0.563 |
| Compound B | IPM | Pseudomonas aeruginosa A | 0.5 |
| Compound B | LVFX | Pseudomonas aeruginosa A | 0.625 |
| Compound B | AMK | Pseudomonas aeruginosa A | 0.375 |
| Compound B | PL-B | Pseudomonas aeruginosa A | 0.531 |
| Compound B | TMP | Pseudomonas aeruginosa A | 0.502 |
| Compound B | RFP | Pseudomonas aeruginosa A | 0.313 |

[Table 42]

| Test compound | Antibacterial substance | Strain | FIC index |
|---|---|---|---|
| Compound C | PIPC | Klebsiella pneumoniae A | < 0.5 |

(continued)

| Test compound | Antibacterial substance | Strain | FIC index |
|---|---|---|---|
| Compound C | CAZ | Klebsiella pneumoniae A | 0.625 |
| Compound C | IPM | Klebsiella pneumoniae A | 0.375 |
| Compound C | MPC | Klebsiella pneumoniae A | 0.188 |
| Compound C | LVFX | Klebsiella pneumoniae A | 0.75 |
| Compound C | AMK | Klebsiella pneumoniae A | 0.75 |
| Compound C | VCM | Klebsiella pneumoniae A | < 0.625 |
| Compound C | PL-B | Klebsiella pneumoniae A | 0.625 |
| Compound C | RFP | Klebsiella pneumoniae A | 0.563 |
| Compound C | PIPC | Pseudomonas aeruginosa A | 0.75 |
| Compound C | CAZ | Pseudomonas aeruginosa A | 0.563 |
| Compound C | IPM | Pseudomonas aeruginosa A | 0.188 |
| Compound C | LVFX | Pseudomonas aeruginosa A | 0.188 |
| Compound C | AMK | Pseudomonas aeruginosa A | 0.5 |
| Compound C | TC | Pseudomonas aeruginosa A | 0.25 |
| Compound C | VCM | Pseudomonas aeruginosa A | 0.266 |
| Compound C | PL-B | Pseudomonas aeruginosa A | 0.188 |
| Compound C | RFP | Pseudomonas aeruginosa A | 0.375 |

All of the compounds A, B, and C exert an additive or synergistic effect on main strains of Gram-negative bacteria and brought about stronger antibacterial effects in a case where these compounds are used in combination with other mainstream antibacterial compounds.

Test Example 6 Evaluation test for antibacterial activity exhibited in case where test compound and β-lactamase inhibitor are used in combination

By using a broth microdilution method, the combinational effect of a test compound and a β-lactamase inhibitor was evaluated.
The evaluation test for antibacterial activity was performed in the same manner as in Test Example 1.
As test compounds, the compounds A, B, C, and E were used.
As control compounds, MEPM, CTX, and CAZ were used.
As β-lactamase inhibitors, SBT (commercially available product), TAZ (commercially available product), CVA (commercially available product), AVI (commercially available product), NAC (manufactured by the present applicant), ZID (manufactured by the present applicant), and VAB (manufactured by the present applicant) were used.
As bacterial strains, the following bacterial strains producing β-lactamase were used.
Escherichia coli TK-1747 strain producing CTX-M-15 (hereinafter, called strain A),

Klebsiella pneumoniae Y-1020 strain producing KPC-3 and SHV-12 (hereinafter, called strain B)
Klebsiella pneumoniae Y-1133 strain producing NDM-1, OXA-48, and CTX-M-15 (hereinafter, called strain C),
Escherichia coli TK-3113 strain producing IMP-8 (hereinafter, called strain D),
Escherichia coli TK-1765 strain producing CMY-2 (hereinafter, called strain E)

The antibacterial activity exhibited in a case where only a test compound was used and in a case where a β-lactamase inhibitor was added to a test compound at a concentration of 4 μg/mL was measured, and the combinational effect was evaluated.
All the β-lactamase inhibitors used in the test did not inhibit the growth of all bacterial strains in a case where the inhibitors added alone at a concentration of 4 μg/mL. The results are shown in Tables 43 to 47.

[Table 43]

| Test compound | β-Lactamase inhibitor | Strain | MIC (μg/mL) |
|---|---|---|---|
| Compound A | N/A | Strain A | 0.12 |
| Compound A | SBT | Strain A | < 0.015 |
| Compound A | TAZ | Strain A | < 0.015 |

(continued)

| Test compound | β-Lactamase inhibitor | Strain | MIC (μg/mL) |
|---|---|---|---|
| Compound A | CVA | Strain A | < 0.015 |
| Compound A | AVI | Strain A | < 0.015 |
| Compound A | NAC | Strain A | < 0.015 |
| Compound A | ZID | Strain A | < 0.015 |
| Compound A | VAB | Strain A | 0.06 |
| Compound C | N/A | Strain A | 0.12 |
| Compound C | SBT | Strain A | 0.06 |
| Compound C | TAZ | Strain A | 0.06 |
| Compound C | CVA | Strain A | 0.03 |
| Compound C | AVI | Strain A | 0.03 |
| Compound C | NAC | Strain A | 0.03 |
| Compound C | ZID | Strain A | < 0.015 |
| Compound E | N/A | Strain A | 0.06 |
| Compound E | SBT | Strain A | < 0.015 |
| Compound E | TAZ | Strain A | < 0.015 |
| Compound E | CVA | Strain A | < 0.015 |
| Compound E | AVI | Strain A | < 0.015 |
| Compound E | NAC | Strain A | < 0.015 |
| Compound E | ZID | Strain A | < 0.015 |
| CTX | N/A | Strain A | > 128 |

[Table 44]

| Test compound | β-Lactamase inhibitor | Strain | MIC (μg/mL) |
|---|---|---|---|
| Compound A | N/A | Strain B | 2 |
| Compound A | AVI | Strain B | 0.25 |
| Compound A | NAC | Strain B | < 0.015 |
| Compound A | ZID | Strain B | < 0.015 |
| Compound A | VAB | Strain B | 1 |
| Compound B | N/A | Strain B | 2 |
| Compound B | AVI | Strain B | 0.25 |
| Compound B | NAC | Strain B | < 0.015 |
| Compound B | ZID | Strain B | < 0.015 |
| Compound B | VAB | Strain B | < 0.015 |
| Compound C | N/A | Strain B | 4 |
| Compound C | AVI | Strain B | 1 |
| Compound C | NAC | Strain B | 0.12 |
| Compound C | ZID | Strain B | 0.03 |
| Compound D | N/A | Strain B | 2 |
| Compound D | AVI | Strain B | 0.25 |
| Compound D | NAC | Strain B | < 0.015 |
| Compound D | ZID | Strain B | < 0.015 |
| Compound E | N/A | Strain B | 4 |
| Compound E | AVI | Strain B | 0.25 |
| Compound E | NAC | Strain B | < 0.015 |
| Compound E | ZID | Strain B | < 0.015 |
| Compound E | VAB | Strain B | 1 |
| MEPM | N/A | Strain B | 32 |

[Table 45]

| Test compound | β-Lactamase inhibitor | Strain | MIC (μg/mL) |
|---|---|---|---|
| Compound A | N/A | Strain C | 0.5 |
| Compound A | AVI | Strain C | 0.12 |
| Compound A | NAC | Strain C | 0.03 |
| Compound A | ZID | Strain C | < 0.015 |
| Compound B | N/A | Strain C | 1 |
| Compound B | AVI | Strain C | 0.12 |
| Compound B | NAC | Strain C | < 0.015 |
| Compound B | ZID | Strain C | < 0.015 |
| Compound C | N/A | Strain C | 1 |
| Compound C | AVI | Strain C | 0.5 |
| Compound C | NAC | Strain C | 0.03 |
| Compound C | ZID | Strain C | < 0.015 |
| Compound D | N/A | Strain C | 0.5 |
| Compound D | AVI | Strain C | 0.12 |
| Compound D | NAC | Strain C | < 0.015 |
| Compound D | ZID | Strain C | < 0.015 |
| Compound E | N/A | Strain C | 1 |
| Compound E | AVI | Strain C | 0.12 |
| Compound E | NAC | Strain C | < 0.015 |
| Compound E | ZID | Strain C | < 0.015 |
| MEPM | N/A | Strain C | > 128 |

[Table 46]

| Test compound | β-Lactamase inhibitor | Strain | MIC (μg/mL) |
|---|---|---|---|
| Compound A | N/A | Strain D | 0.5 |
| Compound A | NAC | Strain D | 0.06 |
| Compound A | ZID | Strain D | < 0.015 |
| Compound B | N/A | Strain D | 0.5 |
| Compound B | AVI | Strain D | 0.25 |
| Compound B | NAC | Strain D | < 0.015 |
| Compound B | ZID | Strain D | < 0.015 |
| Compound C | N/A | Strain D | 4 |
| Compound C | NAC | Strain D | 0.03 |
| Compound C | ZID | Strain D | 0.03 |
| Compound C | VAB | Strain D | 2 |
| Compound D | N/A | Strain D | 1 |
| Compound D | AVI | Strain D | 0.5 |
| Compound D | NAC | Strain D | 0.06 |
| Compound D | ZID | Strain D | 0.03 |
| Compound E | N/A | Strain D | 0.25 |
| Compound E | AVI | Strain D | 0.12 |
| Compound E | NAC | Strain D | < 0.015 |
| Compound E | ZID | Strain D | < 0.015 |
| CAZ | N/A | Strain D | > 128 |
| CAZ | AVI | Strain D | > 128 |

[Table 47]

| Test compound | β-Lactamase inhibitor | Strain | MIC (μg/mL) |
|---|---|---|---|
| Compound A | N/A | Strain E | 1 |
| Compound A | SBT | Strain E | 0.5 |
| Compound A | TAZ | Strain E | 0.25 |
| Compound A | AVI | Strain E | 0.06 |
| Compound A | VAB | Strain E | 0.12 |
| Compound B | N/A | Strain E | 1 |
| Compound B | SBT | Strain E | 0.5 |
| Compound B | TAZ | Strain E | 0.12 |
| Compound B | AVI | Strain E | 0.03 |
| Compound B | VAB | Strain E | 0.03 |
| Compound C | N/A | Strain E | 2 |
| Compound C | TAZ | Strain E | 1 |
| Compound C | AVI | Strain E | 0.25 |
| Compound C | VAB | Strain E | 0.5 |
| Compound D | N/A | Strain E | 0.5 |
| Compound D | SBT | Strain E | 0.25 |
| Compound D | TAZ | Strain E | 0.06 |
| Compound D | CVA | Strain E | 0.25 |
| Compound D | AVI | Strain E | 0.03 |
| Compound D | VAB | Strain E | 0.12 |
| Compound E | N/A | Strain E | 1 |
| Compound E | SBT | Strain E | 0.5 |
| Compound E | TAZ | Strain E | 0.25 |
| Compound E | AVI | Strain E | 0.06 |
| Compound E | VAB | Strain E | 0.12 |

For all the bacterial strain, higher antibacterial activity was exhibited in a case where the compounds A, B, C, D, and E and a β-lactamase inhibitor were used in combination, than in a case where the β-lactamase inhibitor was not used in combination with these compounds.

Test Example 7 Evaluation test for antibacterial activity exhibited in case where plurality of β-lactamase inhibitors and test compound are used in combination

By using a broth microdilution method, the combinational effect of a test compound and a plurality of β-lactamase inhibitors was evaluated.
The evaluation test for antibacterial activity was performed in the same manner as in Test Example 1.
As test compounds, the compounds A and B were used.
As β-lactamase inhibitors, SBT (commercially available product) and AVI (commercially available product) were used.
As a control compound, MEPM was used.
As bacterial strains, Carbapenem-resistant Acinetobacter A514 and A515 strains were used.
Table 48 shows the antibacterial activity exhibited against the Carbapenem-resistant Acinetobacter A514 and A515 strains in a case where the compound A, SBT, and AVI are used in combination.

[Table 48]

| Composition | MIC (μg/mL) |
|---|---|
| SBT | > 32 |
| AVI | > 32 |
| Compound A + SBT + AVI (1:1:1) | 0.25 |
| MEPM | > 32 |

Table 49 shows the antibacterial activity exhibited against the Carbapenem-resistant Acinetobacter A515 strain in a case where the compound B, SBT, and AVI are used in combination.

[Table 49]

| Composition | MIC (μg/mL) |
|---|---|
| SBT | > 32 |
| AVI | > 32 |
| Compound B + SBT + AVI (1:1:1) | 0.25 |
| MEPM | > 32 |

High antibacterial activity was exhibited against all the bacterial strains in a case where the compounds A and B and two β-lactamase inhibitors were used in combination.

Test example 8

An antibacterial activity evaluation test was performed in the same manner as in Test Example 1.

As test compounds, the compounds obtained in Examples 143, 144, 147, 153, 154, and 156 were used.

The results are shown in Table 50.

[Table 50]

| Exampl e No. | Pseudo monas aerugino sa ATCC 27853 | Pseudo monas aerugino sa S-3028 | Pseudo monas aerugino sa S-2838 | Pseudo monas aerugino sa S-3779 | Pseudo monas aerugino sa S-3759 | Escherich ia coli TK-1747 | Klebsiella pneumoniae Y-1007 | Klebsiella pneumoniae Y-995 | Klebsiella pneumoniae Y-1062 |
|---|---|---|---|---|---|---|---|---|---|
| 143 | 0.5 | 0.12 | 0.5 | 0.25 | 1 | 0.12 | < 0.06 | 0.12 | 2 |
| 144 | 0.25 | < 0.12 | 32 | 0.25 | 1 | < 0.12 | < 0.12 | < 0.12 | 0.5 |
| 147 | 1 | 0.5 | 4 | 1 | 1 | 1 | < 0.06 | 0.25 | 4 |
| 153 | 0.5 | 0.12 | 0.5 | 1 | 2 | 0.5 | < 0.06 | 0.25 | 4 |
| 154 | 2 | 0.5 | 4 | 2 | 4 | 0.5 | 0.25 | 0.25 | 2 |
| 156 | 2 | 0.5 | 1 | 1 | 2 | 0.12 | < 0.06 | 0.12 | 0.5 |

Test Example 9

An antibacterial activity evaluation test was performed in the same manner as in Test Example 6.

As test compounds, a compound F and a compound G were used.

As the bacterial strain, the strains B, C, and D producing β-lactamase and a Klebsiella pneumoniae Y-1069 strain (hereinafter, called strain F) producing OXA-48 and CTX-M-15 were used.

The results are shown in Tables 51 and 52.

[Table 51]

| Test compound | β-Lactamase inhibitor | Strain | MIC (μg/mL) |
|---|---|---|---|
| Compound F | N/A | Strain B | 2 |
| Compound F | CVA | Strain B | 1 |
| Compound F | AVI | Strain B | 0.5 |
| Compound F | NAC | Strain B | 0.03 |
| Compound F | ZID | Strain B | 0.03 |
| Compound F | VOB | Strain B | 1 |
| Compound F | N/A | Strain C | 1 |
| Compound F | AVI | Strain C | 0.25 |
| Compound F | NAC | Strain C | 0.06 |
| Compound F | ZID | Strain C | < 0.015 |
| Compound F | N/A | Strain D | 0.5 |
| Compound F | NAC | Strain D | < 0.015 |
| Compound F | ZID | Strain D | < 0.015 |
| Compound F | N/A | Strain F | 8 |
| Compound F | SBT | Strain F | 4 |
| Compound F | TAZ | Strain F | 4 |
| Compound F | CVA | Strain F | 4 |
| Compound F | AVI | Strain F | 0.25 |
| Compound F | NAC | Strain F | 0.12 |
| Compound F | ZID | Strain F | < 0.015 |

[Table 52]

| Test compound | β-Lactamase inhibitor | Strain | MIC (μg/mL) |
|---|---|---|---|
| Compound G | N/A | Strain B | 1 |
| Compound G | CVA | Strain B | 0.25 |
| Compound G | AVI | Strain B | 0.25 |
| Compound G | NAC | Strain B | < 0.015 |
| Compound G | ZID | Strain B | 0.03 |
| Compound G | N/A | Strain C | 1 |
| Compound G | AVI | Strain C | 0.06 |
| Compound G | NAC | Strain C | 0.06 |
| Compound G | ZID | Strain C | < 0.015 |
| Compound G | N/A | Strain D | 0.5 |
| Compound G | SBT | Strain D | 0.25 |
| Compound G | TAZ | Strain D | 0.125 |
| Compound G | CVA | Strain D | 0.25 |
| Compound G | AVI | Strain D | 0.25 |
| Compound G | NAC | Strain D | < 0.015 |
| Compound G | ZID | Strain D | < 0.015 |
| Compound G | N/A | Strain F | 4 |
| Compound G | SBT | Strain F | 2 |
| Compound G | TAZ | Strain F | 2 |

(continued)

| Test compound | β-Lactamase inhibitor | Strain | MIC (μg/mL) |
|---|---|---|---|
| Compound G | CVA | Strain F | 2 |
| Compound G | AVI | Strain F | 0.25 |
| Compound G | NAC | Strain F | 0.06 |
| Compound G | ZID | Strain F | 0.03 |

For all the bacterial strain, higher antibacterial activity was exhibited in a case where the compounds F and G and a β-lactamase inhibitor were used in combination, than in a case where the β-lactamase inhibitor was not used in combination with these compounds.

[Industrial applicability]

The pharmaceutical composition and the kit of the present application have strong antibacterial activity against Gram-negative bacteria such as Pseudomonas aeruginosa and/or drug-resistant Gram-negative bacteria including multidrug-resistant Pseudomonas aeruginosa, and are useful for treating infections caused by these bacteria.

## Claims

**1.** A pharmaceutical composition comprising:

a compound represented by General Formula [1] or a salt thereof; and
one or more compounds selected from a β-lactamase inhibitory compound and an antibacterial compound or salts thereof,

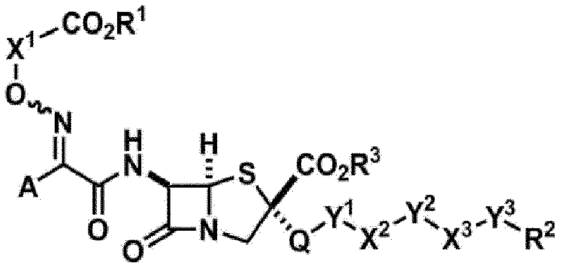

[1]

"in the formula,
$R^1$ represents a hydrogen atom;
$R^2$ represents an aryl group which may be substituted;
$R^3$ represents a hydrogen atom;
$X^1$ represents a $C_{1-6}$ alkylene group which may be substituted, a $C_{2-6}$ alkenylene group which may be substituted, a $C_{2-6}$ alkynylene group which may be substituted, a divalent cyclic hydrocarbon group which may be substituted, or a divalent monocyclic saturated heterocyclic group which may be substituted;
A represents a monocyclic heterocyclic group which may be substituted;
Q represents a divalent cyclic amino group which may be substituted or a divalent heterocyclic group which may be substituted;
$Y^1$ represents a $C_{1-6}$ alkylene group which may be substituted, a $C_{2-6}$ alkenylene group which may be substituted, a $C_{2-6}$ alkynylene group which may be substituted, a group represented by Formula -N=CH-CH=N-, a group represented by Formula -N=CH-CH=N-O-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula -NHC(=O)C(=O)N(OH)-, a group represented by Formula $-NHCH_2C(=O)-$, a group represented by Formula $-NHS(=O)_2NH-C(=O)-$, a group represented by Formula $-NHC(=O)NHS(=O)_2-$, or a bond;
$X^2$ represents a group represented by General Formula $-NR^4-$ (where $R^4$ represents a hydrogen atom, a carbamoyl group, a $C_{1-6}$ alkyl group which may be substituted, or a hydroxyl group which may be protected),

a group represented by General Formula $-N^+R^5R^6-$ (where $R^5$ and $R^6$ are the same as or different from each other and each represent a $C_{1-6}$ alkyl group which may be substituted, or in combination represent a $C_{2-6}$ alkylene which may be substituted or a $C_{2-6}$ alkenylene group which may be substituted), a group represented by General Formula $-NR^7-C(=O)-NR^8-$ (where $R^7$ and $R^8$ are the same as or different from each other and each represent a hydrogen atom, a $C_{1-6}$ alkyl group which may be substituted, or a hydroxyl group which may be protected), a divalent cyclic amino group which may be substituted, a divalent heterocyclic group which may be substituted, or a bond;

$Y^2$ represents a $C_{1-6}$ alkylene group which may be substituted, or a bond;

$X^3$ represents a group represented by General Formula $-NR^9-$ (where $R^9$ represents a hydrogen atom); and

$Y^3$ represents a group represented by Formula -C(=O)-, a group represented by Formula -C(=O)-C(=O)-, or a group represented by General Formula $-C(=O)-C(=NR^{10a})-$(where $R^{10a}$ represents a hydroxyl group).

**2.** The pharmaceutical composition according to claim 1,
wherein $X^1$ represents a $C_{1-6}$ alkylene group which may be substituted or a divalent cyclic hydrocarbon group which may be substituted.

**3.** The pharmaceutical composition according to claims 1 or 2,
wherein Q represents a divalent heterocyclic group which may be substituted.

**4.** The pharmaceutical composition according to any one of claims 1 to 3,
wherein $Y^1$ represents a $C_{1-6}$ alkylene group which may be substituted, a group represented by Formula -N=CH-CH=N-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula $-NHCH_2C(=O)-$, or a bond.

**5.** The pharmaceutical composition according to any one of claims 1 to 4,
wherein $X^2$ represents a group represented by General Formula $-NR^{4a}-$ (where $R^{4a}$ represents a hydrogen atom or a carbamoyl group), a group represented by General Formula $-N^+R^{5a}R^{6a}-$ (where $R^{5a}$ and $R^{6a}$ in combination represent a $C_{2-6}$ alkylene group which may be substituted), a group represented by General Formula $-NR^{7a}-C(=O)-NR^{8a}-$ (where $R^{7a}$ and $R^{8a}$ each represent a hydrogen atom), a divalent cyclic amino group which may be substituted, a divalent heterocyclic group which may be substituted, or a bond.

**6.** The pharmaceutical composition according to any one of claims 1 to 5,

wherein $R^2$ represents a phenyl group which may be substituted,

A represents a monocyclic nitrogen and sulfur-containing heterocyclic group which may be substituted;

Q represents a divalent monocyclic heterocyclic group which may be substituted;

$Y^1$ represents a group represented by Formula -NHC(=O)-, a group represented by Formula -NHC(=O)C(=O)NH-, or a bond;

$X^2$ represents a group represented by General Formula $-NR^{4b}-$ (where $R^{4b}$ represents a hydrogen atom) or a bond;

$Y^2$ represents a $C_{1-3}$ alkylene group or a bond; and

$Y^3$ represents a group represented by Formula -C(=O)- or a group represented by Formula -C(=O)-C(=O)-.

**7.** The pharmaceutical composition according to claim 1,
wherein the compound represented by General Formula [1] is a compound selected from (3R,5R,6R)-6-((Z)-2-(2-aminothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-a zabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thi a-1-azabicyclo[3.2.0]heptane-3-carboxylic acid, (3R,5R,6R)-6-((Z)-2-(2-amino-5-chlorothiazol-4-yl)-2-(((2-carboxypropan-2-yl)oxy)imino)ac etamido)-3-(3-(2-(2-chloro-3,4-dihydroxyphenyl)-2-oxoacetamido)-2-oxoimidazolidin-1-yl)-7 -oxo-4-thia-1-azabicyclo [3.2.0]heptane-3-carboxylic acid, (3R,5R,6R)-6-((Z)-2-(2-aminothiazol-4-yl)-2-(((2-carboxypropan-2-yl)oxy)imino) acetamido)-3-((S)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy) imino)acetamido)-3-((R)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetatamido)-2,3-dioxopiperazin-1-yl)-7-oxo-4-thia-1-azabicyclo[3.2.0]heptane-3-carboxylic acid, and (3R,5R,6R)-6-((Z)-2-(2-amino-5-chlorothiazol-4-yl)-2-(((2-carboxypropan-2-yl)oxy)imino)ac etamido)-3-(3-(2-(2-(2-(2-chloro-3,4-dihydroxyphenyl)-2-oxoacetyl)hydradienyl)-2-oxoaceta mido)-2-oxoimidazolidin-1-yl)-7-oxo-4-thia-1-azabicyclo[3.2.0]heptane-3-carboxylic acid.

**8.** The pharmaceutical composition according to any one of claims 1 to 7, wherein the one or more compounds selected from a β-lactamase inhibitory compound and an antibacterial compound or salts thereof are a β-lactamase inhibitory compound or a salt thereof.

**9.** The pharmaceutical composition according to any one of claims 1 to 8, wherein the β-lactamase inhibitory compound is one or more compounds selected from a diazabicyclo[3.2.1]octane-type inhibitory compound, a boronic acid-type inhibitory compound, and a clavam-type inhibitory compound.

**10.** The pharmaceutical composition according to any one of claims 1 to 9, wherein the β-lactamase inhibitory compound is one or more compounds selected from avibactam, nacubactam, relebactam, zidebactam, vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid, sulbactam, tazobactam, clavulanic acid, (1aR,7bS)-5-fluoro-2-hydroxy-1,1a,2,7b-tetrahydrocyclopropan[c][1,2]benzoxaborinine-4-car boxylic acid, and (25,3S,5R)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide.

**11.** The pharmaceutical composition according to any one of claims 1 to 10 for use in treating infections caused by Gram-negative bacteria.

**12.** An agent for use in treating infections caused by Gram-negative bacteria, comprising: the pharmaceutical composition according to any one of claims 1 to 10.

**13.** A kit for use in treating infections, comprising:

a compound represented by General Formula [1] or a salt thereof, and
one or more compounds selected from a β-lactamase inhibitory compound and an antibacterial compound, or salts thereof,

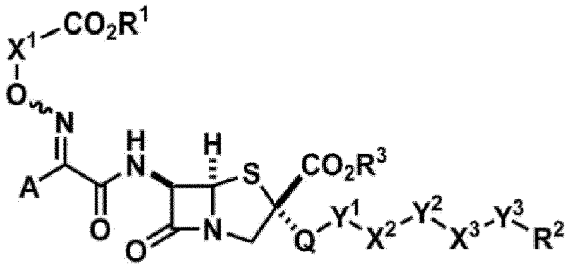

[1]

"in the formula,
$R^1$ represents a hydrogen atom;
$R^2$ represents an aryl group which may be substituted;
$R^3$ represents a hydrogen atom;
$X^1$ represents a $C_{1-6}$ alkylene group which may be substituted, a $C_{2-6}$ alkenylene group which may be substituted, a $C_{2-6}$ alkynylene group which may be substituted, a divalent cyclic hydrocarbon group which may be substituted, or a divalent monocyclic saturated heterocyclic group which may be substituted;
A represents a monocyclic heterocyclic group which may be substituted;
Q represents a divalent cyclic amino group which may be substituted or a divalent heterocyclic group which may be substituted;
$Y^1$ represents a $C_{1-6}$ alkylene group which may be substituted, a $C_{2-6}$ alkenylene group which may be substituted, a $C_{2-6}$ alkynylene group which may be substituted, a group represented by Formula -N=CH-CH=N-, a group represented by Formula -N=CH-CH=N-O-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula -NHC(=O)C(=O)N(OH)-, a group represented by Formula $-NHCH_2C(=O)-$, a group represented by Formula $-NHS(=O)_2NH-C(=O)-$, a group represented by Formula $-NHC(=O)NHS(=O)_2-$, or a bond;
$X^2$ represents a group represented by General Formula $-NR^4-$ (where $R^4$ represents a hydrogen atom, a carbamoyl group, a $C_{1-6}$ alkyl group which may be substituted, or a hydroxyl group which may be protected), a group represented by General Formula $-N^+R^5R^6-$ (where $R^5$ and $R^6$ are the same as or different from each other and each represent a $C_{1-6}$ alkyl group which may be substituted, or in combination represent a $C_{2-6}$ alkylene group which may be substituted or a $C_{2-6}$ alkenylene group which may be substituted), a group represented by General Formula $-NR^7-C(=O)-NR^8-$ (where $R^7$ and $R^8$ are the same as or different from each other and each represent a hydrogen atom, a $C_{1-6}$ alkyl group which may be substituted, or a hydroxyl group which may be protected), a divalent cyclic amino group which may be substituted, a divalent heterocyclic group which may be substituted, or a bond;
$Y^2$ represents a $C_{1-6}$ alkylene group which may be substituted, or a bond;
$X^3$ represents a group represented by General Formula $-NR^9-$ (where $R^9$ represents a hydrogen atom); and
$Y^3$ represents a group represented by Formula -C(=O)-, a group represented by Formula -C(=O)-C(=O)-, or a group represented by General Formula $-C(=O)-C(=NR^{10a})-$(where $R^{10a}$ represents a hydroxyl group).

**14.** The kit for use according to claim 13,

wherein $R^2$ represents an aryl group which may be substituted;
wherein A represents a monocyclic heterocyclic group which may be substituted;
wherein $X^1$ represents a $C_{1-6}$ alkylene group which may be substituted or a divalent cyclic hydrocarbon group which may be substituted;
wherein Q represents a divalent heterocyclic group which may be substituted;
wherein $Y^1$ represents a $C_{1-6}$ alkylene group which may be substituted, a group represented by Formula -N=CH-CH=N-, a group represented by Formula $-N=CH-CH_2-$, a group represented by Formula -N=CHC(=O)-, a group represented by Formula -NHC(=O)-, a group represented by Formula $-NHC(=O)CH_2-$, a group represented by Formula -NHC(=O)NH-, a group represented by Formula -NHC(=O)NH-O-, a group represented by Formula -NHC(=O)C(=O)NH-, a group represented by Formula $-NHCH_2C(=O)-$, or a bond;
wherein $X^2$ represents a group represented by General Formula $-NR^{4a}-$ (where $R^{4a}$ represents a hydrogen atom or a carbamoyl group), a group represented by General Formula $-N^+R^{5a}R^{6a}-$ (where $R^{5a}$ and $R^{6a}$ in combination represent a $C_{2-6}$ alkylene group which may be substituted), a group represented by General Formula $-NR^{7a}-C(=O)-NR^{8a}-$ (where $R^{7a}$ and $R^{8a}$ each represent a hydrogen atom), a divalent cyclic amino group which may be substituted, a divalent heterocyclic group which may be substituted, or a bond;
wherein $Y^2$ represents a $C_{1-6}$ alkylene group which may be substituted or a bond;
wherein $X^3$ represents a group represented by General Formula $-NR^{9a}-$ (where $R^{9a}$ represents a hydrogen atom);
wherein $R^3$ represents a hydrogen atom;
wherein $R^1$ represents a hydrogen atom.

**15.** The kit for use according to claim 13 or 14,

wherein $R^2$ represents a phenyl group which may be substituted;
A represents a monocyclic nitrogen and sulfur-containing heterocyclic group which may be substituted;
Q represents a divalent monocyclic heterocyclic group which may be substituted;
$Y^1$ represents a group represented by Formula -NHC(=O)-, a group represented by Formula -NHC(=O)C(=O)NH-, or a bond;
$X^2$ represents a group represented by General Formula $-NR^{4b}-$ (where $R^{4b}$ represents a hydrogen atom) or a bond;
$Y^2$ represents a $C_{1-3}$ alkylene group or a bond; and
$Y^3$ represents a group represented by Formula -C(=O)- or a group represented by Formula -C(=O)-C(=O)-.

**16.** The kit for use according to claim 13,
wherein the compound represented by General Formula [1] is a compound selected from (3R,5R,6R)-6-((Z)-2-(2-aminothiazol-4-yl)-2-((1-carboxycyclobutoxy)imino)acetamido)-3-(3-(2-(2-chloro-3,4-dihydroxyphenyl)-2-oxoacetamido)-2-oxoimidazolidin-1-yl)-7-oxo-4-thia-1-a zabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(3-(2-(2-chloro-3,4-dihydroxyphenyl)-2-oxoacetamido)-2-oxoimidazolidin-1-yl)-7-oxo-4-thi a-1-azabicyclo[3.2.0]heptane-3-carboxylic acid, (3R,5R,6R)-6-((Z)-2-(2-amino-5-chlorothiazol-4-yl)-2-(((2-carboxypropan-2-yl)oxy)imino)ac etamido)-3-(3-(2-(2-chloro-3,4-dihydroxyphenyl)-2-oxoacetamido)-2-oxoimidazolidin-1-yl)-7 -oxo-4-thia-1-azabicyclo [3.2.0]heptane-3-carboxylic acid, (3R,5R,6R)-6-((Z)-2-(2-aminothiazol-4-yl)-2-(((2-carboxypropan-2-yl)oxy)imino) acetamido)-3-((S)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy) imino)acetamido)-3-((R)-3-(2-(2-chloro-3,4-dihydroxyphenyl)-2-oxoacetamido)-2-oxopyrrolidin-1-yl)-7-oxo-4-t hia-1-azabicyclo[3.2.0]heptane-3-carboxylic acid, (3R,5R,6R)-6-((Z)-2-(2-aminothiazol-4-yl)-2-(((2-carboxypropan-2-yl)oxy)imino)acetamido)-3-(4-(2-(2-chloro-3,4-dihydroxyphenyl)-2-oxoacetatamido)-2,3-dioxopiperazin-1-yl)-7-oxo-4-thia-1-azabicyclo[3.2.0]heptane-3-carboxylic acid, and (3R,5R,6R)-6-((Z)-2-(2-amino-5-chlorothiazol-4-yl)-2-(((2-carboxypropan-2-yl)oxy)imino)ac etamido)-3-(3-(2-(2-(2-(2-chloro-3,4-dihydroxyphenyl)-2-oxoacetyl)hydradienyl)-2-oxoaceta mido)-2-oxoimidazolidin-1-yl)-7-oxo-4-thia-1-azabicyclo[3.2.0]heptane-3-carboxylic acid.

**17.** The kit for use according to any one of claims 13 to 16, wherein the one or more compounds selected from a β-lactamase inhibitory compound and an antibacterial compound or salts thereof are a β-lactamase inhibitory compound or a salt thereof.

**18.** The kit for use according to any one of claims 13 to 17, wherein the β-lactamase inhibitory compound is one or more compounds selected from a diazabicyclo[3.2.1]octane-type inhibitory compound, a boronic acid-type inhibitory compound, and a clavam-type inhibitory compound.

**19.** The kit for use according to any one of claims 13 to 17, wherein the β-lactamase inhibitory compound is one or more compounds selected from avibactam, nacubactam, relebactam, zidebactam, vaborbactam, (3R)-3-(2-[trans-4-[(2-aminoethyl) amino]cyclohexyl]acetamide)-2-hydroxy-3,4-dihydro-2H-1,2-benzoxaborinine-8-carboxylic acid, sulbactam, tazobactam, clavulanic acid, (1aR,7bS)-5-fluoro-2-hydroxy-1,1a,2,7b-tetrahydrocyclopropan[c][1,2]benzoxaborinine-4-car boxylic acid, and (25,3 S,SR)-3-methyl-3-[(3-methyl-1H-1,2,3-triazol-3-ium-1-yl)methyl]-7-oxo-4-thia-1-azabic yclo[3.2.0]heptane-2-carboxylate 4,4-dioxide.

## Patentansprüche

**1.** Pharmazeutische Zusammensetzung, umfassend:

eine Verbindung, die durch allgemeine Formel [1] dargestellt wird, oder ein Salz davon; und
eine oder mehrere Verbindungen, die aus einer β-Lactamase-Hemmverbindung und einer antibakteriellen Verbindung oder Salzen davon ausgewählt werden,

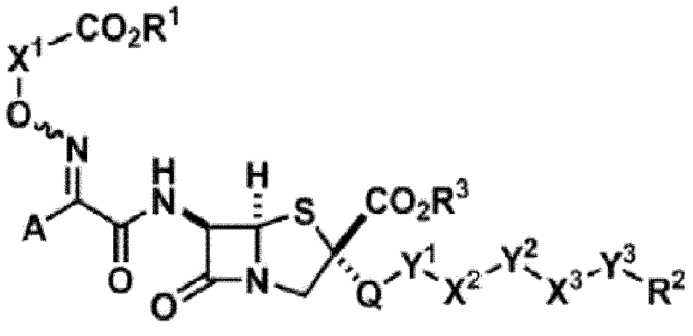

[1]

wobei in der Formel
$R^1$ ein Wasserstoffatom darstellt;
$R^2$ eine Arylgruppe darstellt, die substituiert sein kann;
$R^3$ ein Wasserstoffatom darstellt;
$X^1$ eine $C_{1-6}$-Alkylengruppe, die substituiert sein kann, eine $C_{2-6}$-Alkenylengruppe, die substituiert sein kann, eine $C_{2-6}$-Alkinylengruppe, die substituiert sein kann, eine divalente zyklische Kohlenwasserstoffgruppe, die substituiert sein kann, oder eine divalente monozyklische gesättigte heterozyklische Gruppe, die substituiert sein kann, darstellt;
A eine monozyklische heterozyklische Gruppe darstellt, die substituiert sein kann;
Q eine divalente zyklische Aminogruppe, die substituiert sein kann, oder eine divalente heterozyklische Gruppe,

die substituiert sein kann, darstellt;

$Y^1$ eine $C_{1-6}$-Alkylengruppe, die substituiert sein kann, eine $C_{2-6}$-Alkenylengruppe, die substituiert sein kann, eine $C_{2-6}$-Alkinylengruppe, die substituiert sein kann, eine Gruppe, die durch Formel -N=CH-CH=N- dargestellt wird, eine Gruppe, die durch Formel -N=CH-CH=N-O- dargestellt wird, eine Gruppe, die durch Formel -N=CH-$CH_2$- dargestellt wird, eine Gruppe, die durch Formel -N=CHC(=O)- dargestellt wird, eine Gruppe, die durch Formel -NHC(=O)- dargestellt wird, eine Gruppe, die durch Formel -NHC(=O)$CH_2$- dargestellt wird, eine Gruppe, die durch Formel - NHC(=O)NH- dargestellt wird, eine Gruppe, die durch Formel -NHC(=O)NH-O-dargestellt wird, eine Gruppe, die durch Formel -NHC(=O)C(=O)NH- dargestellt wird, eine Gruppe, die durch Formel -NHC(=O)C(=O)N(OH)- dargestellt wird, eine Gruppe, die durch Formel -NH$CH_2$C(=O)- dargestellt wird, eine Gruppe, die durch Formel - NHS$(=O)_2$NHC(=O)- dargestellt wird, eine Gruppe, die durch Formel - NHC(=O) NHS$(=O)_2$- dargestellt wird, oder eine Bindung darstellt;

$X^2$ eine Gruppe, die durch allgemeine Formel -$NR^4$- dargestellt wird (wobei $R^4$ ein Wasserstoffatom, eine Carbamoylgruppe, eine $C_{1-6}$-Alkylgruppe, die substituiert sein kann, oder eine Hydroxylgruppe, die geschützt sein kann, darstellt), eine Gruppe, die durch allgemeine Formel -$N^+R^5R^6$- dargestellt wird (wobei $R^5$ und $R^6$ gleich oder unterschiedlich zueinander sind und jeweils eine $C_{1-6}$-Alkylgruppe, die substituiert sein kann, darstellen oder in Kombination eine $C_{2-6}$-Alkylengruppe, die substituiert sein kann, oder eine $C_{2-6}$-Alkenylengruppe, die substituiert sein kann, darstellen), eine Gruppe, die durch allgemeine Formel -$NR^7$-C(=O)-$NR^8$- dargestellt wird (wobei $R^7$ und $R^8$ gleich oder unterschiedlich zueinander sind und jeweils ein Wasserstoffatom, eine $C_{1-6}$-Alkylgruppe, die substituiert sein kann, oder eine Hydroxylgruppe, die geschützt sein kann, darstellen), eine divalente zyklische Aminogruppe, die substituiert sein kann, eine divalente heterozyklische Gruppe, die substituiert sein kann, oder eine Bindung darstellt;

$Y_2$ eine $C_{1-6}$-Alkylengruppe, die substituiert sein kann, oder eine Bindung darstellt;

$X^3$ eine Gruppe darstellt, die durch allgemeine Formel -$NR^9$- dargestellt wird (wobei $R^9$ ein Wasserstoffatom darstellt); und

$Y^3$ eine Gruppe, die durch Formel -C(=O)- dargestellt wird, eine Gruppe, die durch Formel -C(=O)-C(=O)- dargestellt wird, oder eine Gruppe, die durch allgemeine Formel -C(=O)-C(=$NR^{10a}$)- dargestellt wird (wobei $R^{10a}$ eine Hydroxylgruppe darstellt), darstellt.

**2.** Pharmazeutische Zusammensetzung nach Anspruch 1, wobei $X^1$ eine $C_{1-6}$-Alkylengruppe, die substituiert sein kann, oder eine divalente zyklische Kohlenwasserstoffgruppe, die substituiert sein kann, darstellt.

**3.** Pharmazeutische Zusammensetzung nach Anspruch 1 oder 2, wobei Q eine divalente heterozyklische Gruppe darstellt, die substituiert sein kann.

**4.** Pharmazeutische Zusammensetzung nach einem der Ansprüche 1 bis 3, wobei $Y^1$ eine $C_{1-6}$-Alkylengruppe, die substituiert sein kann, eine Gruppe, die durch Formel -N=CH-CH=N- dargestellt wird, eine Gruppe, die durch Formel -N=CH-$CH_2$-dargestellt wird, eine Gruppe, die durch Formel -N=CHC(=O)- dargestellt wird, eine Gruppe, die durch Formel -NHC(=O)- dargestellt wird, eine Gruppe, die durch Formel -NHC(=O)$CH_2$- dargestellt wird, eine Gruppe, die durch Formel -NHC(=O)NH-dargestellt wird, eine Gruppe, die durch Formel -NHC(=O)NH-O- dargestellt wird, eine Gruppe, die durch Formel -NHC(=O)C(=O)NH- dargestellt wird, eine Gruppe, die durch Formel -NH$CH_2$C(=O)- dargestellt wird, oder eine Bindung darstellt.

**5.** Pharmazeutische Zusammensetzung nach einem der Ansprüche 1 bis 4, wobei $X^2$ eine Gruppe, die durch allgemeine Formel -$NR4^a$- dargestellt wird (wobei $R4^a$ ein Wasserstoffatom oder eine Carbamoylgruppe darstellt), eine Gruppe, die durch allgemeine Formel -$N^+R^{5a}R^{6a}$- dargestellt wird (wobei $R^{5a}$ und $R^{6a}$ in Kombination eine $C_{2-6}$-Alkylengruppe, die substituiert sein kann, darstellen), eine Gruppe, die durch allgemeine Formel -$NR^{7a}$-C(=O)-$NR^{8a}$- dargestellt wird (wobei $R^{7a}$ und $R^{8a}$ jeweils ein Wasserstoffatom darstellen), eine divalente zyklische Aminogruppe, die substituiert sein kann, eine divalente heterozyklische Gruppe, die substituiert sein kann, oder eine Bindung darstellt.

**6.** Pharmazeutische Zusammensetzung nach einem der Ansprüche 1 bis 5,

wobei $R^2$ eine Phenylgruppe darstellt, die substituiert sein kann;

A eine monozyklische stickstoff- und schwefelhaltige heterozyklische Gruppe darstellt, die substituiert sein kann;

Q eine divalente monozyklische heterozyklische Gruppe darstellt, die substituiert sein kann;

$Y^1$ eine Gruppe, die durch Formel -NHC(=O)- dargestellt wird, eine Gruppe, die durch Formel -NHC(=O)C(=O) NH- dargestellt wird, oder eine Bindung darstellt;

$X^2$ eine Gruppe, die durch allgemeine Formel $-NR^{4b}-$ dargestellt wird (wobei $R^{4b}$ ein Wasserstoffatom darstellt) oder eine Bindung darstellt;
$Y^2$ eine $C_{1-3}$-Alkylengruppe oder eine Bindung darstellt; und
$Y^3$ eine Gruppe, die durch Formel -C(=O)- dargestellt wird, oder eine Gruppe, die durch Formel -C(=O)-C(=O)- dargestellt wird, darstellt.

**7.** Pharmazeutische Zusammensetzung nach Anspruch 1,
wobei die durch allgemeine Formel [1] dargestellte Verbindung eine Verbindung ist, die aus (3R,5R,6R)-6-((Z)-2-(2-Aminothiazol-4-yl)-2-((1-Carboxycyclobutoxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Amino-5-chlorothiazol-4-yl)-2-(((2-Carboxypropan-2-yl)oxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-((S)-3-(2-(2-Chloro-3,4-dihydroxyphenyl)-2-oxoacetamido)-2-oxopyrrolidin-1-yl)-7-oxo-4-thia-1-azabicyclo [3.2.0]heptan-3-carbonsäure, (3R,5R,6R)-6-((Z)-2-(2-Aminothiazol-4-yl)-2-(((2-Carboxypropan-2-yl)oxy)imino) acetamido)-3-((R)-3-(2-(2-Chloro-3,4-dihydroxyphenyl)-2-oxoacetamido)-2-oxopyrrolidin-1-yl)-7-oxo-4-thia-1-azabicyclo [3 .2.0]heptan-3-carbonsäure, (3R,5R,6R)-6-((Z)-2-(2-Aminothiazol-4-yl)-2-(((2-Carboxypropan-2-yl)oxy) imino)acetamido)-3-(4-(2-(2-Chloro-3,4-dihydroxyphenyl)-2-oxoacetamido)-2,3-Dioxopiperazin-1-yl)-7-oxo-4-thia-1-azabicyclo[3.2.0]heptan-3-carbonsäure und (3R,5R,6R)-6-((Z)-2-(2-Amino-5-chlorothiazol-4-yl)-2-(((2-Carboxypropan-2-yl)oxy)imino)acetamido)-3-(3-(2-(2-(2-(2-Chloro-3,4-dihydroxyphenyl)-2-oxoacetyl)hydradienyl)-2-oxoacetamido)-2-oxoimidazolidin-1-yl)-7-oxo-4-thia-1-azabicyclo[3.2.0]heptan-3-carbonsäure ausgewählt wird.

**8.** Pharmazeutische Zusammensetzung nach einem der Ansprüche 1 bis 7,
wobei die eine oder mehrere Verbindungen, die aus einer β-Lactamase-Hemmverbindung und einer antibakteriellen Verbindung oder Salzen davon ausgewählt werden, eine β-Lactamase-Hemmverbindung oder ein Salz davon sind.

**9.** Pharmazeutische Zusammensetzung nach einem der Ansprüche 1 bis 8,
wobei die β-Lactamase-Hemmverbindung eine oder mehrere Verbindungen ist, die aus einer Hemmverbindung des Diazabicyclo[3.2.1]octantyps, einer Hemmverbindung des Boronsäuretyps und einer Hemmverbindung des Clavamtyps ausgewählt werden.

**10.** Pharmazeutische Zusammensetzung nach einem der Ansprüche 1 bis 9,
wobei die β-Lactamase-Hemmverbindung eine oder mehrere Verbindungen ist, die aus Avibactam, Nacubactam, Relebactam, Zidebactam, Vaborbactam, (3R)-3-(2-[trans-4-[(2-Aminoethyl)amino]cyclohexyl]acetamid)-2-hydroxy-3,4-dihydro-2H-1,2-benzoxaborinine-8-carbonsäure, Sulbactam, Tazobactam, Clavulansäure, (1aR,7bS)-5-Fluor-2-hydroxy-1,1a,2,7b-tetrahydrocyclopropan[c][1,2]benzoxaborinine-4-carbonsäure und (2S,3S,5R)-3-Methyl-3-[(3-methyl-1H-1,2,3-triazol-3-ium-1-yl)methyl]-7-oxo-4-thia-1-azabicyclo[3.2.0]heptan-2-carboxylat-4,4-dioxid ausgewählt werden.

**11.** Pharmazeutische Zusammensetzung nach einem der Ansprüche 1 bis 10 zur Verwendung bei Behandlung von Infektionen, die durch gramnegative Bakterien verursacht werden.

**12.** Mittel zur Verwendung bei Behandlung von Infektionen, die durch gramnegative Bakterien verursacht werden, umfassend:
die pharmazeutische Zusammensetzung nach einem der Ansprüche 1 bis 10.

**13.** Kit zur Verwendung bei Behandlung von Infektionen, umfassend:

eine Verbindung, die durch allgemeine Formel [1] dargestellt wird, oder ein Salz davon, und
eine oder mehrere Verbindungen, die aus einer β-Lactamase-Hemmverbindung und einer antibakteriellen Verbindung oder Salzen davon ausgewählt werden,

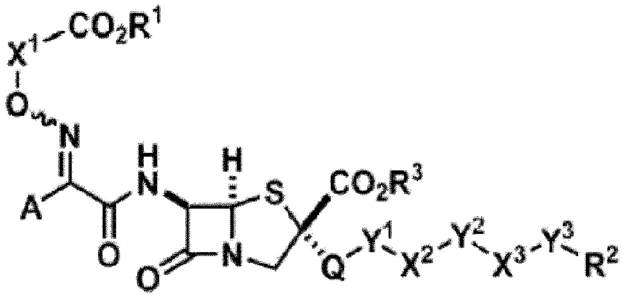

[ 1 ]

wobei in der Formel
$R^1$ ein Wasserstoffatom darstellt;
$R^2$ eine Arylgruppe darstellt, die substituiert sein kann;
$R^3$ ein Wasserstoffatom darstellt;
$X^1$ eine $C_{1-6}$-Alkylengruppe, die substituiert sein kann, eine $C_{2-6}$-Alkenylengruppe, die substituiert sein kann, eine $C_{2-6}$-Alkinylengruppe, die substituiert sein kann, eine divalente zyklische Kohlenwasserstoffgruppe, die substituiert sein kann, oder eine divalente monozyklische gesättigte heterozyklische Gruppe, die substituiert sein kann, darstellt;
A eine monozyklische heterozyklische Gruppe darstellt, die substituiert sein kann;
Q eine divalente zyklische Aminogruppe, die substituiert sein kann, oder eine divalente heterozyklische Gruppe, die substituiert sein kann, darstellt;
$Y^1$ eine $C_{1-6}$-Alkylengruppe, die substituiert sein kann, eine $C_{2-6}$-Alkenylengruppe, die substituiert sein kann, eine $C_{2-6}$-Alkinylengruppe, die substituiert sein kann, eine Gruppe, die durch Formel -N=CH-CH=N- dargestellt wird, eine Gruppe, die durch Formel -N=CH-CH=N-O- dargestellt wird, eine Gruppe, die durch Formel -N=CH-$CH_2$- dargestellt wird, eine Gruppe, die durch Formel -N=CHC(=O)- dargestellt wird, eine Gruppe, die durch Formel -NHC(=O)- dargestellt wird, eine Gruppe, die durch Formel -NHC(=O)$CH_2$- dargestellt wird, eine Gruppe, die durch Formel - NHC(=O)NH- dargestellt wird, eine Gruppe, die durch Formel -NHC(=O)NH-O-dargestellt wird, eine Gruppe, die durch Formel -NHC(=O)C(=O)NH- dargestellt wird, eine Gruppe, die durch Formel -NHC(=O)C(=O)N(OH)- dargestellt wird, eine Gruppe, die durch Formel -NH$CH_2$C(=O)- dargestellt wird, eine Gruppe, die durch Formel - NHS$(=O)_2$NHC(=O)- dargestellt wird, eine Gruppe, die durch Formel - NHC(=O) NHS$(=O)_2$- dargestellt wird, oder eine Bindung darstellt;
$X^2$ eine Gruppe, die durch allgemeine Formel -$NR^4$- dargestellt wird (wobei $R^4$ ein Wasserstoffatom, eine Carbamoylgruppe, eine $C_{1-6}$-Alkylgruppe, die substituiert sein kann, oder eine Hydroxylgruppe, die geschützt sein kann, darstellt), eine Gruppe, die durch allgemeine Formel -$N^+R^5R^6$- dargestellt wird (wobei $R^5$ und $R^6$ gleich oder
unterschiedlich zueinander sind und jeweils eine $C_{1-6}$-Alkylgruppe, die substituiert sein kann, darstellen oder in Kombination eine $C_{2-6}$-Alkylengruppe, die substituiert sein kann, oder eine $C_{2-6}$-Alkenylengruppe, die substituiert sein kann, darstellen), eine Gruppe, die durch allgemeine Formel -$NR^7$-C(=O)-$NR^8$- dargestellt wird (wobei $R^7$ und
$R^8$ gleich oder unterschiedlich zueinander sind und jeweils ein Wasserstoffatom, eine $C_{1-6}$-Alkylgruppe, die substituiert sein kann, oder eine Hydroxylgruppe, die geschützt sein kann, darstellen), eine divalente zyklische Aminogruppe, die substituiert sein kann, eine divalente heterozyklische Gruppe, die substituiert sein kann, oder eine Bindung darstellt;
$Y^2$ eine $C_{1-6}$-Alkylengruppe, die substituiert sein kann, oder eine Bindung darstellt;
$X^3$ eine Gruppe darstellt, die durch allgemeine Formel -$NR^9$- dargestellt wird (wobei $R^9$ ein Wasserstoffatom darstellt); und
$Y^3$ eine Gruppe, die durch Formel -C(=O)- dargestellt wird, eine Gruppe, die durch Formel -C(=O)-C(=O)- dargestellt wird, oder eine Gruppe, die durch allgemeine Formel -C(=O)-C(=$NR^{10a}$)- dargestellt wird (wobei $R^{10a}$ eine Hydroxylgruppe darstellt), darstellt.

**14.** Kit zur Verwendung nach Anspruch 13,

wobei $R^2$ eine Arylgruppe darstellt, die substituiert sein kann;
wobei A eine monozyklische heterozyklische Gruppe darstellt, die substituiert sein kann;
wobei $X^1$ eine $C_{1-6}$-Alkylengruppe, die substituiert sein kann, oder eine divalente zyklische Kohlenwasserstoffgruppe, die substituiert sein kann, darstellt;
wobei Q eine divalente heterozyklische Gruppe darstellt, die substituiert sein kann; wobei $Y^1$ eine $C_{1-6}$-Alkylen-

gruppe, die substituiert sein kann, eine Gruppe, die durch Formel -N=CH-CH=N- dargestellt wird, eine Gruppe, die durch Formel -N=CH-$CH_2$-dargestellt wird, eine Gruppe, die durch Formel -N=CHC(=O)- dargestellt wird, eine Gruppe, die durch Formel -NHC(=O)- dargestellt wird, eine Gruppe, die durch Formel -NHC(=O)$CH_2$-dargestellt wird, eine Gruppe, die durch Formel -NHC(=O)NH-dargestellt wird, eine Gruppe, die durch Formel -NHC(=O)NH-O- dargestellt wird, eine Gruppe, die durch Formel -NHC(=O)C(=O)NH- dargestellt wird, eine Gruppe, die durch Formel -NH$CH_2$C(=O)- dargestellt wird, oder eine Bindung darstellt;

wobei $X^2$ eine Gruppe, die durch allgemeine Formel -NR$4^a$- dargestellt wird (wobei R$4^a$ ein Wasserstoffatom oder eine Carbamoylgruppe darstellt), eine Gruppe, die durch allgemeine Formel -$N^+R^{5a}R^{6a}$- dargestellt wird (wobei $R^{5a}$ und $R^{6a}$ in Kombination eine $C_{2-6}$-Alkylengruppe, die substituiert sein kann, darstellen), eine Gruppe, die durch allgemeine Formel -$NR^{7a}$-C(=O)-$NR^{8a}$- dargestellt wird (wobei $R^{7a}$ und $R^{8a}$ jeweils ein Wasserstoffatom darstellen), eine divalente zyklische Aminogruppe, die substituiert sein kann, eine divalente heterozyklische Gruppe, die substituiert sein kann, oder eine Bindung darstellt;

wobei $Y^2$ eine $C_{1-6}$-Alkylengruppe, die substituiert sein kann, oder eine Bindung darstellt;

wobei $X^3$ eine Gruppe, die durch allgemeine Formel -$NR^{9a}$- dargestellt wird (wobei $R^{9a}$ ein Wasserstoffatom darstellt), darstellt;

wobei $R^3$ ein Wasserstoffatom darstellt;

wobei $R^1$ ein Wasserstoffatom darstellt.

**15.** Kit zur Verwendung nach Anspruch 13 oder 14,

wobei $R^2$ eine Phenylgruppe darstellt, die substituiert sein kann;

A eine monozyklische stickstoff- und schwefelhaltige heterozyklische Gruppe darstellt, die substituiert sein kann;

Q eine divalente monozyklische heterozyklische Gruppe darstellt, die substituiert sein kann;

$Y^1$ eine Gruppe, die durch Formel -NHC(=O)- dargestellt wird, eine Gruppe, die durch Formel -NHC(=O)C(=O)NH- dargestellt wird, oder eine Bindung darstellt;

$X^2$ eine Gruppe, die durch allgemeine Formel -$NR^{4b}$- dargestellt wird (wobei $R^{4b}$ ein Wasserstoffatom darstellt) oder eine Bindung darstellt;

$Y^2$ eine $C_{1-3}$-Alkylengruppe oder eine Bindung darstellt; und

$Y^3$ eine Gruppe, die durch Formel -C(=O)- dargestellt wird, oder eine Gruppe, die durch Formel -C(=O)-C(=O)-dargestellt wird, darstellt.

**16.** Kit zur Verwendung nach Anspruch 13,
wobei die durch allgemeine Formel [1] dargestellte Verbindung eine Verbindung ist, die aus (3R,5R,6R)-6-((Z)-2-(2-Aminothiazol-4-yl)-2-((1-Carboxycyclobutoxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Amino-5-chlorothiazol-4-yl)-2-(((2-Carboxypropan-2-yl)oxy)imino)acetamido)-3-(3-(2-(2-Chloro-3,4-dihydroxyphenyl)-2-oxoacetamido)-2-oxoimidazolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-((S)-3-(2-(2-Chloro-3,4-dihydroxyphenyl)-2-oxoacetamido)-2-oxopyrrolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-((R)-3-(2-(2-Chloro-3,4-dihydroxyphenyl)-2-oxoacetamido)-2-oxopyrrolidin-1-yl)-7-oxo-4-thia-1-azabicyclo[3.2.0]heptan-3-carbonsäure, (3R,5R,6R)-6-((Z)-2-(2-Aminothiazol-4-yl)-2-(((2-Carboxypropan-2-yl)oxy)imino)acetamido)-3-(4-(2-(2-Chloro-3,4-dihydroxyphenyl)-2-oxoacetamido)-2,3-Dioxopiperazin-1-yl)-7-oxo-4-thia-1-azabicyclo[3.2.0]heptan-3-carbonsäure und (3R,5R,6R)-6-((Z)-2-(2-Amino-5-chlorothiazol-4-yl)-2-(((2-Carboxypropan-2-yl)oxy)imino)acetamido)-3-(3-(2-(2-(2-(2-Chloro-3,4-dihydroxyphenyl)-2-oxoacetyl)hydradienyl)-2-oxoacetamido)-2-oxoimidazolidin-1-yl)-7-oxo-4-thia-1-azabicyclo[3.2.0]heptan-3-carbonsäure ausgewählt wird.

**17.** Kit zur Verwendung nach einem der Ansprüche 13 bis 16,
wobei die eine oder mehrere Verbindungen, die aus einer β-Lactamase-Hemmverbindung und einer antibakteriellen Verbindung oder Salzen davon ausgewählt werden, eine β-Lactamase-Hemmverbindung oder ein Salz davon sind.

**18.** Kit zur Verwendung nach einem der Ansprüche 13 bis 17,
wobei die β-Lactamase-Hemmverbindung eine oder mehrere Verbindungen ist, die aus einer Hemmverbindung des Diazabicyclo[3.2.1]octantyps, einer Hemmverbindung des Boronsäuretyps und einer Hemmverbindung des Clavamtyps ausgewählt werden.

**19.** Kit zur Verwendung nach einem der Ansprüche 13 bis 17, wobei die β-Lactamase-Hemmverbindung eine oder mehrere Verbindungen ist, die aus Avibactam, Nacubactam, Relebactam, Zidebactam, Vaborbactam, (3R)-3-(2-[trans-4-[(2-Aminoethyl)amino]cyclohexyl]acetamid)-2-hydroxy-3,4-dihydro-2H-1,2-benzoxaborinine-8-carbonsäure, Sulbactam, Tazobactam, Clavulansäure, (1aR,7bS)-5-Fluor-2-hydroxy-1,1a,2,7b-tetrahydrocyclopropan[c][1,2]benzoxaborinine-4-carbonsäure und (2S,3S,5R)-3-Methyl-3-[(3-methyl-1H-1,2,3-triazol-3-ium-1-yl)methyl]-7-oxo-4-thia-1-azabicyclo[3.2.0]heptan-2-carboxylat-4,4-dioxid ausgewählt werden.

## Revendications

**1.** Composition pharmaceutique comprenant :

un composé représenté par Formule Générale [1] ou un sel de celui-ci ; et
un ou plusieurs composés sélectionnés parmi un composé inhibiteur de β-lactamase et un composé antibactérien ou des sels de ceux-ci,

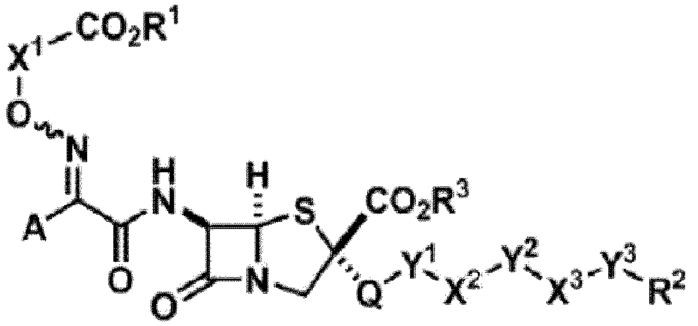

[1]

dans la formule,
$R^1$ représente un atome d'hydrogène ;
$R^2$ représente un groupe aryle qui peut être substitué ;
$R^3$ représente un atome d'hydrogène ;
$X^1$ représente un groupe alkylène $C_{1-6}$ qui peut être substitué, un groupe alcénylène $C_{2-6}$ qui peut être substitué, un groupe alkynylène $C_{2-6}$ qui peut être substitué, un groupe hydrocarboné cyclique divalent qui peut être substitué ou un groupe hétérocyclique saturé monocyclique divalent qui peut être substitué ;
A représente un groupe hétérocyclique monocyclique qui peut être substitué ;
Q représente un groupe aminé cyclique divalent qui peut être substitué ou un groupe hétérocyclique divalent qui peut être substitué ;
$Y^1$ représente un groupe alkylène $C_{1-6}$ qui peut être substitué, un groupe alcénylène $C_{2-6}$ qui peut être substitué, un groupe alkynylène $C_{2-6}$ qui peut être substitué, un groupe représenté par Formule -N=CH-CH=N-, un groupe représenté par Formule -N=CH-CH=N-O-, un groupe représenté par Formule $-N=CH-CH_2-$, un groupe représenté par Formule -N=CHC(=O)-, un groupe représenté par Formule -NHC(=O)-, un groupe représenté par Formule $-NHC(=O)CH_2-$, un groupe représenté par Formule - NHC(=O)NH-, un groupe représenté par Formule -NHC(=O)NH-O-, un groupe représenté par Formule -NHC(=O)C(=O)NH-, un groupe représenté par Formule - NHC(=O)C(=O)N(OH)-, un groupe représenté par Formule $-NHCH_2C(=O)-$, un groupe représenté par Formule $-NHS(=O)_2NHC(=O)-$, un groupe représenté par Formule - $NHC(=O)NHS(=O)_2-$, ou une liaison;
$X^2$ représente un groupe représenté par Formule Générale $-NR^4-$ (où $R^4$ représente un atome d'hydrogène, un groupe carbamoyle, un groupe alkyle $C_{1-6}$ qui peut être substitué, ou un groupe hydroxyle qui peut être protégé), un groupe représenté par Formule Générale $-N^+R^5R^6-$ (où $R^5$ et $R^6$ sont identiques ou différents l'un de l'autre et représentent chacun un groupe alkyle $C_{1-6}$ qui peut être substitué, ou en combinaison représentent un groupe alkylène $C_{2-6}$ qui peut être substitué ou un groupe alcénylène $C_{2-6}$ qui peut être substitué), un groupe représenté par Formule Générale $-NR^7-C(=O)-NR^8-$ (où $R^7$ et $R^8$ sont identiques ou différents l'un de l'autre et représentent chacun un atome d'hydrogène, un groupe alkyle $C_{1-6}$ qui peut être substitué, ou un groupe hydroxyle qui peut être protégé), un groupe aminé cyclique divalent qui peut être substitué, un groupe hétérocyclique divalent qui peut être substitué, ou une liaison ;
$Y_2$ représente un groupe alkylène $C_{1-6}$ qui peut être substitué, ou une liaison ;
$X^3$ représente un groupe représenté par Formule Générale $-NR^9-$ (où $R^9$ représente un atome d'hydrogène) ; et

$Y^3$ représente un groupe représenté par Formule -C(=O)-, un groupe représenté par Formule -C(=O)-C(=O)-, ou un groupe représenté par Formule Générale -C(=O)-C(=$NR^{10a}$)- (où $R^{10a}$ représente un groupe hydroxyle).

**2.** Composition pharmaceutique selon la revendication 1,
dans laquelle $X^1$ représente un groupe alkylène $C_{1-6}$ qui peut être substitué ou un groupe hydrocarboné cyclique divalent qui peut être substitué.

**3.** Composition pharmaceutique selon la revendication 1 ou la revendication 2,
dans laquelle Q représente un groupe hétérocyclique divalent qui peut être substitué.

**4.** Composition pharmaceutique selon l'une quelconque des revendications 1 à 3,
dans laquelle $Y^1$ représente un groupe alkylène $C_{1-6}$ qui peut être substitué, un groupe représenté par Formule -N=CH-CH=N-, un groupe représenté par Formule -N=CH-$CH_2$-, un groupe représenté par Formule -N=CHC(=O)-, un groupe représenté par Formule -NHC(=O)-, un groupe représenté par Formule -NHC(=O)$CH_2$-, un groupe représenté par Formule -NHC(=O)NH-, un groupe représenté par Formule - NHC(=O)NH-O-, un groupe représenté par Formule -NHC(=O)C(=O)NH-, un groupe représenté par Formule -NH$CH_2$C(=O)-, ou une liaison.

**5.** Composition pharmaceutique selon l'une quelconque des revendications 1 à 4,
dans laquelle $X^2$ représente un groupe représenté par Formule Générale -$NR4^a$- (où $R4^a$ représente un atome d'hydrogène ou un groupe carbamoyle), un groupe représenté par Formule Générale -$N^+R^{5a}R^{6a}$- (où $R^{5a}$ et $R^{6a}$ en combinaison représentent un groupe alkylène $C_{2-6}$ qui peut être substitué), un groupe représenté par Formule Générale - $NR^{7a}$-C(=O)-$NR^{8a}$- (où $R^{7a}$ et $R^{8a}$ représentent chacun un atome d'hydrogène), un groupe aminé cyclique divalent qui peut être substitué, un groupe hétérocyclique divalent qui peut être substitué, ou une liaison.

**6.** Composition pharmaceutique selon l'une quelconque des revendications 1 à 5,

dans laquelle $R^2$ représente un groupe phényle qui peut être substitué ;
A représente un groupe hétérocyclique monocyclique contenant de l'azote et soufre qui peut être substitué ;
Q représente un groupe hétérocyclique monocyclique divalent qui peut être substitué ; $Y^1$ représente un groupe représenté par Formule -NHC(=O)-, un groupe représenté par Formule -NHC(=O)C(=O)NH-, ou une liaison ;
$X^2$ représente un groupe représenté par Formule Générale -$NR^{4b}$- (où $R^{4b}$ représente un atome d'hydrogène) ou une liaison ;
$Y^2$ représente un groupe alkylène $C_{1-3}$ ou une liaison; et
$Y^3$ représente un groupe représenté par Formule -C(=O)- ou un groupe représenté par Formule -C(=O)-C(=O)-.

**7.** Composition pharmaceutique selon la revendication 1,
dans laquelle le composé représenté par Formule Générale [1] est un composé sélectionné parmi l'acide (3R,5R,6R)-6-((Z)-2-(2-aminothiazol-4-yl)-2-((1-carboxycyclobutoxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo[3.2.0]heptane-3-carboxylique, l'acide (3R,5R,6R)-6-((Z)-2-(2-amino-5-chlorothiazol-4-yl)-2-(((2-carboxypropan-2-yl)oxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo [3.2.0]heptane-3-carboxylique, l'acide (3R,5R,6R)-6-((Z)-2-(2-aminothiazol-4-yl)-2-(((2-carboxypropan-2-yl)oxy) imino)acétamido)-3-((S)-3-(2-(2-chloro-3,4-dihydroxyphényl)-2-oxoacétamido)-2-oxopyrrolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-((R)-3-(2-(2-chloro-3,4-dihydroxyphényl)-2-oxoacétamido)-2-oxopyrrolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-(4-(2-(2-chloro-3,4-dihydroxyphényl)-2-oxoacétamido)-2,3-dioxopipérazin-1-yl)-7-oxo-4-thia-1-azabicyclo[3.2.0]heptane-3-carboxylique, et l'acide (3R,5R,6R)-6-((Z)-2-(2-amino-5-chlorothiazol-4-yl)-2-(((2-carboxypropan-2-yl)oxy)imino)acétamido)-3-(3-(2-(2-(2-(2-chloro-3,4-dihydroxyphényl)-2-oxoacétyl)hydradienyl)-2-oxoacétamido)-2-oxoimidazolidin-1-yl)-7-oxo-4-thia-1-azabicyclo[3.2.0]heptane-3-carboxylique.

**8.** Composition pharmaceutique selon l'une quelconque des revendications 1 à 7,
dans laquelle un ou plusieurs composés sélectionnés parmi un composé inhibiteur de β-lactamase et un composé antibactérien ou des sels de ceux-ci sont un composé inhibiteur de β-lactamase ou un sel de celui-ci.

**9.** Composition pharmaceutique selon l'une quelconque des revendications 1 à 8, dans laquelle le composé inhibiteur de β-lactamase est un ou plusieurs composés sélectionnés parmi un composé inhibiteur de type diazabicyclo[3.2.1]octane, un composé inhibiteur de type acide boronique et un composé inhibiteur de type clavam.

**10.** Composition pharmaceutique selon l'une quelconque des revendications 1 à 9, dans laquelle le composé inhibiteur de β-lactamase est un ou plusieurs composés sélectionnés parmi l'avibactam, le nacubactam, le relebactam, le zidebactam, le vaborbactam, l'acide (3R)-3-(2-[trans-4-[(2-aminoéthyl)amino]cyclohexyl]acétamide)-2-hydroxy-3,4-dihydro-2H-1,2-benzoxaborinine-8-carboxylique, le sulbactam, le tazobactam, l'acide clavulanique, l'acide (1aR,7bS)-5-fluoro-2-hydroxy-1,1a,2,7b-tétrahydrocyclopropan[c][1,2]benzoxaborinine-4-carboxylique et le 4,4-dioxyde de (2S,3S,5R)-3-méthyl-3-[(3-méthyl-1H-1,2,3-triazol-3-ium-1-yl)méthyl]-7-oxo-4-thia-1-azabicyclo[3.2.0]heptane-2-carboxylate.

**11.** Composition pharmaceutique selon l'une quelconque des revendications 1 à 10 pour une utilisation dans le traitement d'infections causées par des bactéries Gram-négatives.

**12.** Agent pour une utilisation dans le traitement d'infections causées par des bactéries Gram-négatives, comprenant : la composition pharmaceutique selon l'une quelconque des revendications 1 à 10.

**13.** Kit pour une utilisation dans le traitement d'infections, comprenant :

un composé représenté par Formule Générale [1] ou un sel de celui-ci, et
un ou plusieurs composés sélectionnés parmi un composé inhibiteur de β-lactamase et un composé antibactérien, ou des sels de ceux-ci,

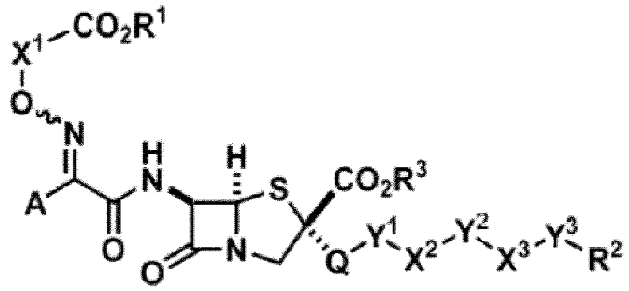
[1]

dans la formule,
$R^1$ représente un atome d'hydrogène;
$R^2$ représente un groupe aryle qui peut être substitué;
$R^3$ représente un atome d'hydrogène ;
$X^1$ représente un groupe alkylène $C_{1-6}$ qui peut être substitué, un groupe alcénylène $C_{2-6}$ qui peut être substitué, un groupe alkynylène $C_{2-6}$ qui peut être substitué, un groupe hydrocarboné cyclique divalent qui peut être substitué ou un groupe hétérocyclique saturé monocyclique divalent qui peut être substitué ;
A représente un groupe hétérocyclique monocyclique qui peut être substitué ;
Q représente un groupe aminé cyclique divalent qui peut être substitué ou un groupe hétérocyclique divalent qui peut être substitué ;
$Y^1$ représente un groupe alkylène $C_{1-6}$ qui peut être substitué, un groupe alcénylène $C_{2-6}$ qui peut être substitué, un groupe alkynylène $C_{2-6}$ qui peut être substitué, un groupe représenté par Formule -N=CH-CH=N-, un groupe représenté par Formule -N=CH-CH=N-O-, un groupe représenté par Formule $-N=CH-CH_2-$, un groupe représenté par Formule -N=CHC(=O)-, un groupe représenté par Formule -NHC(=O)-, un groupe représenté par Formule $-NHC(=O)CH_2-$, un groupe représenté par Formule - NHC(=O)NH-, un groupe représenté par Formule -NHC(=O)NH-O-, un groupe représenté par Formule -NHC(=O)C(=O)NH-, un groupe représenté par Formule -NHC(=O)C(=O)N(OH)-, un groupe représenté par Formule $-NHCH_2C(=O)-$, un groupe représenté par Formule $-NHS(=O)_2NHC(=O)-$, un groupe représenté par Formule - $NHC(=O)NHS(=O)_2-$, ou une liaison;
$X^2$ représente un groupe représenté par Formule Générale $-NR^4-$ (où $R^4$ représente un atome d'hydrogène, un groupe carbamoyle, un groupe alkyle $C_{1-6}$ qui peut être substitué, ou un groupe hydroxyle qui peut être protégé), un groupe représenté par Formule Générale $-N^+R^5R^6-$ (où $R^5$ et $R^6$ sont identiques ou différents l'un de l'autre et représentent chacun un groupe alkyle $C_{1-6}$ qui peut être substitué, ou en combinaison représentent un groupe

alkylène $C_{2-6}$ qui peut être substitué ou un groupe alcénylène $C_{2-6}$ qui peut être substitué), un groupe représenté par Formule Générale -$NR^7$-C(=O)-$NR^8$- (où $R^7$ et $R^8$ sont identiques ou différents l'un de l'autre et représentent chacun un atome d'hydrogène, un groupe alkyle $C_{1-6}$ qui peut être substitué, ou un groupe hydroxyle qui peut être protégé), un groupe aminé cyclique divalent qui peut être substitué, un groupe hétérocyclique divalent qui peut être substitué, ou une liaison ;
$Y^2$ représente un groupe alkylène $C_{1-6}$ qui peut être substitué, ou une liaison ;
$X^3$ représente un groupe représenté par Formule Générale -$NR^9$- (où $R^9$ représente un atome d'hydrogène) ; et
$Y^3$ représente un groupe représenté par Formule -C(=O)-, un groupe représenté par Formule -C(=O)-C(=O)-, ou un groupe représenté par Formule Générale -C(=O)-C(=$NR^{10a}$)- (où $R^{10a}$ représente un groupe hydroxyle).

**14.** Kit à utiliser selon la revendication 13,

dans lequel $R^2$ représente un groupe aryle qui peut être substitué ;
dans lequel A représente un groupe hétérocyclique monocyclique qui peut être substitué ;
dans lequel $X^1$ représente un groupe alkylène $C_{1-6}$ qui peut être substitué ou un groupe hydrocarboné cyclique divalent qui peut être substitué ;
dans lequel Q représente un groupe hétérocyclique divalent qui peut être substitué ; dans lequel $Y^1$ représente un groupe alkylène $C_{1-6}$ qui peut être substitué, un groupe représenté par Formule -N=CH-CH=N-, un groupe représenté par Formule -N=CH-$CH_2$-, un groupe représenté par Formule -N=CHC(=O)-, un groupe représenté par Formule -NHC(=O)-, un groupe représenté par Formule -NHC(=O)$CH_2$-, un groupe représenté par Formule -NHC(=O)NH-, un groupe représenté par Formule - NHC(=O)NH-O-, un groupe représenté par Formule -NHC(=O)C(=O)NH-, un groupe représenté par Formule -$NHCH_2C$(=O)-, ou une liaison ;
dans lequel $X^2$ représente un groupe représenté par Formule Générale -$NR^{4a}$- (où R4ª représente un atome d'hydrogène ou un groupe carbamoyle), un groupe représenté par Formule Générale -$N^+R^{5a}R^{6a}$- (où $R^{5a}$ et $R^{6a}$ en combinaison représentent un groupe alkylène $C_{2-6}$ qui peut être substitué), un groupe représenté par Formule Générale - NR $^{7a}$-C(=O)-NR8ª- (où $R^{7a}$ et $R^{8a}$ représentent chacun un atome d'hydrogène), un groupe aminé cyclique divalent qui peut être substitué, un groupe hétérocyclique divalent qui peut être substitué, ou une liaison ;
dans lequel $Y^2$ représente un groupe alkylène $C_{1-6}$ qui peut être substitué ou une liaison ;
dans lequel $X^3$ représente un groupe représenté par Formule Générale -$NR^{9a}$- (où $R^{9a}$ représente un atome d'hydrogène);
dans lequel $R^3$ représente un atome d'hydrogène;
dans lequel $R^1$ représente un atome d'hydrogène.

**15.** Kit à utiliser selon la revendication 13 ou la revendication 14,

dans lequel $R^2$ représente un groupe phényle qui peut être substitué;
A représente un groupe hétérocyclique monocyclique contenant de l'azote et soufre qui peut être substitué ;
Q représente un groupe hétérocyclique monocyclique divalent qui peut être substitué ; $Y^1$ représente un groupe représenté par Formule -NHC(=O)-, un groupe représenté par Formule -NHC(=O)C(=O)NH-, ou une liaison ;
$X^2$ représente un groupe représenté par Formule Générale -$NR^{4b}$- (où $R^{4b}$ représente un atome d'hydrogène) ou une liaison;
$Y^2$ représente un groupe alkylène $C_{1-3}$ ou une liaison; et
$Y^3$ représente un groupe représenté par Formule -C(=O)- ou un groupe représenté par Formule -C(=O)-C(=O)-.

**16.** Kit à utiliser selon la revendication 13,
dans lequel le composé représenté par Formule Générale [1] est un composé sélectionné parmi l'acide (3R,5R,6R)-6-((Z)-2-(2-aminothiazol-4-yl)-2-((1-carboxycyclobutoxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo[3.2.0]heptane-3-carboxylique, l'acide (3R,5R,6R)-6-((Z)-2-(2-amino-5-chlorothiazol-4-yl)-2-(((2-carboxypropan-2-yl)oxy)imino)acétamido)-3-(3-(2-(2-chloro-3,4-dihydroxyphényl)-2-oxoacétamido)-2-oxoimidazolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-((S)-3-(2-(2-chloro-3,4-dihydroxyphényl)-2-oxoacétamido)-2-oxopyrrolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-((R)-3-(2-(2-chloro-3,4-dihydroxyphényl)-2-oxoacétamido)-2-oxopyrrolidin-1-yl)-7-oxo-4-thia-1-azabicyclo[3.2.0]heptane-3-carboxylique, l'acide (3R,5R,6R)-6-((Z)-2-(2-aminothiazol-4-yl)-2-(((2-carboxypropan-2-yl)oxy)imino)acétamido)-3-(4-(2-(2-chloro-3,4-dihydroxyphényl)-2-oxoacétamido)-2,3-dioxopipérazin-1-yl)-7-oxo-4-thia-1-azabicyclo[3.2.0]heptane-3-carboxylique, et l'acide (3R,5R,6R)-6-((Z)-2-(2-amino-5-chlorothiazol-4-yl)-2-(((2-carboxypropan-2-yl)oxy)imino)acétamido)-3-(3-(2-(2-(2-(2-chloro-3,4-dihydroxyphényl)-2-oxoacétyl)hydradienyl)-2-oxoacétamido)-2-oxoimidazolidin-1-yl)-7-oxo-4-thia-1-azabicyclo[3.2.0]heptane-3-carboxylique.

**17.** Kit à utiliser selon l'une quelconque des revendications 13 à 16,
dans lequel un ou plusieurs composés sélectionnés parmi un composé inhibiteur de β-lactamase et un composé antibactérien ou des sels de ceux-ci sont un composé inhibiteur de β-lactamase ou un sel de celui-ci.

**18.** Kit à utiliser selon l'une quelconque des revendications 13 à 17,
dans lequel le composé inhibiteur de β-lactamase est un ou plusieurs composés sélectionnés parmi un composé inhibiteur de type diazabicyclo[3.2.1]octane, un composé inhibiteur de type acide boronique et un composé inhibiteur de type clavam.

**19.** Kit à utiliser selon l'une quelconque des revendications 13 à 17,
dans lequel le composé inhibiteur de β-lactamase est un ou plusieurs composés sélectionnés parmi l'avibactam, le nacubactam, le relebactam, le zidebactam, le vaborbactam, l'acide (3R)-3-(2-[trans-4-[(2-aminoéthyl)amino]cyclohexyl]acétamide)-2-hydroxy-3,4-dihydro-2H-1,2-benzoxaborinine-8-carboxylique, le sulbactam, le tazobactam, l'acide clavulanique, l'acide (laR,7bS)-5-fluoro-2-hydroxy-1,1a,2,7b-tétrahydrocyclopropan[c][1,2]benzoxaborinine-4-carboxylique et le 4,4-dioxyde de (2S,3S,5R)-3-méthyl-3-[(3-méthyl-1H-1,2,3-triazol-3-ium-1-yl)méthyl]-7-oxo-4-thia-1-azabicyclo[3.2.0]heptane-2-carboxylate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2648818
- WO 2019070052 A1
- JP 4074182 A
- JP H04074182 A
- JP 10182654 A
- JP H10182654 A
- US 5185330 A


### Non-patent literature cited in the description


- *Antimicrobial Agents and Chemotherapy*, 2008, vol. 52, 813-821
- *Scientific Reports*, 2017, vol. 24 (43232)
- *International Journal of Antimicrobial Agents*, 2014, vol. 43, 533-539
- **W. GREENE et al.** Protective Groups in Organic Synthesis. John Wiley & Sons, INC, 2007, 16-299
- **W. GREENE et al.** Protective Groups in Organic Synthesis. John Wiley & Sons, INC, 2007, 696-926
- **W. GREENE et al.** Protective Groups in Organic Synthesis. John Wiley & Sons, INC, 2007, 696-868
- **W. GREENE et al.** Protective Groups in Organic Synthesis. John Wiley & Sons, INC, 2007, 533-643
- **W. GREENE et al.** Protective Groups in Organic Synthesis. John Wiley & Sons, INC., 2007, 533-643
- *Diagnostic Microbiology and Infectious Disease*, 2004, vol. 49, 197